(19) Europäisches Patentamt
European Patent Office
Office européen des brevets

(11) EP 2 895 183 B1

(12) **EUROPEAN PATENT SPECIFICATION**

(45) Date of publication and mention of the grant of the patent:
**24.10.2018 Bulletin 2018/43**

(21) Application number: **13763220.4**

(22) Date of filing: **13.09.2013**

(51) Int Cl.:
***A61K 38/00*** *(2006.01)* ***C07K 7/08*** *(2006.01)*
***C12N 15/113*** *(2010.01)* ***A61P 25/16*** *(2006.01)*
***A61P 25/00*** *(2006.01)* ***A61P 3/00*** *(2006.01)*

(86) International application number:
**PCT/EP2013/068983**

(87) International publication number:
**WO 2014/041111 (20.03.2014 Gazette 2014/12)**

(54) **USP30 INHIBITORS FOR USE IN THE TREATMENT OF PARKINSON'S DISEASE**

USP30 INHIBITOREN ZUR BEHANDLUNG DER PARKINSON-KRANKHEIT

INHIBITEURS DE USP30 POUR LE TRAITEMENT DE LA MALADIE DE PARKINSON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2012 US 201261701963 P**
**09.04.2013 US 201361809927 P**

(43) Date of publication of application:
**22.07.2015 Bulletin 2015/30**

(73) Proprietor: **F. Hoffmann-La Roche AG**
**4070 Basel (CH)**

(72) Inventors:
- **BINGOL, Baris**
**South San Francisco, California 94080 (US)**
- **CORN, Jacob**
**South San Francisco, California 94080 (US)**
- **ZHANG, Yingnan**
**South San Francisco, California 94080 (US)**

(74) Representative: **Bernard, Guillaume**
**F. Hoffmann-La Roche AG**
**Patent Department**
**Grenzacherstrasse 124**
**4070 Basel (CH)**

(56) References cited:
**WO-A2-2011/053825**

- **N. NAKAMURA ET AL: "Regulation of Mitochondrial Morphology by USP30, a Deubiquitinating Enzyme Present in the Mitochondrial Outer Membrane", MOLECULAR BIOLOGY OF THE CELL, vol. 19, no. 5, May 2008 (2008-05), pages 1903-1911, XP055092951, ISSN: 1059-1524, DOI: 10.1091/mbc.E07-11-1103**
- **BUUS R ET AL: "Deubiquitinase Activities Required for Hepatocyte Growth Factor-Induced Scattering of Epithelial Cells", CURRENT BIOLOGY, CURRENT SCIENCE, GB, vol. 19, no. 17, 15 September 2009 (2009-09-15), pages 1463-1466, XP026643210, ISSN: 0960-9822, DOI: 10.1016/J.CUB.2009.07.040 [retrieved on 2009-08-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 895 183 B1

## Description

### FIELD

Inhibitors of USP30 and methods of using inhibitors of USP30 are provided. In some embodiments, methods of treating conditions involving mitochondrial defects are provided.

### BACKGROUND

Mitophagy is a specialized autophagy pathway that eliminates mitochondria through degradation by lysosomes. As such, it removes mitochondria during normal cellular turnover of organelles, during maturation of erythrocytes, and following fertilization to eliminate sperm-derived mitochondria. Mitophagy also mediates the clearance of damaged mitochondria, an important aspect of mitochondria quality control. Defective or excess mitochondria, if left uncleared, may become an aberrant source of oxidative stress and compromise healthy mitochondria through mitochondrial fusion. In yeast, selective blockade of mitophagy causes increased production of reactive oxygen species (ROS) by excess mitochondria and loss of mitochondrial DNA (mt-DNA). Impaired mitochondria quality control could also affect key biosynthetic pathways, ATP production, and Ca2+ buffering, and disturb overall cellular homeostasis.

Parkinson's disease (PD), the second most common neurodegenerative disorder after Alzheimer's disease (AD), is characterized most prominently by loss of dopaminergic neurons in the substantia nigra. Although the pathogenic mechanisms of PD are not clear, several lines of evidence suggest that mitochondrial dysfunction is central to PD. MPTP, a mitochondrial toxin, damages dopamine neurons and produces clinical parkinsonism in humans. Epidemiologic evidence links PD with exposure to pesticides such as rotenone (a complex I inhibitor) and paraquat (an oxidative stressor). Consistent with mitochondrial impairment, reduced complex I activity and high levels of mt-DNA mutations have been found in substantia nigra from PD patients. Similarly, functional and morphological changes in mitochondria are present in genetic models of PD. Perhaps most compellingly, early-onset familial PD can be caused by mutations in Parkin ubiquitin-ligase and PINK1 serine/threonine protein kinase, both of which function to maintain healthy mitochondria through regulating mitochondrial dynamics and quality control.

Genetic studies in flies established that PINK1 acts upstream of Parkin to maintain proper mitochondria morphology and function. PINK1 recruits Parkin from the cytoplasm to the surface of damaged mitochondria, leading to Parkin-mediated ubiquitination of mitochondrial outer membrane proteins and removal of damaged mitochondria by mitophagy. PD-associated mutations in either PINK1 or Parkin impair Parkin recruitment, mitochondrial ubiquitination and mitophagy. Parkin regulates multiple aspects of mitochondrial function such as mitochondrial dynamics and trafficking, and may also influence mitochondria biogenesis. The degradation of a broad range of outer mitochondrial membrane proteins on damaged mitochondria appears to be affected by Parkin. Among these mitochondria associated proteins, MIRO, a component of the mitochondria-kinesin motor adaptor complex, may be a shared substrate of both Parkin and PINK-1.

Parkin expression and/or activity can be impaired through genetic mutations in familial PD or by phosphorylation in sporadic PD. In the context of the inherently high mitochondrial oxidative stress in substantia nigra dopamine neurons, loss of Parkin-mediated mitochondrial quality control could explain the greater susceptibility of substantia nigra neurons to neurodegeneration. Promoting clearance of damaged mitochondria and enhancing mitochondrial quality control could be beneficial in PD.

### SUMMARY

In some embodiments, methods of increasing mitophagy in a cell are provided. In some embodiments, the method comprises contacting the cell with an inhibitor of USP30.

In some embodiments, methods of increasing mitochondrial ubiquitination in a cell are provided. In some embodiments, methods of increasing ubiquitination of at least one, at least two, at least three, at least four, at least five, at least six, at least seven, at least eight, at least nine, at least ten, at least eleven, at least twelve, at least thirteen, or fourteen proteins selected from Tom20, MIRO, MUL1, ASNS, FKBP8, TOM70, MAT2B, PRDX3, IDE, VDAC1, VDAC2, VDAC3, IPO5, PSD13, UBP13, and PTH2 in a cell are provided. In some embodiments, the method comprises contacting the cell with an inhibitor of USP30.

In some embodiments, the method comprises increasing ubiquitination of at least one, at least two, or three amino acids selected from K56, K61, and K68 of Tom 20. In some embodiments, the method comprises increasing ubiquitination of at least one, at least two, at least three, at least four, at least five, at least six, at least seven, or eight amino acids selected from K153, K187, K330, K427, K512, K535, K567, and K572 of MIRO. In some embodiments, the method comprises increasing ubiquitination of at least one, at least two, or three amino acids selected from K273, K299,and K52 of MUL1. In some embodiments, the method comprises increasing ubiquitination of at least one, at least

two, at least three, at least four, at least five, at least six, at least seven, at least eight, or nine amino acids selected from K147, K168, K176, K221, K244, K275, K478, K504, and K556 of ASNS. In some embodiments, the method comprises increasing ubiquitination of at least one, at least two, at least three, at least four, at least five, at least six, at least seven, or eight amino acids selected from K249, K271, K273, K284, K307, K317, K334, and K340 of FKBP8. In some embodiments, the method comprises increasing ubiquitination of at least one, at least two, at least three, at least four, at least five, at least six, at least seven, at least eight, at least nine, or at least ten amino acids selected from K78, K120, K123, K126, K129, K148, K168, K170, K178, K185, K204, K230, K233, K245, K275, K278, K312, K326, K349, K359, K441, K463, K470, K471, K494, K501, K524, K536, K563, K570, K599, K600, and K604 of TOM70. In some embodiments, the method comprises increasing ubiquitination of at least one, at least two, at least three, or four amino acids selected from K209, K245, K316, and K326 of MAT2B. In some embodiments, the method comprises increasing ubiquitination of at least one, at least two, at least three, at least four, or five amino acids selected from K83, K91, K166, K241, and K253 of PRDX3. In some embodiments, the method comprises increasing ubiquitination of at least one, at least two, at least three, at least four, at least five, or six amino acids selected from K558, K657, K854, K884, K929, and K933 of IDE. In some embodiments, the method comprises increasing ubiquitination of at least one, at least two, at least three, at least four, at least five, at least six, or seven amino acids selected from K20, K53, K61, K109, K110, K266, and K274 of VDAC1. In some embodiments, the method comprises increasing ubiquitination of at least one, at least two, at least three, at least four, at least five, or six amino acids selected from K31, K64, K120, K121, K277, and K285 of VDAC2. In some embodiments, the method comprises increasing ubiquitination of at least one, at least two, at least three, at least four, at least five, at least six, at least seven, or eight amino acids selected from K20, K53, K61, K109, K110, K163, K266, and K274 of VDAC3. In some embodiments, the method comprises increasing ubiquitination of at at least one, at least two, at least three, at least four, at least five, at least six, at least seven, at least eight, at least nine, or at least ten amino acids selected from K238, K353, K436, K437, K548, K556, K613, K678, K690, K705, K775, and K806 of IPO5. In some embodiments, the method comprises increasing ubiquitination of at least one, at least two, at least three, at least four, at least five, at least six, at least seven, at least eight, at least nine, or at least ten amino acids selected from K2, K32, K99, K115, K122, K132, K161, K186, K313, K321, K347, K350, and K361 of PSD13. In some embodiments, the method comprises increasing ubiquitination of at least one, at least two, at least three, at least four, at least five, at least six, at least seven, at least eight, at least nine, or at least ten amino acids selected from K18, K190, K259, K326, K328, K401, K405, K414, K418, K435, K586, K587, and K640 of UBP13. In some embodiments, the method comprises increasing ubiquitination of at least one, at least two, at least three, at least four, at least five, at least six, at least seven, at least eight, or nine amino acids selected from 47, 76, 81, 95, 106, 119, 134, 171, 177 of PTH2.

In some embodiments, the cell is under oxidative stress. In some embodiments, methods of reducing oxidative stress in a cell are provided. In some embodiments, a method comprises contacting the cell with an inhibitor of USP30.

In some embodiments, the cell comprises a pathogenic mutation in Parkin, a pathogenic mutation in PINK1, or a pathogenic mutation in Parkin and a pathogenic mutation in PINK1. Nonlimiting exemplary pathogenic mutations in Parkin are shown in Table 1. Thus, in some embodiments, the pathogenic mutation in Parkin is selected from the mutations in Table 1. Nonlimiting exemplary pathogenic mutations in PINK1 are shown in Table 2. In some embodiments, the pathogenic mutation in PINK1 selected from the mutations in Table 2.

In various embodiments, the cell is selected from a neuron, a cardiac cell, and a muscle cell. In some such embodiments, the cell is *ex vivo* or *in vitro.* Alternatively, in some such embodiments, the cell is comprised in a subject.

In some embodiments, methods of treating conditions involving mitochondrial defects in a subject are provided. In some embodiments, the method comprises administering to the subject an effective amount of an inhibitor of USP30. In some embodiments, the condition involving a mitochondrial defect is selected from a condition involving a mitophagy defect, a condition involving a mutation in mitochondrial DNA, a condition involving mitochondrial oxidative stress, a condition involving a defect in mitochondrial shape or morphology, a condition involving a defect in mitochondrial membrane potential, and a condition involving a lysosomal storage defect.

In some embodiments, the condition involving a mitochondrial defect is selected from a neurodegenerative disease; mitochondrial myopathy, encephalopathy, lactic acidosis, and stroke-like episodes (MELAS) syndrome; Leber's hereditary optic neuropathy (LHON); neuropathy, ataxia, retinitis pigmentosa-maternally inherited Leigh syndrome (NARP-MILS); Danon disease; ischemic heart disease leading to myocardial infarction; multiple sulfatase deficiency (MSD); mucolipidosis II (ML II); mucolipidosis III (ML III); mucolipidosis IV (ML IV); GM1-gangliosidosis (GM1); neuronal ceroid-lipofuscinoses (NCL1); Alpers disease; Barth syndrome; Beta-oxidation defects; carnitine-acyl-carnitine deficiency; carnitine deficiency; creatine deficiency syndromes; co-enzyme Q10 deficiency; complex I deficiency; complex II deficiency; complex III deficiency; complex IV deficiency; complex V deficiency; COX deficiency; chronic progressive external ophthalmoplegia syndrome (CPEO); CPT I deficiency; CPT II deficiency; glutaric aciduria type II; Kearns-Sayre syndrome; lactic acidosis; long-chain acyl-CoA dehydrongenase deficiency (LCHAD); Leigh disease or syndrome; lethal infantile cardiomyopathy (LIC); Luft disease; glutaric aciduria type II; medium-chain acyl-CoA dehydrongenase deficiency (MCAD); myoclonic epilepsy and ragged-red fiber (MERRF) syndrome; mitochondrial recessive ataxia syndrome; mitochondrial cytopathy; mitochondrial DNA depletion syndrome; myoneurogastointestinal disorder and encephalopathy;

Pearson syndrome; pyruvate carboxylase deficiency; pyruvate dehydrogenase deficiency; POLG mutations; medium/short-chain 3-hydroxyacyl-CoA dehydrogenase (M/SCHAD) deficiency; and very long-chain acyl-CoA dehydrongenase (VLCAD) deficiency.

In some embodiments, methods of treating neurodegenerative diseases are provided. In some embodiments, the method comprises administering to a subject an effective amount of an inhibitor of USP30.

In some embodiments, the neurodegenerative disease is selected from Alzheimer's disease, Parkinson's disease, amyotrophic lateral sclerosis (ALS), Huntington's disease, ischemia, stroke, dementia with Lewy bodies, and frontotemporal dementia.

In some embodiments, methods of treating Parkinson's disease are provided. In some embodiments, the method comprises administering to a subject an effective amount of an inhibitor of USP30.

In some embodiments, methods of treating conditions involving cells undergoing oxidative stress are provided. In some embodiments, the method comprises administering to a subject an effective amount of an inhibitor of USP30.

In some embodiments involving treatment of a subject, the subject comprises a pathogenic mutation in Parkin, a pathogenic mutation in PINK1, or a pathogenic mutation in Parkin and a pathogenic mutation in PINK1 in at least a portion of the subject's cells. In some embodiments, the pathogenic mutation in Parkin is selected from the mutations in Table 1. In some embodiments, the pathogenic mutation in PINK1 is selected from the mutations in Table 2.

In some embodiments, the inhibitor of USP30 is administered orally, intramuscularly, intravenously, intraarterially, intraperitoneally, or subcutaneously. In some embodiments, the method comprises administering at least one additional therapeutic agent. In some embodiments, the at least one additional therapeutic agent is selected from levodopa, a dopamine agonist, a monoamino oxygenase (MAO) B inhibitor, a catechol O-methyltransferase (COMT) inhibitor, an anticholinergic, amantadine, riluzole, a cholinesterase inhibitor, memantine, tetrabenazine, an antipsychotic, clonazepam, diazepam, an antidepressant, and an anti-convulsant.

In any of the methods described herein, the inhibitor of USP30 may be an inhibitor of USP30 expression. Nonlimiting exemplary inhibitors of USP30 expression include antisense oligonucleotides and short interfering RNAs (siRNAs). In any of the methods described herein, the inhibitor of USP30 may be an inhibitor of USP30 activity. Nonlimiting exemplary inhibitors of USP30 activity include antibodies, peptides, peptibodies, aptamers, and small molecules.

In some embodiments, a peptide inhibitor of USP30 comprises the amino acid sequence:

$X_1X_2CX_3X_4X_5X_6X_7X_8X_9X_{10}X_{11}CX_{12}$ (SEQ ID NO: 48)

wherein:

$X_1$ is selected from L, M, A, S, and V;
$X_2$ is selected from Y, D, E, I, L, N, and S;
$X_3$ is selected from F, I, and Y;
$X_4$ is selected from F, I, and Y;
$X_5$ is selected from D and E;
$X_6$ is selected from L, M, V, and P;
$X_7$ is selected from S, N, D, A, and T;
Xg is selected from Y, D, F, N, and W;
$X_9$ is selected from G, D, and E;
$X_{10}$ is selected from Y and F;
$X_{11}$ is selected from L, V, M, Q, and W; and
$X_{12}$ is selected from F, L, C, V, and Y.

In some embodiments, a peptide inhibitor of USP30 peptide comprises an amino acid sequence that is at least 80%, at least 85%, at least 90%, at least 95%, or 100% identical to an amino acid sequence selected from SEQ ID NOs: 1 to 22. In some embodiments, the peptide inhibits USP30 with an IC50 of less than 10 $\mu$M. In some embodiments, the IC50 of a peptide inhibitor of USP30 for at least one, at least two, or at least three peptidases selected from USP7, USP5, UCHL3, and USP2 is greater than 20 $\mu$M, greater than 30 $\mu$M, greater than 40 $\mu$M, or greater than 50 $\mu$M.

In some embodiments, an antisense oligonucleotide comprises a nucleotide sequence that is at least at least 80%, at least 85%, at least 90%, at least 95%, or 100% complementary to a region of USP30 mRNA and/or a region of USP30 pre-mRNA. In some embodiments, the region of USP30 mRNA or region of USP30 pre-mRNA is at least at least 10, at least 15, at least 20, at least 25, at least 30, at least 40, at least 50, at least 60, at least 70, at least 80, at least 90, or at least 100 nucleotides long. In some embodiments, the antisense oligonucleotide is 10 to 500 nucleotides long, or 10 to 400 nucleotides long, or 10 to 300 nucleotides long, or 10 to 200 nucleotides long, or 10 to 100 nucleotides long, or 15 to 100 nucleotides long, or 10 to 50 nucleotides long, or 15 to 50 nucleotides long. An antisense oligonucleotide may comprise one or more non-nucleotide components.

In some embodiments, an siRNA comprises a nucleotide sequence that is at least at least 80%, at least 85%, at least 90%, at least 95%, or 100% identical to a region of USP30 mRNA and/or a region of USP30 pre-mRNA. In some embodiments, the region of USP30 mRNA or region of USP30 pre-mRNA is at least at least 10, at least 15, at least 20, or at least 25 nucleotides long. In some embodiments, the siRNA is 10 to 200 nucleotides long, or 10 to 100 nucleotides

long, or 15 to 100 nucleotides long, or 10 to 60 nucleotides long, or 15 to 60 nucleotides long, or 10 to 50 nucleotides long, or 15 to 50 nucleotides long, or 10 to 30 nucleotides long, or 15 to 30 nucleotides long. In some embodiments, an siRNA is an shRNA.

An embodiment is an inhibitor of USP30 for the treatment of a condition involving a mitochondrial defect in a subject. In a particular embodiment the condition involving a mitochondrial defect is selected from a condition involving a mitophagy defect, a condition involving a mutation in mitochondrial DNA, a condition involving mitochondrial oxidative stress, a condition involving a defect in mitochondrial shape or morphology, a condition involving a defect in mitochondrial membrane potential, and a condition involving a lysosomal storage defect. in another particular embodiment the condition involving a mitochondrial defect is selected from a neurodegenerative disease; mitochondrial myopathy, encephalopathy, lactic acidosis, and stroke-like episodes (MELAS) syndrome; Leber's hereditary optic neuropathy (LHON); neuropathy, ataxia, retinitis pigmentosa-maternally inherited Leigh syndrome (NARP-MILS); Danon disease; ischemic heart disease leading to myocardial infarction; multiple sulfatase deficiency (MSD); mucolipidosis II (ML II); mucolipidosis III (ML III); mucolipidosis IV (ML IV); GM1-gangliosidosis (GM1); neuronal ceroid-lipofuscinoses (NCL1); Alpers disease; Barth syndrome; Beta-oxidation defects; carnitine-acyl-carnitine deficiency; carnitine deficiency; creatine deficiency syndromes; co-enzyme Q10 deficiency; complex I deficiency; complex II deficiency; complex III deficiency; complex IV deficiency; complex V deficiency; COX deficiency; chronic progressive external ophthalmoplegia syndrome (CPEO); CPT I deficiency; CPT II deficiency; glutaric aciduria type II; Kearns-Sayre syndrome; lactic acidosis; long-chain acyl-CoA dehydrongenase deficiency (LCHAD); Leigh disease or syndrome; lethal infantile cardiomyopathy (LIC); Luft disease; glutaric aciduria type II; medium-chain acyl-CoA dehydrongenase deficiency (MCAD); myoclonic epilepsy and ragged-red fiber (MERRF) syndrome; mitochondrial recessive ataxia syndrome; mitochondrial cytopathy; mitochondrial DNA depletion syndrome; myoneurogastointestinal disorder and encephalopathy; Pearson syndrome; pyruvate carboxylase deficiency; pyruvate dehydrogenase deficiency; POLG mutations; medium/short-chain 3-hydroxyacyl-CoA dehydrogenase (M/SCHAD) deficiency; and very long-chain acyl-CoA dehydrongenase (VLCAD) deficiency. In a more particular embodiment the neurodegenerative disease is selected from Alzheimer's disease, Parkinson's disease, amyotrophic lateral sclerosis (ALS), Huntington's disease, ischemia, stroke, dementia with Lewy bodies, and frontotemporal dementia.

Another embodiment is an inhibitor of USP30 for the treatment of a neurodegenerative disease in a subject comprising administering to the subject. In a raticular embodiment, the neurodegenerative disease is selected from Parkinson's disease, Alzheimer's disease, Huntington's disease, amyotrophic lateral sclerosis (ALS), ischemia, stroke, dementia with Lewy bodies, and frontotemporal dementia.

Also an embodiment of the present invention is an inhibitor of USP30 for the treatment of Parkinson's disease in a subject.

In another embodiment the inhibitor of USP30 is administered orally, intramuscularly, intravenously, intraarterially, intraperitoneally, or subcutaneously.

In a particular embodiment of the present invention. the inhibitor of USP30 for the use in a treatment as described herein is combined with at least one additional therapeutic agent. in a further particular embodiment, the at least one additional therapeutic agent is selected from levodopa, a dopamine agonist, a monoamino oxygenase (MAO) B inhibitor, a catechol O-methyltransferase (COMT) inhibitor, an anticholinergic, amantadine, riluzole, a cholinesterase inhibitor, memantine, tetrabenazine, an antipsychotic, clonazepam, diazepam, an antidepressant, and an anti-convulsant. The invention is defined in the claims.

## BRIEF DESCRIPTION OF THE FIGURES

The patent or application file contains at least one drawing executed in color. Copies of this patent or patent application publication with color drawing(s) will be provided by the Office upon request and payment of the necessary fee.

Figure 1A shows immunostaining of HeLa cells cotransfected with GFP-Parkin, and individual FLAG-tagged DUBs. Following 24 hours of expression, cells were treated with CCP (20 μM, 24h) and immunostained for GFP, FLAG, and endogenous Tom20. Representative images are shown for FLAG-tagged USP30, DUBA2, UCH-L1, USP15 and ATXN3; other DUBs are not shown. Scale bar, 10 μm. Figure 1B shows immunostaining of SH-SY5Y cells cotransfected with GFP-Parkin and the indicated control (β-Gal) and USP30 constructs. Following 24 hours of expression, cells were treated with CCCP (20 μM, 24h) and immunostained for myc, FLAG, and endogenous Tom20 and HSP60 (Scale bar, 5 μm). Figure 1C shows quantification of percent of cells with Tom20 or HSP60 staining from Figure 1B (***$p<0.001$ by One-way ANOVA - Dunnett's Multiple Comparison test. N=3 experiments. Error bars represent SEM). Figure 1D shows quantification of total Tom20 and HSP60 fluorescence intensity per cell from Figure 1B (**$p<0.01$ by One-way ANOVA - Dunnett's Multiple Comparison test. n = 63, 67 and 54 cells for control (β-Gal), USP30-FLAG and USP30-C77S-FLAG groups, respectively. N=3 experiments. Error bars represent SEM). Figure 1E shows quantification of percent of cells containing Parkin clusters from Figure 1B (***$p<0.001$ by One-way ANOVA - Dunnett's Multiple Comparison test. N=3 experiments. Error bars represent SEM).

Figure 2A shows immunostaining of transfected USP30-FLAG (red) and mitochondria-targeted GFP (green) in cultured rat hippocampal neurons. Merge is shown in color; individual channels in gray-scale. Scale bar, 5 μm. Figure 2B shows immunostaining of SH-Sy5Y cells transfected with control or USP30 siRNA. Following 3 days of knockdown, cells were fixed and immunostained for endogenous USP30 and HSP60. USP30 siRNA primarily decreased mitochondrial USP30 antibody staining (Scale bar, 5 μm). Higher magnification images of the boxed regions are shown on the right panel (Scale bar, 2 μm). Figure 2C shows immunoblots of cytoplasm- and mitochondria-enriched fractions from rat brain with USP30, HSP60, and GAPDH antibodies. Figure 2D shows immunostaining of SH-SY5Y cells cotransfected with GFP-Parkin and the indicated control (β-Gal) and USP30 constructs. Following 24 hours of expression, cells were treated with CCCP (20 μM, 4h) and immunostained for GFP, FLAG, and endogenous Tom20 and polyubiquitin chains (detected with the FK2 antibody) (Scale bar, 5 μm). Figure 2E is a plot showing the quantification of mitochondria-associated polyubiquitin staining intensity normalized by mitochondrial area from Figure 2D (integrated fluorescence intensity of mitochondrial FK2 staining / area of Tom20 staining). (***p<0.001 by One-way ANOVA - Dunnett's Multiple Comparison test. n = 61, 45 and 59 cells for β-Gal, USP30-FLAG and USP30-C77S-FLAG groups, respectively. Error bars represent SEM). Figure 2F shows immunoblots of cell lysates from GFP-Parkin expressing stable HEK-293 cells transfected with the indicated control (β-Gal) and USP30 constructs. Following 24 hours of expression, cells were treated with CCCP (5 μM, 2 hours) and lysed. Figure 2G is a plot showing the quantification of immunoblot signal for GFP-Parkin normalized to actin from Figure 2F (***p<0.001 by One-way ANOVA - Dunnett's Multiple Comparison test. N=6 experiments. Error bars represent SEM).

Figure 3A shows that mt-Keima differentially highlights cytoplasmic (green) and lysosomal (red) mitochondria. Cultured hippocampal neurons were transfected with mt-Keima and GFP. Following 2 days of expression, cells were imaged with 458 nm (shown in green) or 543 nm (shown in red) light excitation. GFP signal was used to outline the cell (shown in white). Scale bar, 5 μm. Figure 3B shows mt-Keima imaging in neurons transfected with Parkin shRNA knockdown constructs. Scale bar, 5 μm. Figure 3C is a plot showing the quantification of mitophagy index from Figure 3B (**p<0.01 and ***p<0.001 by One-way ANOVA - Dunnett's Multiple Comparison test. n=52-109 cells per group. N=3-6 experiments. Error bars represent SEM). Figure 3D shows mt-Keima imaging in neurons transfected with PINK1 shRNA knockdown constructs. Scale bar, 5 μm. Figure 3E is a plot showing the quantification of mitophagy index from Figure 3D (**p<0.01 and ***p<0.001 by One-way ANOVA - Dunnett's Multiple Comparison test. n=52-109 cells per group. N=3-6 experiments. Error bars represent SEM). Figure 3F shows mt-Keima imaging in neurons transfected with PINK1-GFP and Parkin-shRNA#1 (luciferase shRNA and β-Gal as controls). Scale bar, 5 μm. Figure 3G is a plot showing the quantification of mitophagy index from Figure 3F (***p<0.001 by One-way ANOVA - Dunnett's Multiple Comparison test. n=55-77 cells. N=3 experiments. Error bars represent SEM).

Figure 4A shows mt-Keima imaging in cultured hippocampal neurons before and after $NH_4Cl$ treatment (50mM, 2 minutes). mt-Keima signal collected with 543 nm or 458 nm laser excitation sources are shown in red and green, respectively. Scale bar, 5 μm. Figure 4B shows imaging of mt-Keima and Lysotracker (shown in gray scale) in hippocampal neurons. Scale bar, 5 μm. Figure 4C shows post-hoc immunostaining for endogenous LAMP-1 in neurons imaged for mt-Keima signal. Immediately following mt-Keima imaging, cells were fixed and stained with anti-LAMP1 antibody (shown in gray scale). Scale bar, 5 μm. Figure 4D is a plot showing quantification of mitophagy index following 1, 3 and 6-7 days of mt-Keima expression in cultured hippocampal neurons (*p<0.05 and ***p<0.001 using One-way ANOVA - Bonferroni's Multiple Comparison test. n=56-146 cells. N=6 experiments. Error bars represent SEM). Figure 4E is an immunoblot of HEK-293 cell lysates transfected with FLAG-Parkin cDNA and Parkin shRNA expression constructs. PSD-95-FLAG was co-transfected as control. Figure 4F is an immunoblot of HEK-293 cell lysates transfected with PINK1-GFP cDNA and PINK shRNA constructs. PSD-95-FLAG was co-transfected as control. Figure 4G shows an immunoblot of endogenous Parkin in cultured hippocampal neurons infected with Adeno-associated virus expressing the indicated shRNAs. Figure 4H shows an immunoblot of endogenous PINK1 in cultured hippocampal neurons infected with Adeno-associated virus expressing the indicated shRNAs. Figure 4I shows mt-Keima imaging in neurons transfected with GFP-Parkin (or GFP as control). Scale bar, 5 μm. Figure 4J is a plot showing quantification of mitophagy index from Figure 4I (p=0.52 by Student's t-test. n=61-67 cells. N=3 experiments. Error bars represent SEM).

Figure 5A shows mt-Keima imaging in neurons transfected with USP30-FLAG or USP30-C77S-FLAG. Scale bar, 5 μm. Figure 5B shows immunoblots of HEK-293 cell lysates transfected with the indicated cDNA and shRNA constructs. PSD-95-FLAG was co-transfected as control. Figure 5C shows an immunoblot of endogenous USP30 in cultured hippocampal neurons infected with Adeno-associated virus particles expressing the USP30 shRNA. Figure 5D shows mt-Keima imaging in neurons transfected with rat USP30 shRNA and human USP30 cDNA expression constructs (luciferase shRNA and β-Gal as controls). Scale bar, 5 μm. Figure 5E is a plot showing quantification of mitophagy index from Figure 5A (***p<0.001 by One-way ANOVA - Bonferroni's Multiple Comparison test. 43-122 cells. N=6 experiments. Error bars represent SEM). Figure 5F is a plot showing the quantification of

mitophagy index from Figure 5B (**p<0.01 and ***p<0.001 by One-way ANOVA - Dunnett's Multiple Comparison test. n=96-101 cells. N=4 experiments. Error bars represent SEM).

Figure 6A shows immunoblots of anti-HA-immunoprecipitates for endogenous MIRO and Tom20 in a parental HEK-293 cell line (that lacks GFP-Parkin) transfected with HA-ubiquitin and the indicated constructs. Following 24 hours of expression, cells were treated with CCCP (5 μM, 2 hours) and ubiquitinated proteins were immunoprecipitated with anti-HA beads. Immunoprecipitates and inputs were blotted with the indicated antibodies. Figure 6B shows immunoblots of anti-HA-immunoprecipitates for endogenous MIRO and Tom20 with USP30 knockdown. GFP-Parkin expressing stable HEK-293 cells were transfected with HA-ubiquitin and the indicated shRNA and cDNA expression constructs. Following 6 days of expression, cells were processed as in Figure 6A. Figure 6C and E show immunoblots of anti-HA-immunoprecipitates for endogenous Miro and Tom20 from cells transfected with the indicated HA-tagged ubiquitin mutants and treated with CCCP (20 μM, 2 hours). Figure 6D and F show quantification of immunoblot signals from (C) and (E). Amount of ubiquitination afforded by the ubiquitin mutants are reported relative to wild-type ubiquitin (**p<0.01 and ***p<0.001 compared to 'wild-type HA-ubiquitin + CCCP' group, using one-way ANOVA with Dunnett's Multiple Comparison test. δ denotes ***p<0.001). Figure 6G shows immunoblots of GFP-Parkin HEK-293 stable cell lysates that were transfected with the indicated FLAG-tagged USP30 constructs and treated with CCCP (5 μM, 1-6 hours). Figure 6H is a plot showing quantification of immunoblot signals normalized to actin shown in Figure 6G (*p<0.05, **p<0.01, ***p<0.001 compared to β-Gal control, using Two-way ANOVA with Bonferroni's Multiple Comparison test. Immunoblot signals for all other proteins (VDAC, Mfn-1, Tom70, Hsp60) did not reach significance. N=3-5 experiments).

Figure 7A shows immunoblots of anti-HA-immunoprecipitates for endogenous MIRO and Tom20 with USP30 overexpression. HEK-293 cells stably expressing GFP-Parkin were transfected with HA-ubiquitin and the indicated constructs. Following 24 hours of expression, cells were treated with CCCP (5 μM, 2 hours) and ubiquitinated proteins were immunoprecipitated with anti-HA beads. Immunoprecipitates and inputs were blotted with the indicated antibodies. Figure 7B is a plot showing quantification of the immunoblot signal for co-IP'ed MIRO from Figure 7A. Figure 7C is a plot showing quantification of the immunoblot signal for co-IP'ed Tom20 from Figure 7A. Protein levels co-precipitated with anti-HA beads are normalized to 'β-Gal + CCCP' group (*p<0.05, **p<0.01 and ***p<0.001 by One-way ANOVA - Dunnett's Multiple Comparison test, compared to β-Gal + CCCP. N=3-5 experiments. Error bars represent SEM). Figure 7D shows immunoblots of anti-HA immunoprecipitates for endogenous MIRO and Tom20 with USP30 knockdown. GFP-Parkin expressing stable HEK-293 cells were transfected with HA-ubiquitin and the indicated shRNA plasmids. Following 6 days of expression, cells were processed as in Figure 7A. Figure 7E is a plot showing quantification of the immunoblot signal for co-IP'ed MIRO from Figure 7D. Figure 7F is a plot showing quantification of the immunoblot signal for co-IP'ed Tom20 from Figure 7D. Protein levels co-precipitated with anti-HA beads is normalized to 'luciferase shRNA + CCCP' group (*p<0.05, **p<0.01 and ***p<0.001 by One-way ANOVA - Dunnett's Multiple Comparison test, compared to 'luciferase shRNA+CCCP'. N=4-6 experiments. Error bars represent SEM).

Figure 8A shows immunoblots of HA-ubiquitin precipitates from GFP-Parkin HEK-293 cells transfected with the indicated constructs. Following transfection and treatment with CCCP (5 μM, 2 hours), ubiquitinated proteins were immunoprecipitated with anti-HA beads, and precipitates and inputs were immunoblotted with the indicated antibodies. Figure 8B shows mt-Keima imaging in neurons transfected with Tom20-myc and USP30 constructs (β-Gal as control). Scale bar, 5 μm. Figure 8C shows mt-Keima imaging in neurons transfected with USP30 shRNA and MIRO cDNA constructs (luciferase RNAi and β-Gal as controls). Scale bar, 5 μm. Figure 8D is a plot showing the quantification of mitophagy index from Figure 8B (***p<0.001 by One-way ANOVA - Dunnett's Multiple Comparison test. n=67-80 cells for all groups. N=3 experiments. Error bars represent SEM). Figure 8E is a plot showing quantification of mitophagy index from Figure 8C (*p<0.05 and ***p<0.001 by One-way ANOVA - Bonferroni's Multiple Comparison test. n=72-75 cells for all groups. N=3 experiments. Error bars represent SEM).

Figure 9A shows extracted ion chromatograms corresponding to K-GG peptides identified from Tom20 in the USP30 knockdown experiment. Relative abundance of each ubiquitinated peptide is shown on the y-axis relative to the most abundant analysis, which precursor ion m/z indicated above each peak. The sequence of each K-GG peptide is shown below in green. Asterisks denote modified lysine residues. Figure 9B shows extracted ion chromatograms corresponding to K-GG peptides identified from USP30 in the Parkin overexpression experiment. The data are presented in a similar manner as in (A). Figure 9C shows immunoblots of anti-HA-immunoprecipitates for endogenous USP30 from cells transfected with wild-type, K161N and G430D GFP-Parkin constructs. After 24 hours of expression, cells were treated with CCCP (20 μM, 2 hours) and ubiquitinated proteins were immunoprecipitated with anti-HA beads. Immunoprecipitates and inputs were blotted with the indicated antibodies. Figure 9D shows quantification of immunoblot signal for co-IP'ed USP30 from (C). Protein levels co-precipitating with anti-HA beads are normalized to the 'wild-type GFP-Parkin + CCCP' group. (***p<0.001 by One-way ANOVA - Dunnett's Multiple Comparison test, compared to 'wild-type GFP-Parkin + CCCP'. N = 4 experiments. Error bars represent S.E.M.) Figure 9E shows immunoblots of lysates prepared from HEK-293 cells transfected with the indicated GFP and GFP-Parkin constructs

and treated with CCCP (20 μM). Figure 9F shows quantification of immunoblot signal for USP30 normalized to actin from (E). (**p<0.01, ***p<0.001 compared to wild-type GFP-Parkin, using Two-way ANOVA with Bonferroni's Multiple Comparison test. N = 4 experiments. Error bars represent S.E.M.)

Figure 10A shows immunostaining in GFP-Parkin-G430D expressing stable SH-SY5Y cells transfected with the indicated siRNAs and cDNA expression constructs. Following 3 days of expression, cells were treated with CCCP (20 μM, 24 hours), and fixed and stained for GFP, FLAG, and endogenous Tom20. Scale bar, 5 μm. Figure 10B is a plot showing quantification of Tom20 fluorescence intensity from Figure 10A (***p<0.001 by One-way ANOVA - Dunnett's Multiple Comparison test, Error bars represent SEM). Figure 10C is a plot showing quantification of GFP-Parkin-G430D puncta area from Figure 10A (***p<0.001 by One-way ANOVA - Dunnett's Multiple Comparison test, Error bars represent SEM). Figure 10D shows mt-Keima imaging in neurons transfected with Parkin shRNA and USP30-C77A-FLAG. Scale bar, 5 μm. Figure 10E is a plot showing quantification of mitophagy index from Figure 10D (***p<0.001 by One-way ANOVA - Dunnett's Multiple Comparison test. N=71-77 cells. N=3 experiments. Error bars represent SEM).

Figure 11A shows an immunoblot for endogenous USP30 in SH-SY5Y cells transfected with USP30 siRNA for 3 days. Figures 11B and 11C show immunostaining in GFP-Parkin-G430D expressing stable SH-SY5Y cells transfected with the indicated siRNAs. Following 3 days of knockdown, cells were treated with CCCP (20 μM, 24 hours), and fixed and stained for GFP and endogenous Tom20. Scale bar, 5 μm. Figure 11D is a plot showing quantification of fold change in Tom20 staining intensity from Figures 11B and 11C normalized to control siRNA (***p<0.001 by One-way ANOVA - Dunnett's Multiple Comparison test. Error bars represent SEM). Figures 11E and 11F show immunostaining in GFP-Parkin-G430D (E) and GFP-Parkin-K161N (F) expressing SH-SY5Y cells transfected with USP30 siRNA. Following 3 days of knockdown, cells were treated with CCCP (20 μM, 24 hours), and fixed and stained for GFP and endogenous Tom20 and HSP60. Scale bar, 5 μm. Figures 11G and 11H are plots showing quantification of fold change in Tom20 (G) and HSP60 (H) staining intensity from Figures 11E and 11F normalized to control siRNA. (*p<0.05, **p<0.01 and ***<0.001 by Student's t-test. N=2-3 experiments. Error bars represent S.E.M.)

Figure 12A shows transverse sections of indirect flight muscles (IFMs) from *wild-type,* parkin mutant *(park$^{25}$)* and "parkin mutant; dUSP30 knockdown" *(park$^{25}$; Actin-GAL4 > UAS-dUSP30$^{RNAi}$)* flies. Electron-dense mitochondria are marked with arrowheads. Mitochondria with reduced and disorganized cristae (hence pale in appearance) are outlined with dashed lines (top panel - Scale bar, 1 μm). Higher magnification images are shown in the lower panels (Scale bar, 0.2 μm). Figure 12B and C show quantification of mitochondrial integrity from (A). Percent area of mitochondria containing disorganized cristae over total mitochondrial area (B), and percent of muscle area containing disorganized mitochondria (C) are blindly quantified. (*p<0.05, **p<0.01 and ***p<0.001, compared to *wild-type* by Two-way ANOVA - Bonferroni's Multiple Comparison test. ***p<0.001 for *park$^{25}$* versus *park$^{25}$; Actin-GAL4 > UAS-dUSP30$^{RNAi}$.* 34-55 imaging fields per fly, N = 3-4 flies. Error bars represent S.E.M.) Figure 12D and E show effect of dUSP30 knockdown and paraquat on climbing assay in Drosophila. Percent of flies climbing >15 cm in 30 seconds, treated with vehicle (5% sucrose) or paraquat (10mM, 48 hours), for the indicated genotypes. (**p<0.01 and ***p<0.001 by One-Way ANOVA with Bonferroni's multiple comparisons test. N = 4-10 experiments. Error bars represent S.E.M.) Figure 12F shows dopamine neurotransmitter levels per *Drosophila* head for the indicated genotypes, as determined by ELISA. (*p<0.05 and ***p<0.001 by One-way ANOVA - Bonferroni's Multiple Comparison test. n=28 heads per genotype. N=4 experiments. Error bars represent S.E.M.). Figure 12G and H show effect of dUSP30 knockdown and paraquat on survival in *Drosophila.* Percent of flies still alive, treated with vehicle or paraquat (10 mM, up to 96 hours), for the indicated genotypes. (**p<0.01 and ***p<0.001 using Two-Way ANOVA with Bonferroni's multiple comparisons test. N=3 (G) and 4 (H) experiments. Error bars represent S.E.M.)

Figure 13 shows asymmetric "volcano plot" demonstrating the subset of 41 proteins whose ubiquitination significantly increased (p<0.05) for the "Combo" treatment versus CCCP-treatment alone in both USP30 knockdown (left side) and GFP-Parkin overexpression (right side) experiments. "Combo" refers to cells treated with CCCP and expressing USP30-shRNA, or treated with CCCP and expressing GFP-Parkin, in the two experiments, respectively. For this subset of proteins, fold-increase in ubiquitination (x-axis) and the p-value (y-axis) are reported. Mitochondrial proteins (identified based on the Human MitoCarta database) are shown in red.

Figure 14 shows inhibition of various peptidases, including USP30, by inhibitory peptides USP30_3 ("pep3"; SEQ ID NO: 1) and USP30_8 ("pep8"; SEQ ID NO: 2), as described in Example 10.

Figure 15 shows a graph of residue probability by peptide position for USP30_3 and certain affinity-matured peptides, along with the signal to noise ratio ("S/N"), ELISA signal ("signal"), number of clones for each sequence ("n"), total number of clones ("total"), and the number of unique sequences ("Uniq"), as described in Example 10.

Figure 16 shows a graph of signal to noise ratio for USP30_3 and three affinity matured peptides, as described in Example 10. For each peptide, the targets tested were, from left to right, USP2, USP7, USP14, USP30, UCHL1, UCHL3, and UCHL5. The sequences for each peptide are shown below.

Figure 17A shows ratiometric mito-roGFP imaging in hippocampal neurons transfected with USP30 shRNA. The

"relative oxidation index" was shown in a 'color scale' from 0 (mito-roGFP ratio after DTT treatment, 1mM, shown in black) to 1 (mito-roGFP ratio after aldrithiol treatment, 100 μM, shown in red). Figure 17B is a plot showing quantification of relative oxidation from Figure 17A (***p<0.001 by Student's t-test. n=24 cells for luciferase shRNA and 36 cells for USP30 shRNA. N = 3 experiments. Error bars represent SEM). Figure 17C shows quantitative RT-PCR of dUSP30 mRNA. qRT-PCR in *Actin-GAL4, UAS-dUSP30$^{RNAi}$,* and *Actin-GAL4> UAS-dUSP30$^{RNAi}$* flies, expressed relative to *Actin-GAL4.* dUSP30 mRNA levels were normalized to *Drosophila* RpII140 mRNA levels in each group. N = 7 experiments. ***p<0.001 by One-Way ANOVA with Bonferroni's multiple comparisons test. Figure 17D shows climbing assay in control flies (*Actin-GAL4*). Flies were treated with vehicle control (5% sucrose) or paraquat (10 mM, 48 hours). L-DOPA (1mM, 48 hours) was administered simultaneously with paraquat, as indicated. (***p<0.001 by One-Way ANOVA - Dunnett's Multiple Comparison test. N=6 experiments. Error bars represent S.E.M.). Figure 17E shows serotonin levels per fly head, as assessed by ELISA. Flies were treated with paraquat (10 mM, 48 hours) or vehicle control (5% sucrose). (p-values calculated by One-Way ANOVA - Bonferroni's Multiple Comparison test. n=8 heads, N=2 experiments. Error bars represent S.E.M.). Figure 17F and G show quantitative RT-PCR measurement of (F) dUSP47 and (G) dYOD1 mRNA levels in flies of the indicated genotypes, expressed as relative to *Actin-GAL4* genotype. TaqMan assays Dm01795269_g1 (*Drosophila* CG5486 (USP47)) and Dm018401l5_s1 (*Drosophila* CG4603 (YOD1)) were used. Dm02134593_g1 (RpII140) was used for normalization. (p**<0.01 and p***<0.001 using One-Way ANOVA - Dunnett's Multiple Comparison test. N=3 replicates. Error bars represent S.E.M.) Figure 17H and I show survival curves of flies of the indicated genotype, treated with vehicle or paraquat (10 mM). Graph shows percent flies alive at indicated times after feeding with paraquat. (*p<0.05, p**<0.01, and p***<0.001 using Two-Way ANOVA with Bonferroni's Multiple Comparisons test. N=5 (H) and 4 (I) experiments. Error bars represent S.E.M.)

## DETAILED DESCRIPTION

The present inventors have identified USP30, a mitochondria-localized deubiquitinase (DUB) as an atagonist of Parkin-mediated mitophagy. USP30, through its deubiquitinase activity, counteracts ubiquitination and degradation of damaged mitochondria, and inhibition of USP30 rescues mitophagy defects caused by mutant Parkin. Further, USP30 inhibition of USP30 decreases oxidative stress and provides protection against the mitochondrial toxin, rotenone. Since damaged mitochondria are more likely to accumulate Parkin, USP30 inhibition should preferentially clear unhealthy mitochondria. In addition to neurons (such as substantia nigra neurons, which are especially vulnerable to mitochondria dysfunction in Parkinson's disease), long-lived metabolically active cells such as cardiomyocytes also rely on an efficient mitochondria quality control system. In this context, Parkin has been shown to protect cardiomyocytes against ischemia/reperfusion injury through activating mitophagy and clearing damaged mitochondria in response to ischemic stress. Thus, inhibitors of USP30 are provided for us in treating a conditions involving mitochondrial defects, including neurological conditions, cardiac conditions, and systemic conditions.

## I. DEFINITIONS

An "inhibitor" refers to an agent capable of blocking, neutralizing, inhibiting, abrogating, reducing and/or interfering with one or more of the activities of a target and/or reducing the expression of the target protein (or the expression of nucleic acids encoding the target protein). Inhibitors include, but are not limited to, antibodies, polypeptides, peptides, nucleic acid molecules, short interfering RNAs (siRNAs) and other inhibitory RNAs, small molecules (e.g., small inorganic molecules), polysaccharides, polynucleotides, antisense oligonucleotides, aptamers, and peptibodies. An inhibitor may decrease the activity and/or expression of a target protein by at least 10% (e.g., by at least 15%, 20%, 25%, 30%, 35%, 40%, 45%, 50%, 55%, 60%, 65%, 70%, 75%, 80%, 85%, 90%, or even 100% decrease) as compared to the expression and/or activity of the target protein that is untreated with the inhibitor.

An "inhibitor of USP30" refers to an agent capable of blocking, neutralizing, inhibiting, abrogating, reducing and/or interfering with one or more of the activities of USP30 and/or reducing the expression of USP30 (or the expression of nucleic acids encoding USP30). In some embodiments, an inhibitor of USP30 reduces the deubiquitinase activity of USP30. In some embodiments, an inhibitor of USP30 reduces deubiquitinase activity by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or 100%. Deubiquitinase activity may be reduced by an inhibitor by any mechanism, including, but not limited to, interfering with the active site of USP30, interfering with target recognition, altering the conformation of USP30, interfering with proper subcellular localization of USP30, etc. In some embodiments, an inhibitor of USP30 inhibits USP30 expression, which may be expression as the mRNA (e.g., it inhibits transcription of the USP30 gene to produce USP30 mRNA) and/or protein level (e.g., it inhibits translation of the USP30 mRNA to produce USP30 protein). In some embodiments, an inhibitor of USP30 expression reduces the level of USP30 protein by at least 10%, at least 15%, at least 20%, at least 25%, at least 30%, at least 35%,

at least 40%, at least 45%, at least 50%, at least 55%, at least 60%, at least 65%, at least 70%, at least 75%, at least 80%, at least 85%, at least 90%, or 100%.

The terms "mitophagy" and "mitochondrial degradation" are used interchangeably to refer to the regulated degradation of mitochondria through the lysosomal machinery of a cell.

A "condition involving a mitochondrial defect" refers to a condition involving a defect or defects in mitochondrial function, mitochondrial shape/morphology, mitochondrial membrane potential, and/or mitophagy in a cell population. Conditions involving a mitochondrial defect include, but are not limited to, conditions involving a defect in mitophagy, such that mitophagy occurs in the cell population at a slower rate or to a lesser extent than in a normal cell population. In some embodiments, the defect in mitophagy is accompanied by other mitochondrial defects such that the decreased mitophagy results in the increased presence of defective mitochondria. Conditions involving a mitochondrial defect also include, but are not limited to, conditions involving mutations in mitochondrial DNA that result in altered mitochondrial function. Conditions involving a mitochondrial defect also include conditions involving mitochondrial oxidative stress, in which increased levels of reactive oxygen species (ROS) and/or reactive nitrogen species (RNS) in a cell are associated with protein aggregation and/or mitochondrial dysfunction. Mitochondrial oxidative stress may result in mitochondrial dysfunction, or mitochondrial dysfunction may result in oxidative stress. Conditions involving a mitochondrial defect also include, but are not limited to, conditions involving defects in mitochondrial shape/morphology and conditions involving defects in mitochondrial membrane potential. Exemplary conditions involving mitochondrial defects include, but are not limited to, neurodegenerative diseases (such as Parkinson's disease, Huntington's disease, amyotrophic lateral sclerosis (ALS), Alzheimer's disease, ischemia, stroke, dementia with Lewy bodies, and frontotemporal dementia); mitochondrial myopathy, encephalopathy, lactic acidosis, and stroke-like episodes (MELAS) syndrome; Leber's hereditary optic neuropathy (LHON); neuropathy, ataxia, retinitis pigmentosa-maternally inherited Leigh syndrome (NARP-MILS); Danon disease; myoclonic epilepsy with ragged red fibers (MERFF) syndrome; ischemic heart disease leading to myocardial infarction; multiple sulfatase deficiency (MSD); mucolipidosis II (ML II); mucolipidosis III (ML III); mucolipidosis IV (ML IV); GM1-gangliosidosis (GM1); neuronal ceroid-lipofuscinoses (NCL1); Alpers disease; Barth syndrome; Beta-oxidation defects; carnitine-acyl-carnitine deficiency; carnitine deficiency; creatine deficiency syndromes; co-enzyme Q10 deficiency; complex I deficiency; complex II deficiency; complex III deficiency; complex IV deficiency; complex V deficiency; COX deficiency; chronic progressive external ophthalmoplegia syndrome (CPEO); CPT I deficiency; CPT II deficiency; glutaric aciduria type II; Kearns-Sayre syndrome; lactic acidosis; long-chain acyl-CoA dehydrongenase deficiency (LCHAD); Leigh disease or syndrome; lethal infantile cardiomyopathy (LIC); Luft disease; glutaric aciduria type II; medium-chain acyl-CoA dehydrongenase deficiency (MCAD); myoclonic epilepsy and ragged-red fiber (MERRF) syndrome; mitochondrial recessive ataxia syndrome; mitochondrial cytopathy; mitochondrial DNA depletion syndrome; myoneurogastointestinal disorder and encephalopathy; Pearson syndrome; pyruvate carboxylase deficiency; pyruvate dehydrogenase deficiency; POLG mutations; medium/short-chain 3-hydroxyacyl-CoA dehydrogenase (M/SCHAD) deficiency; and very long-chain acyl-CoA dehydrongenase (VLCAD) deficiency.

A "pathogenic mutation" in Parkin or PINK1 refers to a mutation or mutations in the respective protein or gene that results in reduced activity in a cell, and may involve loss of function and/or gain of function (such as dominant negative mutations, for example, Parkin Q311stop). Such reduced activity in a cell may include, but is not limited to, reduced enzymatic activity (such as reduced ubiquitination or kinase activity), reduced activity due to the presence of a dominant negative mutant protein, reduced binding to another cellular factor, reduced activity due to subcellular localization changes, and/or reduced activity due to reduced levels of protein in the cell or in a cellular compartment. In some embodiments, a pathogenic mutation in Parkin and/or PINK1 results in reduced ubiquitination of mitochondria, which may result in reduced mitophagy. Pathogenic mutations may also occur outside of the coding region of the protein, e.g., in an intron (affecting, for example, splicing), the promoter, the 5' untranslated region, the 3' untranslated region, etc. Further, Parkin mutations may involve substitutions, deletions, insertions, duplications, etc., or any combination of those. Nonlimiting exemplary pathogenic mutations in Parkin are shown in Table 1. Nonlimiting exemplary pathogenic mutations in PINK1 protein are shown in Table 2. Databases of Parkinson's disease mutations are publicly available, such as Parkinson Disease Mutation Database, http://www.molgen.ua.ac.be/PDmutDB/.

Table 1: Exemplary pathogenic mutations in Parkin (PARK2)

| | | | |
|---|---|---|---|
| Ala291fs | ex10del | ex4-7del | Gln311Stop |
| Ala31Asp | ex10dup | ex4del | Gln34fs |
| Ala398Thr | ex11del | ex4dup | Gln34fs |
| Arg234Gln | ex11dup | ex5-12del | Gln40Stop |
| Arg334Cys | ex12dup | ex5-6del | Glu395Stop |
| Arg33Gln | ex1-4del | ex5-7del | Glu409Stop |

(continued)

| | | | |
|---|---|---|---|
| Arg33Stop | ex1del | ex5-8dup | Glu444Gln |
| Arg348fs | ex1dup | ex5-9dup | Glu79Stop |
| Arg366Trp | ex2-3del | ex5del | Gly179fs |
| Arg392fs | ex2-3dup | ex5dup | Gly328Glu |
| Arg42His | ex2-4del | ex6-7del | Gly359Asp |
| Arg42Pro | ex2-4dup | ex6-8dup | Gly429Glu |
| Asn428fs | ex2-4trip | ex6del | Gly430Asp |
| Asn52fs | ex2-5del | ex6dup | IVS1+1G>A |
| Asp280Asn | ex2del | ex7-8del | IVS11-3C>G |
| Asp460fs | ex2dup | ex7-9del | Leu283Pro |
| Asp53Stop | ex2trip | ex7del | Lys161Asn |
| c.-39G>T | ex3-4del | ex7dup | Lys211Asn |
| Cys212Gly | ex3-4dup | ex8-10del | Lys349fs |
| Cys212Tyr | ex3-5del | ex8-11del | Met192Leu |
| Cys238fs | ex3-6del | ex8-9del | Met192Val |
| Cys268Stop | ex3-7del | ex8del | Met1Leu |
| Cys289Gly | ex3-9del | ex8dup | partial ex4del |
| Cys323fs | ex3del | ex9del | Pro113fs / ex3 Δ40bp |
| Cys431Phe | ex3dup | ex9dup | Pro133del |
| ex10-12del | ex4-5del | Gln171Stop | Thr240Arg |
| ex10-12dup | ex4-6del | Gln311His | Thr240Met |
| Val56Glu | Val258Met | Trp453Stop | Thr351Pro |
| prom+ex1del | Va1324fs | Trp74fs | Thr415Asn |
| del=deletion; dup=duplication; fs=frameshift; ex=exon; IVS=intervening sequence; prom=promoter | | | |

Table 2: Exemplary pathogenic mutations in PINK1

| | | | |
|---|---|---|---|
| Tyr258Stop | IVS7+1G>A | ex6-8del | Asp297fs |
| Trp437Stop | Gly440Glu | ex4-8del | Arg492Stop |
| Thr313Met | Glu239Stop | ex3-8del | Arg464His |
| Stop582Leu | Gln456Stop | delPINK1 | Arg246Stop |
| Pro196fs | Gln129Stop | Cys92Phe | Ala168Pro |
| Lys520fs | Gln129fs | Cys549fs | 23bp del ex7 |
| Lys24fs | ex7del | Asp525fs | |
| del=deletion; fs=frameshift; ex=exon | | | |

The term "oxidative stress" refers to an increase in reactive oxygen species (ROS) and/or reactive nitrogen species (RNS) in a cell. In some embodiments, oxidative stress leads to protein aggregation and/or mitochondrial dysfunction. In some embodiments, mitochondrial dysfunction leads to oxidative stress.

The term "USP30," as used herein, refers to any native USP30 ("ubiquitin specific peptidase 30" or "ubiquitin specific protease 30") from any vertebrate source, including mammals such as primates (e.g. humans) and rodents

(e.g., mice and rats), unless otherwise indicated. The term encompasses "full-length," unprocessed USP30 as well as any form of USP30 that results from processing in the cell. The term also encompasses naturally occurring variants of USP30, e.g., splice variants or allelic variants. The amino acid sequence of an exemplary human USP30 is shown in SEQ ID NO: 26 (Table 4).

The term "Parkin" as used herein, refers to any native Parkin from any vertebrate source, including mammals such as primates (e.g. humans) and rodents (e.g., mice and rats), unless otherwise indicated. The term encompasses "full-length," unprocessed Parkin as well as any form of Parkin that results from processing in the cell. The term also encompasses naturally occurring variants of Parkin, e.g., splice variants or allelic variants. The amino acid sequence of an exemplary human Parkin is shown in SEQ ID NO: 29 (Table 4).

The term "PINK1" as used herein, refers to any native PINK1 (PTEN-induced putative kinase protein 1) from any vertebrate source, including mammals such as primates (e.g. humans) and rodents (e.g., mice and rats), unless otherwise indicated. The term encompasses "full-length," unprocessed PINK1 as well as any form of PINK1 that results from processing in the cell. The term also encompasses naturally occurring variants of PINK1, e.g., splice variants or allelic variants. The amino acid sequence of an exemplary human PINK1 is shown in SEQ ID NO: 30 (Table 4).

The term "Tom20" as used herein, refers to any native Tom20 from any vertebrate source, including mammals such as primates (e.g. humans) and rodents (e.g., mice and rats), unless otherwise indicated. The term encompasses "full-length," unprocessed Tom20 as well as any form of Tom20 that results from processing in the cell. The term also encompasses naturally occurring variants of Tom20, e.g., splice variants or allelic variants. The amino acid sequence of an exemplary human Tom20 is shown in SEQ ID NO: 27 (Table 4).

The terms "MIRO1" and "MIRO" as used herein, refer to any native MIRO1 (mitochondrial Rho GTPase 1) from any vertebrate source, including mammals such as primates (e.g. humans) and rodents (e.g., mice and rats), unless otherwise indicated. The term encompasses "full-length," unprocessed MIRO1 as well as any form of MIRO1 that results from processing in the cell. The term also encompasses naturally occurring variants of MIRO1, e.g., splice variants or allelic variants. The amino acid sequence of an exemplary human MIRO1 is shown in SEQ ID NO: 28 (Table 4).

The term "MUL1" as used herein, refers to any native MUL1 (mitochondrial ubiquitin ligase activator of NFκB) from any vertebrate source, including mammals such as primates (e.g. humans) and rodents (e.g., mice and rats), unless otherwise indicated. The term encompasses "full-length," unprocessed MUL1 as well as any form of MUL1 that results from processing in the cell. The term also encompasses naturally occurring variants of MUL1, e.g., splice variants or allelic variants. The amino acid sequence of an exemplary human MUL1 is shown in SEQ ID NO: 32 (Table 4).

The term "ASNS" as used herein, refers to any native ASNS (asparagine synthetase [glutamine hydrolyzing]) from any vertebrate source, including mammals such as primates (e.g. humans) and rodents (e.g., mice and rats), unless otherwise indicated. The term encompasses "full-length," unprocessed ASNS as well as any form of ASNS that results from processing in the cell. The term also encompasses naturally occurring variants of ASNS, e.g., splice variants or allelic variants. The amino acid sequence of an exemplary human ASNS is shown in SEQ ID NO: 33 (Table 4).

The term "FKBP8" as used herein, refers to any native FKBP8 (FK506 binding protein 8) from any vertebrate source, including mammals such as primates (e.g. humans) and rodents (e.g., mice and rats), unless otherwise indicated. The term encompasses "full-length," unprocessed ASNS as well as any form of FKBP8 that results from processing in the cell. The term also encompasses naturally occurring variants of FKBP8, e.g., splice variants or allelic variants. The amino acid sequence of an exemplary human FKBP8 is shown in SEQ ID NO: 34 (Table 4).

The term "TOM70" as used herein, refers to any native TOM70 (translocase of outer membrane 70 kDa subunit) from any vertebrate source, including mammals such as primates (e.g. humans) and rodents (e.g., mice and rats), unless otherwise indicated. The term encompasses "full-length," unprocessed TOM70 as well as any form of TOM70 that results from processing in the cell. The term also encompasses naturally occurring variants of TOM70, e.g., splice variants or allelic variants. The amino acid sequence of an exemplary human TOM70 is shown in SEQ ID NO: 35 (Table 4).

The term "MAT2B" as used herein, refers to any native MAT2B (methionine adenosyltransferase 2 subunit beta) from any vertebrate source, including mammals such as primates (e.g. humans) and rodents (e.g., mice and rats), unless otherwise indicated. The term encompasses "full-length," unprocessed MAT2B as well as any form of MAT2B that results from processing in the cell. The term also encompasses naturally occurring variants of MAT2B, e.g., splice variants or allelic variants. The amino acid sequence of an exemplary human MAT2B is shown in SEQ ID NO: 36 (Table 4).

The term "PRDX3" as used herein, refers to any native PRDX3 (peroxiredoxin III) from any vertebrate source, including mammals such as primates (e.g. humans) and rodents (e.g., mice and rats), unless otherwise indicated. The term encompasses "full-length," unprocessed PRDX3 as well as any form of PRDX3 that results from processing in the cell. The term also encompasses naturally occurring variants of PRDX3, e.g., splice variants or allelic variants. The amino acid sequence of an exemplary human PRDX3 is shown in SEQ ID NO: 37 (Table 4).

The term "IDE" as used herein, refers to any native IDE (insulin degrading enzyme) from any vertebrate source, including mammals such as primates (e.g. humans) and rodents (e.g., mice and rats), unless otherwise indicated. The term encompasses "full-length," unprocessed IDE as well as any form of IDE that results from processing in the cell. The term also encompasses naturally occurring variants of IDE, e.g., splice variants or allelic variants. The amino acid

sequence of an exemplary human IDE is shown in SEQ ID NO: 38 (Table 4).

The term "VDAC1" as used herein, refers to any native VDAC1 (voltage-dependent anion selective channel protein 1) from any vertebrate source, including mammals such as primates (e.g. humans) and rodents (e.g., mice and rats), unless otherwise indicated. The term encompasses "full-length," unprocessed VDAC1 as well as any form of VDAC1 that results from processing in the cell. The term also encompasses naturally occurring variants of VDAC1, e.g., splice variants or allelic variants. The amino acid sequence of an exemplary human VDAC1 is shown in SEQ ID NO: 39 (Table 4).

The term "VDAC2" as used herein, refers to any native VDAC2 (voltage-dependent anion selective channel protein 2) from any vertebrate source, including mammals such as primates (e.g. humans) and rodents (e.g., mice and rats), unless otherwise indicated. The term encompasses "full-length," unprocessed VDAC2 as well as any form of VDAC2 that results from processing in the cell. The term also encompasses naturally occurring variants of VDAC2, e.g., splice variants or allelic variants. The amino acid sequence of an exemplary human VDAC2 is shown in SEQ ID NO: 44 (Table 4).

The term "VDAC3" as used herein, refers to any native VDAC3 (voltage-dependent anion selective channel protein 3) from any vertebrate source, including mammals such as primates (e.g. humans) and rodents (e.g., mice and rats), unless otherwise indicated. The term encompasses "full-length," unprocessed VDAC3 as well as any form of VDAC3 that results from processing in the cell. The term also encompasses naturally occurring variants of VDAC3, e.g., splice variants or allelic variants. The amino acid sequence of an exemplary human VDAC3 is shown in SEQ ID NO: 45 (Table 4).

The term "IPO5" as used herein, refers to any native IPO5 (importin 5) from any vertebrate source, including mammals such as primates (e.g. humans) and rodents (e.g., mice and rats), unless otherwise indicated. The term encompasses "full-length," unprocessed IPO5 as well as any form of IPO5 that results from processing in the cell. The term also encompasses naturally occurring variants of IPO5, e.g., splice variants or allelic variants. The amino acid sequence of an exemplary human IPO5 is shown in SEQ ID NO: 40 (Table 4).

The term "PTH2" as used herein, refers to any native PTH2 (peptidyl-tRNA hydrolase 2, mitochondrial) from any vertebrate source, including mammals such as primates (e.g. humans) and rodents (e.g., mice and rats), unless otherwise indicated. The term encompasses "full-length," unprocessed PTH2 as well as any form of PTH2 that results from processing in the cell. The term also encompasses naturally occurring variants of PTH2, e.g., splice variants or allelic variants. The amino acid sequence of an exemplary human PTH2 is shown in SEQ ID NO: 41 (Table 4).

The term "PSD13" as used herein, refers to any native PSD13 (26S proteasome non-ATPase regulatory subunit 13) from any vertebrate source, including mammals such as primates (e.g. humans) and rodents (e.g., mice and rats), unless otherwise indicated. The term encompasses "full-length," unprocessed PSD13 as well as any form of PSD13 that results from processing in the cell. The term also encompasses naturally occurring variants of PSD13, e.g., splice variants or allelic variants. The amino acid sequence of an exemplary human PSD13 is shown in SEQ ID NO: 42 (Table 4).

The term "UBP13" as used herein, refers to any native UBP13 (ubiquitin carboxyl-terminal hydrolase 13) from any vertebrate source, including mammals such as primates (e.g. humans) and rodents (e.g., mice and rats), unless otherwise indicated. The term encompasses "full-length," unprocessed UBP13 as well as any form of UBP13 that results from processing in the cell. The term also encompasses naturally occurring variants of UBP13, e.g., splice variants or allelic variants. The amino acid sequence of an exemplary human UBP13 is shown in SEQ ID NO: 43 (Table 4).

The term "package insert" is used to refer to instructions customarily included in commercial packages of therapeutic products, that contain information about the indications, usage, dosage, administration, combination therapy, contraindications and/or warnings concerning the use of such therapeutic products.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be

compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

Administration "in combination with" one or more further therapeutic agents includes simultaneous (concurrent) and consecutive administration, in any order.

An "effective amount" of an agent, e.g., a pharmaceutical formulation, refers to an amount effective, at dosages and for periods of time necessary, to achieve the desired therapeutic or prophylactic result.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

## II. COMPOSITIONS AND METHODS

In various aspects, the invention is based, in part, on inhibitors of USP30 and methods of treating diseases and disorders comprising inhibiting USP30.

### A. Exemplary Inhibitors of USP30

The present invention is based in part on the discovery that inhibitors of USP30 activity and/or expression are effective for increasing and/or restoring mitochondrial ubiquitination and mitophagy. In some embodiments, inhibitors of USP30 are effective for treating neurodegenerative diseases, such as Parkinson's disease, as well as conditions that involve mitochondrial defects, such as those involving mitophagy defects, mutations in mitochondrial DNA, mitochondrial oxidative stress, and/or lysosomal storage defects.

Inhibitors of USP30 include inhibitors of USP30 activity and inhibitors of USP30 expression. Nonlimiting exemplary such inhibitors include antisense oligonucleotides, short interfering RNAs (siRNAs), antibodies, peptides, peptibodies, aptamers, and small molecules. In some embodiments, antisense oligonucleotides or short interfering RNAs (siRNAs) may be used to inhibit USP30 expression. In some embodiments, antibodies, peptides, peptibodies, aptamers, and small molecules may be used to inhibit USP30 activity. Some nonlimiting examples of inhibitors of USP30 are described herein. Further inhibitors can be identified using standard methods in the art, including those discussed herein.

Antisense oligonucleotides

In some embodiments, antisense oligonucleotides that hybridize to USP30 mRNA and/or USP30 pre-mRNA are provided. A nonlimiting exemplary human mRNA sequence encoding USP30 is shown in SEQ ID NO: 30 (Table 4). In some embodiments, an antisense oligonucleotide hybridizes to a region of USP30 mRNA and/or USP30 pre-mRNA and directs its degradation through RNase H, which cleaves double-stranded RNA/DNA hybrids. By mediating cleavage of USP30 mRNA and/or USP30 pre-mRNA, an antisense oligonucleotide may reduce the amount of USP30 protein in a cell (i.e., may inhibit expression of USP30). In some embodiments, an antisense oligonucleotide does not mediate degradation through RNase H, but rather "blocks" translation of the mRNA, e.g., through interference with translational machinery binding or processivity, or "blocks" proper splicing of the pre-mRNA, e.g., through interference with the splicing machinery and/or accessibility of a splice site. In some embodiments, an antisense oligonucleotide may mediate degradation of an mRNA nad/or pre-mRNA through a mechanism other than RNase H Any inhibitory mechanism of an antisense oligonucleotide is contemplated herein.

In some embodiments, an antisense oligonucleotide is 10 to 500 nucleotides long, or 10 to 400 nucleotides long, or 10 to 300 nucleotides long, or 10 to 200 nucleotides long, or 10 to 100 nucleotides long, or 15 to 100 nucleotides long, or 10 to 50 nucleotides long, or 15 to 50 nucleotides long. In various embodiments, an antisense oligonucleotide hybridizes to a region of the USP30 mRNA and/or pre-mRNA comprising at least 10, at least 15, at least 20, at least 25, at least 30, at least 40, at least 50, at least 60, at least 70, at least 80, at least 90, or at least 100 nucleotides. Further, in various embodiments, an antisense oligonucleotide need not be 100% complementary to a region USP30 mRNA and/or a region of USP30 pre-mRNA, but may have 1 or more mismatches. Thus, in some embodiments, an antisense oligonucleotide is at least 80% complementary, at least 85% complementary, at least 90% complementary, at least 95% complementary, or 100% complementary to a region of USP30 mRNA and/or a region of USP30 pre-mRNA. In some embodiments, the region of USP30 mRNA or the region of USP pre-mRNA is at least at least 10, at least 15, at least 20, at least 25, at least 30, at least 40, at least 50, at least 60, at least 70, at least 80, at least 90, or at least 100 nucleotides long.

Antisense oligonucleotides may comprise modifications to one or more of the internucleoside linkages, sugar moieties, and/or nucleobases. Further, the sequence of nucleotides may be interrupted by non-nucleotide components, and/or non-nucleotide components may be attached at one or both ends of the oligonucleotide.

Nonlimiting exemplary nucleotide modifications include sugar modifications, in which any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Oligonucleotides can also contain analogous forms of ribose or deoxyribose sugars that are generally known in the art, including, for example, 2'-O-methyl-2'-O-allyl, 2'-fluoro- or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by modified internucleoside linkages. These modified internucleoside linkages include, but are not limited to, embodiments wherein phosphate is replaced by P(O)S ("thioate"), P(S)S ("dithioate"), (O)$NR_2$ ("amidate"), P(O)R, P(O)OR', CO or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all oligonucleotides referred to herein, including antisense oligonucleotides and siRNA.

In some embodiments, one or more internucleoside linkages in an antisense oligonucleotide are phosphorothioates. In some embodiments, one or more sugar moieties in an antisense oligonucleotide comprise 2' modifications, such as 2'-O-alkyl (such as 2'-OMe) and 2'-fluoro; or are bicyclic sugar moieties (such as LNA). Nonlimiting exemplary nucleobase modifications include 5-methylcytosine. An antisense oligonucleotide may comprise more than one type of modification within a single oligonucleotide. That is, as a nonlimiting example, an antisense oligonucleotide may comprise 2'-O alkyl modifications, bicyclic nucleotides, and phosphorothioate linkages in the same oligonucleotide. In some embodiments, an antisense oligonucleotide is a "gapmer." Gapmers comprise a central region of deoxyribonucleotides for mediating RNase H cleavage, and 5' and 3' "wings" comprising modified sugar moieties that increase the stability of the duplex.

Antisense oligonucleotide design and mechanisms are described, *e.g.,* in van Roon-Mom et al., Methods Mol. Biol., 867: 79-96 (20120); Prakash, Chem. Biodivers., 8: 1616-1641 (2011); Yamamoto et al., Future Med. Chem., 3: 339-365 (2011); Chan et al., Clin. Exper. Pharmacol. Physiol., 33: 533-540 (2006); Kurreck et al., Nucl. Acids Res., 30: 1911-1918 (2002); Kurreck, Eur. J. Biochem., 270: 1628-1644 (2003); Geary, Expert Opin. Drug Metab. Toxicol., 5: 381-391 (2009); "Designing Antisense Oligonucleotides," available online from Integrated DNA Technologies (2011).

Short interfering RNAs (siRNAs)

In some embodiments, the expression of USP30 is inhibited with a short interfering RNA (siRNA). As used herein, siRNAs are synonymous with double-stranded RNA (dsRNA) and include double-stranded RNA oligomers with or without hairpin structures at each end (also referred to as small hairpin RNA, or shRNA). Short interfering RNAs are also known as small interfering RNAs, silencing RNAs, short inhibitory RNA, and/or small inhibitory RNAs, and these terms are considered to be equivalent herein.

The term "short-interfering RNA (siRNA)" refers to small double-stranded RNAs that interfere with gene expression. siRNAs are mediators of RNA interference, the process by which double-stranded RNA silences homologous genes. In some embodiments, siRNAs are comprised of two single-stranded RNAs of about 15-25 nucleotides in length that form a duplex, which may include single-stranded overhang(s). In some embodiments, siRNAs are comprised of a single RNA that forms a hairpin structure that includes a double-stranded portion that may be 15-25 nucleotides in length and may include a single-stranded overhang. Such hairpin siRNAs may be referred to as a short hairpin RNA (shRNA). Processing of the double-stranded RNA by an enzymatic complex, for example, polymerases, may result in cleavage of the double-stranded RNA to produce siRNAs. The antisense strand of the siRNA is used by an RNA interference (RNAi) silencing complex to guide mRNA cleavage, thereby promoting mRNA degradation. To silence a specific gene using siRNAs, for example, in a mammalian cell, a base pairing region is selected to avoid chance complementarity to an unrelated mRNA. RNAi silencing complexes have been identified in the art, such as, for example, by Fire et al., Nature 391:806-811, 1998, and McManus et al., Nat. Rev. Genet. 3(10):737-747, 2002.

In some embodiments, small interfering RNAs comprise at least about 10 to about 200 nucleotides, including at least about 16 nucleotides, at least about 17 nucleotides, at least about 18 nucleotides, at least about 19 nucleotides, at least about 20 nucleotides, at least about 21 nucleotides, at least about 22 nucleotides, at least about 23 nucleotides, at least about 24 nucleotides, at least about 25 nucleotides, at least about 26 nucleotides, at least about 27 nucleotides, at least about 28 nucleotides, at least about 29 nucleotides, at least about 30 nucleotides, at least about 35 nucleotides, at least about 40 nucleotides, at least about 45 nucleotides, at least about 50 nucleotides, at least about 55 nucleotides, at least about 60 nucleotides, at least about 65 nucleotides, at least about 70 nucleotides, at least about 75 nucleotides, at least about 80 nucleotides, at least about 85 nucleotides, at least about 90 nucleotides, at least about 95 nucleotides, at least about 100 nucleotides, at least about 110 nucleotides, at least about 120 nucleotides, at least about 130 nucleotides, at least about 140 nucleotides, at least about 150 nucleotides, or greater than 150 nucleotides. In some embodiments, an siRNA is 10 to 200 nucleotides long, or 10 to 100 nucleotides long, or 15 to 100 nucleotides long, or 10 to 60 nucleotides long, or 15 to 60 nucleotides long, or 10 to 50 nucleotides long, or 15 to 50 nucleotides long, or 10 to 30 nucleotides long, or 15 to 30 nucleotides long. In certain embodiments, the siRNA comprises an oligonucleotide from about 21 to about 25 nucleotides in length. In some embodiments, the siRNA molecule is a heteroduplex of RNA and DNA.

As with antisense oligonucleotides, siRNAs can include modifications to the sugar, internucleoside linkages, and/or nucleobases. Nonlimiting exemplary modifications suitable for use in siRNAs are described herein and also, e.g., in Peacock et al., J. Org. Chem., 76: 7295-7300 (2011); Bramsen et al., Methods Mol. Biol., 721: 77-103 (2011); Pasternak et al., Org. Biomol. Chem., 9: 3591-3597 (2011); Gaglione et al., Mini Rev. Med. Chem., 10: 578-595 (2010); Chernolovskaya et al., Curr. Opin. Mol. Ther., 12: 158-167 (2010).

A process for inhibiting expression of USP30 in a cell comprises introduction of an siRNA with partial or fully double-stranded character into the cell. In some embodiments, an siRNA comprises a nucleotide sequence that is at least 75%, at least 80%, at least 85%, at least 90%, at least 95%, or 100% identical to a nucleotide sequence found in the USP30 gene coding region or pre-mRNA.

In some embodiments, an siRNA specific to the USP30 gene is synthesized and introduced directly into a subject. In other embodiments, the siRNA can be formulated as part of a targeted delivery system, such as a target specific liposome, which specifically recognizes and delivers the siRNA to an appropriate tissue or cell type. Upon administration of the targeted siRNA to a subject, the siRNA is delivered to the appropriate cell type, thereby increasing the concentration siRNA within the cell type. Depending on the dose of siRNA delivered, this process can provide partial or complete loss of USP30 protein expression.

In other embodiments, an appropriate cell or tissue is provided with an expression construct that comprises a nucleic acid encoding one or both strands of an siRNA that is specific to the USP30 gene. In these embodiments, the nucleic acid that encodes one or both strands of the siRNA can be placed under the control of either a constitutive or a regulatable promoter. In some embodiments, the nucleic acid encodes an siRNA that forms a hairpin structure, e.g., a

shRNA.

Various carriers and drug-delivery systems for siRNAs are described, e.g., in Seth et al., Ther. Deliv., 3: 245-261 (2012); Kanasty et al., Mol. Ther., 20: 513-524 (2012); Methods Enzymol., 502: 91-122 (2012); Vader et al., Curr. Top. Med. Chem., 12: 108-119 (2012); Naeye et al., Curr. Top. Med. Chem., 12: 89-96 (2012); Foged, Curr. Top. Med. Chem., 12: 97-107 (2012); Chaturvedi et al., Expert Opin. Drug Deliv., 8: 1455-1468 (2011); Gao et al., Int. J. Nanomed., 6: 1017-1025 (2011); Shegokar et al., Pharmazie., 66: 313-318 (2011); Kumari et al., Expert Opin. Drug Deliv., 11: 1327-1339 (2011).

Antibodies

In some embodiments, an inhibitor of USP30 is an antibody. The term "antibody" is used herein in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. The term "antibody" as used herein refers to a molecule comprising at least complementarity-determining region (CDR) 1, CDR2, and CDR3 of a heavy chain and at least CDR1, CDR2, and CDR3 of a light chain, wherein the molecule is capable of binding to antigen. The term antibody includes, but is not limited to, fragments that are capable of binding antigen, such as Fv, single-chain Fv (scFv), Fab, Fab', and $(Fab')_2$. The term antibody also includes, but is not limited to, chimeric antibodies, humanized antibodies, and antibodies of various species such as mouse, human, cynomolgus monkey, etc.

In some embodiments, an antibody comprises a heavy chain variable region and a light chain variable region, one or both of which may or may not comprise a respective constant region. A heavy chain variable region comprises heavy chain CDR1, framework (FR) 2, CDR2, FR3, and CDR3. In some embodiments, a heavy chain variable region also comprises at least a portion of an FR1, which is N-terminal to CDR1, and/or at least a portion of an FR4, which is C-terminal to CDR3. Similarly, a light chain variable region comprises light chain CDR1, framework (FR) 2, CDR2, FR3, and CDR3. In some embodiments, a light chain variable region also comprises an FR1 and/or an FR4.

Nonlimiting exemplary heavy chain constant regions include $\gamma$, $\delta$, and $\alpha$. Nonlimiting exemplary heavy chain constant regions also include $\varepsilon$ and $\mu$. Each heavy constant region corresponds to an antibody isotype. For example, an antibody comprising a $\gamma$ constant region is an IgG antibody, an antibody comprising a $\delta$ constant region is an IgD antibody, and an antibody comprising an $\alpha$ constant region is an IgA antibody. Certain isotypes can be further subdivided into subclasses. For example, IgG antibodies include, but are not limited to, IgG1 (comprising a $\gamma_1$ constant region), IgG2 (comprising a $\gamma_2$ constant region), IgG3 (comprising a $\gamma_3$ constant region), and IgG4 (comprising a $\gamma_4$ constant region) antibodies. Nonlimiting exemplary light chain constant regions include $\lambda$ and $\kappa$.

In some embodiments, an antibody is a chimeric antibody, which comprises at least one variable region from a first species (such as mouse, rat, cynomolgus monkey, etc.) and at least one constant region from a second species (such as human, cynomolgus monkey, chicken, etc.). The human constant region of a chimeric antibody need not be of the same isotype as the non-human constant region, if any, it replaces. Chimeric antibodies are discussed, e.g., in U.S. Patent No. 4,816,567; and Morrison et al. Proc. Natl. Acad. Sci. USA 81: 6851-55 (1984).

In some embodiments, an antibody is a humanized antibody, in which at least one amino acid in a framework region of a non-human variable region (such as mouse, rat, cynomolgus monkey, chicken, etc.) has been replaced with the corresponding amino acid from a human variable region. In some embodiments, a humanized antibody comprises at least one human constant region or fragment thereof. In some embodiments, a humanized antibody is an Fab, an scFv, a $(Fab')_2$, etc. Exemplary humanized antibodies include CDR-grafted antibodies, in which the complementarity determining regions (CDRs) of a first (non-human) species have been grafted onto the framework regions (FRs) of a second (human) species. Humanized antibodies are useful as therapeutic molecules because humanized antibodies reduce or eliminate the human immune response to non-human antibodies (such as the human anti-mouse antibody (HAMA) response), which can result in an immune response to an antibody therapeutic, and decreased effectiveness of the therapeutic. An antibody may be humanized by any method. Nonlimiting exemplary methods of humanization include methods described, e.g., in U.S. Patent Nos. 5,530,101; 5,585,089; 5,693,761; 5,693,762; 6,180,370; Jones et al., Nature 321: 522-525 (1986); Riechmann et al., Nature 332: 323-27 (1988); Verhoeyen et al., Science 239: 1534-36 (1988); and U.S. Publication No. US 2009/0136500.

In some embodiments, an antibody is a human antibody, such as an antibody produced in a non-human animal that comprises human immunoglobulin genes, such as XenoMouse®, and antibodies selected using *in vitro* methods, such as phage display, wherein the antibody repertoire is based on a human immunoglobulin sequences. Transgenic mice that comprise human immunoglobulin loci and their use in making human antibodies are described, e.g., in Jakobovits et al., Proc. Natl. Acad. Sci. USA 90: 2551-55 (1993); Jakobovits et al., Nature 362: 255-8 (1993); Lonberg et al., Nature 368: 856-9 (1994); and U.S. Patent Nos. 5,545,807; 6,713,610; 6,673,986; 6,162,963; 5,545,807; 6,300,129; 6,255,458; 5,877,397; 5,874,299; and 5,545,806. Methods of making human antibodies using phage display libraries are described, e.g., in Hoogenboom et al., J. Mol. Biol. 227: 381-8 (1992); Marks et al., J. Mol. Biol. 222: 581-97 (1991);

and PCT Publication No. WO 99/10494.

The choice of heavy chain constant region can determine whether or not an antibody will have effector function *in vivo.* Such effector function, in some embodiments, includes antibody-dependent cell-mediated cytotoxicity (ADCC) and/or complement-dependent cytotoxicity (CDC), and can result in killing of the cell to which the antibody is bound. Typically, antibodies comprising human IgG1 or IgG3 heavy chains have effector function. In some embodiments, effector function is not desirable. In some such embodiments, a human IgG4 or IgG2 heavy chain constant region may be selected or engineered.

Peptides

In some embodiments, an inhibitor of USP30 is a peptide. A peptide is a sequence of amino acids of made up of a single chain of D- or L-amino acids or a mixture of D- and L-amino acids joined by peptide bonds. The amino acid subunits of the peptide may be naturally-occurring amino acids or may be non-naturally occurring amino acids. Many non-naturally occurring amino acids are known in the art and are available commercially. Further, the peptide bonds joining the amino acid subunits may be modified. *See, e.g.,* Sigma-Aldrich; Gentilucci et al., Curr. Pharm. Des. 16: 3185-3203 (2010); US 2008/0318838. Generally, peptides contain at least two amino acid residues and are less than about 50 amino acids in length. In various embodiments, peptide inhibitors may comprise or consist of between 3 and 50, between 5 and 50, between 10 and 50, between 10 and 40, between 10 and 35, between 10 and 30, or between 10 and 25 amino acids. In various embodiments, peptide inhibitors may comprise 10, 11, 12, 13, 14, 15, 16, 17, 18, 19, 20, 21, 22, 23, 24, or 25 amino acids. In various embodiments, peptide inhibitors may consist of 10, 11, 12, 13, 14, 15, 16, 17, 18, 19, 20, 21, 22, 23, 24, or 25 amino acids.

Methods of developing peptides that specifically bind a target molecule are known in the art, including phage display methods. *See, e.g.,* US. Patent No. 5,010,175; WO 1996/023899; WO 1998/015833; Bratkovic, Cell. Mol. Life Sci., 67: 749-767 (2010); Pande et al., Biotech. Adv. 28: 849-858 (2010). In some embodiments, following selection of a peptide, the peptide may be modified, e.g., by incorporating non-natural amino acids and/or peptide bonds. A nonlimiting exemplary method of selecting a peptide inhibitor of USP30 is described herein.

Amino acids that are important for peptide inhibition may be determined, in some embodiments, by alanine scanning mutagenesis. Each residue is replaced in turn with a single amino acid, typically alanine, and the effect on USP30 inhibition is assessed. See, e.g., U.S. Patent Nos. 5,580,723 and 5,834,250. Truncation analyses may also be used to determine not only the importance of the amino acids at the ends of a peptide, but also the importance of the length of the peptide, on inhibitory activity. In some instances, truncation analysis may reveal a shorter peptide that binds more tightly than the parent peptide. The results of various mutational analyses, such as alanine scanning mutagenesis and truncation analyses, may be used to inform further modifications of an inhibitor peptide.

Nonlimiting exemplary peptide inhibitors are described herein, e.g., in Example 10 and Figure 15. One skilled in the art will appreciate that, in some embodiments, the peptide sequences described herein may be modified in order to generate further peptide inhibitors with desirable properties, such as improved specificity for USP30, stronger binding to USP30, improved solubility, and/or improved cell membrane permeability. In some embodiments, a peptide inhibitor of USP30 comprises an amino acid sequence that is at least 80%, at least 85%, at least 90%, at least 95%, or 100% identical to a sequence selected from SEQ ID NOs: 1 to 22.

In some embodiments, a peptide inhibitor comprises the amino acid sequence:

$X_1X_2CX_3X_4X_5X_6X_7X_8X_9X_{10}X_{11}CX_{12}$ (SEQ ID NO: 48)

wherein:

$X_1$ is selected from L, M, A, S, and V;
$X_2$ is selected from Y, D, E, I, L, N, and S;
$X_3$ is selected from F, I, and Y;
$X_4$ is selected from F, I, and Y;
$X_5$ is selected from D and E;
$X_6$ is selected from L, M, V, and P;
$X_7$ is selected from S, N, D, A, and T;
$X_8$ is selected from Y, D, F, N, and W;
$X_9$ is selected from G, D, and E;
$X_{10}$ is selected from Y and F;
$X_{11}$ is selected from L, V, M, Q, and W; and
$X_{12}$ is selected from F, L, C, V, and Y;

In some embodiments, the peptide inhibits USP30 with an IC50 of less than 10 $\mu$M. In some embodiments, $X_1$ is selected from L and M. In some embodiments, $X_3$ is selected from Y and D. In some embodiments, $X_3$ is F. In some embodiments, $X_4$ is selected from Y and F. In some embodiments, $X_4$ is Y. In some embodiments, $X_5$ is D. In some embodiments, $X_6$ is selected from L and M. In some embodiments, $X_7$ is selected from S, N, and D. In some embodiments, $X_8$ is Y. In

some embodiments, $X_9$ is G. In some embodiments, $X_{10}$ is Y. In some embodiments, $X_{11}$ is L. In some embodiments, $X_{12}$ is selected from F and L. In some embodiments, $X_{12}$ is F.

In some embodiments, , a peptide inhibitor comprises the amino acid sequence:

$X_AX_1X_2CX_3X_4X_5X_6X_7X_8X_9X_{10}X_{11}CX_{12}X_B$ (SEQ ID NO: 49)

wherein $X_1$ to $X_{12}$ are as defined above, and $X_A$ and $X_B$ are each independently any amino acid. In some embodiments, $X_A$ is selected from S, A, T, E Q, D, and R. In some embodiments, $X_B$ is selected from D, Y, E, H, S, and I.

Peptibodies

In some embodiments, an inhibitor of USP30 is a peptibody. A peptibody is peptide sequence linked to vehicle. In some embodiments, the vehicle portion of the peptibody reduces degradation and/or increases half-life, reduces toxicity, reduces immunogenicity, and/or increases biological activity of the peptide. In some embodiments, the vehicle portion of the peptibody is an antibody Fc domain. Other vehicles include linear polymers (e.g., polyethylene glycol (PEG), polylysine, dextran, etc.); branched-chain polymers (see, e.g., U.S. Pat. Nos. 4,289,872 and U.S. Pat. No. 5,229,490; WO 1993/0021259); a lipid; a cholesterol group (such as a steroid); a carbohydrate or oligosaccharide; or any natural or synthetic protein, polypeptide, or peptide vehicle. The peptide portion of the peptibody typically binds to the target, e.g., USP30. In some emodiments, the peptide portion of the peptibody is a peptide described herein.

In some embodiments, peptibodies retain certain desirable characteristics of antibodies, such as a long lifetime in plasma and increased affinity for binding partners (for example, due to the dimerization of Fc domains). The production of peptibodies is generally described, e.g., in WO 2000/0024782 and U.S. Pat. No. 6,660,843.

Aptamers

In some embodiments, an inhibitor of USP30 is an aptamer. The term "aptamer" as used herein refers to a nucleic acid molecule that specifically binds to a target molecule, such as USP30. Aptamers can be selected to be highly specific, relatively small in size, and/or non-immunogenic. *See, e.g.,* Ni, et al., Curr. Med. Chem. 18: 4206 (2011). In some embodiments, a aptamer is a small RNA, DNA, or mixed RNA/DNA molecule that forms a secondary and/or tertiary structure capable of specifically binding and inhibiting USP30.

In some embodiments, an aptamer includes one or more modified nucleosides (e.g., nucleosides with modified sugars, modified nucleobases, and/or modified internucleoside linkages), for example, that increase stability *in vivo,* increase target affinity, increase solubility, increase serum half-life, increase resistance to degradation, and/or increase membrane permeability, etc. In some embodiments, aptamers comprise one or more modified or inverted nucleotides at their termini to prevent terminal degradation, e.g., by an exonuclease.

The generation and therapeutic use of aptamers are well established in the art. *See, e.g.,* U.S. Pat. No. 5,475,096. In some embodiments, aptamers are produced by systematic evolution of ligands by exponential enrichment (SELEX), e.g., as described in Ellington et al., Nature 346: 818 (1990); and Tuerk et al., Science 249: 505 (1990). In some embodiments, aptamers are produced by an AptaBid method, *e.g.,* as described in Berezovski et al., J. Am. Chem. Soc. 130: 913 (2008). Slow off-rate aptamers and methods of selecting such aptamers are described, e.g., in Brody et al., Expert Rev. Mol. Diagn., 10: 1013-22 (2010); and U.S. Patent No. 7,964,356.

Small Molecules

In some embodiments, small molecule inhibitors of USP30 are provided. In some embodiments, a small molecule inhibitor of USP30 binds to USP30 and inhibits USP30 enzymatic activity (e.g., peptidase activity) and/or interferes with USP30 target binding and/or alters USP30 conformation such that the efficiency of enzymatic activity or target binding is reduced.

A "small molecule" is defined herein to have a molecular weight below about 1000 Daltons, for example, below about 900 Daltons, below about 800 Daltons, below about 700 Daltons, below about 600 Daltons, or below about 500 Daltons. Small molecules may be organic or inorganic, and may be isolated from, for example, compound libraries or natural sources, or may be obtained by derivatization of known compounds.

In some embodiments, a small molecule inhibitor of USP30 is identified by screening a library of small molecules. The generation and screening of small molecule libraries is well known in the art. *See, e.g.,* Thompson et al., Chem. Rev. 96: 555-600 (1996); and the National Institutes of Health Molecular Libraries Program. A combinatorial chemical library, for example, may be formed by mixing a set of chemical building blocks in various combinations, and may result in millions of chemical compounds. For example, the systematic, combinatorial mixing of 100 interchangeable chemical building blocks theoretically results in the synthesis of 100 million tetrameric compounds or 10 billion pentameric compounds. *See, e.g.,* Gallop et al. 1994, J. Med. Chem. 37: 1233-1250). Various other types of small molecule libraries may also be designed and used, such as, for example, natural product libraries. Small molecule libraries can be obtained

from various commercial vendors. *See, e.g.,* ChemBridge, Enzo Life Sciences, Sigma-Aldrich, AMRI Global, etc.

To identify a small molecule inhibitor of USP30, in some embodiments, a small molecule library may be screened using an assay described herein. In some embodiments, the characteristics of each small molecule that inhibits USP30 are considered in order to identify features common to the small molecule inhibitors, which may be used to inform further modifications of the small molecules.

In some embodiments, one or more small molecule inhibitors of USP30 identified, for example, in an initial library screen, may be used to generate a subsequent library comprising modifications of the initial small molecule inhibitors. Using this method, subsequent iterations of candidate compounds may be developed that possess greater specificity for USP30 (versus other DUBs), and/or greater binding affinity for USP30, and/or other desirable properties, such as low toxicity, greater solubility, greater cell permeability, etc.

Various small molecule inhibitors of deubiquitylating enzymes are known in the art, some of which are shown in Table 3.

Table 3: Inhibitors of ubiquitin specific proteases

| Name | Structure | Target | Reference |
|---|---|---|---|
| HBX 41,108 | 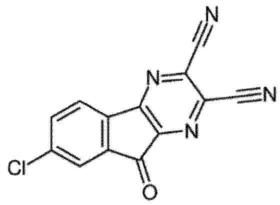 | USP7 | Colland et al., Mol. Cancer Therap., 8: 2286 (2009) |
| HBX 90,397 | 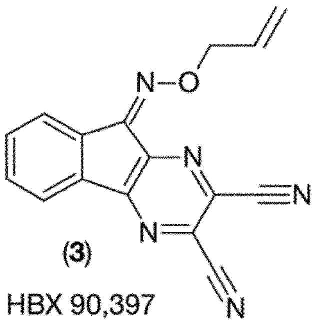 (3) HBX 90,397 | USP8 | WO 2007/017758; |
| IU1 | 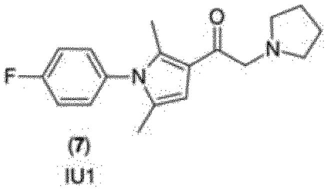 (7) IU1 | USP14 | Lee et al., Nature, 467: 179-184 (2010) |
| PR619 | 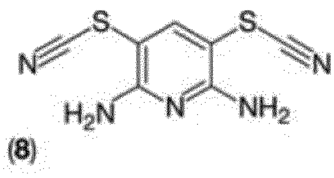 (8) | Broad specificity DUB inhibitor | Tian et al., Assay Drug Develop. Technol., 9: 165-173 (2011) |
| Isatin O-acyl oxime | 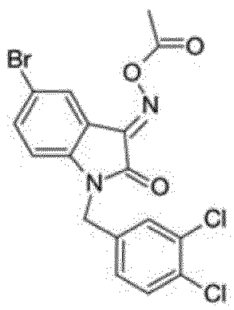 | UCH-L1 | Liu et al., Chemistry & Biology, 10: 837-846 (2003) |

(continued)

| Name | Structure | Target | Reference |
|---|---|---|---|
| Isatin derivative | 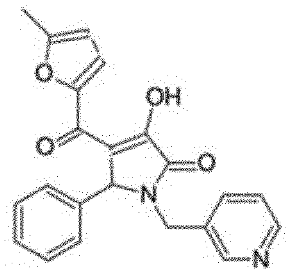 | UCH-L3 | Liu et al., Neurobiol. Disease, 41: 318-328 (2010); Koharudin et al., PNAS, 107: 6835-6840 (2010) |
| $PGA_1$ | 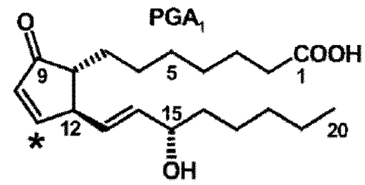 | Ubiquitin isopeptidase | Mullally et al., J. Biol. Chem., 276: 30366-73 (2001) |
| $PGA_2$ | 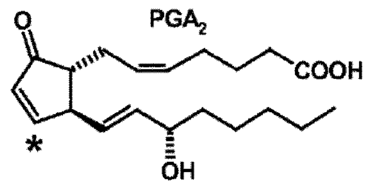 | Ubiquitin isopeptidase | Mullally et al., J. Biol. Chem., 276: 30366-73 (2001) |
| $\Delta$12-$PGJ_2$ | 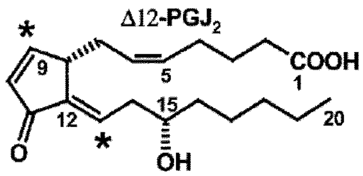 | Ubiquitin isopeptidase | Mullally et al., J. Biol. Chem., 276: 30366-73 (2001) |
| Dibenzylideneacetone (DBA) | 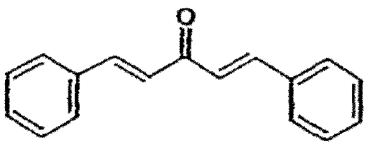 | Ubiquitin isopeptidase | WO 2004/009023 |
| Curcumin | 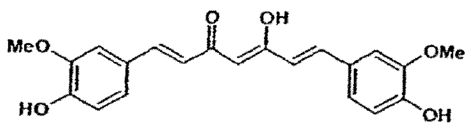 | Ubiquitin isopeptidase | WO 2004/009023 |
| Shikoccin (NSC-302979) | 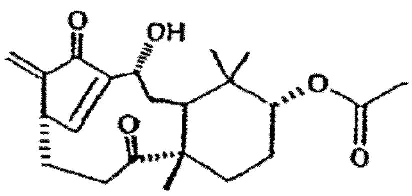 | Ubiquitin isopeptidase | WO 2004/009023 |

The inhibitors shown in Table 3 and the references cited therein, as well as additional inhibitors known in the art, can form the basis for developing additional deubiquitylation enzyme inhibitors, including specific inhibitors of USP30. *See also* WO 2007/009715. One skilled in the art can, for example, make modifications to any of the above structures to form a library of putative deubiquitylation enzyme inhibitors and screen for modified compounds with specificity for USP30 using the assays described herein.

**B.** Assays

Various assays may be used to identify and test inhibitors of USP30. For inhibitors that reduce expression of USP30 protein, any assay that detects protein levels may be suitable for measuring inhibition. As an example, protein levels can be detected by various immunoassays using antibodies that bind USP30, such as ELISA, Western blotting, immunohistochemistry, etc. If an inhibitor affects the subcellular localization of USP30, changes in subcellular localization may be detected, e.g., by immunohistochemistry, or by fractionating cellular components and detecting levels of USP30 in the various fractions using one or more antibodies. For inhibitors that reduce levels of USP30 mRNA, amplification-based assays, such as reverse transcriptase PCR (RT-PCR) may be used to detect changes in mRNA levels.

For inhibitors that affect USP30 enzymatic activity, a nonlimiting exemplary assay is as follows: USP30 is contacted with the inhibitor or candidate inhibitor in the presence of a USP30 substrate. Nonlimiting exemplary USP30 substrates include a Ub-β-galactosidase fusion protein (see, e.g., Quesada et al., Biochem. Biophys. Res. Commun 314:54-62 (2004)), Ub4 chains (e.g., Lys-48- and Lys-63-linked Ub), the linear product of UBIQ gene translation; the post-translationally formed branched peptide bonds in mono- or multi-ubiquitylated conjugates; ubiquitylated remnants resulting from proteasome-mediated degradation, and other small amide or ester adducts. USP30 activity (e.g., processing of Ub substrates) is measured in the presence of USP30 and the inhibitor or candidate inhibitor. This activity is compared with the processing of Ub substrates in the presence USP30 without the inhibitor or candidate inhibitor. If the inhibitor or candidate inhibitor inhibits the activity of USP30, the amount of UB substrate processing will decrease compared to the amount of UB substrate processing in the presence of USP30 without the inhibitor or candidate inhibitor.

A further nonlimiting exemplary assay to determine inhibition and/or specificity is described in Example 10. Briefly, a range of concentrations of inhibitor are mixed with ubiquitin-AMC and USP30 (the inhibitor may be mixed with the substrate, and then USP30 added to start the reaction). If specificity is to be determined, similar reactions may be set up with one or more additional DUBs or ubiquitin C-terminal hydrolases (UCHs) in place of USP30. Immediately after addition of the enzyme, fluorescence is monitored (with excitation at 340 nm and emission at 465 nm). The initial rate of enzymatic activity may be calculated as described in Example 10.

**C. Pharmaceutical Formulations**

Pharmaceutical formulations of an inhibitor of USP30 as described herein are prepared by mixing such inhibitor having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

The formulation herein may also contain more than one active ingredient as necessary for the particular indication being treated, preferably those with complementary activities that do not adversely affect each other.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the inhibitor, which matrices are in the form of shaped articles, e.g. films, or microcapsules.

The formulations to be used for *in vivo* administration are generally sterile. Sterility may be readily accomplished, e.g., by filtration through sterile filtration membranes.

### D. Therapeutic Methods and Compositions

Any of the inhibitors of USP30 provided herein may be used in methods, e.g., therapeutic methods. In some embodiments, a method of increasing mitophagy in a cell is provided, the method comprising contacting the cell with an inhibitor of USP30 under conditions allowing inhibition of USP30 in the cell. In some embodiments, a method of increasing mitochondrial ubiquitination in a cell is provided, the method comprising contacting the cell with an inhibitor of USP30 under conditions allowing inhibition of USP30 in the cell. Increased mitophagy may be determined, e.g., using immunofluorescence as described in Example 6. Increased ubiquitination may be determined, e.g., by immunoaffinity enrichment of ubiquitinated peptides after trypsin digestion, followed by mass spectrometry as described in Example 5. In some embodiments, an increase in mitochondrial ubiquitination may be determined by comparing the ubiquitination of a mitochondrial proteins a cell or population of cells contacted with an inhibitor of USP30 with the ubiquitination of mitochondrial proteins in a matched cell or population of cells not contacted with the inhibitor.

In some embodiments, increased mitophagy means a reduction in the average number of mitochondria per cell of at least 10%, at least 15%, at least 20%, at least 25%, at least 30%, at least 35%, at least 40%, at least 45%, at least 50%, at least 55%, at least 60%, at least 65%, at least 70%, or at least 75%, in a population of cells contacted with an inhibitor of USP30, as compared to matched population of cells not contacted with the inhibitor. In some embodiments, increased mitochondrial ubiquitination means an increase in overall ubiquitination of mitochondrial proteins in a cell or population of cells contacted with an inhibitor of USP30 of at least 10%, at least 20%, at least 30%, at least 40%, at least 50%, at least 60%, at least 70%, at least 80%, at least 90%, at least 100% (i.e., 2-fold), at least 150%, or at least 200% (i.e., 3-fold) as compared to a matched cell or population of cells not contacted with the inhibitor.

In some embodiments, a method of increasing ubiquitination of at least one protein selected from Tom20, MIRO, MUL1, ASNS, FKBP8, TOM70, MAT2B, PRDX3, IDE, VDAC, IPO5, PSD13, UBP13, and PTH2 in a cell is provided, the method comprising contacting the cell with an inhibitor of USP30 under conditions allowing inhibition of USP30 in the cell. In some such embodiments, ubiquitination increases at at least one, at least two, or three amino acids selected from K56, K61, and K68 of Tom 20; and/or ubiquitination increases at at least one, at least two, at least three, at least four, at least five, at least six, at least seven, or eight amino acids selected from K153, K187, K330, K427, K512, K535, K567, and K572 of MIRO. In some such embodiments, ubiquitination increases at at least one, at least two, or three amino acids selected from K56, K61, and K68 of Tom 20; and/or ubiquitination increases at at least one, at least two, at least three, at least four, at least five, at least six, at least seven, or eight amino acids selected from K153, K187, K330, K427, K512, K535, K567, and K572 of MIRO; and/or ubiquitination increases at at least one, at least two, or three amino acids selected from K273, K299,and K52 of MUL1; and/or ubiquitination increases at at least one, at least two, at least three, at least four, at least five, at least six, at least seven, or eight amino acids selected from K249, K271, K273, K284, K307, K317, K334, and K340 of FKBP8; and/or ubiquitination increases at at least one, at least two, at least three, at least four, at least five, at least six, at least seven, at least eight, or nine amino acids selected from K147, K168, K176, K221, K244, K275, K478, K504, and K556 of ASNS; and/or ubiquitination increases at at least one, at least two, at least three, at least four, at least five, at least six, at least seven, at least eight, at least nine, or at least ten amino acids selected from K78, K120, K123, K126, K129, K148, K168, K170, K178, K185, K204, K230, K233, K245, K275, K278, K312, K326, K349, K359, K441, K463, K470, K471, K494, K501, K524, K536, K563, K570, K599, K600, and K604 of TOM70; and/or ubiquitination increases at at least one, at least two, at least three, or four amino acids selected from K209, K245, K316, and K326 of MAT2B; and/or ubiquitination increases at at least one, at least two, at least three, at least four, or five amino acids selected from K83, K91, K166, K241, and K253 of PRDX3; and/or ubiquitination increases at at least one, at least two, at least three, at least four, at least five, or six amino acids selected from K558, K657, K854, K884, K929, and K933 of IDE; and/or ubiquitination increases at at least one, at least two, at least three, at least four, at least five, at least six, or seven amino acids selected from K20, K53, K61, K109, K110, K266, and K274 of VDAC1; and/or ubiquitination increases at at least one, at least two, at least three, at least four, at least five, or six amino acids selected from K31, K64, K120, K121, K277, and K285 of VDAC2; and/or ubiquitination increases at at least one, at least two, at least three, at least four, at least five, at least six, at least seven, or eight amino acids selected from K20, K53, K61, K109, K110, K163, K266, and K274 of VDAC3; and/or ubiquitination increases at at least one, at least two, at least three, at least four, at least five, at least six, at least seven, at least eight, at least nine, or at least ten amino acids selected from K238, K353, K436, K437, K548, K556, K613, K678, K690, K705, K775, and K806 of IPO5; and/or ubiquitination increases at at least one, at least two, at least three, at least four, at least five, at least six, at least seven, at least eight, at least nine, or at least ten amino acids selected from K2, K32, K99, K115, K122, K132, K161, K186, K313, K321, K347, K350, and K361 of PSD13; and/or ubiquitination increases at at least one, at least two, at least three, at least four, at least five, at least six, at least seven, at least eight, at least nine, or at least ten amino acids selected from K18, K190, K259, K326, K328, K401, K405, K414, K418, K435, K586, K587, and K640 of UBP13; and/or ubiquitination increases at at least one, at least two, at least three, at least four, at least five, at least six, at least seven, at least eight, or nine amino acids selected from K47, K76, K81, K95, K106, K119, K134, K171, K177 of PTH2. In some embodiments, ubiquitination of one or more additional proteins increases upon contacting a cell with an inhibitor of USP30. Nonlimiting

exemplary proteins whose ubiquitination may be increased in the presence of an inhibitor of USP30 are listed in Appendix A. Increased ubiquitination of a target protein can be determined, e.g., by immunoaffinity enrichment of ubiquitinated peptides after trypsin digestion, followed by mass spectrometry, as described in Example 5. In some embodiments, an increase in ubiquitination may be determined by comparing the ubiquitination of a target protein a cell or population of cells contacted with an inhibitor of USP30 with the ubiquitination of the same target protein in a matched cell or population of cells not contacted with the inhibitor.

In some embodiments, increased ubiquitination of a protein means an increase in ubiquitination of the protein in a cell or population of cells contacted with an inhibitor of USP30 of at least 10%, at least 20%, at least 30%, at least 40%, at least 50%, at least 60%, at least 70%, at least 80%, at least 90%, at least 100% (i.e., 2-fold), at least 150%, or at least 200% (i.e., 3-fold) as compared to a matched cell or population of cells not contacted with the inhibitor.

In some embodiments, the cell is under oxidative stress. Further, in some embodiments, a method of reducing oxidative stress in a cell is provided, the method comprising contacting the cell with an inhibitor of USP30 under conditions allowing inhibition of USP30 in the cell.

In any of the foregoing methods, the cell may comprise a pathogenic mutation in Parkin, a pathogenic mutation in PINK1, or a pathogenic mutation in Parkin and a pathogenic mutation in PINK1. Nonlimiting exemplary pathogenic mutations in Parkin and PINK1 are shown, e.g., in Tables 1 and 2 herein.

In some embodiments, the cell is a neuron. In some embodiments, the cell is a substantia nigra neuron. In some embodiments, the cell is a cardiac cell. In some embodiments, the cell is a cardiomyocyte cell. In some embodiments, the cell is a muscle cell.

In some embodiments of any of the foregoing methods, the cell is comprised in a subject. In some embodiments of any of the foregoing methods, the cell may be *in vitro* or *ex vivo.*

In another aspect, an inhibitor of USP30 for use as a medicament is provided. In further aspects, an inhibitor of USP30 for use in a method of treatment is provided. In some embodiments, a method of treating a condition involving a mitochondrial defect in a subject is provided, the method comprising administering to the subject an effective amount of an inhibitor of USP30. A condition involving a mitochondrial defect may involve a mitophagy defect, one or more mutations in mitochondrial DNA, mitochondrial oxidative stress, defects in mitochondrial shape/morphology, mitochondrial membrane potential defects, and/or a lysosomal storage defect. Nonlimiting exemplary conditions involving mitochondrial defects include neurodegenerative diseases; mitochondrial myopathy, encephalopathy, lactic acidosis, and stroke-like episodes (MELAS) syndrome; Leber's hereditary optic neuropathy (LHON); neuropathy, ataxia, retinitis pigmentosa-maternally inherited Leigh syndrome (NARP-MILS); Danon disease; ischemic heart disease leading to myocardial infarction; multiple sulfatase deficiency (MSD); mucolipidosis II (ML II); mucolipidosis III (ML III); mucolipidosis IV (ML IV); GM1-gangliosidosis (GM1); neuronal ceroid-lipofuscinoses (NCL1); Alpers disease; Barth syndrome; Beta-oxidation defects; carnitine-acyl-carnitine deficiency; carnitine deficiency; creatine deficiency syndromes; co-enzyme Q10 deficiency; complex I deficiency; complex II deficiency; complex III deficiency; complex IV deficiency; complex V deficiency; COX deficiency; chronic progressive external ophthalmoplegia syndrome (CPEO); CPT I deficiency; CPT II deficiency; glutaric aciduria type II; Kearns-Sayre syndrome; lactic acidosis; long-chain acyl-CoA dehydrongenase deficiency (LCHAD); Leigh disease or syndrome; lethal infantile cardiomyopathy (LIC); Luft disease; glutaric aciduria type II; medium-chain acyl-CoA dehydrongenase deficiency (MCAD); myoclonic epilepsy and ragged-red fiber (MERRF) syndrome; mitochondrial recessive ataxia syndrome; mitochondrial cytopathy; mitochondrial DNA depletion syndrome; myoneurogastointestinal disorder and encephalopathy; Pearson syndrome; pyruvate carboxylase deficiency; pyruvate dehydrogenase deficiency; POLG mutations; medium/short-chain 3-hydroxyacyl-CoA dehydrogenase (M/SCHAD) deficiency; and very long-chain acyl-CoA dehydrongenase (VLCAD) deficiency. Nonlimiting exemplary neurodegenerative diseases that involve mitochondrial defects include Parkinson's disease, Alzheimer's disease, Huntington's disease, amyotrophic lateral sclerosis (ALS), ischemia, stroke, dementia with Lewy bodies, and frontotemporal dementia. Additional exemplary neurodegenerative diseases that may involve mitochondrial defects include, but are not limited to, intracranial hemorrhage, cerebral hemorrhage, trigeminal neuralgia, glossopharyngeal neuralgia, Bell's Palsy, myasthenia gravis, muscular dystrophy, progressive muscular atrophy, primary lateral sclerosis (PLS), pseudobulbar palsy, progressive bulbar palsy, spinal muscular atrophy, inherited muscular atrophy, invertebrate disk syndromes, cervical spondylosis, plexus disorders, thoracic outlet destruction syndromes, peripheral neuropathies, prophyria, multiple system atrophy, progressive supranuclear palsy, corticobasal degeneration, demyelinating diseases, Guillain-Barre syndrome, multiple sclerosis, Charcot-Marie-Tooth disease, prion disease, Creutzfeldt-Jakob disease, Gerstmann-Straussler-Scheinker syndrome (GSS), and fatal familial insomnia. In some such embodiments, the method further comprises administering to the individual an effective amount of at least one additional therapeutic agent, e.g., as described below.

In some embodiments, an inhibitor of USP30 is provided for use in the manufacture or preparation of a medicament. In some such embodiments, the medicament is for treatment of conditions involving a mitochondrial defect, such as, for example, conditions involving a mitophagy defect, conditions involving mutations in mitochondrial DNA, conditions involving mitochondrial oxidative stress, conditions involving defects in mitochondrial shape/morphology, conditions involving defects in mitochondrial membrane potential, and conditions involving lysosomal storage defects.

In further embodiments, the medicament is for use in a method of treating a condition involving a mitochondrial defect, the method comprising administering to an individual having the condition involving a mitochondrial defect an effective amount of the medicament. In one such embodiment, the method further comprises administering to the individual an effective amount of at least one additional therapeutic agent, e.g., as described below.

An "individual" according to any of the embodiments herein may be a human.

In a further aspect, pharmaceutical formulations comprising any of the inhibitors of USP30 provided herein, e.g., for use in any of the above therapeutic methods are provided. In one embodiment, a pharmaceutical formulation comprises any of the inhibitors of USP30 provided herein and a pharmaceutically acceptable carrier. In another embodiment, a pharmaceutical formulation comprises any of the inhibitors of USP30 provided herein and at least one additional therapeutic agent, e.g., as described below.

Inhibitors of USP30 can be used either alone or in combination with other agents in a therapy. For instance, an inhibitor of USP30 may be co-administered with at least one additional therapeutic agent.

Exemplary therapeutic agents that may be combined with an inhibitor of USP30, e.g., for the treatment of Parkinson's disease, include levodopa, dopamine agonists (such as pramipexole, ropinirole, and apomorphine), monoamine oxygenase (MAO) B inhibitors (such as selegiline and rasagiline), catechol O-methyltransferase (COMT) inhibitors (such as entacapone and tolcapone), anticholinergics (such as benzotropine and trihexylphenidyl), and amantadine. A further exemplary therapeutic agent that may be combined with an inhibitor of USP30, e.g., for the treatment of amyotrophic lateral sclerosis, is riluzole. Exemplary therapeutic agents that may be combined with an inhibitor of USP30, e.g., for the treatment of Alzheimer's disease, include cholinesterase inhibitors (such as donepezil, rivastigmine, galantamine, and tacrine), and memantine. Exemplary therapeutic agents that may be combined with an inhibitor of USP30, e.g., for the treatment of Huntington's disease, include tetrabenazine, antipsychotic drugs (such as haloperidol and clozapine), clonazepam, diazepam, antidepressants (such as escitalopram, fluoxetine, and sertraline), and mood-stabilizing drugs (such as lithium), and anti-convulsants (such as valproic acid, divalproex, and lamotrigine).

Administration "in combination" encompasses combined administration (where two or more therapeutic agents are included in the same or separate formulations), and separate administration, in which case, administration of the inhibitor of the invention can occur prior to, simultaneously, and/or following, administration of the additional therapeutic agent and/or adjuvant. In some embodiments, administration of the inhibitor of USP30 and administration of an additional therapeutic agent occur within about one month, or within about one, two, or three weeks, or within about one, two, three, four, five, or six days of one another. Inhibitors of the invention can also be used in combination with other types of therapies.

An inhibitor of the invention (and any additional therapeutic agent) can be administered by any suitable means, including oral, parenteral, intrapulmonary, intranasal, and intralesional administration. Parenteral administration includes, but is not limited to, intramuscular, intravenous, intraarterial, intracerebral, intracerebroventricular, intrathecal, intraocular, intraperitoneal, and subcutaneous administration. An inhibitor of the invention (and any additional therapeutic agent) may also be administered using an implanted delivery device, such as, for example, an intracerebral implant. Nonlimiting exemplary central nervous system delivery methods are reviewed, e.g., in Pathan et al., Recent Patents on Drug Delivery & Formulation, 2009, 3: 71-89. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Inhibitors of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The inhibitor need not be, but is optionally formulated with one or more agents currently used to prevent or treat the disorder in question. The effective amount of such other agents depends on the amount of inhibitor present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an inhibitor of USP30 (when used alone or in combination with one or more other additional therapeutic agents) will depend on the type of disease to be treated, the type of inhibitor, the severity and course of the disease, whether the inhibitor is administered for preventive or therapeutic purposes, previous therapy, the patient's clinical history and response to the inhibitor, and the discretion of the attending physician. The inhibitor is suitably administered to the patient at one time or over a series of treatments. Depending on the type and severity of the disease, about 1 μg/kg to 15 mg/kg (e.g. 0.1mg/kg-10mg/kg) of inhibitor can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the

condition, the treatment would generally be sustained until a desired suppression of disease symptoms occurs. One exemplary dosage of the inhibitor would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the inhibitor). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using more than one inhibitor of USP30.

### E. Articles of Manufacture

In another aspect an article of manufacture containing materials useful for the treatment, prevention and/or diagnosis of the disorders described herein is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the disorder and may have a sterile access port (for example the container may be an intravenous solution bag or a vial having a stopper pierceable by a hypodermic injection needle). At least one active agent in the composition is an inhibitor of the invention. The label or package insert indicates that the composition is used for treating the condition of choice. Moreover, the article of manufacture may comprise (a) a first container with a composition contained therein, wherein the composition comprises an inhibitor of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

## III. EXAMPLES

The following are examples of methods and compositions of the invention. It is understood that various other embodiments may be practiced, given the general description provided above.

### Example 1: Materials and Methods

DUB cDNA overexpression screen:

To identify regulators of mitophagy, individual cDNAs from a FLAG-tagged DUB library were cotransfected into HeLa cells with GFP-Parkin using Lipofectamine 2000 (Invitrogen) (1:3 DUB-FLAG : GFP-Parkin cDNA ratio). After 24 hours of expression, cells were treated with 10 μM CCCP for 24 hours, and fixed and stained using anti-Tom20 (Santa Cruz Biotechnology), anti-GFP (Aves Labs) and anti-FLAG (Sigma) primary antibodies. Following staining with secondary antibodies, images of random fields were acquired with a Leica SP5 Laser Scanning Confocal Microscope using a 40X/1.25 oil-objective (0.34 μm/pixel resolution, 1 μm confocal z-step size). Percent of GFP-Parkin and FLAG-DUB cotransfected cells containing Tom20 staining was scored blindly.

Hippocampal culture, transfection and mt-Keima imaging:

Dissociated hippocampal neuron cultures were prepared as described (Seeburg et al., Neuron 58: 571-583 (2008)) and transfected using Lipofectamine LTX PLUS at DIV 8-10. Constructs were expressed for 1-3 days for overexpression experiments and 3-4 days for knockdown experiments. mt-Keima-transfected neurons were imaged with a Leica TCS SP5 Laser Scanning Confocal microscope with a 40X/1.25 oil objective (0.07 μm/pixel resolution, 1 μm confocal z-step size). Cells were kept in a humidified chamber maintained at 37°C / 5% $CO_2$ during imaging. Two images were acquired using a hybrid detector in sequential mode with 458 nm (neutral pH signal) and 543 nm (acidic pH signal) laser excitation, and emission fluorescence collected between 630-710 nm. All image quantification was performed by custom-written macros in ImageJ. For mt-Keima quantification, cell bodies were manually outlined and total area of high ratio (543nm/458nm) lysosomal signal was divided by the total area of somatic mitochondrial signal (mitophagy index).

Mass Spectrometry

To determine Parkin substrates, HEK-293 GFP-Parkin inducible cells were treated with doxycycline for 24 hours, and then treated with 5 μM CCCP or DMSO vehicle control for 2 hours. To determine USP30 substrates, HEK-293T cells were transfected with human USP30 shRNA using Lipofectamine 2000 (Invitrogen) for 6 days, then treated as before. In both experiments, cells were lysed (20 mM HEPES pH 8.0, 8M urea, 1 mM sodium orthovanadate, 2.5 mM sodium pyrophosphate, 1 mM β-glycerophosphate), sonicated, and cleared by centrifugation prior to proteolytic digestion and immunoaffinity enrichment of peptides bearing the ubiquitin remnant, and mass spectrometry analysis.

Preparation of cellular lysates and immunoprecipitation

For total lysate experiments, transfected HEK-293 cells were lysed at 24 hours post-transfection in SDS sample buffer (Invitrogen) containing sample reducing agent (Invitrogen). For immunoprecipitation experiments, cells were lysed at 24 hours (overexpression experiments) or 6 days (knockdown experiments) post-transfection in TBS buffer containing 0.5% SDS, and lysates were diluted with a buffer containing 1% Triton-X-100 and protease and phosphatase inhibitors. Ubiquitinated proteins were immunoprecipated from lysates of HA-ubiquitin transfected cells with anti-HA affinity matrix beads (Roche Applied Science). Inputs and precipitates were resolved by SDS-PAGE and analyzed by immunoblotting.

Statistical analysis

Error bars indicate standard error of the mean (S.E.M.). To compute p values, non-paired Student's t-test, One-way ANOVA with Dunnett's Multiple Comparison test (for comparisons to a single condition) or Bonferroni's Multiple Comparison test (for comparisons between multiple conditions), and Two-way ANOVA were used, as indicated in figure legends. All statistical analysis was performed in GraphPad Prism v.5 software.

DNA construction

For the DUB overexpression screen, a FLAG-tagged DUB library consisting of 100 cDNAs was used. For transfection, the following constructs were subcloned into β-actin promoter-based pCAGGS plasmid: USP30-FLAG (rat), USP30-FLAG (human), GFP-Parkin (human), FLAG-Parkin (human), PINK1-GFP (human), myc-Parkin (human), RHOT1 (MIRO)-myc-FLAG (human), TOM20-myc (human), HA-ubiquitin, PSD-95-FLAG, and mt-mKeima (Katayama et al., Chemistry & Biology 18: 1042-1094 (2011)). Point mutations were generated using QuikChange II XL (Agilent Technologies) for the following constructs: USP30-C77S-FLAG (rat), USP30-C77A-FLAG (rat), USP30-C77S-FLAG (human), GFP-Parkin K161N (human), and GFP-Parkin G430D (human). Mito-tagGFP2 (Evrogen), Tom20-3KR-myc, and HA-ubiquitin mutants (Blue Heron) were purchased. β-Gal (Seeburg and Sheng, J. Neurosci. 28: 6583-6591 (2008)) and mito-ro-GFP (Dooley et al., J. Biol. Chem. 279: 22284-22293 (2004)) expression plasmids were previously described. Short-hairpin sequences targeting the following regions were cloned into pSuper or pSuper-GFP-neo plasmids: rat PINK1 #1 (TCAGGAGATCCAGGCAATT), rat PINK1 #2 (CCAGTACCTTGAAGAGCAA), rat Parkin #1 (GGAAGTGGTTGCTAAGCGA), rat Parkin #2 (GAGGAAAAGTCACGAAACA), rat USP30 (CCAGAGCCCTGTTCGGTTT), human USP30 (CCAGAGTCCTGTTCGATTT), and firefly luciferase (CGTACGCGGAATACTTCGA).

Antibodies and Reagents:

The following antibodies were used for immunocytochemistry: rabbit anti-TOM20, mouse anti-TOM20, goat anti-HSP60 (Santa Cruz Biotechnology); mouse anti-FLAG, rabbit anti-FLAG, mouse anti-myc (Sigma-Aldrich); and chicken anti-GFP (Aves Labs).

The following antibodies were used for immunoblotting: rabbit anti-TOM20, goat anti-HSP60 (Santa Cruz Biotechnology); mouse anti-MFNI, HRP-conjugated anti-FLAG, mouse anti-myc, rabbit anti-USP30, rabbit anti-RHOT1 (MIRO), rabbit anti-TIMM8A (Sigma-Aldrich); rabbit anti-GFP, chicken anti-GFP (Invitrogen); HRP-conjugated anti-GAPDH, HRP-conjugated anti-α-actin, HRP-conjugated anti-β-tubulin, rabbit anti-VDAC (Cell Signaling Technology); rabbit anti-TOM70 (Proteintech Group); anti-ubiquitin (FK2) (Enzo Life Sciences); mouse anti-LAMP1 (StressGen); HRP-conjugated anti-HA (Roche); and anti-USP30 rabbit (generated by immunizing rabbits with purified human USP30 amino acids 65-517).

For immunoprecipitation experiments anti-FLAG M2 affinity gel beads (Sigma) and anti-HA affinity matrix beads (Roche Applied Science) were used.

Adeno-associated virus type2 (AAV2) particles expressing Parkin, PINK1 and USP30 shRNAs were produced by Vector Biolabs, Inc. from pAAV-BASIC-CAGeGFP-WPRE vector containing the H1 promoter and shRNA expression cassette of the pSuper vectors.

The following reagents were purchased as indicated: blasticidin S, zeocin, Lipofectamine 2000, Lipofectamine LTX PLUS, LysoTracker Green DND-626 (Invitrogen); PhosSTOP phosphatase inhibitor tablets, cOmplete EDTA-free protease inhibitor tablets, DNase I (Roche Applied Science); carbonyl cyanide 3-chlorophenylhydrazone (CCCP), doxycycline, dimethyl sulfoxide, ammonium chloride, rotenone, DTT, aldrithiol, paraquat dichloride (Sigma-Aldrich); N-Ethylmaleimide (Thermo Scientific); and hygromycin (Clontech Laboratories).

Transfection and Immunocytochemistry:

All heterologous cells were transfected with Lipofectamine 2000 for cDNA expression and Lipofectamine RNAiMAX for siRNA knockdown experiments, according to manufacturer's instructions (Invitrogen). siRNAs were purchased from Dharmacon as siGenome pools (non-Silencing pool #2 was used control siRNA transfection). Hippocampal cultures were prepared as described previously (Seeburg et al., Neuron 58: 571-583 (2008)) and transfected with Lipofectamine LTX PLUS (Invitrogen) with 1.8 μg DNA, 1.8 μl PLUS reagent and 6.3 μl LTX reagent. Following drug treatments, cells were fixed with 4% paraformaldehyde/4% sucrose in phosphate-buffered saline (PBS, pH 7.4) (Electron Microscopy Sciences). Following permeabilization (0.1% Triton-X in PBS), blocking (2% BSA in PBS) and primary antibody incubation, antibodies were visualized using Alexa dye-conjugated secondary antibodies (Invitrogen). All immunocytochemistry images were acquired with a Leica SP5 laser scanning microscope with a 40X/1.25 oil objective (0.34 μm/pixel resolution, 1 μm confocal z-step size).

HEK293 and SH-SY5Y stable cell line generation

Stably transfected HEK cell lines expressing GFP-Parkin (human) wild-type, K161N, and G430D were generated by co-transfecting FLP-In 293 cells with a pOG44 Flp-recombinase expression vector (Invitrogen) and a pcDNA5-FRT vector (Invitrogen) expressing the corresponding constructs under a CMV promoter. Cell lines were selected and maintained using 50 μg/mL hygromycin selection. Inducible HEK stable cell line expressing GFP-Parkin (human) was generated by co-transfecting FLP-In T-Rex 293 cells with pOG44 and a pcDNA5-FRT-TO vector (Invitrogen) expressing GFP-Parkin (human). The line was selected and maintained using 50 μg/mL hygromycin and 15 μg/mL blasticidin. SH-SY5Y stable cells were generated similarly with a Flp-In inducible parental cell line using pcDNA5-FRT-TO and maintained under 75 μg/ml hygromycin and 3 μg/ml blasticidin.

Isolation and identification of ubiquitin modifications by mass spectrometry

To identify Parkin substrates, HEK 293T cells stably expressing inducible GFP-Parkin (human) were induced using doxycycline (1μg/mL) for 24 hours, then treated with 5 μM CCCP or DMSO vehicle control for 2 hours. To determine USP30 substrates, HEK 293T cells were transfected with human USP30 shRNA using Lipofectamine 2000 (Invitrogen) for 6 days, then treated as above.

Immunoaffinity isolation and mass spectrometry methods were used to enrich and identify K-GG peptides from digested protein lysates as previously described (Xu et al., Nat. Biotech., 28: 868-873 (2010); Kim et al., Mol. Cell, 44: 325-340 (2011)). Cell lysates were prepared in lysis buffer (8M urea 20 mM HEPES pH 8.0 with 1 mM sodium orthovanadate, 2.5 mM sodium pyrophosphate, 1 mM β-glycerophosphate) by brief sonication on ice. Protein samples (60 mg) were reduced at 60°C for 20 min in 4.1 mM DTT, cooled 10 min on ice, and alkylated with 9.1 mM iodoacetamide for 15 min at room temperature in the dark. Samples were diluted 4X using 20 mM HEPES pH 8.0 and digested in 10 μg/ml trypsin overnight at room temperature. Following digestion, TFA was added to a final concentration of 1% to acidify the peptides prior to desalting on a Sep-Pak C18 cartridge (Waters). Peptides were eluted from the cartridge in 40 % ACN/ 0.1 % TFA, flash frozen and lyophilized for 48 hr. Dry peptides were gently resuspended in 1.4 ml 1X IAP buffer (Cell Signaling Technology) and cleared by centrifugation for 5 min at 1800 x g. Precoupled anti-KGG beads (Cell Signaling Technology) were washed in 1X IAP buffer prior to contacting the digested peptides.

Immunoaffinity enrichment was performed for 2 hours at 4°C. Beads were washed 2X with IAP buffer and 4X with water prior to 2X elution of peptides in 0.15% TFA for 10 min each at room temperature. Immunoaffinity enriched peptides were desalted using STAGE-Tips as previously described (Rappsilber et al., Anal. Chem., 75: 663-670 (2003)).

Liquid chromatography-mass spectrometry (LC-MS) analysis was performed on an LTQ-Orbitrap Velos mass spectrometer operating in data dependent top 15 mode. Peptides were injected onto a 0.1 x 100-mm Waters 1.7-um BEH-130 C18 column using a NanoAcquity UPLC and separated at 1 ul/min using a two stage linear gradient where solvent B ramped first from 2% to 25% over 85 min and then 25% to 40% over 5 min. Peptides eluting from the column were ionized and introduced to the mass spectrometer using an ADVANCE source (Michrom-Bruker). In each duty cycle, one full MS scan collected was at 60,000 resolution in the Orbitrap followed by up to 15 MS/MS scans in the ion trap on monoisotopic, charge state defined precursors ($z > 1$). Ions selected for MS/MS (±20 ppm) were subjected to dynamic exclusion for 30 sec duration.

Mass spectral data were converted to mzxml for loading into a relational database. MS/MS spectra were searched using Mascot against a concatenated target-decoy database of tryptic peptides from human proteins (Uniprot) and common contaminants. Precursor ion mass tolerance was set to ±50 ppm. Fixed modification of carbamidomethyl cysteine (+57.0214) and variable modifications of oxidized methionine (+15.9949) and K-GG (+114.0429) considered. Linear discriminant analysis (LDA) was used to filter peptide spectral matches (PSMs) from each run to 5% false discovery rate (FDR) at the peptide level, and subsequently to a 2% protein level FDR as an aggregate of all runs (< 0.5% peptide level FDR). Localization scores were generated for each K-GG PSM using a modified version of the AScore algorithm and positions of the modifications localized accordingly as the AScore sequence. (Beausoleil et al., Nat. Biotech. 24(10): 1285-1292 (2006)). Given work showing that trypsin cannot cut adjacent to ubiquitin modified lysines PSMs where the AScore sequence reports a - GG modification on the C-terminal lysine are dubious (Bustos et al., Mol. Cell. Proteomics, published online June 23, 2012, doi: 10.1074/mcp.R112.019117; Seyfried et al., Anal. Chem., 80: 4161-4169 (2008)). Possible exceptions to this would be lysines at the C-termini of proteins (or *in vivo* truncation products), PSMs stemming from in source fragmentation of a bona fide K-GG peptide. To establish the most reliable dataset for downstream analysis, PSMs where the AScore sequence reports a C-terminal lysine were split into two groups: those with an available internal lysine residue to which the -GG could be alternatively localized, and those which lacked an available lysine. PSMs bearing a C-terminal K-GG but lacking an available lysine were removed from consideration in downstream analyses. For the remaining PSMs, the -GG modification was relocalized to the available lysine closest to the C-terminus.

Confidently identified peptides with ambiguous localization (AScore < 13) bearing only a single internal lysine residue were reported with the modification localized to that internal lysine. Peptides where the modification has been assigned to the C-terminal lysine with an AScore > 13 were discarded based on evidence suggesting that trypsin cannot cleave at a ubiquitin modified lysine residue.

A modified version of the VistaGrande algorithm, termed XQuant, was employed to interrogate the unlabeled peak areas for individual K-GG peptides, guided by direct PSMs or accurate precursor ion and retention time matching (cross quantitation). For direct PSMs, quantification of the unlabeled peak area was performed as previously described using fixed mass and retention tolerances (Bakalarski et al., J. Proteome Res., 7: 4756-4765 (2008)). To enable cross quantitation within XQuant, retention time correlation across pairwise instrument analyses was determined based on high-scoring peptide sequences identified by between one and four PSMs across all analyses within an experiment. Matched retention time pairings were modeled using a linear least squares regression model to yield the retention time correlation equation. In instrument analyses where a peptide was not identified by a discrete MS/MS, cross quantification was carried out by seeding the XQuant algorithm with the calculated mass of the precursor ion and its predicted retention time derived from the regression model. While the m/z tolerance was fixed, the retention time tolerance was dynamically adjusted for each pairwise instrument run. In cases where peptides were not confidently identified within a given instrument run but were identified in multiple other runs, multiple cross quantification events were performed to ensure data quality. XQuant results were filtered to a heuristic confidence score of 83 or greater, as previously described (Bakalarski et al., J. Proteome Res., 7: 4756-4765 (2008)). Full scan peak area measurements arising from multiple quantification events of the same m/z within a single run were grouped together if their peak boundaries in retention time overlapped. From such a group, the peak with the largest total peak area was chosen as its single representative.

To identify candidate substrates of Parkin and USP30, graphical analysis and mixed-effect modeling were applied to the XQuant data. A mixed-effect model was fit to the AUC data for each protein. "Treatment" (e.g. Control, Parkin overexpression/USP30 knockdown , CCCP, Combo) was a categorical fixed effect and "Peptide" was fit as random effect. False discovery rates (FDR) are calculated based on the P-values of each treatment vs. Control. Fold-changes and P-values of mean AUC from Combo vs. Control and Combo vs. CCCP were utilized in preparing plots. Mixed-effect model was fit in R by 'nlme' (Pinheiro et al., nlme: Linear and Nonlinear Mixed Effects Models. R package version 3, 1-101 (2011)).

Preparation of cell lysates, and immunoprecipitation

For total lysate experiments, cells were lysed after 24 hours in SDS sample buffer (Invitrogen) containing sample reducing agent (Invitrogen) and boiled at 95°C for 10 minutes. Total lysates were resolved by SDS-PAGE and analyzed by immunoblotting. For immunoprecipitation experiments, cells were treated with 5 μM MG132 and the indicated concentrations and durations of CCCP at 24 hours (overexpression experiments) or 6 days (knockdown experiments) in 0.5% SDS in Tris-Buffered Saline (10mM TRIS, 150mM NaCl, pH 8.0) and boiled at 70°C for 10 minutes. Lysates were diluted in immunoprecipitation buffer (50mM HEPES, 150mM NaCl, 10% glycerol, 1% Triton-X, protease inhibitors (Roche Applied Science), phosphatase inhibitors (Roche Applied Science), DNAse I (Roche Applied Science), 2 mM N-Ethylmaleimide (Thermo Scientific), pH 7.4), cleared by centrifugation at 31,000g for 10 minutes, and incubated overnight with anti-HA affinity matrix beads (Roche Applied Science). Inputs and anti-HA immunoprecipitates were resolved by SDS-PAGE and analyzed by immunoblotting.

Mitochondria fractionation

Subcellular fractionation was performed using the FOCUS SubCell Kit (G Biosciences) from ~P60 adult male rat forebrain.

Drosophila stocks

The following Drosophila lines were obtained for analysis: y, w; *Actin5C-GAL4/CyO, y+* (Bloomington Drosophila Stock Center, 4414), *UAS-CG3016$^{RNAi}$* (referred to here as *UAS-dUSP30$^{RNAi}$*; NIG-Fly Stock Center, 3016R-2). For USP30 knockdown experiments, *Actin5C-GAL4* and *UAS-dUSP30$^{RNAi}$* were recombined onto the same chromosome using standard genetic techniques.

Flies were raised on Nutri-Fly "German Food" Formulation (Genesee, 66-115), prepared per manufacturer's instructions. All flies were raised at 25°C and crossed using standard genetic techniques. All experiments were performed using age-matched male flies.

Quantitative RT-PCR

RNA and subsequent cDNA was obtained from single flies following manufacturer's instructions (Qiagen RNeasy Plus kit, Applied Biosystems High Capacity cDNA Reverse Transcription kit). Quantitative RT-PCR was performed using an Applied Biosystems ViiA7 Real-Time PCR system using TaqMan Assays Dm01796115_g1 and Dm01796116_g1 (Drosophila CG3016 (USP30)), Dm01795269_g1 (Drosophila CG5486 (USP47)), and Dm01840115_s1 (Drosophila CG4603 (YOD1)). Dm02134593_g1 (RpII140) was used as a control.

Determination of ingested paraquat concentration

1-day old adult males were fed a solution containing 5% sucrose only (in water) or 5% sucrose + 10mM paraquat (in water) on saturated Whatman paper. After 48 hours of treatment, 15 flies were collected per condition and homogenized in 100 μL water. Standard curve samples were generated by spiking appropriate amounts of paraquat to homogenates from untreated flies. Then the samples were vortex mixed, 200 μL of acetonitrile containing internal standard (Propranolol) was added. The samples were vortexed again and centrifuged at 10,000 × *g* for 10 min. Supernatants were transferred to a new plate that contained 200 μL of water and analyzed by LC-MS/MS to quantify for concentrations of paraquat. The LC-MS/MS consisted of an Agilent 1100 series HPLC system (Santa Clara, CA) and an HTS PAL autosampler from CTC Analytics (Carrboro, NC) coupled with a 4000 Q TRAP® MS and TurboIonSpray® ion source from Applied Biosystems (Foster City, CA). HPLC separation was performed on a Waters Atlantis dC18 column (3 μm 100 x 2.1mm) with a Krud Katcher guard column from Phenomenex. Quantitation was carried out using the multiple reaction monitoring (MRM) with transition 185.1→165.1 for paraquat and 260.2→183.1 for propranolol. The lower and upper limit of the assay is 10 μM and 1000 μM, respectively. The quatitation of the assay employed a calibration curve which was constructed through plotting the analyte/IS peak area ration versus the nominal concentration of paraquat with a weighed $1/x^2$ linear regression.

Transmission electron microscopy of *Drosophila* indirect flight muscles

Adult male thoraxes were isolated from the remainder of the body, then longitudinally hemi-sectioned and immediately fixed and processed as previously described (Greene et al., Proc. Natl. Acad. Sci. USA, 100: 4078-4083 (2003)).

Climbing assays

Climbing assays were performed using the following *Drosophila* lines: y, w; *Actin5C-GAL4/CyO, y+* (Actin only); *y*, *w*; *UAS-CG3016-RNAi/CyO, y+* (RNAi only); *y*, *w*; *UAS-CG3016$^{RNAi}$, Actin5C-GALA/CyO, y+* (USP30 knockdown).

1-day old adult males were fed a solution containing 5% sucrose only (in water) or 5% sucrose + 10mM paraquat (in water) on saturated Whatman paper. After 48 hours of treatment, flies were anesthetized using carbon dioxide and transferred in groups of ten to vials containing only 1% agarose for a 1-hour recovery period from the effects of carbon dioxide. The flies were then transferred into a new glass tube, gently tapped to the bottom and scored for their ability to climb. The number of flies climbing vertically >15 cm in 30 seconds was recorded.

Survival assays

Ten adult 1-day old males per vial were fed a solution containing 5% sucrose only (in water) or 5% sucrose + 10 mM paraquat (in water) on saturated Whatman paper. The number of live flies was counted at described intervals.

MultiTox Cell Death Assay

SH-SY5Y cells transfected with control or USP30 siRNAs are treated with rotenone in normal growth medium (DMEM/F12 and 1X GlutaMax) containing 1% Fetal Bovine Serum. Following 24 hours of incubation, Multi-Tox Fluor assay (Promega) is used to measure cell viability according to manufacturer's instructions. GF-AFC fluorescence is normalized to bis-AAF-R110 fluorescence for each condition and presented as a fraction of control (control RNAi + DMSO).

**Example 2: USP30 antagonizes Parkin-mediated clearance of damaged mitochondria**

To identify DUBs that regulate mitochondria clearance, a FLAG-tagged human DUB cDNA library (97 DUBs) was screened in an established mitochondrial degradation assay (Narendra et al., J. Cell Biol., 183: 795-803 (2008)). In this assay, mitochondria depolarization induced by protonophore carbonyl cyanide 3-chlorophenylhydrazone (CCCP, 20 μM, 24 hours) results in marked loss of mitochondria in cultured cells overexpressing Parkin (as measured by staining for mitochondria outer membrane protein marker Tom20). CCCP treatment led to a robust disappearance of Tom20 staining in the great majority of cells transfected with GFP-Parkin (>80% of Parkin-transfected cells lacked Tom20 staining after CCCP - Figure 1A). Individual FLAG-tagged DUB cDNAs were cotransfected with GFP-Parkin, and their effects on CCCP-induced mitochondrial (Tom20) clearance were measured. Out of the library of ~100 different DUBs, 2 DUBs, USP30 and DUBA2, robustly blocked the loss of Tom20 staining in CCCP-treated GFP-Parkin-transfected cells, whereas others had little effect (Figure 1A - % of cells with Tom20 staining: control (β-Gal): 15.3%, USP30: 97.4%, DUBA2: 94.7%, UCH-L1: 36%, USP15: 23.3%, ATXN3: 8.3%; other negative DUBs not shown). USP30 rather than DUBA2 was selected for further study since USP30 has been reported to be localized in the mitochondrial outer membrane with its enzymatic domain putatively facing the cytoplasm (Nakamura and Hirose, Mol. Biol. Cell, 19: 1903-1914 (2008)); thus it would be in the right subcellular compartment to counteract the action of Parkin on mitochondria. The specific mitochondrial association of USP30 was confirmed by immuno-colocalization of transfected USP30-FLAG and of endogenous USP30 with mitochondrial markers in neurons (Figure 2A, B), as well as by cofractionation of USP30 with purified mitochondria from rat brain (Figure 2C).

The ability of USP30 overexpression to prevent CCCP-induced mitophagy was also shown in a different cell line (dopaminergic SH-SY5Y cells) transfected with myc-Parkin (Figure 1B). To confirm that the effects of USP30 were not specific to Tom20, whether USP30 overexpression also prevented the CCCP-induced loss of the mitochondrial matrix protein HSP60 was tested. Indeed, USP30 overexpression also prevented the CCCP-induced loss of HSP60, implying USP30 blocks en masse degradation of the organelle (Figure 1B-D). In contrast, expression of an catalytically-inactive USP30 C77S mutant (Nakamura and Hirose, Mol. Biol. Cell, 19: 1903-1914 (2008)) was ineffective at preventing Parkin-mediated mitochondria degradation, supporting the idea that USP30 counteracts mitophagy through deubiquitination of mitochondrial substrates (Figure 1B-D).

Since USP30 enzymatic activity was necessary for blocking mitophagy, whether USP30 and Parkin have opposing effects on mitochondria ubiquitination was examined. As reported previously, short-term CCCP treatment (20 μM, 4 hours) caused Parkin redistribution to mitochondria (marked by Tom20) and led to accumulation of ubiquitination signal on mitochondria (measured by staining with polyubiquitin antibody FK2, Figure 2D; (Lee et al., J. Cell Biol., 189: 671-680 (2010)). When USP30 was co-expressed with Parkin, the amount of ubiquitin signal accumulated on mitochondria was reduced by ~75% - an effect that also required USP30 enzymatic activity (Figure 2D, E). These data support the idea that USP30 functions as a DUB that opposes the ubiquitin ligase action of Parkin on mitochondrial proteins, thereby inhibiting mitophagy.

Previous studies indicated that Parkin pathogenic mutants defective in ligase activity cannot support mitochondrial degradation in response to CCCP, leading to clustering of uncleared mitochondria in the perinuclear region in association with translocated Parkin (Geisler et al., Nat. Cell Biol., 12: 119-131 (2010); Lee et al., J. Cell Biol., 189: 671-680 (2010)). Remarkably, in cells co-transfected with USP30 plus Parkin and treated with CCCP, wild-type myc-Parkin behaved similarly to mutant Parkin in that it remained associated with the perinuclear clusters of non-degraded mitochondria (Figure 1B (white arrow), E). Co-expression of USP30 did not alter Parkin expression level (Figure 2F, G). These data indicate that USP30 blocks mitophagy by enzymatic removal of ubiquitin signal on damaged mitochondria, rather than by inhibiting the translocation of Parkin to mitochondria.

**Example 3: Pinkl, Parkin required for mitophagy in neurons**

To measure mitophagy in neurons, mt-Keima, a ratiometric pH-sensitive fluorescent protein that is targeted into the mitochondrial matrix, was monitored. A low ratio mt-Keima-derived fluorescence (543nm/458nm) reports neutral environment whereas a high ratio fluorescence reports acidic pH (Katayama et al., Chemistry & Biology 18: 1042-1094 (2011)). Thus mt-Keima enables differential imaging of mitochondria in the cytoplasm and mitochondria in acidic lysosomes. Because mt-Keima is resistant to lysosomal proteases, it allows for measurement of cumulative lysosomal delivery of mitochondria over time.

Following transfection in rat dissociated hippocampal cultures, mt-Keima signal accumulated initially in elongated structures characteristic of mitochondria and with low 543/458 ratio values (shown in green - Figure 3A). After 2-3 days of expression, multiple round mt-Keima structures with high ratio (acidic) signal also appeared throughout the cell body (shown in red - Figure 3A). These round mt-Keima-positive structures most likely represent lysosomes since (1) neutralizing cells with NH4Cl completely reversed the high ratio (543/458) pixels to low ratio signal specifically in these round structures without affecting the tubular-reticular mitochondrial signal (Figure 4A); (2) an independent lysosomal marker dye (lysotracker green DND-26) stained high ratio mt-Keima structures, though there were also many Lysotracker-positive structures that were not associated with mt-Keima (Figure 4B); (3) in post-hoc immunostaining experiments, high ratio pixels colocalized with endogenous lysosomal protein LAMP-1 (Figure 4C). Since almost all of the "acidic" mt-Keima signal was found in neuronal cell bodies (cell body contained 95.6 ± 2.2% of the total high ratio (543/458) signal), the ratio of the area of lysosomal (red) signal / mitochondrial (green) signal within the cell body was used as a measure of lysosomal delivery of mitochondria in neurons ("mitophagy index") (Katayama et al., Chemistry & Biology 18: 1042-1094 (2011)). As quantified by this mitophagy index, the abundance of mt-Keima in lysosomes increased over a time course of days (Figure 4D), implying active mitophagy in cultured neurons under basal conditions.

In heterologous cells, Parkin overexpression can drive mitochondrial degradation upon mitochondria depolarization; however, it is not yet established whether endogenous Parkin and PINK1 are required for mitophagy in non-neural or neural cells (Youle and Narendra, Nat. Rev. Mol. Cell Biol. 12: 9-14 (2011)). To examine the role of the PINK1/Parkin pathway in neuronal mitophagy, Parkin or PINK1 was knocked down using small hairpin RNAs (shRNAs) expressed from pSuper-based vectors. These Parkin and PINK1 shRNAs efficiently knocked down the cDNA-driven expression of their respective targets in heterologous cells (Figure 4E, F), and suppressed the protein levels of endogenous Parkin or PINK1 in neuronal cultures by ~80% and ~90%, respectively (Figure 4G, H). Compared to control luciferase shRNA, neurons transfected with Parkin shRNAs (two independent sequences) showed ~50% reduction in the mitophagy index, indicative of decreased mitochondria delivery to lysosomes (Figure 3B, C). PINK1 shRNAs were even more effective in reducing the acidic mt-Keima signal (~80-90% reduction in mitophagy index (Figure 3D, E)). Previous genetic studies placed PINK1 upstream of Parkin in maintaining healthy mitochondria (Clark et al., Nature, 441: 1162-1166 (2006); Park et al. Nature, 441: 1157-1161 (2006)). Consistent with the genetic epistasis, our mt-Keima experiments showed that PINK1 overexpression strongly enhanced mitophagy in neurons, an effect that was completely eliminated by Parkin knockdown (Figure 3F, G). On the other hand, Parkin overexpression by itself had no apparent effect on basal mitophagy, as measured by the mt-Keima assay (Figure 4I, J). Thus, neuronal mitophagy requires both PINK1 and Parkin, with PINK1 - apparently limiting - acting upstream of Parkin.

**Example 4: USP30 antagonizes mitophagy in neurons**

Next, whether USP30 suppresses mitophagy in neurons as in heterologous cells was investigated. Compared with control neurons transfected with β-Gal and mt-Keima, co-expression of wild-type USP30 caused a ~60% reduction in mitophagy index at 3 days, indicating that USP30 inhibits lysosomal delivery of mitochondria in neurons (Figure 5A, E). In contrast, overexpression of enzymatically-inactive USP30 (C77S or C77A) induced a robust increase in mitophagy signal (Figure 5A, E). The enhanced delivery of mitochondria to lysosomes likely reflects a dominant-negative action of catalytically-inactive USP30, presumably by interacting with substrates or pro-mitophagy ubiquitin chains, and sequestering them from endogenous USP30 (Berlin et al., J. Biol. Chem., 285: 34909-34921 (2010); Bomberger et al., J. Biol. Chem., 284: 18778-18789 (2009); Ogawa et al., J. Biol. Chem., 286: 41455-41465 (2011)).

To test the function of endogenous USP30, USP30 was knocked down using shRNAs. In heterologous cells, rat USP30 shRNA plasmid specifically eliminated the expression of transfected rat USP30 cDNA (Figure 5B). The same rat USP30 shRNA led to a ~85% reduction in endogenous USP30 in neuronal cultures (Figure 5C). In neurons, USP30 knockdown increased the lysosomal delivery of mt-Keima (~60% increase in mitophagy index), relative to negative control luciferase shRNA (Figure 5D, F). Co-transfection of shRNA-resistant human USP30 cDNA "rescued" this effect, i.e. it restored the brake on mitochondrial degradation, indicating that USP30 shRNA was not exerting a non-specific effect (Figure 5B, D, F). In fact, neurons co-transfected with human USP30 cDNA plus rat USP30 shRNA showed lower levels of lysosomal accumulation of mt-Keima than controls, similar to neurons overexpressing wild-type USP30 by itself (Figure 5D, F). Moreover, enzymatically-inactive human USP30 (C77S) failed to reverse the enhanced mitophagy induced

by USP30 shRNA, and actually enhanced mitophagic activity even more than USP30 shRNA (Figure 5D, F), the latter result suggesting that USP30 knockdown is incomplete. These results provide strong evidence that endogenous USP30 restrains mitophagy in neurons through its DUB activity.

**Example 5: USP30 deubiquitinates multiple mitochondrial proteins**

Since Parkin and USP30 antagonistically regulate mitochondrial degradation, it was hypothesized that this E3 ligase and DUB act on some common substrates. To identify Parkin and USP30 substrates, global ubiquitination in cells was analyzed by mass spectrometry (MS) following immunoaffinity enrichment of ubiquitinated peptides from trypsin-digested extracts using the ubiquitin branch-specific (K-GG) antibodies. Global ubiquitination was analyzed and quantified by MS in HEK-293 cells in two different sets of conditions: 1) inducible Parkin overexpression, or 2) USP30 knockdown (USP30 knockdown efficiency was 85 ± 5% (see Figure 7C)). In each set, cells were treated with CCCP (5 μM, 2 hours) or vehicle control (DMSO). In aggregate, MS analysis revealed >15,000 unique ubiquitination sites on ~3200 proteins of which a subset responded to either CCCP alone (endogenous Parkin and USP30 levels) or Parkin overexpression/USP30 knockdown (*see* Appendix A for a list of the ~3200 proteins). MS identified 233 and 335 proteins whose ubiquitination increased by parkin overexpression or USP30 knockdown, respectively (i.e. exhibited significantly more ubiquitination in 'parkin overexpression + CCCP' or 'USP30 knockdown + CCCP' vs. CCCP-alone. 41 of these proteins were regulated by both Parkin overexpression and USP30 knockdown (Figure 13). Twelve of these 41 proteins are mitochondrial or associated with mitochondria (Tom20, MIRO1, FKBP8, PTH2, MUL1, MAT2B, TOM70, PRDX3, IDE, and all three VDAC isoforms - based on Human MitoCarta database). Others included nuclear import proteins (e.g. IPO5), demethylases (e.g. KDM3B), and components of the ubiquitin/proteasome system (e.g., PSD13, UBP13) (Figure 13).

We focused additional studies on two mitochondrial proteins -- Tom20 and MIRO -- that showed large increases in ubiquitination with USP30 knockdown (USP30 shRNA + CCCP / CCCP ubiquitination ratio for Tom20 = 3.52, p = 0.005; for MIRO = 2.95, p= 0.019; see Figure 13, left). Tom20 and MIRO also showed large magnitude and highly significant increases in ubiquitination with Parkin overexpression (Figure 13, right). To confirm that USP30 can deubiquitinate these proteins, cell lines stably overexpressing GFP-Parkin were transfected with HA-ubiquitin and immunoprecipitated (IP) ubiquitinated proteins using anti-HA antibodies. Following mitochondrial depolarization (CCCP, 5 μM, 2 hours), GFP-Parkin stable cells showed robust enhanced ubiquitination of endogenous MIRO, as measured by immunoblotting for MIRO in the anti-HA-immunoprecipitates (Figure 7A). In control transfections without HA-ubiquitin, anti-HA-beads did not immunoprecipitate MIRO, indicating the specificity of MIRO ubiquitination signal (Figure 7A, left lanes). Compared to β-Gal control, cotransfection of wildtype USP30, but not DUB-dead USP30-C77S, decreased the amount of ubiquitininated MIRO by ~85% (Figure 7A, B). Similarly, wildtype USP30 overexpression reduced the ubiquitination of Tom20 (Figure 7A); whereas USP30-C77S actually increased basal Tom20 ubiquitination ~2-fold (without CCCP), and CCCP-induced ubiquitination ~8-fold, consistent with a dominant-negative mechanism (Figure 7A, C). CCCP did not induce detectable Tom20 or MIRO ubiquitination in the parental HEK-293 cell line (lacking GFP-Parkin) (Figure 6A). In this cell line, however, overexpression of USP30-C77S was still able to enhance basal Tom20 ubiquitination and USP30 to suppress it (Figure 6A). Taken together, our data indicate that MIRO and Tom20 are substrates of USP30 and that USP30 can counteract Parkin-mediated ubiquitination of both MIRO and Tom20 following mitochondria damage.

It was found that a subset of the shared substrates were regulated by USP30 even under basal conditions (exemplified by Tom20, discussed above). MUL1, ASNS and FKBP8 - but not MIRO -- were substrates that behaved similarly to Tom20; i.e. they also exhibited a basal increase in ubiquitination with USP30 knockdown in the absence of CCCP. Thus, USP30 basally deubiquitinates this set of proteins, possibly counterbalancing against a mitochondrial E3 ligase that is active in the absence of CCCP and that acts on Tom20 but not MIRO. On the other hand, proteins such as TOM70, MAT2B and PTH2 behaved similarly to MIRO in that they exhibited enhanced ubiquitination with USP30 knockdown only following CCCP, suggesting that USP30 engages in deubiquitination of these proteins only after Parkin is recruited to mitochondria. Parkin, following recruitment to damaged mitochondria, may target both Tom20 and MIRO types of USP30 substrates, shifting the balance towards their polyubiquitination.

Using the same experimental system (cells overexpressing GFP-Parkin and HA-ubiquitin), the function of endogenous USP30 was tested by shRNA suppression. USP30 knockdown did not affect basal ubiquitination of MIRO (in the absence of CCCP-induced mitochondria damage). After mitochondrial depolarization (CCCP 5 μM, 2 hours), however, and consistent with the MS experiments, USP30 knockdown increased the level of ubiquitinated MIRO ~2.5-fold, as measured in HA-ubiquitin immunoprecipitates (Figure 7D, E). Notably, USP30 knockdown increased both basal and CCCP-induced Tom20 ubiquitination, similar to enzymatically-inactive USP30 (Figure 7D, F). The increase in MIRO and Tom20 ubiquitination caused by USP30 shRNA was prevented by expression of the rat USP30 cDNA that is insensitive to human USP30 shRNA, indicating the specificity of the RNAi effect (Figure 6B). Thus, these biochemical data corroborate the MS findings that endogenous USP30 acts as a brake on ubiquitination of both Tom20 and MIRO.

Parkin has previously been shown to assemble K27-, K48- and K63-type polyubiquitin chains on various mitochondrial substrates (Geisler et al., Nat. Cell Biol., 12: 119-131 (2010)). To examine the polyubiquitin chain topology on Tom20 and Miro, we repeated the ubiquitination assays with HA-ubiquitin mutants where all seven lysine residues were individually replaced with arginine (single K-to-R mutants), or with mutants where a single lysine was left intact and all other six lysines were replaced with arginine. We compared the amount of CCCP-induced Tom20 and Miro ubiquitination afforded by these ubiquitin mutants to wild-type ubiquitin. Among all "single K-to-R mutants", only the K27R mutation blocked the CCCP-induced ubiquitination of Tom20, whereas the other K-to-R mutants (K6R, K11R, K29R, K33R, K48R, K63R) supported normal Tom20 ubiquitination (Figure 6C, D). Conversely, normal Tom20 ubiquitination was only supported by ubiquitin with K27 intact (all other lysines mutated), whereas all other single lysine mutants (K6, K11, K29, K33, K48, K63) had impaired Tom20 ubiquitination (Figure 6E, F). Thus, K27 on ubiquitin is both necessary and sufficient for building polyubiquitin chains on Tom20, suggesting the primary polyubiquitin topology on Tom20 is K27-type chains. Similar to Tom20, Miro also required K27 (and not the other lysines on ubiquitin) for its normal ubiquitination (Figure 6C, D). Although the ubiquitin mutant that contains only K27 supported Miro ubiquitination the best, significantly less ubiquitin was attached on Miro as compared to wild-type ubiquitin (~65% of wild-type ubiquitin), suggesting that Miro accumulates other chain-types in addition to K27 (Figure 6E, F). Our data are consistent with Parkin's ability to assembly K27-linked chains on other substrates (Geisler et al., Nat. Cell Biol., 12: 119-131 (2010)).

Beyond ubiquitination, does USP30 regulate protein turnover in addition to ubiquitination? Published evidence suggests that Parkin mediates the degradation of multiple mitochondrial outer membrane proteins (Chan et al., Human Mol. Genet., 20: 1726-1737 (2011)). Consistent with this, all of the several outer membrane proteins examined (MIRO, MFN-1, TOM70, VDAC, Tom20) showed significant drop in protein level during the 6 hours of CCCP treatment (5 $\mu$M) of GFP-Parkin stable cell lines (Figure 6G, H). Tom20 levels were also reduced but to a lesser extent than MIRO and other outer membrane proteins (10 +/- 1% decrease with CCCP at t = 6h, $p < 0.01$) (Figure 6G, H). In contrast to the outer membrane proteins, mitochondrial matrix protein HSP60 and inner membrane protein TIMM8A were unchanged by CCCP within this time frame. Overexpression of USP30 in GFP-Parkin stable cells greatly attenuated or abolished the CCCP-induced depletion of MIRO and Tom20 (Figure 6G, H). Stabilization by USP30 overexpression appeared to be relatively specific for MIRO and Tom20, since CCCP-induced degradation of other mitochondria membrane proteins (MFN-1, TOM70, VDAC) was unaffected (Figure 6G, H). Unlike wildtype USP30, the inactive C77A- or C77S-USP30 mutants did not inhibit degradation of MIRO or Tom20 induced by CCCP, implying requirement for DUB activity (Figure 6G, H). These data indicate USP30 can specifically counteract degradation of MIRO and Tom20 without affecting the turnover of other mitochondrial proteins.

Since MIRO and Tom20 degradation accompanies mitophagy (Figure 6G, H and (Chan et al., Human Mol. Genet., 20: 1726-1737 (2011)) and USP30 knockdown enhances mitophagy (Figure 5C, E) and ubiquitination of MIRO and Tom20 (Figure 7), it was speculated that the depletion of these proteins might trigger mitophagy. In this model, overexpression of these proteins would block mitophagy induced by USP30-knockdown. Instead, it was found that overexpression of MIRO or Tom20 in neurons - even by themselves -- led to a robust increase in mitophagy in the mt-Keima assay (Figure 8B, C, D, E), an effect similar to USP30 knockdown. It was therefore hypothesized that it is the ubiquitination of MIRO and Tom20 that serves as the signal for mitophagy (rather than their degradation, which occurs secondary to ubiquitination), and that overexpression of MIRO and Tom20 promotes mitophagy by increasing the pool of these substrates available for ubiquitination.

MS analysis of ubiquitinated peptides derived from Tom20 identified 3 lysine residues (K56, K61 and K68) whose ubiquitination increased upon CCCP or USP30 knockdown, and that increased even further in response to the combination of CCCP treatment + USP30 knockdown (Figure 9). To confirm ubiquitination on these particular sites, the three lysine residues in Tom20 were mutated to arginine ("3KR-Tom20" (K56R, K61R, K68R mutant)). In GFP-Parkin overexpressing cells, myc-tagged wildtype Tom20 exhibited an increase in ubiquitination with coexpression of enzymatically-inactive USP30-C77S (Figure 8A), similar to endogenous Tom20 (Figure 7A, C). In contrast, 3KR-Tom20 showed less basal ubiquitination than wild-type Tom20 and additionally it was unaffected by the dominant negative USP30-C77S (Figure 8A), indicating that these three lysine residues are the major USP30 target residues on Tom20.

In the mt-Keima assay in neurons, overexpression of wild-type Tom20 enhanced mitophagy, whereas the 3KR-Tom20 mutant failed to do so (Figure 8B, D); thus Tom20 is sufficient to drive mitophagy, but this ability depends on its ubiquitination. Moreover, 3KR-Tom20 blocked the increase in mitophagy induced by USP30-C77S (Figure 8B, D), implying that the increased mitophagic flux caused by dominant-negative USP30 requires Tom20 ubiquitination. Alternatively, overexpressed 3KR-Tom20 may be able to oppose USP30-C77S-induced mitophagy by physically associating with USP30 in a non-catalytic manner.

Mass spectrometry identified nine lysine-ubiquitination sites on MIRO regulated by USP30 and Parkin, some of which are known to be required for normal MIRO function (e.g. K427 required for GTPase activity (Fransson et al., Bioch. Biophys. Res. Comm., 344: 500-510 (2006)). Thus, instead of a pursuing a combinatorial mutagenesis, the effect of USP30 on MIRO's ability to induce mitophagy was studied since USP30 knockdown increases MIRO ubiquitination (Figure 7D, E). Consistent with the idea that MIRO ubiquitination drives mitophagy, USP30 knockdown further enhanced mitophagy beyond what was observed following MIRO overexpression alone (Figure 8C, E). Taken together, these data

indicate that ubiquitination of MIRO or Tom20 can drive mitophagy in neurons, and that inhibition of mitophagy by USP30 can be explained at least in part by deubiquitination of these proteins.

**Example 6: USP30 knockdown rescues mitophagy defect associated with PD mutations**

If mitochondrial degradation defects associated with PD-linked mutations of Parkin are due to impaired ubiquitination of damaged mitochondria, and USP30 indeed functions as a biochemical and functional antagonist of Parkin, then inhibiting USP30 should restore mitochondria ubiquitination and degradation. To test this hypothesis, we focused on PD-linked Parkin pathogenic mutants, such as G430D and K161N, that display attenuated ligase activity (Sriram et al., Human Mol. Genet., 14: 2571-2586 (2005)) with accompanying defects in mitophagy (Geisler et al., Nat. Cell Biol., 12: 119-131 (2010); Lee et al., J. Cell Biol., 189: 671-680 (2010)) were studied (e.g. G430D and K161N).

In SH-SY5Y cells transfected with pathogenic mutant GFP-Parkin-G430D and treated with CCCP, mitochondria fail to be cleared and form perinuclear clusters in association with the defective Parkin protein (Figure 10A, first column). The same cells doubly transfected with Parkin-G430D and USP30 siRNA, which led to knockdown of USP30 protein by ~60% (Figure 11A), showed a 60% decrease in mitochondria (as measured by total Tom20 fluorescence) compared to cells transfected with Parkin-G430D and control siRNA (Figure 10A, quantified in B). This result shows that siRNA knockdown of USP30 protein level can largely rescue mitophagy in the face of defective Parkin. Mitochondria degradation was not rescued by knockdown of other DUBs (USP6, USP14) (Figure 11B-D). Re-introduction of an RNAi-resistant wildtype USP30 (rat USP30 cDNA), but not the inactive rat USP30-C77S mutant, prevented the rescue of mitochondrial degradation by USP30 siRNA (Figure 10A, B). Rescue of mitochondrial degradation was correlated with loss of perinuclear clusters of mutant G430D Parkin (usually associated with mitochondria) and appearance of smaller dispersed Parkin-containing puncta throughout the cytoplasm (Figure 10A, C; see also Figure 11B, C). In CCCP-treated GFP-Parkin-G430D expressing cells, USP30 knockdown not only led to loss of Tom20 immunoreactivity but also decreased staining for matrix protein HSP60 suggesting that USP30 suppression restored degradation of the whole mitochondrion (Figure 11E, G). The mitochondrial degradation defect associated with another PD-associated Parkin mutant (K161N) was similarly rescued with USP30 siRNA knockdown (Figure 11F, H). In neurons, reduced mitophagy associated with Parkin knockdown (as measured in the mt-Keima assay) was also rescued with dominant-negative USP30-C77A (Figure 10D, E). Thus, suppressing the expression or activity of USP30 allows cells to overcome defective Parkin or Parkin knockdown and restore the clearance of damaged mitochondria.

While not intending to be bound by any particular theory, since Parkin ligase activity marks mitochondria through ubiquitination, some residual ligase activity present in Parkin mutants may be needed in order for USP30 knockdown to rescue mitophagy. It is currently unknown whether USP30 knockdown would be effective with complete loss of Parkin activity. It is possible, however that there are other E3s that have overlapping substrates or that can compensate for lack of Parkin.

**Example 7: USP30 is a Parkin substrate**

Since Parkin has broad activity towards outer mitochondrial membrane proteins, we wondered whether Parkin ubiquitinates USP30, which also resides at this mitochondrial compartment. Supporting this possibility, we identified USP30-derived ubiquitinated peptides in proteomics experiments in GFP-Parkin expressing cells treated with CCCP (fold change in ubiquitination of USP30 in 'GFP-Parkin + CCCP' over 'DMSO' = 27.23, $p < 0.001$). To confirm USP30 ubiquitination by Parkin, we repeated the ubiquitination assay in cells overexpressing GFP-Parkin and HA-ubiquitin, and found GFP-Parkin induced ubiquitination of endogenous USP30 following CCCP treatment (20 $\mu$M, 2 hours, Figure 9C, D). The ubiquitination sites of USP30 (K235 and K289) identified by mass spectrometry were not required for its ubiquitination suggesting other lysine residues in USP30 can accept ubiquitin (data not shown). CCCP treatment (20 $\mu$M) also induced a significant drop in USP30 levels in GFP-Parkin expressing cells (Figure 9E, F). Importantly, Parkin with pathogenic mutations G430D or K161N were not able to ubiquitinate (Figure 9C, D) or degrade USP30 (Figure 9E-F). These data indicate that Parkin ubiquitinates and degrades USP30, thus removing the brake on mitophagy.

**Example 8: USP30 knockdown decreases oxidative stress and provides protection *in vivo***

Whether USP30 knockdown provides functional benefit to mitochondria and cells was examined next. ROS - which largely derive from mitochondria - is associated with neurodegenerative disorders and mitochondria dysfunction may contribute to increased oxidative stress in PD (Lee et al., Biochem. J., 441: 523-540 (2012)). To measure oxidative stress in mitochondria, mitochondria matrix-targeted ro-GFP (mito-roGFP), a redox-sensitive fluorescent protein that allows quantitative ratiometric imaging of mitochondrial redox potential was used (Dooley et al., J. Biol. Chem. 279: 22284-22293 (2004)). Following measurement of ratiometric mito-roGFP signal in individual cells, the dynamic range of the probe was calibrated by treating cultures sequentially with DTT (1 mM) to fully reduce the probe and aldrithiol

(100 μM) to fully oxidize the probe (Guzman et al., Nature, 468: 696-700 (2010). Ratios of mito-roGFP measured after DTT and aldrithiol were set to 0 and 1, respectively, to calibrate the relative oxidation index. In control cells transfected with control luciferase shRNA, neurons had a mean relative oxidation index of ~0.6 (Figure 17A, B). USP30 knockdown dropped the relative oxidation index to ~0.4, suggesting that suppression of USP30 protein led to a reduction in mitochondrial oxidative stress.

To test whether knocking down USP30 would provide protection under stress conditions *in vivo,* we used *Drosophila,* which has emerged as an effective model system for studying PD molecular pathogenesis (Guo, Cold Spring Harb. Perspect. Med. 2(11) pii: a009944 (2012)). To knock down fly USP30 (CG3016, hereafter called dUSP30), we employed the GAL4/UAS system (Brand et al., Development, 118: 401-415 (1993)). We crossed an *Actin-GAL4* driver line with a *UAS-dUSP30$^{RNAi}$* transgenic line, which allows expression of dUSP30 RNAi under the control of the Actin promoter (this *Actin-GAL4 > dUSP30$^{RNAi}$* line is referred to as 'dUSP30 knockdown' line). Activation of *UAS-dUSP30$^{RNAi}$* by *Actin-GAL4* led to a ~90% reduction of dUSP30 mRNA by quantitative RT-PCR, compared to control parental lines containing only *Actin-GAL4* or only *UAS-dUSP30$^{RNAi}$* (Figure 17C). To test the protective effects of dUSP30 knockdown, we crossed the 'dUSP30 knockdown' line with *parkin* mutant flies (*park$^{25}$*) (Greene et al., Proc. Natl. Acad. Sci. USA, 100: 4078-4083 (2003)). Flies lacking *parkin* show severe defects in mitochondrial morphology in their indirect flight muscles (IFMs), with mitochondria that are malformed with sparse, disorganized cristae, giving rise to a "pale" appearance of mitochondria under EM (Figure 12A, and Greene et al., Proc. Natl. Acad. Sci. USA, 100: 4078-4083 (2003)). In contrast, wild-type flies have many dark-staining mitochondria evenly packed with cristae (Figure 12A). To determine the effect of USP30 inhibition on Parkin-deficient mitochondria, we crossed parkin mutants to dUSP30 knockdown flies. In the "parkin mutant; dUSP30 knockdown" flies, most of the IFM mitochondria were electron-dense and contained numerous cristae, although "pale" mitochondria with fragmented cristae were also occasionally found (Figure 12A). Quantification of the percent area of mitochondria containing disorganized cristae over total mitochondria area revealed a strong improvement in mitochondrial integrity with dUSP30 knockdown (Figure 12B - ~90% in parkin mutants versus ~25% in "parkin mutant; dUSP30 knockdown"). "Parkin mutant; dUSP30 knockdown" flies also had less damaged mitochondria per muscle area (Figure 12C). Thus, suppressing dUSP30 expression is able to largely restore morphological mitochondrial integrity *in vivo* in parkin-deficient Drosophila.

To examine the effect of suppressing USP30 in neurons that are relevant to PD, we used dopamine decarboxylase (Ddc)-GAL439 to drive dUSP30$^{RNAi}$ specifically in aminergic neurons of the fly nervous system. As a model of mitochondrial damage and PD, we treated flies with paraquat, a mitochondrial toxin linked to PD (Castello et al., J. Biol. Chem., 282: 14186-14193 (2007); Cocheme et al., J. Biol. Chem., 283: 1786-1798 (2008); Tanner et al., Environmental Health Perspect., 119: 866-872 (2011)). Following treatment with paraquat (10 mM, 48 hours), both the *Ddc-GAL4* and *UAS-dUSP30$^{RNAi}$* control fly lines showed reduced ability to climb up beyond 15 cm (Figure 12D). This climbing defect was fully rescued by additional treatment with L-DOPA (Figure 17D), showing that this behavioral deficit is likely due to depletion of dopamine. Consistently, in control fly lines (*Ddc-GAL4* or *UAS-dUSP30$^{RNAi}$* transgenics alone), paraquat treatment (10 mM, 48 hours) caused a 30-60% reduction in dopamine levels in fly heads without altering serotonin neurotransmitter levels (indicating specific toxicity of paraquat on the dopaminergic system in this model - Figure 12F and Figure 17E). Similar to L-DOPA, dUSP30 knockdown in *Ddc-GAL4 > UAS-dUSP30$^{RNAi}$* flies also completely rescued the paraquat-induced climbing impairment (Figure 12D), indicating that complete protection against paraquat toxicity in this behavioral test can be afforded by suppression of USP30 specifically in aminergic neurons. A similar complete protection was also observed with whole body knockdown of USP30 in *Actin-GAL4 > UAS-dUSP30$^{RNAi}$* flies (Figure 12E). Strikingly, USP30 knockdown in Ddc neurons (*Ddc-GAL4 > UAS-dUSP30$^{RNAi}$* flies) also prevented the paraquat-induced dopamine depletion (Figure 12F). Since USP30 knockdown rescued both depletion of dopamine and motor impairment, these results show that suppression of USP30 can benefit dopaminergic neurons and the organism in both neurochemical and functional terms.

To test whether USP30 knockdown has an effect on organism survival, we monitored the percentage of live flies over prolonged treatment with paraquat (10 mM, 96 hours). Flies expressing dUSP30 RNAi survived significantly longer than controls (Figure 12G). Only <15% of flies treated with paraquat were alive in *Actin-GAL4* and *UAS-dUSP30$^{RNAi}$* control groups at 96 hours whereas ~45% of flies were alive in the *Actin-GAL4> UAS-dUSP30$^{RNAi}$* ('whole body dUSP30 knockdown') group (Figure 12G). We confirmed that the benefit of USP30 knockdown was not due to differences in exposure to paraquat since all three fly lines ingested roughly equal amounts of paraquat as measured by LC-MS/MS (average mass of paraquat per fly: *UAS-dUSP30$^{RNAi}$*: 3.2 μg, *Actin-GAL4*: 2.7 μg, *Actin-GAL4 >UAS-dUSP30$^{RNAi}$*:2.7 μg). Knockdown of other DUBs in flies (dUSP47 (CG5486) or dYOD1 (CG4603)) did not provide benefit in the survival assay; if anything, they exacerbated the rate of death in response to paraquat (Figure 17F-H). Furthermore, introduction of a human USP30 cDNA into flies expressing *dUSP30$^{RNAi}$* reversed the survival benefit provided by *dUSP30$^{RNAi}$* (Figure 17I), demonstrating the specificity of the RNAi effect. Remarkably, USP30 knockdown specifically in Ddc neurons was sufficient to provide significant survival benefit, albeit less than the whole body USP30 knockdown (Figure 12H). This result implies that a significant portion of the organismal benefit of USP30 suppression is mediated in dopaminergic neurons, and it further reinforces the idea that USP30 plays a critical role in dopaminergic neuron

dysfunction.

**Example 9: Discussion**

Better understanding of the pathogenic mechanisms in PD would be helpful for rational design of disease-modifying therapies for this neurodegenerative disease. Impaired activity of oxidative phosphorylation enzymes (Schapira et al., Lancet, 1: 1269 (1989)), elevated levels of oxidative stress markers (Lee et al., Biochem. J., 441: 523-540 (2012)) and mtDNA mutations (Bender et al., Nature Genet., 38: 515-517 (2006); Kraytsberg et al., Nature Genet., 38: 518-520 (2006)) in PD suggest accumulation of defective mitochondria (Zheng et al., Science Transl. Med., 2: 52ra73 (2010)). PINK1/Parkin genetics further implicate aberrant mitochondrial biology and point to impaired mitochondrial quality control as a causative factor in the etiology of PD (Youle et al., J. Biol. Chem., 12: 9-23 (2011)). Uncleared damaged mitochondria can be a source of toxicity and "pollute" the mitochondrial network through fusion with healthy mitochondria (Tanaka et al., J. Cell. Biol., 191: 1367-1380 (2010)).

We have identified USP30, a DUB localized to mitochondria, as a negative regulator of mitophagy. USP30, through its deubiquitinase activity, opposes Parkin-mediated ubiquitination and degradation of mitochondrial proteins and reverses the marking of damaged mitochondria for mitophagy. Knockdown inhibition of USP30 accelerated mitophagy, and it restored CCCP-induced mitochondrial degradation in cells expressing PD-associated mutants of Parkin. USP30 knockdown improves mitochondrial integrity in parkin mutant flies, confirming that Parkin and USP30 have opposing actions on mitochondrial quality in vivo. USP30 knockdown also conferred motor behavior and survival benefits in wildtype flies treated with paraquat, further supporting the idea that USP30 inhibition might ameliorate the effects of mitochondrial damage.

<u>Parkin, USP30 and mitochondrial quality control</u>

Although Parkin and PINK1 are identified as key players in mitophagy, a detailed mechanistic understanding of the mitophagy pathway, especially in mammalian cells, is lacking. The fact that basal mitophagy in neurons depends on Parkin and PINK1 (Figure 3) suggests that these proteins actively monitor normally occurring mitochondrial damage. Mitochondria fission - which appears to be required for Parkin-mediated mitophagy (Tanaka et al., J. Cell. Biol., 191: 1367-1380 (2010)) - may contribute to basal mitochondrial turnover by eliciting a transient drop in membrane potential in one of the two daughter mitochondria (Twig et al., EMBO J., 27: 433-446 (2008)). This transient drop in membrane potential creates an opportunity for PINK1 accumulation and Parkin recruitment, leading to eventual mitophagy if membrane potential is not quickly re-established. Thus under basal conditions, USP30 knockdown may accelerate mitophagy by favoring Parkin-mediated ubiquitination during the fission-associated drops in mitochondrial membrane potential. As damaged mitochondria are more likely to accumulate Parkin, it is expected that suppression of USP30 function will preferentially clear unhealthy mitochondria.

Since Parkin ligase activity marks mitochondria through ubiquitination, some residual ligase activity present in Parkin mutants is presumably required in order for USP30 knockdown to rescue mitophagy. It would be expected that with complete loss of Parkin activity, USP30 knockdown would be ineffective at rescuing clearance unless other E3 ligases have overlapping substrates and can compensate for lack of Parkin. The rescue of mitochondrial integrity with USP30 knockdown in parkin mutant flies, even though a large portion of parkin gene is missing, supports the latter possibility.

As part of normal turnover, the cleared mitochondria presumably need to be replaced through mitochondrial biogenesis. In culture cell lines, mitochondrial damage increases overall mitochondrial mass (Narendra et al., PLoS Biology, 8: e1000298 (2010)). In this context it is interesting to note that Parkin can also boost mitochondrial biogenesis by degrading negative transcriptional regulators (Shin et al., Cell 144: 689-702 (2011)). Further studies are required to determine whether USP30 also regulates the biogenesis pathway and whether mitophagy induced by USP30 inhibition is accompanied by new mitochondria production.

<u>USP30 versus Parkin on common substrates</u>

Global ubiquitination site mapping experiments identified multiple substrates whose ubiquitination is affected by both Parkin overexpression and USP30 knockdown. Amongst these shared presumptive substrates, we confirmed that Miro and Tom20 have ubiquitination levels that are antagonistically regulated by Parkin and USP30, i.e. GFP-Parkin overexpression or USP30 knockdown increased ubiquitination induced by CCCP treatment. Interestingly, a subset of these shared substrates, exemplified by Tom20, was regulated by USP30 even under basal conditions. Mull, Asns and Fkbp8 - but not Miro - were mitochondrial substrates that behaved similarly to Tom20, exhibiting a basal increase in ubiquitination with USP30 knockdown in the absence of CCCP. Thus, USP30 basally deubiquitinates this set of proteins, presumably by counterbalancing against a mitochondrial E3 ligase that is active in the absence of CCCP and that acts

on Tom20 but not Miro. Following CCCP, USP30 also counteracts Parkin dependent ubiquitination of this set of substrates. On the other hand, proteins such as Tom70, Mat2b and Pth2, behaved similarly to Miro in that they exhibited enhanced ubiquitination with USP30 knockdown only following CCCP. This observation suggests that these set of proteins undergo low levels of basal ubiquitination in the absence of recruited Parkin (i.e. Parkin is their major E3 ligase), or that USP30 is inactive toward those proteins under basal conditions. Mitochondrial depolarization regulates Parkin's E3 ligase activity (Matsuda et al., J. Cell Biol., 189: 211-221 (2010)) possibly via PINK1-mediated phosphorylation (Kondapalli et al., Open Biology, 2: 120080 (2012); *but see* Vives-Bauza et al., Proc. Natl. Acad. Sci. USA, 107: 378-383 (2010)); it remains to be studied whether the activity of USP30 - which is constitutively localized on mitochondria - is also regulated by mitochondrial damage.

Global ubiquitination analysis also revealed a series of non-mitochondrial proteins whose ubiquitination was inversely regulated by Parkin and USP30 (including nuclear proteins, metabolic enzymes and components of the ubiquitin-proteasome system (UPS)). This suggests that the antagonistic functional relationship between Parkin and USP30 may extend beyond mitochondria. These non-mitochondrial "substrates" may be indirectly regulated by Parkin and USP30, or may have subpopulations on mitochondria, but have not formally been assigned as having mitochondrial localization due to the strict filtering criteria employed by computational tools (Pagliarini et al., Cell, 134: 112-123 (2008)). MS also identified some proteins that showed enhanced ubiquitination with CCCP (under endogenous Parkin and USP30 levels) with no further increase in ubiquitination upon Parkin overexpression or USP30 knockdown (e.g. Ssbp, ZO1, Rab10, Vamp1), suggesting engagement of alternative UPS pathways following mitochondrial depolarization. It is appropriate to note that in some cases, elevated levels of ubiquitinated species can derive from increases in the level of the total protein substrate itself.

Interestingly, Parkin also ubiquitinates and degrades USP30, and pathogenic Parkin mutations blocked this ability to downregulate USP30. Failure to remove a negative regulator of mitophagy may exacerbate the inefficient ubiquitination associated with these Parkin mutants, and could also partly explain the rescue of mitophagy by USP30 knockdown.

Parkin, USP30 and Neurodegeneration

PD-linked mutations in Parkin may lead to decreased catalytic activity, enhanced aggregation and/or reduced expression (Hampe et al., Human Mol. Genet., 15: 2059-2075 (2006); Matsuda et al., J. Biol. Chem., 281: 3204-3209 (2006); Wang et al., J. Neurochem., 93: 422-431 (2005); Winklhofer et al., J. Biol. Chem. 278: 47199-47208 (2003)). In sporadic PD, Parkin activity can be also inhibited due to cellular stress (Corti et al., Physiol. Rev., 91: 1161-1218 (2011)). PD-linked PINK1 mutations impair translocation of Parkin to damaged mitochondria (Matsuda et al., J. Cell Biol., 189: 211-221 (2010); Narendra et al., PLoS Biology, 8:e1000298 (2010); Vives-Bauza et al., Proc. Natl. Acad. Sci. USA, 107: 378-383 (2010)). Thus, reduced function of Parkin in mitochondrial quality control is likely more prevalent in PD than as represented by rare Parkin mutations, providing further support for a possible utility of USP30 inhibition in idiopathic PD. Consistent with a benefit of USP30 inhibition, knockdown of USP30 restores mitophagy in cells expressing PD-associated mutant Parkin and reduces oxidative stress in neurons. In Drosophila parkin mutants, knockdown of USP30 improves mitochondrial integrity. Furthermore, USP30 knockdown provides a benefit in behavior and survival assays against paraquat, an oxidative stressor linked to mitochondria (Castello et al., J. Biol. Chem., 282: 14186-14193 (2007); Cocheme et al., J. Biol. Chem., 283: 1786-1798 (2008)). Since paraquat is a mitochondrial poison epidemiologically linked to PD (Tanner et al., Environmental Health Perspect., 119: 866-872 (2011)), our findings provide in vivo evidence that inhibition of USP30 might be helpful in diseases caused by mitochondrial damage and dysfunction.

In PD, mitochondrial dysfunction is not specific to substantia nigra neurons and is present systemically (Schapira et al, Parkinson's Dis., 2011: 159160 (2011)). Since USP30 expression seems to be widespread (Nakamura and Hirose, Mol. Biol. Cell, 19: 1903-1914 (2008)), USP30 inhibition has the potential to provide wide benefit by promoting clearance of damaged mitochondria. In addition to neurons, long-lived metabolically active cells such as cardiomyocytes also rely on an efficient mitochondrial quality control system (Gottlieb et al., Am. J. Physiol. Cell Physiol., 299: C203-210 (2010)). In this context, Parkin has been shown to protect cardiac myocytes against ischemia/reperfusion injury through activating mitophagy and clearing damaged mitochondria in response to ischemic stress (Huang et al., PLoS One, 6: e20975 (2011)). In inherited mitochondrial diseases, mtDNA mutations co-exist with wildtype mtDNA within the same cells, and mitochondrial dysfunction and disease ensue only when the proportion of mutated mtDNAs is high (Bayona-Bafaluy et al., Proc. Natl. Acad. Sci. USA, 102: 14392-14397 (2005)). Interestingly, Parkin overexpression eliminates mitochondria with deleterious mtDNA mutations and restores mitochondrial function, presumably by degrading mitochondria containing mutant mtDNA (Suen et al., Proc. Natl. Acad. Sci. USA, 107: 11835-11840 (2010)). Thus, USP30 inhibition has the potential to benefit diseases beyond PD by enhancing mitochondrial quality.

**Example 10: Peptide inhibitors of USP30**

Two types of phage-displayed naïve peptide libraries, Linear-lib and Cyclic-lib, were cycled through rounds of binding selections with biotinylated USP30_cd (USP30 catalytic domain with C77A mutation) in solution as described previously (Stanger, et al., 2012, Nat. Chem. Bio., 7: 655-660). The selection identified peptide USP30_3 and USP30_8, which had moderate spot ELISA signals (signal/noise ratios of ~5). USP_30 and USP_8 have the sequences:

USP30_3: PLYCFYDLTYGYLCFY (SEQ ID NO: 1);
USP30_8: VSRCYIFWNEMFCDVE (SEQ ID NO: 2).

USP30_3 and USP30_8 were then assayed for inhibition of USP30 and also inhibition of USP7, USP5, UCHL3, and USP2, to determine each peptide's specificity for USP30, as follows. USP30 peptide ligands at a concentration range of 0-100 μM (for USP30_3) and 0-500 μM (for USP30_8) were mixed with 250 nM ubiquitin-AMC (Boston Biochem., Boston, MA; Cat. No. U-550). A panel of DUBs at 5.6, 2, 2, 5 and 0.05 nM for USP30, USP7, USP2, USP5, and UCHL3, respectively, in PBS buffer containing 0.05% Tween20, 0.1% BSA and ImM DTT for 30 minutes were added to the ubiquitin-AMC/USP30 peptide ligands mixture and the initial velocity was immediately measured by monitoring fluorescence (excitation at 340nm and emission at 465nm) using SpectraMax®M5e (Molecular Device, Sunnyvale, CA). The initial rates were calculated based on slopes of increasing fluorescence signal. The velocity was normalized to the percentage of the rate when the peptide ligand concentration was zero, and the data was processed using KaleidaGraph by fitting to the following equation:

$$V = V_0 + \frac{V_{\max} - V_0}{1 + \left(\frac{I}{IC_{50}}\right)^n}$$

in which $v$ is the percentage of maximum rate; $I$ is the concentration of inhibitor (USP30 peptide ligands); $v_0$ and $v_{max}$ are minimum and maximum percentage of the rate, respectively.

The results of that experiment are shown in Figure 14. Peptide USP30_3 showed good specificity for USP30, with an IC50 of 8.0 μM. The IC50 of peptide USP30_3 for USP5 was more than 6-fold higher, at 49.4 μM, and for USP2 was more than 10-fold higher, at about 100 μM. The IC50 of peptide USP30_3 for UCHL3 and USP2 was > 200 μM.

Peptide USP30_3 was selected for affinity maturation. To improve the affinity, a soft randomized library was constructed using the USP30_3 sequence as the targeted parent, and panned against USP30_cd in solution as described previously (Stanger, et al., 2012, Nat. Chem. Bio., 7: 655-660). After four rounds of solution panning, 20 peptides were identified that bound to USP30 catalytic domain with stronger spot phage ELISA signal than the parent USP30_3, manifested by an improvement in the signal/noise ratio of about 3-6 fold.

Figure 15 shows a graph of residue probability by position in USP30_3 and the affinity matured peptides. The sequences for certain affinity matured peptides are shown below the graph, along with the signal to noise ratio ("S/N"), which is the ratio of the spot phage ELISA signal ("signal") detected against biotinylated USP30 cd captured on NeutrAvidin-coated 384 well Maxisorb plates versus the ELISA signal against the NeutrAvidin-coated plate alone. Figure 15 also shows the number of occurrences of each sequence in the selection ("n"), the total number of clones ("Total"; 66), and the number of unique sequences ("Uniq"; 20). All of the affinity matured peptides shown had signal to noise ratios of greater than 10 except for USP30_3.27 and USP30_3.62.

Certain peptides were then tested for specificity for USP30 versus other deubiquitinating enzymes. Binding was tested by ELISA. Figure 16 shows the signal to noise ratio ("s/n ratio") for binding of parent USP30_3 peptide and affinity matured peptides USP30_3.2, USP30_3.23, USP30_3.65, and USP30_3.88 to the catalytic domains of USP2, USP7, USP14, and USP30 (each with the active site cysteine mutated to alanine), and to the catalytic domains of UCHL1, UCHL3, and UCHL5. For the set of bar graphs for each peptide, the targets tested, from left to right, were USP2, USP7, USP14, USP30, UCHL1, UCHL3, and UCHL5.

Table 4: Table of Sequences

| SEQ ID NO | Description | Sequence |
|---|---|---|
| 26 | Human ubiquitin-specific peptidase 30 (USP30); SwissProt Q70CQ3.1 | MLSSRAEAAM TAADRAIQRF LRTGAAVRYK VMKNWGVIGG IAAALAAGIY VIWGPITERK KRRKGLVPGL VNLGNTCFMN SLLQGLSACP AFIRWLEEFT SQYSRDQKEP PSHQYLSLTL LHLLKALSCQ EVTDDEVLDA SCLLDVLRMY RWQISSFEEQ DAHELFHVIT SSLEDERDRQ PRVTHLFDVH SLEQQSEITP KQITCRTRGS PHPTSNHWKS QHPFHGRLTS NMVCKHCEHQ SPVRFDTFDS LSLSIPAATW GHPLTLDHCL HHFISSESVR DVVCDNCTKI EAKGTLNGEK VEHQRTTFVK QLKLGKLPQC LCIHLQRLSW SSHGTPLKRH EHVQFNEFLM MDIYKYHLLG HKPSQHNPKL NKNPGPTLEL QDGPGAPTPV LNQPGAPKTQ IFMNGACSPS LLPTLSAPMP FPLPVVPDYS SSTYLFRLMA VVVHHGDMHS GHFVTYRRSP PSARNPLSTS NQWLWVSDDT VRKASLQEVL SSSAYLLFYE RVLSRMQHQS QECKSEE |
| 27 | Human mitochondrial import receptor subunit 20 homolog (Tom20); GenBank NP_055580.1 | MVGRNSAIAA GVCGALFIGY CIYFDRKRRS DPNFKNRLRE RRKKQKLAKE RAGLSKLPDL KDAEAVQKFF LEEIQLGEEL LAQGEYEKGV DHLTNAIAVC GQPQQLLQVL QQTLPPPVFQ MLLTKLPTIS QRIVSAQSLA EDDVE |
| 28 | Human MIRO1; SwissProt Q8IXI2.2 | MKKDVRILLV GEPRVGKTSL IMSLVSEEFP EEVPPRAEEI TIPADVTPER VPTHIVDYSE AEQSDEQLHQ EISQANVICI VYAVNNKHSI DKVTSRWIPL INERTDKDSR LPLILVGNKS DLVEYSSMET ILPIMNQYTE IETCVECSAK NLKNISELFY YAQKAVLHPT GPLYCPEEKE MKPACIKALT RIFKISDQDN DGTLNDAELN FFQRICFNTP LAPQALEDVK NVVRKHISDG VADSGLTLKG FLFLHTLFIQ RGRHETTWTV LRRFGYDDDL DLTPEYLFPL LKIPPDCTTE LNHHAYLFLQ STFDKHDLDR DCALSPDELK DLFKVFPYIP WGPDVNNTVC TNERGWITYQ GFLSQWTLTT YLDVQRCLEY LGYLGYSILT EQESQASAVT VTRDKKIDLQ KKQTQRNVFR CNVIGVKNCG KSGVLQALLG RNLMRQKKIR EDHKSYYAIN TVYVYGQEKY LLLHDISESE FLTEAEIICD VVCLVYDVSN PKSFEYCARI FKQHFMDSRI PCLIVAAKSD LHEVKQEYSI SPTDFCRKHK MPPPQAFTCN TADAPSKDIF VKLTTMAMYP HVTQADLKSS TFWLRASFGA TVFAVLGFAM YKALLKQR |
| 29 | Human Parkin; GenBank NP_ 004553.2 | MIVFVRFNSS HGFPVEVDSD TSIFQLKEVV AKRQGVPADQ LRVIFAGKEL RNDWTVQNCD LDQQSIVHIV QRPWRKGQEM NATGGDDPRN AAGGCEREPQ SLTRVDLSSS VLPGDSVGLA VILHTDSRKD SPPAGSPAGR SIYNSFYVYC KGPCQRVQPG KLRVQCSTCR QATLTLTQGP SCWDDVLIPN RMSGECQSPH CPGTSAEFFF KCGAHPTSDK ETSVALHLIA TNSRNITCIT CTDVRSPVLV FQCNSRHVIC LDCFHLYCVT RLNDRQFVHD PQLGYSLPCV AGCPNSLIKE LHHFRILGEE QYNRYQQYGA EECVLQMGGV LCPRPGCGAG LLPEPDQRKV TCEGGNGLGC GFAFCRECKE AYHEGECSAV FEASGTTTQA YRVDERAAEQ ARWEAASKET IKKTTKPCPR CHVPVEKNGG CMHMKCPQPQ CRLEWCWNCG CEWNRVCMGD HWFDV |
| 30 | Human PINK1; SwissProt Q9BXM7.1 | MAVRQALGRG LQLGRALLLR FTGKPGRAYG LGRPGPAAGC VRGERPGWAA GPGAEPRRVG LGLPNRLRFF RQSVAGLAAR LQRQFVVRAW GCAGPCGRAV FLAFGLGLGL IEEKQAESRR AVSACQEIQA IFTQKSKPGP DPLDTRRLQG FRLEEYLIGQ SIGKGCSAAV YEATMPTLPQ NLEVTKSTGL LPGRGPGTSA PGEGQERAPG APAFPLAIKM MWNISAGSSS EAILNTMSQE LVPASRVALA GEYGAVTYRK SKRGPKQLAP HPNIIRVLRA FTSSVPLLPG ALVDYPDVLP SRLHPEGLGH GRTLFLVMKN YPCTLRQYLC VNTPSPRLAA MMLLQLLEGV DHLVQQGIAH RDLKSDNILV ELDPDGCPWL VIADFGCCLA DESIGLQLPF |
| | | SSWYVDRGGN GCLMAPEVST ARPGPRAVID YSKADAWAVG AIAYEIFGLV NPFYGQGKAH LESRSYQEAQ LPALPESVPP DVRQLVRALL QREASKRPSA RVAANVLHLS LWGEHILALK NLKLDKMVGW LLQQSAATLL ANRLTEKCCV ETKMKMLFLA NLECETLCQA ALLLCSWRAA L |

(continued)

| SEQ ID NO | Description | Sequence |
|---|---|---|
| 31 | USP30 mRNA; GenBank NM_ 032663.3 | TGCGGCCGCA GGTTCCGCTG TCTCGGGAAC CGTCGTATCC CTCGGTCCGG CGGCGGCGGC GGCGGTAGCG GAGGAGACGG TTTCAGGCCT CCGGTGCGGC TGCAATGCTG AGCTCCCGGG CCGAGGCGGC GATGACCGCG GCCGACAGGG CCATCCAGCG CTTCCTGCGG ACCGGGGCGG CCGTCAGATA TAAAGTCATG AAGAACTGGG GAGTTATAGG TGGAATTGCT GCTGCTCTTG CAGCAGGAAT ATATGTTATT TGGGGTCCCA TTACAGAAAG AAAGAAGCGT AGAAAAGGGC TTGTGCCTGG CCTTGTTAAT TTAGGGAACA CCTGCTTCAT GAACTCCCTG CTACAAGGCC TGTCTGCCTG TCCTGCTTTC ATCAGGTGGC TGGAAGAGTT CACCTCCCAG TACTCCAGGG ATCAGAAGGA GCCCCCCTCA CACCAGTATT TATCCTTAAC ACTCTTGCAC CTTCTGAAAG CCTTGTCCTG CCAAGAAGTT ACTGATGATG AGGTCTTAGA TGCAAGCTGC TTGTTGGATG TCTTAAGAAT GTACAGATGG CAGATCTCAT CATTTGAAGA ACAGGATGCT CACGAATTAT TCCATGTCAT TACCTCGTCA TTGGAAGATG AGCGAGACCG CCAGCCTCGG GTCACACATT TGTTTGATGT GCATTCCCTG GAGCAGCAGT CAGAAATAAC TCCCAAACAA ATTACCTGCC GCACAAGAGG GTCACCTCAC CCTACATCCA ATCACTGGAA GTCTCAACAT CCTTTTCATG GAAGACTCAC TAGTAATATG GTCTGCAAAC ACTGTGAACA CCAGAGTCCT GTTCGATTTG ATACCTTTGA TAGCCTTTCA CTAAGTATTC CAGCCGCCAC ATGGGGTCAC CCATTGACCC TGGACCACTG CCTTCACCAC TTCATCTCAT CAGAATCAGT GCGGGATGTT GTGTGTGACA ACTGTACAAA GATTGAAGCC AAGGGAACGT TGAACGGGGA AAAGGTGGAA CACCAGAGGA CCACTTTTGT TAAACAGTTA AAACTAGGGA AGCTCCCTCA GTGTCTCTGC ATCCACCTAC AGCGGCTGAG CTGGTCCAGC CACGGCACGC CTCTGAAGCG GCATGAGCAC GTGCAGTTCA ATGAGTTCCT GATGATGGAC ATTTACAAGT ACCACCTCCT TGGACATAAA CCTAGTCAAC ACAACCCTAA ACTGAACAAG AACCCAGGGC CTACACTGGA GCTGCAGGAT GGGCCGGGAG CCCCCACACC AGTTCTGAAT CAGCCAGGGG CCCCCAAAAC ACAGATTTTT ATGAATGGCG CCTGCTCCCC ATCTTTATTG CCAACGCTGT CAGCGCCGAT GCCCTTCCCT CTCCCAGTTG TTCCCGACTA CAGCTCCTCC ACATACCTCT TCCGGCTGAT GGCAGTTGTC GTCCACCATG GAGACATGCA CTCTGGACAC TTTGTCACTT ACCGACGGTC CCCACCTTCT GCCAGGAACC CTCTCTCAAC TAGCAATCAG TGGCTGTGGG TCTCCGATGA CACTGTCCGC AAGGCCAGCC TGCAGGAGGT CCTGTCCTCC AGCGCCTACC TGCTGTTCTA CGAGCGCGTC CTTTCCAGGA TGCAGCACCA GAGCCAGGAG TGCAAGTCTG AAGAATGACT GTGCCCTCCT GCAAGGCTAG AGCTGATGGC ACTGTCTGCA CTGTCCAGGA AAAAAGTAAA ACTGTACTGT TGCGTGTGCA AGCGGCCCCA CTAGAGCCTT CCAGCCTTCT GGTGTGTTCT AAGAGCAGGC TCCACCTGGG AGCCAGCCCC AGTTCACACC AAACCAGGCT CCCTGAACAG TCCTGTTCAT GTGTGTAGGT GGTTCTGTTG TGTTAAGAAA GCATTCATTA TGTCCGGAGT GTCTTTTTAC TCATCTGATA CAGGTAATTA AAAGAACTCA GATTCTTGAA GCCACCGTTT TCATATTGTA ATGTTAGGTG TTCTCAGAGG GGAGGTACCT TTGTCTAATC AACGTTTCCA CTTAGATCTT TTATTTTTAA TAAGCAGGCC CATAAAAATT GTTGACAAGA ATTAATGAAA TTATTAAAGG CAACAATTTA GAAGAAAAAG TGCCTTTCAC TTTCGATTGC TTTTGTAGCA CGTCCATTGT GAAATATTCC TTCCAGGCTA CTCAAAGGAT AGCAAGAGAA CAGGTAAATG ATGCCTAAAG AACACCTTCC TTTTTCTATG CCTTTTCTAA TCTTTCAATT CTTTCTATGG AGTAAAGGCT CATCTGCCAA ATCTGCCCCC TGGGGAAACT CTTTCACTAC TTTGTCAGTT ATAAGTGAAG AGCTTACTTG TTGCTTTTAT CTTTTGTATA TTGGACTGAG ATGTAATTAC ACTGTATTAT AAAACTCTGT GAATAGCCAG AACTGAGCTG GATCTTTGCA ACACCTGATT CCTCTGCTCT GTGGAAAACT TTTTCTTACA CAAGGATCCA CTGTGGACGG TTACTTTCAT CTGTTTATTT ATTGCCCATG CAGAGCTCTT AAGGTTTACA GGTGGGAGCT TGGGGCTGTA TAAAAAAATA ATCCCTGCCC TGAGTTGACA CCTGGCTTAG GAAGGAAGGG CTGACTATGG GGCTGCAGTC TCTCTGAACC TCAGTTTCCT |

(continued)

| SEQ ID NO | Description | Sequence |
|---|---|---|
| | | CATTTGTGAA GTGAAGGGTT AGATTTGATG ACCACCAAAG TTCAGCCCTT TTCACGAAAA GGAGAAAGCA GCTTTTGACT TTTTAAAAAA CATATAACTA CAGCTGGCAT CTAGTATTGT CATGTTGCTC TAGGTCCATA TTCTGAATTT ATTCATTTCC AATAGCCTAA TACAAAAAGT ATATATTGAG CACTTTCTTC CCTTTTCAGG TAAGTCTCTG AATGCAGCCC AGGGCCAAAG GAATTTTGAT GACACAGTAG TACCTATGTT TTAAGCTATA TTTTTAATTT AGAAAAATGG ATACCAAATT CAAACCGACT CATCAGAGGT AAGATTTGGA ATCAGACCTT TCCAAAAGGT CATCTGAGGT AAGGCTAAGA CCGCACTTCC TCTGCTGGGG GTGAGCTGGC AGACACACCA AACAGTGCCT TGGCAGCAGC TCACAGTGCA GGAAGCCCAG GTGATCACTC TTCTGCTGGG CCCAGGCTGC ACCCTGAGGA CTCAGTAACT CACTCTCAAC AGAATATTCT GTGCAGGCTC TCCAGGCTCT GGGCGTCAGG GTGCAAGGGG CAGCTTGAAC TGTACGGTCC GTCCTGCACT CACCCGATGC AGACCTTGAC TTTGATGTTG AAATGAACAC ACTTGTTTTA CCCAAGTCTG GTGGAACAAA TGCCCAATCA TGTGACCTTA AAGTGTACTG CAAAGCTGTA GCTTTAAGTA ATTGCTGTTC TGCCACTGCT TACTCTGAAA TCTACCATCA AAGAAAGATA GAGAAAAGGG GCTGAGCCTT GGAATATATG GTTATAAGCA GATCTTTCTT TGGTCAGAGA CCAGGGTTTG AGCCAAGGCT GTAAATGTGA ACAATAGCTG TGCAAAGCCT TTTAACCTGA CTTCTTCATT TTGTAAATTA TTATGCATTA AGTAGCAGCC CAATAATCTG ATTTCTAGTT TTATTTTCAA AGTAAGTAGC TTCTTTTGGG AAAAACCTAA GTTAAACTAG TAGTTTTGCC ATAATAACTG CTGATTTATG TATTTGCTAA AGGTACTTTT GTATCTGCTG TGTATTATAG CAATAAAATA ATCATTTTGT TAGAAAAAAA TCAAAAAAAA AAAAAA |
| 32 | Human MUL1; SwissProt Q969V5.1 | MESGGRPSLC QFILLGTTSV VTAALYSVYR QKARVSQELK GAKKVHLGED LKSILSEAPG KCVPYAVIEG AVRSVKETLN SQFVENCKGV IQRLTLQEHK MVWNRTTHLW NDCSKIIHQR TNTVPFDLVP HEDGVDVAVR VLKPLDSVDL GLETVYEKFH PSIQSFTDVI GHYISGERPK GIQETEEMLK VGATLTGVGE LVLDNNSVRL QPPKQGMQYY LSSQDFDSLL QRQESSVRLW KVLALVFGFA TCATLFFILR KQYLQRQERL RLKQMQEEFQ EHEAQLLSRA KPEDRESLKS ACVVCLSSFK SCVFLECGHV CSCTECYRAL PEPKKCPICR QAITRVIPLY NS |
| 33 | Human ASNS; GenBank NP_ 899199.2 | MCGIWALFGS DDCLSVQCLS AMKIAHRGPD AFRFENVNGY TNCCFGFHRL AVVDPLFGMQ PIRVKKYPYL WLCYNGEIYN HKKMQQHFEF EYQTKVDGEI ILHLYDKGGI EQTICMLDGV FAFVLLDTAN KKVFLGRDTY GVRPLFKAMT EDGFLAVCSE AKGLVTLKHS ATPFLKVEPF LPGHYEVLDL KPNGKVASVE MVKYHHCRDV PLHALYDNVE KLFPGFEIET VKNNLRILFN NAVKKRLMTD RRIGCLLSGG LDSSLVAATL LKQLKEAQVQ YPLQTFAIGM EDSPDLLAAR KVADHIGSEH YEVLFNSEEG IQALDEVIFS LETYDITTVR ASVGMYLISK YIRKNTDSVV IFSGEGSDEL TQGYIYFHKA PSPEKAEEES ERLLRELYLF DVLRADRTTA AHGLELRVPF LDHRFSSYYL SLPPEMRIPK NGIEKHLLRE TFEDSNLIPK EILWRPKEAF SDGITSVKNS WFKILQEYVE HQVDDAMMAN AAQKFPFNTP KTKEGYYYRQ VFERHYPGRA DWLSHYWMPK WINATDPSAR TLTHYKSAVK A |
| 34 | Human FKBP8; SwissProt Q14318.2 | MASCAEPSEP SAPLPAGVPP LEDFEVLDGV EDAEGEEEEE EEEEEEDDLS ELPPLEDMGQ PPAEEAEQPG ALAREFLAAM EPEPAPAPAP EEWLDILGNG LLRKKTLVPG PPGSSRPVKG QVVTVHLQTS LENGTRVQEE PELVFTLGDC DVIQALDLSV PLMDVGETAM VTADSKYCYG PQGRSPYIPP HAALCLEVTL KTAVDGPDLE MLTGQERVAL ANRKRECGNA HYQRADFVLA ANSYDLAIKA ITSSAKVDMT FEEEAQLLQL KVKCLNNLAA SQLKLDHYRA ALRSCSLVLE HQPDNIKALF RKGKVLAQQG EYSEAIPILR AALKLEPSNK TIHAELSKLV KKHAAQRSTE TALYRKMLGN PSRLPAKCPG KGAWSIPWKW LFGATAVALG GVALSVVIAA RN |

(continued)

| SEQ ID NO | Description | Sequence |
|---|---|---|
| 35 | Human TOM70; SwissProt 094826.1 | MAASKPVEAA VVAAAVPSSG SGVGGGGTAG PGTGGLPRWQ LALAVGAPLL LGAGAIYLWS RQQRRREARG RGDASGLKRN SERKTPEGRA SPAPGSGHPE GPGAHLDMNS LDRAQAAKNK GNKYFKAGKY EQAIQCYTEA ISLCPTEKNV DLSTFYQNRA AAFEQLQKWK EVAQDCTKAV ELNPKYVKAL FRRAKAHEKL |
| | | DNKKECLEDV TAVCILEGFQ NQQSMLLADK VLKLLGKEKA KEKYKNREPL MPSPQFIKSY FSSFTDDIIS QPMLKGEKSD EDKDKEGEAL EVKENSGYLK AKQYMEEENY DKIISECSKE IDAEGKYMAE ALLLRATFYL LIGNANAAKP DLDKVISLKE ANVKLRANAL IKRGSMYMQQ QQPLLSTQDF NMAADIDPQN ADVYHHRGQL KILLDQVEEA VADFDECIRL RPESALAQAQ KCFALYRQAY TGNNSSQIQA AMKGFEEVIK KFPRCAEGYA LYAQALTDQQ QFGKADEMYD KCIDLEPDNA TTYVHKGLLQ LQWKQDLDRG LELISKAIEI DNKCDFAYET MGTIEVQRGN MEKAIDMFNK AINLAKSEME MAHLYSLCDA AHAQTEVAKK YGLKPPTL |
| 36 | Human MAT2B; SwissProt Q9NZL9.1 | MVGREKELSI HFVPGSCRLV EEEVNIPNRR VLVTGATGLL GRAVHKEFQQ NNWHAVGCGF RRARPKFEQV NLLDSNAVHH IIHDFQPHVI VHCAAERRPD VVENQPDAAS QLNVDASGNL AKEAAAVGAF LIYISSDYVF DGTNPPYREE DIPAPLNLYG KTKLDGEKAV LENNLGAAVL RIPILYGEVE KLEESAVTVM FDKVQFSNKS ANMDHWQQRF PTHVKDVATV CRQLAEKRML DPSIKGTFHW SGNEQMTKYE MACAIADAFN LPSSHLRPIT DSPVLGAQRP RNAQLDCSKL ETLGIGQRTP FRIGIKESLW PFLIDKRWRQ TVFH |
| 37 | Human PRDX3; SwissProt P30048.3 | MAAAVGRLLR ASVARHVSAI PWGISATAAL RPAACGRTSL TNLLCSGSSQ AKLFSTSSSC HAPAVTQHAP YFKGTAVVNG EFKDLSLDDF KGKYLVLFFY PLDFTFVCPT EIVAFSDKAN EFHDVNCEVV AVSVDSHFSH LAWINTPRKN GGLGHMNIAL LSDLTKQISR DYGVLLEGSG LALRGLFIID PNGVIKHLSV NDLPVGRSVE ETLRLVKAFQ YVETHGEVCP ANWTPDSPTI KPSPAASKEY FQKVNQ |
| 38 | Human IDE; SwissProt P14735.4 | MRYRLAWLLH PALPSTFRSV LGARLPPPER LCGFQKKTYS KMNNPAIKRI GNHITKSPED KREYRGLELA NGIKVLLISD PTTDKSSAAL DVHIGSLSDP PNIAGLSHFC EHMLFLGTKK YPKENEYSQF LSEHAGSSNA FTSGEHTNYY FDVSHEHLEG ALDRFAQFFL CPLFDESCKD REVNAVDSEH EKNVMNDAWR LFQLEKATGN PKHPFSKFGT GNKYTLETRP NQEGIDVRQE LLKFHSAYYS SNLMAVCVLG RESLDDLTNL VVKLFSEVEN KNVPLPEFPE HPFQEEHLKQ LYKIVPIKDI RNLYVTFPIP DLQKYYKSNP GHYLGHLIGH EGPGSLLSEL KSKGWVNTLV GGQKEGARGF MFFIINVDLT EEGLLHVEDI ILHMFQYIQK LRAEGPQEWV FQECKDLNAV AFRFKDKERP RGYTSKIAGI LHYYPLEEVL TAEYLLEEFR PDLIEMVLDK LRPENVRVAI VSKSFEGKTD RTEEWYGTQY KQEAIPDEVI KKWQNADLNG KFKLPTKNEF IPTNFEILPL EKEATPYPAL IKDTAMSKLW FKQDDKFFLP KACLNFEFFS PFAYVDPLHC NMAYLYLELL KDSLNEYAYA AELAGLSYDL QNTIYGMYLS VKGYNDKQPI LLKKIIEKMA TFEIDEKRFE IIKEAYMRSL NNFRAEQPHQ HAMYYLRLLM TEVAWTKDEL KEALDDVTLP RLKAFIPQLL SRLHIEALLH GNITKQAALG IMQMVEDTLI EHAHTKPLLP SQLVRYREVQ LPDRGWFVYQ QRNEVHNNCG IEIYYQTDMQ STSENMFLEL FCQIISEPCF NTLRTKEQLG YIVFSGPRRA NGIQGLRFII QSEKPPHYLE SRVEAFLITM EKSIEDMTEE AFQKHIQALA IRRLDKPKKL SAECAKYWGE IISQQYNFDR DNTEVAYLKT LTKEDIIKFY KEMLAVDAPR RHKVSVHVLA REMDSCPVVG EFPCQNDINL SQAPALPQPE VIQNMTEFKR GLPLFPLVKP HINFMAAKL |

(continued)

| SEQ ID NO | Description | Sequence |
|---|---|---|
| 39 | Human VDAC1; SwissProt P21796.2 | MAVPPTYADL GKSARDVFTK GYGFGLIKLD LKTKSENGLE FTSSGSANTE TTKVTGSLET KYRWTEYGLT FTEKWNTDNT LGTEITVEDQ LARGLKLTFD SSFSPNTGKK NAKIKTGYKR EHINLGCDMD FDIAGPSIRG ALVLGYEGWL AGYQMNFETA KSRVTQSNFA VGYKTDEFQL HTNVNDGTEF GGSIYQKVNK KLETAVNLAW TAGNSNTRFG IAAKYQIDPD ACFSAKVNNS SLIGLGYTQT LKPGIKLTLS ALLDGKNVNA GGHKLGLGLE FQA |
| 44 | Human VDAC2; SwissProt P45880.2 | MATHGQTCAR PMCIPPSYAD LGKAARDIFN KGFGFGLVKL DVKTKSCSGV EFSTSGSSNT DTGKVTGTLE TKYKWCEYGL TFTEKWNTDN TLGTEIAIED QICQGLKLTF DTTFSPNTGK KSGKIKSSYK RECINLGCDV DFDFAGPAIH GSAVFGYEGW LAGYQMTFDS AKSKLTRNNF AVGYRTGDFQ LHTNVNDGTE FGGSIYQKVC EDLDTSVNLA WTSGTNCTRF GIAAKYQLDP TASISAKVNN SSLIGVGYTQ TLRPGVKLTL SALVDGKSIN AGGHKVGLAL ELEA |
| 45 | Human | MCNTPTYCDL GKAAKDVFNK GYGFGMVKID LKTKSCSGVE FSTSGHAYTD |
| | VDAC3; SwissProt Q9Y277.1 | TGKASGNLET KYKVCNYGLT FTQKWNTDNT LGTEISWENK LAEGLKLTLD TIFVPNTGKK SGKLKASYKR DCFSVGSNVD IDFSGPTIYG WAVLAFEGWL AGYQMSFDTA KSKLSQNNFA LGYKAADFQL HTHVNDGTEF GGSIYQKVNE KIETSINLAW TAGSNNTRFG IAAKYMLDCR TSLSAKVNNA SLIGLGYTQT LRPGVKLTLS ALIDGKNFSA GGHKVGLGFE LEA |
| 40 | Human IPO5; SwissProt 000410.4 | MAAAAAEQQQ FYLLLGNLLS PDNVVRKQAE ETYENIPGQS KITFLLQAIR NTTAAEEARQ MAAVLLRRLL SSAFDEVYPA LPSDVQTAIK SELLMIIQME TQSSMRKKVC DIAAELARNL IDEDGNNQWP EGLKFLFDSV SSQNVGLREA ALHIFWNFPG IFGNQQQHYL DVIKRMLVQC MQDQEHPSIR TLSARATAAF ILANEHNVAL FKHFADLLPG FLQAVNDSCY QNDDSVLKSL VEIADTVPKY LRPHLEATLQ LSLKLCGDTS LNNMQRQLAL EVIVTLSETA AAMLRKHTNI VAQTIPQMLA MMVDLEEDED WANADELEDD DFDSNAVAGE SALDRMACGL GGKLVLPMIK EHIMQMLQNP DWKYRHAGLM ALSAIGEGCH QQMEGILNEI VNFVLLFLQD PHPRVRYAAC NAVGQMATDF APGFQKKFHE KVIAALLQTM EDQGNQRVQA HAAAALINFT EDCPKSLLIP YLDNLVKHLH SIMVLKLQEL IQKGTKLVLE QVVTSIASVA DTAEEKFVPY YDLFMPSLKH IVENAVQKEL RLLRGKTIEC ISLIGLAVGK EKFMQDASDV MQLLLKTQTD FNDMEDDDPQ ISYMISAWAR MCKILGKEFQ QYLPVVMGPL MKTASIKPEV ALLDTQDMEN MSDDDGWEFV NLGDQQSFGI KTAGLEEKST ACQMLVCYAK ELKEGFVEYT EQVVKLMVPL LKFYFHDGVR VAAAESMPLL LECARVRGPE YLTQMWHFMC DALIKAIGTE PDSDVLSEIM HSFAKCIEVM GDGCLNNEHF EELGGILKAK LEEHFKNQEL RQVKRQDEDY DEQVEESLQD EDDNDVYILT KVSDILHSIF SSYKEKVLPW FEQLLPLIVN LICPHRPWPD RQWGLCIFDD VIEHCSPASF KYAEYFLRPM LQYVCDNSPE VRQAAAYGLG VMAQYGGDNY RPFCTEALPL LVRVIQSADS KTKENVNATE NCISAVGKIM KFKPDCVNVE EVLPHWLSWL PLHEDKEEAV QTFNYLCDLI ESNHPIVLGP NNTNLPKIFS IIAEGEMHEA IKHEDPCAKR LANVVRQVQT SGGLWTECIA QLSPEQQAAI QELLNSA |
| 41 | Human PTH2; SwissProt Q9Y3E5.1 | MPSKSLVMEY LAHPSTLGLA VGVACGMCLG WSLRVCFGML PKSKTSKTHT DTESEASILG DSGEYKMILV VRNDLKMGKG KVAAQCSHAA VSAYKQIQRR NPEMLKQWEY CGQPKVVVKA PDEETLIALL AHAKMLGLTV SLIQDAGRTQ IAPGSQTVLG IGPGPADLID KVTGHLKLY |
| 42 | Human PSD13; SwissProt Q9UNM6.2 | MKDVPGFLQQ SQNSGPGQPA VWHRLEELYT KKLWHQLTLQ VLDFVQDPCF AQGDGLIKLY ENFISEFEHR VNPLSLVEII LHVVRQMTDP NVALTFLEKT REKVKSSDEA VILCKTAIGA LKLNIGDLQV TKETIEDVEE MLNNLPGVTS VHSRFYDLSS KYYQTIGNHA SYYKDALRFL GCVDIKDLPV SEQQERAFTL GLAGLLGEGV FNFGELLMHP VLESLRNTDR QWLIDTLYAF NSGNVERFQT LKTAWGQQPD LAANEAQLLR KIQLLCLMEM TFTRPANHRQ LTFEEIAKSA KITVNEVELL VMKALSVGLV KGSIDEVDKR VHMTWVQPRV LDLQQIKGMK DRLEFWCTDV KSMEMLVEHQ AHDILT |

(continued)

| SEQ ID NO | Description | Sequence |
|---|---|---|
| 43 | Human UBP13; SwissProt Q92995.2 | MQRRGALFGM PGGSGGRKMA AGDIGELLVP HMPTIRVPRS GDRVYKNECA FSYDSPNSEG GLYVCMNTFL AFGREHVERH FRKTGQSVYM HLKRHVREKV RGASGGALPK RRNSKIFLDL DTDDDLNSDD YEYEDEAKLV IFPDHYEIAL PNIEELPALV TIACDAVLSS KSPYRKQDPD TWENELPVSK YANNLTQLDN GVRIPPSGWK CARCDLRENL WLNLTDGSVL CGKWFFDSSG GNGHALEHYR DMGYPLAVKL GTITPDGADV YSFQEEEPVL DPHLAKHLAH FGIDMLHMHG TENGLQDNDI KLRVSEWEVI QESGTKLKPM YGPGYTGLKN LGNSCYLSSV MQAIFSIPEF QRAYVGNLPR IFDYSPLDPT QDFNTQMTKL GHGLLSGQYS KPPVKSELIE QVMKEEHKPQ QNGISPRMFK AFVSKSHPEF SSNRQQDAQE FFLHLVNLVE RNRIGSENPS DVFRFLVEER IQCCQTRKVR YTERVDYLMQ LPVAMEAATN KDELIAYELT RREAEANRRP LPELVRAKIP FSACLQAFSE PENVDDFWSS ALQAKSAGVK TSRFASFPEY LVVQIKKFTF GLDWVPKKFD VSIDMPDLLD INHLRARGLQ PGEEELPDIS PPIVIPDDSK DRLMNQLIDP SDIDESSVMQ LAEMGFPLEA CRKAVYFTGN MGAEVAFNWI IVHMEEPDFA EPLTMPGYGG AASAGASVFG ASGLDNQPPE EIVAIITSMG FQRNQAIQAL RATNNNLERA LDWIFSHPEF EEDSDFVIEM ENNANANIIS EAKPEGPRVK DGSGTYELFA FISHMGTSTM SGHYICHIKK EGRWVIYNDH KVCASERPPK |
| | | DLGYMYFYRR IPS |

Appendix A

| | | | |
|---|---|---|---|
| 1433B_HUMAN | ABCE1_HUMAN | AGM1_HUMAN | AN32B_HUMAN |
| 1433E_HUMAN | ABCF1_HUMAN | AGO1_HUMAN | AN32E_HUMAN |
| 1433F_HUMAN | ABCF2_HUMAN | AGO2_HUMAN | ANFY1_HUMAN |
| 1433G_HUMAN | ABCF3_HUMAN | AHNK_HUMAN | ANLN_HUMAN |
| 1433T_HUMAN | ABHD2_HUMAN | AHSA1_HUMAN | ANM1_HUMAN |
| 1433Z_HUMAN | ABT1_HUMAN | AIBP_HUMAN | ANM5_HUMAN |
| 1A01_HUMAN | ACACA_HUMAN | AIF1L_HUMAN | ANR26_HUMAN |
| 1A02_HUMAN | ACBD6_HUMAN | AIFM1_HUMAN | ANR46_HUMAN |
| 1B07_HUMAN | ACBP_HUMAN | AIMP1_HUMAN | ANX11_HUMAN |
| 1C07_HUMAN | ACHA5_HUMAN | AIMP2_HUMAN | ANXA1_HUMAN |
| 2A5D_HUMAN | ACINU_HUMAN | AINX_HUMAN | ANXA2_HUMAN |
| 2AAA_HUMAN | ACLY_HUMAN | AIP_HUMAN | ANXA5_HUMAN |
| 2ABA_HUMAN | ACO13_HUMAN | AKA11_HUMAN | ANXA6_HUMAN |
| 2ABD_HUMAN | ACOD_HUMAN | AKA12_HUMAN | AOFA_HUMAN |
| 3BP5_HUMAN | ACSL1_HUMAN | AKAP1_HUMAN | AP1G1_HUMAN |
| 41_HUMAN | ACSL3_HUMAN | AKAP9_HUMAN | AP1M1_HUMAN |
| 4F2_HUMAN | ACSL4_HUMAN | AKIB1_HUMAN | AP2A1_HUMAN |
| 5NT3_HUMAN | ACTA_HUMAN | AKP13_HUMAN | AP2A2_HUMAN |
| 6PGD_HUMAN | ACTB_HUMAN | AKP8L_HUMAN | AP2A_HUMAN |
| 6PGL_HUMAN | ACTN1_HUMAN | AKT2_HUMAN | AP2B1_HUMAN |
| A0PJ76_HUMAN | ACTN4_HUMAN | AL3A2_HUMAN | AP2M1_HUMAN |
| A219Y7_HUMAN | ACTZ_HUMAN | AL7A1_HUMAN | AP2S1_HUMAN |
| A4UCU2_HUMAN | ACV1B_HUMAN | AL9A1_HUMAN | AP3B1_HUMAN |
| A4_HUMAN | ACYP1_HUMAN | ALBU_HUMAN | AP3D1_HUMAN |
| A7UJ17_HUMAN | ADAM9_HUMAN | ALDOA_HUMAN | AP3M1_HUMAN |
| A8K781_HUMAN | ADCY3_HUMAN | ALDR_HUMAN | AP3S1_HUMAN |
| A8K7N0_HUMAN | ADCY9_HUMAN | ALG5_HUMAN | AP3S2_HUMAN |
| A8KAM7_HUMAN | ADDA_HUMAN | ALG6_HUMAN | APBA2_HUMAN |

(continued)

| | | | |
|---|---|---|---|
| AAAS_HUMAN | ADHX_HUMAN | ALKB5_HUMAN | APC1_HUMAN |
| AAAT_HUMAN | ADNP_HUMAN | ALO17_HUMAN | APC4_HUMAN |
| AACS_HUMAN | ADPPT_HUMAN | AMD_HUMAN | APC5_HUMAN |
| AAKG1_HUMAN | ADRM1_HUMAN | AMOL1_HUMAN | APC7_HUMAN |
| AAMP_HUMAN | ADT1_HUMAN | AMOT_HUMAN | APC_HUMAN |
| AAPK2_HUMAN | ADT2_HUMAN | AMPL_HUMAN | APEX1_HUMAN |
| AATF_HUMAN | ADT3_HUMAN | AMPM2_HUMAN | API5_HUMAN |
| ABC3C_HUMAN | AES_HUMAN | AMRA1_HUMAN | APLP2_HUMAN |
| ABCA3_HUMAN | AF1Q_HUMAN | AN13A_HUMAN | APOL2_HUMAN |
| ABCB6_HUMAN | AFF4_HUMAN | AN13B_HUMAN | APOO_HUMAN |
| ABCBA_HUMAN | AGAL_HUMAN | AN13C_HUMAN | APR_HUMAN |
| ABCD3_HUMAN | AGK_HUMAN | AN32A_HUMAN | APT_HUMAN |
| AR6P1_HUMAN | AT12A_HUMAN | B3KPC1_HUMAN | BCCIP_HUMAN |
| AR6P4_HUMAN | AT131_HUMAN | B3KRI9_HUMAN | BCD1_HUMAN |
| ARF1_HUMAN | AT132_HUMAN | B3KTN8_HUMAN | BCLF1_HUMAN |
| ARF4_HUMAN | AT1A1_HUMAN | B4DE27_HUMAN | BCOR_HUMAN |
| ARF5_HUMAN | AT2A2_HUMAN | B4DIH6_HUMAN | BDH2_HUMAN |
| ARF6_HUMAN | AT2B1_HUMAN | B4DIM0_HUMAN | BET1L_HUMAN |
| ARFG1_HUMAN | AT2B4_HUMAN | B4DKA3_HUMAN | BET1_HUMAN |
| ARFG2_HUMAN | AT2C1_HUMAN | B4DKB3_HUMAN | BEX4_HUMAN |
| ARH40_HUMAN | AT5F1_HUMAN | B4DL94_HUMAN | BHLH9_HUMAN |
| ARHG7_HUMAN | ATAD1_HUMAN | B4DLR3_HUMAN | BI1_HUMAN |
| ARI1A_HUMAN | ATBD4_HUMAN | B4DMT9_HUMAN | BIEA_HUMAN |
| ARI1_HUMAN | ATF1_HUMAN | B4DNE0_HUMAN | BIRC2_HUMAN |
| ARI2_HUMAN | ATF2_HUMAN | B4DSP0_HUMAN | BIRC5_HUMAN |
| ARID2_HUMAN | ATF7_HUMAN | B4DW33_HUMAN | BIRC6_HUMAN |
| ARIP4_HUMAN | ATG3_HUMAN | B4E184_HUMAN | BL1S1_HUMAN |
| ARL1_HUMAN | ATLA2_HUMAN | B4E2Y0_HUMAN | BMP2K_HUMAN |
| ARL2_HUMAN | ATLA3_HUMAN | B7H6_HUMAN | BNI3L_HUMAN |
| ARL3_HUMAN | ATM_HUMAN | B7Z2A7_HUMAN | BNIP2_HUMAN |
| ARL6_HUMAN | ATOX1_HUMAN | B7Z4W9_HUMAN | BOD1L_HUMAN |
| ARL8B_HUMAN | ATP7B_HUMAN | B7Z613_HUMAN | BOD1_HUMAN |
| ARM10_HUMAN | ATPA_HUMAN | B7Z6F8_HUMAN | BOP1_HUMAN |
| ARMC1_HUMAN | ATPB_HUMAN | B7Z780_HUMAN | BOREA_HUMAN |
| ARMC6_HUMAN | ATPG_HUMAN | B7Z8Y4_HUMAN | BORG5_HUMAN |
| ARMC8_HUMAN | ATPO_HUMAN | B9D1_HUMAN | BPNT1_HUMAN |
| ARMX3_HUMAN | ATR_HUMAN | BABA1_HUMAN | BRAP_HUMAN |
| ARP19_HUMAN | ATX10_HUMAN | BACD3_HUMAN | BRAT1_HUMAN |
| ARP2_HUMAN | ATX2_HUMAN | BACH_HUMAN | BRCA1_HUMAN |
| ARP3_HUMAN | ATX3_HUMAN | BAF_HUMAN | BRCC3_HUMAN |
| ARP5L_HUMAN | AUP1_HUMAN | BAG2_HUMAN | BRD2_HUMAN |
| ARP8_HUMAN | AURKA_HUMAN | BAG5_HUMAN | BRD4_HUMAN |
| ARPC2_HUMAN | AURKB_HUMAN | BAG6_HUMAN | BRE1A_HUMAN |
| ARPC3_HUMAN | AVEN_HUMAN | BAP18_HUMAN | BRE1B_HUMAN |
| ARPC4_HUMAN | AZI1-HUMAN | BAP29_HUMAN | BRE_HUMAN |
| ARPC5_HUMAN | AZI2_HUMAN | BAP31_HUMAN | BRK1_HUMAN |
| ARV1_HUMAN | AZIN1_HUMAN | BARD1_HUMAN | BROX_HUMAN |
| ASB13_HUMAN | B1AK87-HUMAN | BASI_HUMAN | BRWD3_HUMAN |
| ASCC2_HUMAN | B1ALK7_HUMAN | BASP1_HUMAN | BSDC1_HUMAN |
| ASNA_HUMAN | B2CI53_HUMAN | BAX_HUMAN | BT2A1_HUMAN |
| ASNS_HUMAN | B2L12_HUMAN | BAZ1A_HUMAN | BT3L4_HUMAN |

(continued)

| | | | |
|---|---|---|---|
| ASPP1_HUMAN | B2L13_HUMAN | BAZ1B_HUMAN | BTBD1_HUMAN |
| ASPP2_HUMAN | B2RDE1_HUMAN | BAZ2A_HUMAN | BTBD2_HUMAN |
| ASXL2_HUMAN | B3A2_HUMAN | BBS1_HUMAN | BTBDA_HUMAN |
| AT11C_HUMAN | B3KNS4_HUMAN | BBS2_HUMAN | BTF3_HUMAN |
| BUB1B_HUMAN | CBR3_HUMAN | CDC16_HUMAN | CFDP1_HUMAN |
| BUB1_HUMAN | CBS_HUMAN | CDC20_HUMAN | CG044_HUMAN |
| BUB3_HUMAN | CBWD1_HUMAN | CDC23_HUMAN | CG074 _ HUMAN |
| BYST_HUMAN | CBX1_HUMAN | CDC27_HUMAN | CGL_HUMAN |
| BZW1_HUMAN | CBX2_HUMAN | CDC37_HUMAN | CH055_HUMAN |
| BZW2_HUMAN | CBX3_HUMAN | CDC42_HUMAN | CH059_HUMAN |
| C19L1_HUMAN | CBX5_HUMAN | CDC45_HUMAN | CH10_HUMAN |
| C1QBP_HUMAN | CBX6_HUMAN | CDC5L_HUMAN | CH60_HUMAN |
| C1TC_HUMAN | CC037_HUMAN | CDC73_HUMAN | CHC10_HUMAN |
| C8AP2_HUMAN | CC038_HUMAN | CDC7_HUMAN | CHCH2_HUMAN |
| C99L2_HUMAN | CC075_HUMAN | CDIPT_HUMAN | CHCH3_HUMAN |
| CA031_HUMAN | CC104_HUMAN | CDK1_HUMAN | CHD1_HUMAN |
| CA043_HUMAN | CC138_HUMAN | CDK2_HUMAN | CHD4_HUMAN |
| CA052_HUMAN | CC167_HUMAN | CDK4_HUMAN | CHD8_HUMAN |
| CA055_HUMAN | CC85B_HUMAN | CDK5_HUMAN | CHIC1_HUMAN |
| CA124_HUMAN | CCD14_HUMAN | CDKAL_HUMAN | CHIC2_HUMAN |
| CAB39_HUMAN | CCD22_HUMAN | CDV3_HUMAN | CHK1_HUMAN |
| CAB45_HUMAN | CCD47_HUMAN | CDYL1_HUMAN | CHM1A_HUMAN |
| CACO2_HUMAN | CCD50_HUMAN | CE025_HUMAN | CHM1B_HUMAN |
| CADH2_HUMAN | CCD58_HUMAN | CE170_HUMAN | CHM2A_HUMAN |
| CADM1_HUMAN | CCD72_HUMAN | CE192_HUMAN | CHM2B_HUMAN |
| CAF1A_HUMAN | CCD86_HUMAN | CE290_HUMAN | CHM4B_HUMAN |
| CAF1B_HUMAN | CCD94_HUMAN | CEBPZ_HUMAN | CHMP5_HUMAN |
| CAH2_HUMAN | CCD97_HUMAN | CEGT_HUMAN | CHRD1_HUMAN |
| CAH8_HUMAN | CCDB1_HUMAN | CELF1_HUMAN | CHSP1_HUMAN |
| CALD1_HUMAN | CCDC6_HUMAN | CENPB_HUMAN | CHTOP_HUMAN |
| CALM_HUMAN | CCDC8_HUMAN | CENPF_HUMAN | CI040_HUMAN |
| CALU_HUMAN | CCNA2_HUMAN | CENPH_HUMAN | CI041_HUMAN |
| CALX_HUMAN | CCNB1_HUMAN | CENPL_HUMAN | CI064_HUMAN |
| CAN1_HUMAN | CCNB2_HUMAN | CENPN_HUMAN | CI078_HUMAN |
| CAN7_HUMAN | CCND1_HUMAN | CENPQ_HUMAN | CIB1_HUMAN |
| CANB1_HUMAN | CCNK_HUMAN | CEP44_HUMAN | CING_HUMAN |
| CAND1_HUMAN | CCZ1L_HUMAN | CEP55_HUMAN | CIP2A_HUMAN |
| CAP1_HUMAN | CD032_HUMAN | CEP78_HUMAN | CIR1A_HUMAN |
| CAPR1_HUMAN | CD11A_HUMAN | CERS2_HUMAN | CISD1_HUMAN |
| CAPZB_HUMAN | CD123_HUMAN | CETN1_HUMAN | CISD2_HUMAN |
| CARM1_HUMAN | CD151_HUMAN | CETN2_HUMAN | CISY_HUMAN |
| CASC3_HUMAN | CD276_HUMAN | CF072_HUMAN | CJ032_HUMAN |
| CASC5_HUMAN | CD2AP_HUMAN | CF106_HUMAN | CK046_HUMAN |
| CAV1_HUMAN | CD320_HUMAN | CF115_HUMAN | CK067_HUMAN |
| CAZA1_HUMAN | CD81_HUMAN | CF130_HUMAN | CK5P2_HUMAN |
| CBPD_HUMAN | CD97_HUMAN | CF192_HUMAN | CK5P3_HUMAN |
| CBR1_HUMAN | CD99_HUMAN | CF211_HUMAN | CKAP2_HUMAN |
| CKAP5_HUMAN | COPG_HUMAN | CSN4_HUMAN | CYB5_HUMAN |
| CKS1_HUMAN | COPZ1_HUMAN | CSN5_HUMAN | CYBP_HUMAN |
| CL023_HUMAN | COQ2_HUMAN | CSN6_HUMAN | CYC_HUMAN |
| CL16A_HUMAN | COR1B_HUMAN | CSN7A_HUMAN | CYFP1_HUMAN |

(continued)

| | | | |
|---|---|---|---|
| CLCA_HUMAN | COR1C_HUMAN | CSN7B_HUMAN | CYFP2_HUMAN |
| CLCB_HUMAN | COX17_HUMAN | CSPG5_HUMAN | CYLD_HUMAN |
| CLCC1_HUMAN | COX41_HUMAN | CSTF2_HUMAN | CYTB_HUMAN |
| CLH1_HUMAN | CP013_HUMAN | CSTF3_HUMAN | CYTSA_HUMAN |
| CLIC1_HUMAN | CP072_HUMAN | CSTFT_HUMAN | CYTSB_HUMAN |
| CLIC4_HUMAN | CP080_HUMAN | CT004_HUMAN | D3DQ69_HUMAN |
| CMIP_HUMAN | CP110_HUMAN | CT011_HUMAN | D3VVH3_HUMAN |
| CN166_HUMAN | CP135_HUMAN | CT030_HUMAN | D6RDG3_HUMAN |
| CN37_HUMAN | CP250_HUMAN | CTBP1_HUMAN | DACH1_HUMAN |
| CNBP_HUMAN | CP51A_HUMAN | CTBP2_HUMAN | DAD1_HUMAN |
| CND1_HUMAN | CPIN1_HUMAN | CTCF_HUMAN | DAG1_HUMAN |
| CND2_HUMAN | CPNE1_HUMAN | CTNA1_HUMAN | DAXX_HUMAN |
| CND3_HUMAN | CPNE3_HUMAN | CTNB1_HUMAN | DAZP1_HUMAN |
| CNDG2_HUMAN | CPNE5_HUMAN | CTND1_HUMAN | DBLOH_HUMAN |
| CNN3_HUMAN | CPNE8_HUMAN | CTR1_HUMAN | DBNL_HUMAN |
| CNNM3_HUMAN | CPNS1_HUMAN | CTR2_HUMAN | DBPA_HUMAN |
| CNNM4_HUMAN | CPSF1_HUMAN | CU059_HUMAN | DC1L1_HUMAN |
| CNOT1_HUMAN | CPSF2_HUMAN | CUED2_HUMAN | DC1L2_HUMAN |
| CNOT8_HUMAN | CPSF3_HUMAN | CUL1_HUMAN | DCA13_HUMAN |
| CNOTA_HUMAN | CPSF5_HUMAN | CUL2_HUMAN | DCAF5_HUMAN |
| CNO_HUMAN | CPSF6_HUMAN | CUL3_HUMAN | DCAF6_HUMAN |
| CO038_HUMAN | CPSF7_HUMAN | CUL4A_HUMAN | DCAF7_HUMAN |
| CO044_HUMAN | CPT1A_HUMAN | CUL4B_HUMAN | DCAF8_HUMAN |
| CO057_HUMAN | CR021_HUMAN | CUL5_HUMAN | DCAKD_HUMAN |
| COBL1_HUMAN | CREB5_HUMAN | CUL7_HUMAN | DCAM_HUMAN |
| COF1_HUMAN | CRIPT_HUMAN | CUL9_HUMAN | DCK_HUMAN |
| COF2_HUMAN | CRKL_HUMAN | CUTA_HUMAN | DCNL1_HUMAN |
| COG2_HUMAN | CRNL1_HUMAN | CUTC_HUMAN | DCNL5_HUMAN |
| COG4_HUMAN | CS010_HUMAN | CWC15_HUMAN | DCPS_HUMAN |
| COMD1_HUMAN | CS043_HUMAN | CWC22_HUMAN | DCTN1_HUMAN |
| COMD4_HUMAN | CSDE1_HUMAN | CWC27_HUMAN | DCTN2_HUMAN |
| COMD9_HUMAN | CSK21_HUMAN | CX026_HUMAN | DCTN4_HUMAN |
| COMT_HUMAN | CSK22_HUMAN | CX056_HUMAN | DCTP1_HUMAN |
| COPA_HUMAN | CSK2B_HUMAN | CX057_HUMAN | DCUP_HUMAN |
| COPB2_HUMAN | CSKP_HUMAN | CX6B1_HUMAN | DCXR_HUMAN |
| COPB_HUMAN | CSK_HUMAN | CX7A2_HUMAN | DD19A_HUMAN |
| COPD_HUMAN | CSN1_HUMAN | CXA1_HUMAN | DDB1_HUMAN |
| COPE_HUMAN | CSN2_HUMAN | CY561_HUMAN | DDB2_HUMAN |
| COPG2_HUMAN | CSN3_HUMAN | CYB5B_HUMAN | DDHD2_HUMAN |
| DDI1_HUMAN | DHX40_HUMAN | DRG1_HUMAN | EDRF1_HUMAN |
| DDI2_HUMAN | DHX57_HUMAN | DRG2_HUMAN | EEA1_HUMAN |
| DDIT4_HUMAN | DHX9_HUMAN | DRS7B_HUMAN | EF1A1_HUMAN |
| DDTL_HUMAN | DHYS_HUMAN | DSC3_HUMAN | EF1A2_HUMAN |
| DDX17_HUMAN | DIAP1_HUMAN | DSCR3_HUMAN | EF1B_HUMAN |
| DDX18_HUMAN | DICER_HUMAN | DSG2_HUMAN | EF1D_HUMAN |
| DDX1_HUMAN | DIDO1_HUMAN | DSRAD_HUMAN | EF1G_HUMAN |
| DDX20_HUMAN | DIM1_HUMAN | DTL_HUMAN | EF2K_HUMAN |
| DDX21_HUMAN | DIP2B_HUMAN | DUS3L_HUMAN | EF2_HUMAN |
| DDX23_HUMAN | DJC11_HUMAN | DUS3_HUMAN | EFHD1_HUMAN |
| DDX24_HUMAN | DJC21_HUMAN | DUT_HUMAN | EFNB1_HUMAN |
| DDX27_HUMAN | DKC1_HUMAN | DVL1L_HUMAN | EFTU_HUMAN |

(continued)

| | | | |
|---|---|---|---|
| DDX3X_HUMAN | DLL1_HUMAN | DVL2_HUMAN | EHD4_HUMAN |
| DDX41_HUMAN | DLRB1_HUMAN | DX39A_HUMAN | EHMT1_HUMAN |
| DDX46_HUMAN | DMD_HUMAN | DX39B_HUMAN | EHMT2_HUMAN |
| DDX47_HUMAN | DMKN_HUMAN | DYH7_HUMAN | EIA_HUMAN |
| DDX59_HUMAN | DNA2L_HUMAN | DYHC1_HUMAN | EIBB_HUMAN |
| DDX5_HUMAN | DNJA1_HUMAN | DYHC2_HUMAN | EI2BD_HUMAN |
| DDX6_HUMAN | DNJA2_HUMAN | DYL1_HUMAN | EID1_HUMAN |
| DEK_HUMAN | DNJB1_HUMAN | DYL2_HUMAN | EIF1A_HUMAN |
| DEN4C_HUMAN | DNJB2_HUMAN | DYLT1_HUMAN | EIF1_HUMAN |
| DENR_HUMAN | DNJB3_HUMAN | DYM_HUMAN | EIF3A_HUMAN |
| DEP1A_HUMAN | DNJB4_HUMAN | DYN1_HUMAN | EIF3B_HUMAN |
| DESM_HUMAN | DNJB6_HUMAN | DYN2_HUMAN | EIF3C_HUMAN |
| DESP_HUMAN | DNJC7_HUMAN | DYR_HUMAN | EIF3D_HUMAN |
| DEST_HUMAN | DNJC8_HUMAN | DZIP3_HUMAN | EIF3_HUMAN |
| DFFA_HUMAN | DNJC9_HUMAN | E2AK2_HUMAN | EIF3F_HUMAN |
| DHAK_HUMAN | DNLI1_HUMAN | E41L2_HUMAN | EIF3G_HUMAN |
| DHB11_HUMAN | DNLI3_HUMAN | E41L5_HUMAN | EIF3H_HUMAN |
| DHB12_HUMAN | DNM1L_HUMAN | E7EW20_HUMAN | EIF31_HUMAN |
| DHB4_HUMAN | DNMT1_HUMAN | E9PDP1_HUMAN | EIF3K_HUMAN |
| DHB7_HUMAN | DOCK7_HUMAN | E9PHA7_HUMAN | EIF3L_HUMAN |
| DHC24_HUMAN | DP13A_HUMAN | E9PI5_HUMAN | EIF3M_HUMAN |
| DHCR7_HUMAN | DPM1_HUMAN | EAA1_HUMAN | ELAV1_HUMAN |
| DHRS1_HUMAN | DPOA2_HUMAN | EAPP_HUMAN | ELAV2_HUMAN |
| DHRS3_HUMAN | DPOD1_HUMAN | EBP2_HUMAN | ELMD2_HUMAN |
| DHRS4_HUMAN | DPOE1_HUMAN | ECH1_HUMAN | ELOB_HUMAN |
| DHRS7_HUMAN | DPOE2_HUMAN | ECHA_HUMAN | ELOC_HUMAN |
| DHSO_HUMAN | DPOE3_HUMAN | ECHM_HUMAN | ELP1_HUMAN |
| DHX15_HUMAN | DPOLA_HUMAN | ECM29_HUMAN | ELP2_HUMAN |
| DHX30_HUMAN | DPY30_HUMAN | EDC3_HUMAN | ELP3_HUMAN |
| DHX32_HUMAN | DPYL2_HUMAN | EDC4_HUMAN | EM55_HUMAN |
| DHX36_HUMAN | DREB_HUMAN | EDf1_HUMAN | EMAL3_HUMAN |
| EMAL4_HUMAN | EXOS9_HUMAN | FBX42_HUMAN | FWCH2_HUMAN |
| EMD_HUMAN | EZRI_HUMAN | FBXL3_HUMAN | FXR1_HUMAN |
| ENAH_HUMAN | F10A1_HUMAN | FBXL4_HUMAN | FYV1_HUMAN |
| ENOA_HUMAN | F115A_HUMAN | FCF1_HUMAN | FZD1_HUMAN |
| ENOPH_HUMAN | F120A_HUMAN | FCHO2_HUMAN | fZR_HUMAN |
| ENPLL_HUMAN | F125A_HUMAN | FCL_HUMAN | G2E3_HUMAN |
| ENPL_HUMAN | F127A_HUMAN | FDFT_HUMAN | G3BP1_HUMAN |
| ENSA_HUMAN | F127B_HUMAN | FEM1A_HUMAN | G3BP2_HUMAN |
| EP15R_HUMAN | F136A_HUMAN | FEM1B_HUMAN | G3P_HUMAN |
| EP400_HUMAN | F17SB_HUMAN | FEN1_HUMAN | G6PI_HUMAN |
| EPCAM_HUMAN | F188A_HUMAN | FETUA_HUMAN | GA45A_HUMAN |
| EPHA2_HUMAN | F19SB_HUMAN | FHL1_HUMAN | GAK_HUMAN |
| EPHA7_HUMAN | F208A_HUMAN | FHL3_HUMAN | GANAB_HUMAN |
| EPIPL_HUMAN | F263_HUMAN | FIBP_HUMAN | GAPD1_HUMAN |
| EPN1_HUMAN | F6XY72_HUMAN | FIP1_HUMAN | GAR1_HUMAN |
| EPN2_HUMAN | F8VZ13_HUMAN | FIS1_HUMAN | GASP2_HUMAN |
| Ef'N4_HUMAN | F92A1_HUMAN | FKB1_HUMAN | GATL1_HUMAN |
| EPS15_HUMAN | FA40A_HUMAN | FKBP3_HUMAN | GBB1_HUMAN |
| ERBB4_HUMAN | FA49B_HUMAN | FKBP4_HUMAN | GBB2_HUMAN |
| ERC6L_HUMAN | FA50A_HUMAN | FKBPS_HUMAN | GBB4_HUMAN |

(continued)

| | | | |
|---|---|---|---|
| ERCC2_HUMAN | FA54A_HUMAN | FKBP8_HUMAN | GBF1_HUMAN |
| ERCC3_HUMAN | FAS4B_HUMAN | FL2D_HUMAN | GBG12_HUMAN |
| ERCC5_HUMAN | FA63A_HUMAN | FLII_HUMAN | GBG5_HUMAN |
| ERCC6_HUMAN | FA98A_HUMAN | FLNA_HUMAN | GBLP_HUMAN |
| ERF1_HUMAN | FABP5_HUMAN | FLNB_HUMAN | GBRAP_HUMAN |
| ERF3A_HUMAN | FACD2_HUMAN | FLOT1_HUMAN | GBRL2_HUMAN |
| ERG1_HUMAN | FACE1_HUMAN | FLOT2_HUMAN | GCC2_HUMAN |
| ERG7_HUMAN | FACR1_HUMAN | FLVC1_HUMAN | GCF_HUMAN |
| ERH_HUMAN | FADS2_HUMAN | FMR1_HUMAN | GCN1L_HUMAN |
| ERI3_HUMAN | FAF1_HUMAN | FNBP1_HUMAN | GCP2_HUMAN |
| ESPL1_HUMAN | FAF2_HUMAN | FOPNL_HUMAN | GCP4_HUMAN |
| ESTD_HUMAN | FAIM1_HUMAN | FOXC1_HUMAN | GCP60_HUMAN |
| ESYT1_HUMAN | FAKD1_HUMAN | FPPS_HUMAN | GDAP1_HUMAN |
| ETFA_HUMAN | FANCA_HUMAN | FRYL_HUMAN | GDAP2_HUMAN |
| ETUD1_HUMAN | FANCI_HUMAN | FTM_HUMAN | GDE_HUMAN |
| EWS_HUMAN | FANCJ_HUMAN | FTO_HUMAN | GDIA_HUMAN |
| EXD2_HUMAN | FAS_HUMAN | FUBP1_HUMAN | GDIB_HUMAN |
| EXOC1_HUMAN | FBRL_HUMAN | FUBP2_HUMAN | GDIR1_HUMAN |
| EXOC2_HUMAN | FBX21_HUMAN | FUBP3_HUMAN | GDPD1_HUMAN |
| EXOC4_HUMAN | FBX28_HUMAN | FUMH_HUMAN | GDS1_HUMAN |
| EXOS5_HUMAN | FBX32_HUMAN | FUND1_HUMAN | GEMI4_HUMAN |
| EXOS6_HUMAN | FBX38_HUMAN | FUND2_HUMAN | GEMI5_HUMAN |
| EXOS8_HUMAN | FBX3_HUMAN | FUS_HUMAN | GEMI6_HUMAN |
| GEMI_HUMAN | GORS2_HUMAN | H2B1A_HUMAN | HES1_HUMAN |
| GFPT1_HUMAN | GOSR1_HUMAN | H2B1B_HUMAN | HEXI1_HUMAN |
| GFRP_HUMAN | GOT1B_HUMAN | H2B1C_HUMAN | HGB1A_HUMAN |
| GGA1_HUMAN | GPAA1_HUMAN | H2B1D_HUMAN | HGS_HUMAN |
| GGA3_HUMAN | GPAT1_HUMAN | H2B1H_HUMAN | HIF1N_HUMAN |
| GGCT_HUMAN | GPHRA_HUMAN | H2B1J_HUMAN | HINT1_HUMAN |
| GGPPS_HUMAN | GPI8_HUMAN | H31T_HUMAN | HINT3_HUMAN |
| GIPC1_HUMAN | GPKOW_HUMAN | H33_HUMAN | HIP1_HUMAN |
| GKAP1_HUMAN | GPM6B_HUMAN | H4_HUMAN | HLTF_HUMAN |
| GLCNE_HUMAN | GPTC4_HUMAN | H90B2_HUMAN | HM13_HUMAN |
| GLMN_HUMAN | GPTC8_HUMAN | H90B3_HUMAN | HMCS1_HUMAN |
| GLNA_HUMAN | GRB2_HUMAN | HACD2_HUMAN | HMDH_HUMAN |
| GLO2_HUMAN | GRHL2_HUMAN | HACD3_HUMAN | HMG3M_HUMAN |
| GLOD4_HUMAN | GRHPR_HUMAN | HAP28_HUMAN | HMGB1_HUMAN |
| GLP3L_HUMAN | GRK6_HUMAN | HAT1_HUMAN | HMGB2_HUMAN |
| GLPK3_HUMAN | GRP75_HUMAN | HAUS1_HUMAN | HMGB3_HUMAN |
| GLPK5_HUMAN | GRP78_HUMAN | HAUS3_HUMAN | HMGN1_HUMAN |
| GLPK_HUMAN | GRSF1_HUMAN | HAUS5_HUMAN | HMGN2_HUMAN |
| GLRX3_HUMAN | GSHR_HUMAN | HAUS6_HUMAN | HMGN3_HUMAN |
| GLTP_HUMAN | GSK3A_HUMAN | HAUS7_HUMAN | HMGN4_HUMAN |
| GLYC_HUMAN | GSTA4_HUMAN | HAUS8_HUMAN | HMGN5_HUMAN |
| GLYR1_HUMAN | GSTM3_HUMAN | HAX1_HUMAN | HMOX2_HUMAN |
| GMFB_HUMAN | GSTO1_HUMAN | HBS1L_HUMAN | HN1_HUMAN |
| GMPPB_HUMAN | GSTP1_HUMAN | HCD2_HUMAN | HNRCL_HUMAN |
| GNA11_HUMAN | GTF2I_HUMAN | HCFC1_HUMAN | HNRDL_HUMAN |
| GNA13_HUMAN | GTPB1_HUMAN | HDAC1_HUMAN | HNRH1_HUMAN |
| GNA1_HUMAN | GTR1_HUMAN | HDAC2_HUMAN | HNRH2_HUMAN |
| GNAI1_HUMAN | GUAA_HUMAN | HDDC2_HUMAN | HNRH3_HUMAN |

(continued)

| | | | |
|---|---|---|---|
| GNA3_HUMAN | GWL_HUMAN | HDGF_HUMAN | HNRL1_HUMAN |
| GNAL_HUMAN | GYS1_HUMAN | HDGR2_HUMAN | HNRL2_HUMAN |
| GNAQ_HUMAN | H11_HUMAN | HD_HUMAN | HNRLL_HUMAN |
| GNAS1_HUMAN | H12_HUMAN | HEAT1_HUMAN | HNRPC_HUMAN |
| GNAS2_HUMAN | H1X_HUMAN | HEAT2_HUMAN | HNRPD_HUMAN |
| GNAZ_HUMAN | H2A1A_HUMAN | HEAT3_HUMAN | HNRPF_HUMAN |
| GNL3_HUMAN | H2A1B_HUMAN | HECD1_HUMAN | HNRPG_HUMAN |
| GNPAT_HUMAN | H2A1D_HUMAN | HECD3_HUMAN | HNRPK_HUMAN |
| GNPI1_HUMAN | H2A2B_HUMAN | HELC1_HUMAN | HNRPL_HUMAN |
| GOGA5_HUMAN | H2A2C_HUMAN | HELLS_HUMAN | HNRPM_HUMAN |
| GOGA7_HUMAN | H2AV_HUMAN | HEM3_HUMAN | HNRPQ_HUMAN |
| GOGB1_HUMAN | H2AW_HUMAN | HERC1_HUMAN | HNRPR_HUMAN |
| GOLI_HUMAN | H2AX_HUMAN | HERC2_HUMAN | HNRPU_HUMAN |
| GOLP3_HUMAN | H2AY_HUMAN | HERC3_HUMAN | HOIL1_HUMAN |
| GOPC_HUMAN | H2AZ_HUMAN | HERC5_HUMAN | HOOK1_HUMAN |
| HPBP1_HUMAN | IF2P_HUMAN | IQGA2_HUMAN | KAPO_HUMAN |
| HPRT_HUMAN | IF4A1_HUMAN | IQGA3_HUMAN | KAP2_HUMAN |
| HPS3_HUMAN | IF4A2_HUMAN | IR31P_HUMAN | KAPCA_HUMAN |
| HS105_HUMAN | IF4A3_HUMAN | IRAK1_HUMAN | KATS_HUMAN |
| HS71L_HUMAN | IF4B_HUMAN | IREB2_HUMAN | KBRS2_HUMAN |
| HS74L_HUMAN | IF4E2_HUMAN | IRF3_HUMAN | KC1A_HUMAN |
| HS902_HUMAN | IF4E_HUMAN | IRS4_HUMAN | KC1D_HUMAN |
| HS904_HUMAN | IF4G1_HUMAN | ISOC2_HUMAN | KC1G1_HUMAN |
| HS905_HUMAN | IF4G2_HUMAN | IST1_HUMAN | KC1G3_HUMAN |
| HS90A_HUMAN | IF4H_HUMAN | ITB1_HUMAN | KCC2B_HUMAN |
| HS90B_HUMAN | IF5A1_HUMAN | ITCH_HUMAN | KCC2D_HUMAN |
| HSBP1_HUMAN | IF5_HUMAN | ITFG3_HUMAN | KCMF1_HUMAN |
| HSDL1_HUMAN | IFT27_HUMAN | ITM2B_HUMAN | KCRB_HUMAN |
| HSF2_HUMAN | IFT43_HUMAN | ITM2C_HUMAN | KCT2_HUMAN |
| HSP71_HUMAN | IGBP1_HUMAN | ITPA_HUMAN | KCTD3_HUMAN |
| HSP72_HUMAN | IKKB_HUMAN | ITPR2_HUMAN | KCTD5_HUMAN |
| HSP74_HUMAN | ILF2_HUMAN | ITPR3_HUMAN | KCTD9_HUMAN |
| HSP7C_HUMAN | ILF3_HUMAN | ITSN1_HUMAN | KDIS_HUMAN |
| HSPB1_HUMAN | ILKAP_HUMAN | ITSN2_HUMAN | KDM1A_HUMAN |
| HTAI2_HUMAN | ILK_HUMAN | IWS1_HUMAN | KDM3A_HUMAN |
| HTR5A_HUMAN | ILVBL_HUMAN | JAK1_HUMAN | KDM3B_HUMAN |
| HTSF1_HUMAN | IMA2_HUMAN | JAM1_HUMAN | KDM4A_HUMAN |
| HUWE1_HUMAN | IMA3_HUMAN | JIP4_HUMAN | KDM4B_HUMAN |
| HXB9_HUMAN | IMB1_HUMAN | JMJD6_HUMAN | KDMSC_HUMAN |
| HXK1_HUMAN | IMDH1_HUMAN | JOS1_HUMAN | KDM6A_HUMAN |
| HXK2_HUMAN | IMDH2_HUMAN | JUN_HUMAN | KEAP1_HUMAN |
| HYOU1_HUMAN | IMMT_HUMAN | K0090_HUMAN | KHDR1_HUMAN |
| I2BP1_HUMAN | IMPCT_HUMAN | K0195_HUMAN | KHNYN_HUMAN |
| I2BP2_HUMAN | INAR1_HUMAN | K0664_HUMAN | KI20A_HUMAN |
| ICAL_HUMAN | INGR1_HUMAN | K0889_HUMAN | KI67_HUMAN |
| ICLN_HUMAN | INO1_HUMAN | K1328_HUMAN | KIF11_HUMAN |
| ID4_HUMAN | INT3_HUMAN | K1797_HUMAN | KIF14_HUMAN |
| IDE_HUMAN | INT7_HUMAN | K1967_HUMAN | KIF1A_HUMAN |
| IDHC_HUMAN | IPO11_HUMAN | K1C18_HUMAN | KIF1B_HUMAN |
| IDI1_HUMAN | IPO4_HUMAN | K1C19_HUMAN | KIF22_HUMAN |
| IF1AX_HUMAN | IPO5_HUMAN | K2C8_HUMAN | KIF23_HUMAN |

(continued)

| | | | |
|---|---|---|---|
| IF2A_HUMAN | IPO7_HUMAN | K6PF_HUMAN | KIF2A_HUMAN |
| IF2B1_HUMAN | IPO8_HUMAN | K6PL_HUMAN | KIF2C_HUMAN |
| IF2B2_HUMAN | IPO9_HUMAN | K6PP_HUMAN | KIF4A_HUMAN |
| IF2B3_HUMAN | IPYR2_HUMAN | KAD1_HUMAN | KIF5A_HUMAN |
| IF2B_HUMAN | IPYR_HUMAN | KAD2_HUMAN | KIF7_HUMAN |
| IF2GL_HUMAN | IQCB1_HUMAN | KAD6_HUMAN | KIFC1_HUMAN |
| IF2G_HUMAN | IQGA1_HUMAN | KAISO_HUMAN | KIN17_HUMAN |
| KINH_HUMAN | LIMS1_HUMAN | LZTL1_HUMAN | MD1L1_HUMAN |
| KIRR1_HUMAN | LIN7C_HUMAN | LZTR1_HUMAN | MD2L1_HUMAN |
| KLC1_HUMAN | LIPA1_HUMAN | M1IP1_HUMAN | MD2L2_HUMAN |
| KLH11_HUMAN | LIS1_HUMAN | M89BB_HUMAN | MDC1_HUMAN |
| KLH13_HUMAN | LITFL_HUMAN | MA7D1_HUMAN | MDHC_HUMAN |
| KLH15_HUMAN | LKHA4_HUMAN | MA7D3_HUMAN | MDHM_HUMAN |
| KLHL7_HUMAN | LLPH_HUMAN | MACOI_HUMAN | MDM2_HUMAN |
| KLHL9_HUMAN | LLR1_HUMAN | MAGD1_HUMAN | MDN1_HUMAN |
| KNTC1_HUMAN | LMAN1_HUMAN | MAGD2_HUMAN | MED10_HUMAN |
| KPCD_HUMAN | LMBD1_HUMAN | MAGD4_HUMAN | MED1_HUMAN |
| KPCI_HUMAN | LMBD2_HUMAN | MAGE1_HUMAN | MED22_HUMAN |
| KPRA_HUMAN | LMBL3_HUMAN | MALD2_HUMAN | MED25_HUMAN |
| KPRB_HUMAN | LMCD1_HUMAN | MAP1B_HUMAN | MED29_HUMAN |
| KPYM_HUMAN | LMNA_HUMAN | MAP4_HUMAN | MED4_HUMAN |
| KT3K_HUMAN | LMNB1_HUMAN | MARCS_HUMAN | MEIS1_HUMAN |
| KTN1_HUMAN | LMNB2_HUMAN | MARE1_HUMAN | MEIS2_HUMAN |
| KTNA1_HUMAN | LN28B_HUMAN | MARHS_HUMAN | MELK_HUMAN |
| L2GL1_HUMAN | LNP_HUMAN | MARH6_HUMAN | MERL_HUMAN |
| L2GL2_HUMAN | LPP3_HUMAN | MARK3_HUMAN | MERTK_HUMAN |
| LAMC1_HUMAN | LPPRC_HUMAN | MAT1_HUMAN | MET7A_HUMAN |
| LANC1_HUMAN | LRBA_HUMAN | MAT2B_HUMAN | METH_HUMAN |
| LANC2_HUMAN | LRC20_HUMAN | MATR3_HUMAN | METK2_HUMAN |
| LAP2A_HUMAN | LRC40_HUMAN | MAZ_HUMAN | MET_HUMAN |
| LAP2B_HUMAN | LRC41_HUMAN | MBB1A_HUMAN | MFA3L_HUMAN |
| LAP4A_HUMAN | LRC47_HUMAN | MBD3_HUMAN | MFAP1_HUMAN |
| LAR4B_HUMAN | LRC57_HUMAN | MBIP1_HUMAN | MFF_HUMAN |
| LARP1_HUMAN | LRC58_HUMAN | MBLC2_HUMAN | MFN1_HUMAN |
| LARP4_HUMAN | LRC59_HUMAN | MBNL1_HUMAN | MFN2_HUMAN |
| LAS1L_HUMAN | LRRC3_HUMAN | MBRL_HUMAN | MFSD1_HUMAN |
| LAT1_HUMAN | LRSM1_HUMAN | MCA3_HUMAN | MGAP_HUMAN |
| LAT3_HUMAN | LS14B_HUMAN | MCAF1_HUMAN | MGN2_HUMAN |
| LAT4_HUMAN | LSM12_HUMAN | MCES_HUMAN | MGRN1_HUMAN |
| LA_HUMAN | LSM4_HUMAN | MCL1_HUMAN | MIA3_HUMAN |
| LBR_HUMAN | LSM7_HUMAN | MCM10_HUMAN | MIA40_HUMAN |
| LC7L2_HUMAN | LSR_HUMAN | MCM2_HUMAN | MIB1_HUMAN |
| LC7L3_HUMAN | LST8_HUMAN | MCM3_HUMAN | MIB2_HUMAN |
| LCHN_HUMAN | LTOR1_HUMAN | MCM4_HUMAN | MICA3_HUMAN |
| LDHA_HUMAN | LTV1_HUMAN | MCM5_HUMAN | MID49_HUMAN |
| LDHB_HUMAN | LYN_HUMAN | MCM6_HUMAN | MIF_HUMAN |
| LEG8_HUMAN | LYPA1_HUMAN | MCM7_HUMAN | MIMIT_HUMAN |
| LEO1_HUMAN | LYPA2_HUMAN | MCM8_HUMAN | MINA_HUMAN |
| LGUL_HUMAN | LYPL1_HUMAN | MCMBP_HUMAN | MINT_HUMAN |
| LIFR_HUMAN | LYRIC_HUMAN | MCRS1_HUMAN | MIO_HUMAN |
| MIRO1_HUMAN | MRP_HUMAN | NAA15_HUMAN | NELFA_HUMAN |

(continued)

| | | | |
|---|---|---|---|
| MIRO2_HUMAN | MRT4_HUMAN | NAA16_HUMAN | NEMF_HUMAN |
| MK01_HUMAN | MS18A_HUMAN | NAA25_HUMAN | NEMO_HUMAN |
| MK03_HUMAN | MSH2_HUMAN | NAA40_HUMAN | NEP1_HUMAN |
| MK14_HUMAN | MSH6_HUMAN | NAA50_HUMAN | NEUA_HUMAN |
| MK67I_HUMAN | MTA1_HUMAN | NACAD_HUMAN | NEUL4_HUMAN |
| MKLN1_HUMAN | MTA2_HUMAN | NACA_HUMAN | NEUL_HUMAN |
| MKRN1_HUMAN | MTAP_HUMAN | NACC1_HUMAN | NFIP1_HUMAN |
| MKRN2_HUMAN | MTBP_HUMAN | NADAP_HUMAN | NFIP2_HUMAN |
| MLL1_HUMAN | MTCH2_HUMAN | NAMPT_HUMAN | NFL_HUMAN |
| MLL2_HUMAN | MTFR1_HUMAN | NASP_HUMAN | NFX1_HUMAN |
| MMGT1_HUMAN | MTL13_HUMAN | NAT10_HUMAN | NFXL1_HUMAN |
| MMS19_HUMAN | MTL14_HUMAN | NB5R1_HUMAN | NFYC_HUMAN |
| MMS22_HUMAN | MTMR3_HUMAN | NB5R3_HUMAN | NGLY1_HUMAN |
| MMTA2_HUMAN | MTMR6_HUMAN | NBN_HUMAN | NH2L1_HUMAN |
| MO4L1_HUMAN | MTMR8_HUMAN | NBR1_HUMAN | NHP2_HUMAN |
| MO4L2_HUMAN | MTMR9_HUMAN | NC2A_HUMAN | NIBL1_HUMAN |
| MOB1A_HUMAN | MTOR_HUMAN | NCBP1_HUMAN | NIP7_HUMAN |
| MOC2A_HUMAN | MTPN_HUMAN | NCDN_HUMAN | NIPA_HUMAN |
| MOC2B_HUMAN | MTR1_HUMAN | NCKP1_HUMAN | NIPBL_HUMAN |
| MOES_HUMAN | MTRR_HUMAN | NCOAT_HUMAN | NISCH_HUMAN |
| MOFA1_HUMAN | MTX1_HUMAN | NDC1_HUMAN | NIT2_HUMAN |
| MON2_HUMAN | MTX2_HUMAN | NDK3_HUMAN | NKAPL_HUMAN |
| MORC3_HUMAN | MTX3_HUMAN | NDK8_HUMAN | NKAP_HUMAN |
| MORC4_HUMAN | MUL1_HUMAN | NDKA_HUMAN | NKRF_HUMAN |
| MOSC1_HUMAN | MXRA7_HUMAN | NDKB_HUMAN | NLTP_HUMAN |
| MOSC2 _ HUMAN | MYCB2_HUMAN | NDRG1_HUMAN | NMD3_HUMAN |
| MOT10_HUMAN | MYCBP_HUMAN | NDUA1_HUMAN | NMNA1_HUMAN |
| MOT1_HUMAN | MYC_HUMAN | NDUA4_HUMAN | NMT1_HUMAN |
| MOV10_HUMAN | MYH10_HUMAN | NDUA5_HUMAN | NOB1_HUMAN |
| MP2K1_HUMAN | MYH11_HUMAN | NDUA6_HUMAN | NOC2L_HUMAN |
| MP2K3_HUMAN | MYH9_HUMAN | NDUA8_HUMAN | NOL11_HUMAN |
| MP2K6_HUMAN | MYL6B_HUMAN | NDUA9_HUMAN | NOL9_HUMAN |
| MPCP_HUMAN | MYL6_HUMAN | NDUAD_HUMAN | NOLC1_HUMAN |
| MPI_HUMAN | MYO19_HUMAN | NDUB6_HUMAN | NOMO1_HUMAN |
| MPP6_HUMAN | MYO1B_HUMAN | NDUB8_HUMAN | NOMO2_HUMAN |
| MPRIP_HUMAN | MYO1C_HUMAN | NDUBA_HUMAN | NONO_HUMAN |
| MPRI_HUMAN | MYO1D_HUMAN | NDUC2_HUMAN | NOP56_HUMAN |
| MPZL1_HUMAN | MYO6_HUMAN | NDUS5_HUMAN | NOP58_HUMAN |
| MR1L1_HUMAN | MYPT1_HUMAN | NECP1_HUMAN | NOSIP_HUMAN |
| MRE11_HUMAN | MYSM1_HUMAN | NEDD8_HUMAN | NOTC3_HUMAN |
| MRP1_HUMAN | MZT1_HUMAN | NEK2_HUMAN | NP1L1_HUMAN |
| MRP4_HUMAN | NAA10_HUMAN | NEK9_HUMAN | NP1L4_HUMAN |
| NPA1P_HUMAN | NUP62_HUMAN | P66B_HUMAN | PDCD5_HUMAN |
| NPDC1_HUMAN | NUP85_HUMAN | P73_HUMAN | PDCL3_HUMAN |
| NPL4_HUMAN | NUP93_HUMAN | PA1B2_HUMAN | PDE12_HUMAN |
| NPM_HUMAN | NUP98_HUMAN | PA2G4_HUMAN | PDIA1_HUMAN |
| NPRL3_HUMAN | NVL_HUMAN | PAAF1_HUMAN | PDIA3_HUMAN |
| NRDC_HUMAN | NXT1_HUMAN | PABP1_HUMAN | PDIP3_HUMAN |
| NRP1_HUMAN | NYNRI_HUMAN | PABP2_HUMAN | PDK1L_HUMAN |
| NSD1_HUMAN | OBSL1_HUMAN | PABP4_HUMAN | PDLI1_HUMAN |
| NSD2_HUMAN | OCAD1_HUMAN | PACE1_HUMAN | PDLI5_HUMAN |

(continued)

| | | | |
|---|---|---|---|
| NSDHL_HUMAN | OCLN_HUMAN | PACN3_HUMAN | PDPK1_HUMAN |
| NSE4A_HUMAN | OCRL_HUMAN | PAF1_HUMAN | PDRG1_HUMAN |
| NSF1C_HUMAN | ODFP2_HUMAN | PAF_HUMAN | PDS5A_HUMAN |
| NSF_HUMAN | ODPB_HUMAN | PAG16_HUMAN | PDXD1_HUMAN |
| NSMA3_HUMAN | OFD1_HUMAN | PAIP2_HUMAN | PDZ11_HUMAN |
| NSUN2_HUMAN | OGFD1_HUMAN | PAIRB_HUMAN | PEA15_HUMAN |
| NSUN5_HUMAN | OGFR_HUMAN | PALM_HUMAN | PEBP1_HUMAN |
| NT5D1_HUMAN | OGT1_HUMAN | PAMM_HUMAN | PEG10_HUMAN |
| NTCP4_HUMAN | OLA1_HUMAN | PANK3_HUMAN | PELO_HUMAN |
| NTF2_HUMAN | OPTN_HUMAN | PANX1_HUMAN | PEPD_HUMAN |
| NTM1A_HUMAN | ORC2_HUMAN | PAPOA_HUMAN | PERI_HUMAN |
| NTPCR_HUMAN | ORC5_HUMAN | PAPS1_HUMAN | PERQ2_HUMAN |
| NU107_HUMAN | ORN_HUMAN | PAPS2_HUMAN | PESC_HUMAN |
| NU133_HUMAN | OSB10_HUMAN | PAR12_HUMAN | PEX13_HUMAN |
| NU153_HUMAN | OSBL3_HUMAN | PAR1_HUMAN | PEX19_HUMAN |
| NU155_HUMAN | OSBL9_HUMAN | PARG_HUMAN | PEX3_HUMAN |
| NU160_HUMAN | OSGEP_HUMAN | PARK7_HUMAN | PEX5_HUMAN |
| NU188_HUMAN | OST48_HUMAN | PARP1_HUMAN | PFD2_HUMAN |
| NU205_HUMAN | OSTC_HUMAN | PAWR_HUMAN | PFD3_HUMAN |
| NUB1_HUMAN | OSTM1_HUMAN | PB1_HUMAN | PFD5_HUMAN |
| NUCKS_HUMAN | OTU1_HUMAN | PBX2_HUMAN | PFD6_HUMAN |
| NUCL_HUMAN | OTU6B_HUMAN | PCBP1_HUMAN | PGAM1_HUMAN |
| NUD19_HUMAN | OTUB1_HUMAN | PCBP2_HUMAN | PGAM5_HUMAN |
| NUDC1_HUMAN | OTUD5_HUMAN | PCGF6_HUMAN | PGES2_HUMAN |
| NUDC2_HUMAN | OXA1L_HUMAN | PCH2_HUMAN | PGK1_HUMAN |
| NUDC_HUMAN | OXR1_HUMAN | PCID2_HUMAN | PGM1_HUMAN |
| NUDT5_HUMAN | P121A_HUMAN | PCM1_HUMAN | PGM2_HUMAN |
| NUF2_HUMAN | P20D2_HUMAN | PCNA_HUMAN | PGP_HUMAN |
| NUFP2_HUMAN | P3C2A_HUMAN | PCNP_HUMAN | PGRC1_HUMAN |
| NUMA1_HUMAN | P3C2B_HUMAN | PCNT_HUMAN | PGRC2_HUMAN |
| NUP37_HUMAN | P4K2A_HUMAN | PCX3_HUMAN | PGTB2_HUMAN |
| NUP50_HUMAN | P4K2B_HUMAN | PDC10_HUMAN | PHB2_HUMAN |
| NUP53_HUMAN | P4R3A_HUMAN | PDC6I_HUMAN | PHB_HUMAN |
| NUP54_HUMAN | P53_HUMAN | PDCD4_HUMAN | PHC2_HUMAN |
| PHF10_HUMAN | PLST_HUMAN | PPME1_HUMAN | PRS8_HUMAN |
| PHF14_HUMAN | PLXA1_HUMAN | PPP5_HUMAN | PSA1_HUMAN |
| PHF5A_HUMAN | PLXA2_HUMAN | PPT1_HUMAN | PSA2_HUMAN |
| PHF6_HUMAN | PLXB2_HUMAN | PPWD1_HUMAN | PSA3_HUMAN |
| PHIP_HUMAN | PM14_HUMAN | PR38A_HUMAN | PSA4_HUMAN |
| PHLP_HUMAN | PMF1_HUMAN | PR38B_HUMAN | PSA5_HUMAN |
| PHP14_HUMAN | PMGE_HUMAN | PR40A_HUMAN | PSA6_HUMAN |
| PI42A_HUMAN | PML_HUMAN | PRAF1_HUMAN | PSA7L_HUMAN |
| PI42C_HUMAN | PMVK_HUMAN | PRAF3_HUMAN | PSA7_HUMAN |
| PI4KA_HUMAN | PNKP_HUMAN | PRAME_HUMAN | PSA_HUMAN |
| PI51A_HUMAN | PNMA1_HUMAN | PRC1_HUMAN | PSB1_HUMAN |
| PI51C_HUMAN | PNMA2_HUMAN | PRC2A_HUMAN | PSB2_HUMAN |
| PIAS1_HUMAN | PNML1_HUMAN | PRC2C_HUMAN | PSB3_HUMAN |
| PIBF1_HUMAN | PNO1_HUMAN | PRCC_HUMAN | PSB4_HUMAN |
| PICAL_HUMAN | PNPH_HUMAN | PRDX1_HUMAN | PSB5_HUMAN |
| PIGU_HUMAN | PO2F1_HUMAN | PRDX2_HUMAN | PSB7_HUMAN |
| PIMT_HUMAN | POGK_HUMAN | PRDX3_HUMAN | PSD10_HUMAN |

(continued)

| | | | |
|---|---|---|---|
| PIN1_HUMAN | POLH_HUMAN | PRDX4_HUMAN | PSD11_HUMAN |
| PIN4_HUMAN | POLI_HUMAN | PRDX5_HUMAN | PSD12_HUMAN |
| PININ_HUMAN | POLK_HUMAN | PRDX6_HUMAN | PSD13_HUMAN |
| PIPNA_HUMAN | POMP_HUMAN | PREB_HUMAN | PSD7_HUMAN |
| PIPNB_HUMAN | POP1_HUMAN | PRI1_HUMAN | PSDE_HUMAN |
| PIPSL_HUMAN | POP7_HUMAN | PRI2_HUMAN | PSF1_HUMAN |
| PJA1_HUMAN | PP1A_HUMAN | PRKDC_HUMAN | PSIP1_HUMAN |
| PJA2_HUMAN | PP1G_HUMAN | PRKN2_HUMAN | PSMD1_HUMAN |
| PK3CA_HUMAN | PP1RA_HUMAN | PROF1_HUMAN | PSMD2_HUMAN |
| PKHA1_HUMAN | PP2AA_HUMAN | PROF2_HUMAN | PSMD3_HUMAN |
| PKHA7_HUMAN | PP2AB_HUMAN | PROSC_HUMAN | PSMD4_HUMAN |
| PKHH3_HUMAN | PP4C_HUMAN | PRP16_HUMAN | PSMD6_HUMAN |
| PKN1_HUMAN | PP4R2_HUMAN | PRP19_HUMAN | PSMD8_HUMAN |
| PKN2_HUMAN | PP6R3_HUMAN | PRP31_HUMAN | PSMD9_HUMAN |
| PKNX1_HUMAN | PPAC_HUMAN | PRP4_HUMAN | PSME1_HUMAN |
| PKP4_HUMAN | PPCEL_HUMAN | PRP6_HUMAN | PSME2_HUMAN |
| PLAK_HUMAN | PPCE_HUMAN | PRP8_HUMAN | PSME3_HUMAN |
| PLAP_HUMAN | PPDPF_HUMAN | PRPF3_HUMAN | PSMG1_HUMAN |
| PLCE_HUMAN | PPIA_HUMAN | PRPS1_HUMAN | PSMG2_HUMAN |
| PLCG1_HUMAN | PPIB_HUMAN | PRPS2_HUMAN | PSMG3_HUMAN |
| PLD3_HUMAN | PPID_HUMAN | PRR11_HUMAN | PTBP1_HUMAN |
| PLEC_HUMAN | PPIG_HUMAN | PRS10_HUMAN | PTBP2_HUMAN |
| PLIN3_HUMAN | PPIH_HUMAN | PRS4_HUMAN | PTH2_HUMAN |
| PLK1_HUMAN | PPIL4_HUMAN | PRS6A_HUMAN | PTK7_HUMAN |
| PLRG1_HUMAN | PPM1B_HUMAN | PRS6B_HUMAN | PTMA_HUMAN |
| PLSL_HUMAN | PPM1G_HUMAN | PRS7_HUMAN | PTMS_HUMAN |
| PTN11_HUMAN | QRIC1_HUMAN | RB39A_HUMAN | RER1_HUMAN |
| PTN23_HUMAN | QTRD1_HUMAN | RB3GP_HUMAN | RERE_HUMAN |
| PTN2_HUMAN | R13AX_HUMAN | RB612_HUMAN | RFA1_HUMAN |
| PTOV1_HUMAN | RA1L2_HUMAN | RBBP4_HUMAN | RFA2_HUMAN |
| PTPRA_HUMAN | RA51C_HUMAN | RBBP5_HUMAN | RFA3_HUMAN |
| PTPRF_HUMAN | RAB10_HUMAN | RBBP6_HUMAN | RFC2_HUMAN |
| PTPRG_HUMAN | RAB13_HUMAN | RBBP7_HUMAN | RFC3_HUMAN |
| PTPS_HUMAN | RAB14_HUMAN | RBGPR_HUMAN | RFC4_HUMAN |
| PTRF_HUMAN | RAB1A_HUMAN | RBM12_HUMAN | RFC5_HUMAN |
| PTSS1_HUMAN | RAB21_HUMAN | RBM14_HUMAN | RFIP1_HUMAN |
| PTTG1_HUMAN | RAB24_HUMAN | RBM15_HUMAN | RFWD3_HUMAN |
| PTTG_HUMAN | RAB2A_HUMAN | RBM22_HUMAN | RGAP1_HUMAN |
| PUF60_HUMAN | RAB34_HUMAN | RBM23_HUMAN | RHBD2_HUMAN |
| PUM1_HUMAN | RAB35_HUMAN | RBM26_HUMAN | RHBT3_HUMAN |
| PUR2_HUMAN | RAB3B_HUMAN | RBM27_HUMAN | RHEB_HUMAN |
| PUR6_HUMAN | RAB5A_HUMAN | RBM28_HUMAN | RHG05_HUMAN |
| PUR8_HUMAN | RAB5B_HUMAN | RBM39_HUMAN | RHG22_HUMAN |
| PUR9_HUMAN | RAB5C_HUMAN | RBM42_HUMAN | RHOA_HUMAN |
| PURA2_HUMAN | RAB7A_HUMAN | RBM4B_HUMAN | RHOU_HUMAN |
| PUS7_HUMAN | RAB8A_HUMAN | RBM4_HUMAN | RIF1_HUMAN |
| PVRL2_HUMAN | RABE1_HUMAN | RBMS1_HUMAN | RIFK_HUMAN |
| PVRL3_HUMAN | RABE2_HUMAN | RBP2_HUMAN | RING2_HUMAN |
| PWP1_HUMAN | RABP2_HUMAN | RBP56_HUMAN | RINI_HUMAN |
| PWP2_HUMAN | RABX5_HUMAN | RBX1_HUMAN | RIOK1_HUMAN |
| PYGB_HUMAN | RAC1_HUMAN | RB_HUMAN | RIOK2_HUMAN |

(continued)

| | | | |
|---|---|---|---|
| PYGL_HUMAN | RAD18_HUMAN | RCC1_HUMAN | RIOK3_HUMAN |
| PYR1_HUMAN | RAD1_HUMAN | RCC2_HUMAN | RIR1_HUMAN |
| PYRG1_HUMAN | RAD21_HUMAN | RCCD1_HUMAN | RIR2B_HUMAN |
| Q13384_HUMAN | RAD50_HUMAN | RCD1_HUMAN | RIR2_HUMAN |
| Q59GX9_HUMAN | RADI_HUMAN | RCL1_HUMAN | RL10A_HUMAN |
| Q5FWY2_HUMAN | RAE1L_HUMAN | RCN1_HUMAN | RL10L_HUMAN |
| Q5JWE8_HUMAN | RAGP1_HUMAN | RCN2_HUMAN | RL10_HUMAN |
| Q5LJA5_HUMAN | RAI14_HUMAN | RD23A_HUMAN | RL11_HUMAN |
| Q6FG99_HUMAN | RALYL_HUMAN | RD23B_HUMAN | RL12_HUMAN |
| Q6IPH7_HUMAN | RALY_HUMAN | RDH11_HUMAN | RL13A_HUMAN |
| Q6IQ27_HUMAN | RANG_HUMAN | RDH14_HUMAN | RL13_HUMAN |
| Q7Z5V0_HUMAN | RAN_HUMAN | RECQ1_HUMAN | RL14_HUMAN |
| O8NDP0_HUMAN | RAP1A_HUMAN | RED_HUMAN | RL1S_HUMAN |
| Q9HBI2_HUMAN | RAP2B_HUMAN | REEP4_HUMAN | RL17_HUMAN |
| Q9ULW9_HUMAN | RASK_HUMAN | REEP5_HUMAN | RL18A_HUMAN |
| QCR2_HUMAN | RASN_HUMAN | REN3B_HUMAN | RL18_HUMAN |
| QCR9_HUMAN | RB11A_HUMAN | RENT1_HUMAN | RL19_HUMAN |
| QKI_HUMAN | RB11B_HUMAN | REPI1_HUMAN | RL1D1_HUMAN |
| RL21_HUMAN | RN122_HUMAN | RPC2_HUMAN | RS2_HUMAN |
| RL22_HUMAN | RN123_HUMAN | RPC4_HUMAN | RS30_HUMAN |
| RL23A_HUMAN | RN138_HUMAN | RPF1_HUMAN | RS3A_HUMAN |
| RL23_HUMAN | RN141_HUMAN | RPIA_HUMAN | RS3_HUMAN |
| RL24_HUMAN | RN146_HUMAN | RPN1_HUMAN | RS4X_HUMAN |
| RL26L_HUMAN | RN166_HUMAN | RPN2_HUMAN | RS5_HUMAN |
| RL27A_HUMAN | RN167_HUMAN | RPP29_HUMAN | RS6_HUMAN |
| RL27_HUMAN | RN168_HUMAN | RPP30_HUMAN | RS7_HUMAN |
| RL28_HUMAN | RN185_HUMAN | RPR1B_HUMAN | RS8_HUMAN |
| RL29_HUMAN | RN187_HUMAN | RPRD2_HUMAN | RS9_HUMAN |
| RL30_HUMAN | RN213_HUMAN | RRAGA_HUMAN | RSBNL_HUMAN |
| RL31_HUMAN | RN216_HUMAN | RRAGC_HUMAN | RSCA1_HUMAN |
| RL32_HUMAN | RN219_HUMAN | RRBP1_HUMAN | RSF1_HUMAN |
| RL34_HUMAN | RN220_HUMAN | RRMJ1_HUMAN | RSMB_HUMAN |
| RL35A_HUMAN | RNBP6_HUMAN | RRMJ3_HUMAN | RSPRY_HUMAN |
| RL35_HUMAN | RNF10_HUMAN | RRP12_HUMAN | RSRC2_HUMAN |
| RL36A_HUMAN | RNF12_HUMAN | RRP1B_HUMAN | RSSA_HUMAN |
| RL36_HUMAN | RNF13_HUMAN | RRP1_HUMAN | RSU1_HUMAN |
| RL37A_HUMAN | RNF25_HUMAN | RRP44_HUMAN | RT06_HUMAN |
| RL37_HUMAN | RNF31_HUMAN | RRP5_HUMAN | RT21_HUMAN |
| RL38_HUMAN | RNF4_HUMAN | RRS1_HUMAN | RT27_HUMAN |
| RL3L_HUMAN | RNF5_HUMAN | RS10L_HUMAN | RTC1_HUMAN |
| RL3_HUMAN | RNH2A_HUMAN | RS10_HUMAN | RTCB_HUMAN |
| RL40_HUMAN | RNPS1_HUMAN | RS11_HUMAN | RTF1_HUMAN |
| RL4_HUMAN | RNZ2_HUMAN | RS12_HUMAN | RTN3_HUMAN |
| RL5_HUMAN | RO60_HUMAN | RS13_HUMAN | RTN4_HUMAN |
| RL6_HUMAN | ROA0_HUMAN | RS14_HUMAN | RU17_HUMAN |
| RL7A_HUMAN | ROA1_HUMAN | RS15A_HUMAN | RU1C_HUMAN |
| RL7L_HUMAN | ROA2_HUMAN | RS15_HUMAN | RU2A_HUMAN |
| RL7_HUMAN | ROA3_HUMAN | RS16_HUMAN | RU2B_HUMAN |
| RL8_HUMAN | ROAA_HUMAN | RS17L_HUMAN | RUFY1_HUMAN |
| RL9_HUMAN | ROBO1_HUMAN | RS18_HUMAN | RUVB1_HUMAN |
| RLA0L_HUMAN | RPA1_HUMAN | RS19_HUMAN | RUVB2_HUMAN |

(continued)

| | | | |
|---|---|---|---|
| RLA0_HUMAN | RPA49_HUMAN | RS20_HUMAN | RUXE_HUMAN |
| RLA1_HUMAN | RPAB1_HUMAN | RS21_HUMAN | RUXF_HUMAN |
| RLA2_HUMAN | RPAB5_HUMAN | RS23_HUMAN | RUXG_HUMAN |
| RM12_HUMAN | RPAP2_HUMAN | RS24_HUMAN | RWDD1_HUMAN |
| RM20_HUMAN | RPB11_HUMAN | RS25_HUMAN | RXRB_HUMAN |
| RM43_HUMAN | RPB1_HUMAN | RS26L_HUMAN | RYBP_HUMAN |
| RM53_HUMAN | RPB2_HUMAN | RS26_HUMAN | S10AA_HUMAN |
| RMD2_HUMAN | RPB7_HUMAN | RS27A_HUMAN | S10AB_HUMAN |
| RMD3_HUMAN | RPC10_HUMAN | RS28_HUMAN | S12A2_HUMAN |
| RN114_HUMAN | RPC1_HUMAN | RS29_HUMAN | S12A4_HUMAN |
| S12A6_HUMAN | SAS10_HUMAN | SETB1_HUMAN | SLK_HUMAN |
| S12A7_HUMAN | SAT1_HUMAN | SETD7_HUMAN | SLN11_HUMAN |
| S14L1_HUMAN | SATT_HUMAN | SET_HUMAN | SLU7_HUMAN |
| S15A4_HUMAN | SBDS_HUMAN | SF01_HUMAN | SMAD3_HUMAN |
| S18L2_HUMAN | SC11A_HUMAN | SF3A1_HUMAN | SMAD4_HUMAN |
| S19A1_HUMAN | SC11C_HUMAN | SF3A3_HUMAN | SMAP_HUMAN |
| S20A1_HUMAN | SC22B_HUMAN | SF3B1_HUMAN | SMC1A_HUMAN |
| S20A2_HUMAN | SC23A_HUMAN | SF3B2_HUMAN | SMC2_HUMAN |
| S22A5_HUMAN | SC23B_HUMAN | SF3B3_HUMAN | SMC3_HUMAN |
| S23A2_HUMAN | SC24C_HUMAN | SF3B5_HUMAN | SMC4_HUMAN |
| S23IP_HUMAN | SC31A_HUMAN | SFPQ_HUMAN | SMC6_HUMAN |
| S2546_HUMAN | SC5A3_HUMAN | SFR15_HUMAN | SMCA1_HUMAN |
| S2611_HUMAN | SC5D_HUMAN | SFR19_HUMAN | SMCA2_HUMAN |
| S26A6_HUMAN | SC6A8_HUMAN | SFSWA_HUMAN | SMCA4_HUMAN |
| S27A2_HUMAN | SCAFB_HUMAN | SFT2C_HUMAN | SMCA5_HUMAN |
| S29A1_HUMAN | SCAM1_HUMAN | SFXN1_HUMAN | SMCE1_HUMAN |
| S29A2_HUMAN | SCAM3_HUMAN | SGPL1_HUMAN | SMD1_HUMAN |
| S30BP_HUMAN | SCFD1_HUMAN | SGT1_HUMAN | SMD2_HUMAN |
| S35B2_HUMAN | SCLY_HUMAN | SGTA_HUMAN | SMD3_HUMAN |
| S35E1_HUMAN | SCML2_HUMAN | SGTB_HUMAN | SMG1_HUMAN |
| S38A1_HUMAN | SCO2_HUMAN | SH3G1_HUMAN | SMG8_HUMAN |
| S38A2_HUMAN | SCOC_HUMAN | SH3L1_HUMAN | SMHD1_HUMAN |
| S38A9_HUMAN | SCPDL_HUMAN | SH3L2_HUMAN | SMN_HUMAN |
| S39A6_HUMAN | SCRIB_HUMAN | SHIP1_HUMAN | SMOX_HUMAN |
| S39AA_HUMAN | SDC2_HUMAN | SHIP2_HUMAN | SMRC1_HUMAN |
| S39AE_HUMAN | SDCB1_HUMAN | SHKB1_HUMAN | SMRC2_HUMAN |
| S4A7_HUMAN | SDCG3_HUMAN | SHLB2_HUMAN | SMRCD_HUMAN |
| S61A1_HUMAN | SDSL_HUMAN | SHOT1_HUMAN | SMRD1_HUMAN |
| S6A15_HUMAN | SEC20_HUMAN | SHPK_HUMAN | SMU1_HUMAN |
| SAAL1_HUMAN | SEC62_HUMAN | SHPRH_HUMAN | SNAA_HUMAN |
| SAE1_HUMAN | SEC63_HUMAN | SHQ1_HUMAN | SNAG_HUMAN |
| SAE2_HUMAN | SEH1_HUMAN | SHRPN_HUMAN | SND1_HUMAN |
| SAFB1_HUMAN | SELR1_HUMAN | SIAS_HUMAN | SNF5_HUMAN |
| SAHH2_HUMAN | SENP3_HUMAN | SIN3A_HUMAN | SNF8_HUMAN |
| SAHH_HUMAN | SEP11_HUMAN | SIRT1_HUMAN | SNP23_HUMAN |
| SALL2_HUMAN | SEPT2_HUMAN | SIRT2_HUMAN | SNP29_HUMAN |
| SAM50_HUMAN | SEPT6_HUMAN | SIVA_HUMAN | SNP47_HUMAN |
| SAMH1_HUMAN | SEPT7_HUMAN | SK2L2_HUMAN | SNR40_HUMAN |
| SAP18_HUMAN | SEPT9_HUMAN | SKA2L_HUMAN | SNR48_HUMAN |
| SAR1A_HUMAN | SERA_HUMAN | SKIV2_HUMAN | SNRPA_HUMAN |
| SARM1_HUMAN | SERC1_HUMAN | SKI_HUMAN | SNTB2_HUMAN |

(continued)

| | | | |
|---|---|---|---|
| SARNP_HUMAN | SERC_HUMAN | SKP1_HUMAN | SNUT1_HUMAN |
| SART3_HUMAN | SESN1_HUMAN | SKP2_HUMAN | SNW1_HUMAN |
| SNX12_HUMAN | SRPK2_HUMAN | STRUM_HUMAN | SYRC_HUMAN |
| SNX1_HUMAN | SRPRB_HUMAN | STT3A_HUMAN | SYSC_HUMAN |
| SNX27_HUMAN | SRRM1_HUMAN | STX10_HUMAN | SYTC_HUMAN |
| SNX2_HUMAN | SRRM2_HUMAN | STX12_HUMAN | SYVC_HUMAN |
| SNX32_HUMAN | SRRT_HUMAN | STX16_HUMAN | SYWC_HUMAN |
| SNX5_HUMAN | SRR_HUMAN | STX17_HUMAN | SYYC_HUMAN |
| SNX6_HUMAN | SRS11_HUMAN | STX18_HUMAN | T106B_HUMAN |
| SNX8_HUMAN | SRSF1_HUMAN | STX4_HUMAN | T22D3_HUMAN |
| SO4A1_HUMAN | SRSF2_HUMAN | STX5_HUMAN | T2AG_HUMAN |
| SOAT1_HUMAN | SRSF3_HUMAN | STX6_HUMAN | T2EB_HUMAN |
| SODC_HUMAN | SRSF4_HUMAN | STX7_HUMAN | T2FB_HUMAN |
| SON_HUMAN | SRSF5_HUMAN | STX8_HUMAN | T2H2L_HUMAN |
| SORCN_HUMAN | SRSF6_HUMAN | STXB1_HUMAN | TAB2_HUMAN |
| SP16H_HUMAN | SRSF7_HUMAN | STXB2_HUMAN | TACC3_HUMAN |
| SPA5L_HUMAN | SRSF9_HUMAN | STXB3_HUMAN | TADBP_HUMAN |
| SPAG7_HUMAN | SSA27_HUMAN | SUFU_HUMAN | TAF10_HUMAN |
| SPB6_HUMAN | SSBP_HUMAN | SUGT1_HUMAN | TAF1B_HUMAN |
| SPC24_HUMAN | SSF1_HUMAN | SUMO1_HUMAN | TAF5L_HUMAN |
| SPDLY_HUMAN | SSNA1_HUMAN | SUMO2_HUMAN | TAF7_HUMAN |
| SPEE_HUMAN | SSPN_HUMAN | SUMO3_HUMAN | TAF9B_HUMAN |
| SF30_HUMAN | SSRA_HUMAN | SUN1_HUMAN | TAF9_HUMAN |
| SPF45_HUMAN | SSRD_HUMAN | SURF4_HUMAN | TAGL2_HUMAN |
| SPG20_HUMAN | SSRG_HUMAN | SUV91_HUMAN | TALDO_HUMAN |
| SPIT2_HUMAN | SSRP1_HUMAN | SUV92_HUMAN | TANC2_HUMAN |
| SPOP_HUMAN | SSU72_HUMAN | SUZ12_HUMAN | TAP2_HUMAN |
| SPRY7_HUMAN | ST1A1_HUMAN | SYAC_HUMAN | TARB1_HUMAN |
| SPSY_HUMAN | STABP_HUMAN | SYAP1_HUMAN | TATD1_HUMAN |
| SPT5H_HUMAN | STAG1_HUMAN | SYCC_HUMAN | TAXB1_HUMAN |
| SPT6H_HUMAN | STAG2_HUMAN | SYDC_HUMAN | TB10A_HUMAN |
| SPTA2_HUMAN | STAM1_HUMAN | SYEP_HUMAN | TB10B_HUMAN |
| SPTB2_HUMAN | STAM2_HUMAN | SYF1_HUMAN | TBA1A_HUMAN |
| SPTC1_HUMAN | STAT2_HUMAN | SYFA_HUMAN | TBA1B_HUMAN |
| SPTCS_HUMAN | STAT3_HUMAN | SYFB_HUMAN | TBA1C_HUMAN |
| SQSTM_HUMAN | STAU1_HUMAN | SYG_HUMAN | TBB2A_HUMAN |
| SR140_HUMAN | STEA3_HUMAN | SYHC_HUMAN | TBB3_HUMAN |
| SRC8_HUMAN | STIP1_HUMAN | SYIC_HUMAN | TBB4A_HUMAN |
| SREK1_HUMAN | STML2_HUMAN | SYJ2B_HUMAN | TBB4B_HUMAN |
| SRP09_HUMAN | STMN1_HUMAN | SYK_HUMAN | TBB5_HUMAN |
| SRP14_HUMAN | STPAP_HUMAN | SYLC_HUMAN | TBB6_HUMAN |
| SRP54_HUMAN | STRAP_HUMAN | SYMC_HUMAN | TBC15_HUMAN |
| SRP68_HUMAN | STRBP_HUMAN | SYMPK_HUMAN | TBC17_HUMAN |
| SRP72_HUMAN | STRN3_HUMAN | SYNC_HUMAN | TBCA_HUMAN |
| SRPK1_HUMAN | STRN4_HUMAN | SYQ_HUMAN | TBCB_HUMAN |
| TBCD4_HUMAN | TFG_HUMAN | TM7S3_HUMAN | TPC12_HUMAN |
| TBCD_HUMAN | TFR1_HUMAN | TM87A_HUMAN | TPD52_HUMAN |
| TBCE_HUMAN | TGFR1_HUMAN | TM9S3_HUMAN | TPD53_HUMAN |
| TBG1_HUMAN | TGS1_HUMAN | TM9S4_HUMAN | TPD54_HUMAN |
| TBL1R_HUMAN | THIC_HUMAN | TMCC1_HUMAN | TPIS_HUMAN |
| TBL2_HUMAN | THIO_HUMAN | TMCO1_HUMAN | TPM1_HUMAN |

(continued)

| | | | |
|---|---|---|---|
| TBL3_HUMAN | THOC2_HUMAN | TMCO7_HUMAN | TPM4_HUMAN |
| TBP_HUMAN | THOC3_HUMAN | TMED4_HUMAN | TPP2_HUMAN |
| TCAL1_HUMAN | THOC4_HUMAN | TMED9_HUMAN | TPPC1_HUMAN |
| TCAL4_HUMAN | THOC6_HUMAN | TMEDA_HUMAN | TPPC3_HUMAN |
| TCAL8_HUMAN | THOP1_HUMAN | TMM31_HUMAN | TPPC4_HUMAN |
| TCEA1_HUMAN | THTM_HUMAN | TMM59_HUMAN | TPPC5_HUMAN |
| TCOF_HUMAN | THTPA_HUMAN | TMM66_HUMAN | TPPC8_HUMAN |
| TCP4_HUMAN | THUM3_HUMAN | TMOD3_HUMAN | TPR_HUMAN |
| TCPA_HUMAN | TIAR_HUMAN | TMUB1_HUMAN | TPX2_HUMAN |
| TCPB_HUMAN | TIF1A_HUMAN | TMUB2_HUMAN | TR10B_HUMAN |
| TCPD_HUMAN | TIF1B_HUMAN | TMX1_HUMAN | TR10D_HUMAN |
| TCPE_HUMAN | TIFA_HUMAN | TMX2_HUMAN | TR150_HUMAN |
| TCPG_HUMAN | TIGAR_HUMAN | TNKS1_HUMAN | TRA2A_HUMAN |
| TCPH_HUMAN | TIM10_HUMAN | TNKS2_HUMAN | TRA2B_HUMAN |
| TCPQ_HUMAN | TIM13_HUMAN | TNPO1_HUMAN | TRABD_HUMAN |
| TCPW_HUMAN | TIM50_HUMAN | TNPO2_HUMAN | TRAD1_HUMAN |
| TCPZ_HUMAN | TIM8A_HUMAN | TNPO3_HUMAN | TRAF2_HUMAN |
| TCRG1_HUMAN | TIM8B_HUMAN | TNR6_HUMAN | TRAF4_HUMAN |
| TCTP_HUMAN | TIM9_HUMAN | TOIP1_HUMAN | TRAF7_HUMAN |
| TDIF2_HUMAN | TIM_HUMAN | TOLIP_HUMAN | TRAP1_HUMAN |
| TDRKH_HUMAN | TIPIN_HUMAN | TOM1_HUMAN | TRI11_HUMAN |
| TE2IP_HUMAN | TIPRL_HUMAN | TOM20_HUMAN | TRI18_HUMAN |
| TEAN2_HUMAN | TITIN_HUMAN | TOM22_HUMAN | TRI25_HUMAN |
| TEBP_HUMAN | TKT_HUMAN | TOM34_HUMAN | TRI26_HUMAN |
| TECR_HUMAN | TLE1_HUMAN | TOM40_HUMAN | TRI27_HUMAN |
| TECT3_HUMAN | TLE3_HUMAN | TOM70_HUMAN | TRI32_HUMAN |
| TELO2_HUMAN | TLK2_HUMAN | TOM7_HUMAN | TRI33_HUMAN |
| TERA_HUMAN | TLN1_HUMAN | TOP1_HUMAN | TRI44_HUMAN |
| TES_HUMAN | TM115_HUMAN | TOP2A_HUMAN | TRI56_HUMAN |
| TF2B_HUMAN | TM165_HUMAN | TOP2B_HUMAN | TRI65_HUMAN |
| TF2H3_HUMAN | TM192_HUMAN | TOPB1_HUMAN | TRIM1_HUMAN |
| TF2H5_HUMAN | TM1L1_HUMAN | TOPK_HUMAN | TRIM4_HUMAN |
| TF3C1_HUMAN | TM1L2_HUMAN | TP4A1_HUMAN | TRIP4_HUMAN |
| TF3C3_HUMAN | TM209_HUMAN | TP4A2_HUMAN | TRIPB_HUMAN |
| TF3C4_HUMAN | TM237_HUMAN | TP4AP_HUMAN | TRIPC_HUMAN |
| TF3C5_HUMAN | TM41B_HUMAN | TPC10_HUMAN | TRM1L_HUMAN |
| TFDP1_HUMAN | TM45A_HUMAN | TPC11_HUMAN | TRM1_HUMAN |
| TRM6_HUMAN | UB2D3_HUMAN | UBP25_HUMAN | UTP18_HUMAN |
| TRRAP_HUMAN | UB2E1_HUMAN | UBP28_HUMAN | UTP23_HUMAN |
| TRUA_HUMAN | UB2G2_HUMAN | UBP2L_HUMAN | UTP6_HUMAN |
| TRXR1_HUMAN | UB2L3_HUMAN | UBP30_HUMAN | UTRO_HUMAN |
| TS101_HUMAN | UB2Q1_HUMAN | UBP33_HUMAN | UXT_HUMAN |
| TSC2_HUMAN | UB2R1_HUMAN | UBP34_HUMAN | VA0D1_HUMAN |
| TSN10_HUMAN | UB2R2_HUMAN | UBP36_HUMAN | VAMP1_HUMAN |
| TSNAX_HUMAN | UB2V1_HUMAN | UBP3_HUMAN | VAMP2_HUMAN |
| TSN_HUMAN | UB2V2_HUMAN | UBP48_HUMAN | VAMP4 HUMAN |
| TSR3_HUMAN | UBA1_HUMAN | UBP5_HUMAN | VAMP7_HUMAN |
| TSYL1_HUMAN | UBA3_HUMAN | UBP7_HUMAN | VAMP8_HUMAN |
| TSYL2_HUMAN | UBA6_HUMAN | UBQL1_HUMAN | VANG1_HUMAN |
| TTC12_HUMAN | UBAC1_HUMAN | UBQL2_HUMAN | VAPA_HUMAN |
| TTC26_HUMAN | UBAP1_HUMAN | UBR4_HUMAN | VAPB_HUMAN |

(continued)

| | | | |
|---|---|---|---|
| TTC27_HUMAN | UBB_HUMAN | UBR5_HUMAN | VAS1_HUMAN |
| TTC32_HUMAN | UBC12_HUMAN | UBR7_HUMAN | VASP_HUMAN |
| TTC37_HUMAN | UBCP1_HUMAN | UBX2A_HUMAN | VAT1_HUMAN |
| TTC5_HUMAN | UBE2C_HUMAN | UBXN1_HUMAN | VATA_HUMAN |
| TTC9C_HUMAN | UBE2H_HUMAN | UBXN4_HUMAN | VATB2_HUMAN |
| TTF2_HUMAN | UBE2K_HUMAN | UBXN6_HUMAN | VATC1_HUMAN |
| TTK_HUMAN | UBE2N_HUMAN | UBXN7_HUMAN | VATF_HUMAN |
| TTL12_HUMAN | UBE2O_HUMAN | UBXN8_HUMAN | VATH_HUMAN |
| TULP3_HUMAN | UBE2S_HUMAN | UCHL1_HUMAN | VCIP1_HUMAN |
| TUT4_HUMAN | UBE2T_HUMAN | UCHL5_HUMAN | VDAC1_HUMAN |
| TX264_HUMAN | UBE3A_HUMAN | UCK2_HUMAN | VDAC2_HUMAN |
| TXD17_HUMAN | UBE3C_HUMAN | UCRIL_HUMAN | VDAC3_HUMAN |
| TXLNA_HUMAN | UBE4A_HUMAN | UEVLD_HUMAN | VIGLN_HUMAN |
| TXN4A_HUMAN | UBE4B_HUMAN | UFC1_HUMAN | VIME_HUMAN |
| TXN4B_HUMAN | UBF1_HUMAN | UFD1_HUMAN | VINC_HUMAN |
| TXND9_HUMAN | UBFD1_HUMAN | UHRF1_HUMAN | VIR_HUMAN |
| TXNIP_HUMAN | UBL4A_HUMAN | UIMC1_HUMAN | VP13A_HUMAN |
| TXNL1_HUMAN | UBL5_HUMAN | UK114_HUMAN | VP13C_HUMAN |
| TYDP2_HUMAN | UBL7_HUMAN | ULA1_HUMAN | VP13D_HUMAN |
| TYSY_HUMAN | UBP10_HUMAN | ULK3_HUMAN | VP26A_HUMAN |
| TYW1_HUMAN | UBP11_HUMAN | UMPS_HUMAN | VP33A_HUMAN |
| TYY1_HUMAN | UBP13_HUMAN | UN45A_HUMAN | VP33B_HUMAN |
| U2AF1_HUMAN | UBP14_HUMAN | UNC5C_HUMAN | VPP1_HUMAN |
| U2AF2_HUMAN | UBP16_HUMAN | UPK3L_HUMAN | VPP2_HUMAN |
| U520_HUMAN | UBP19_HUMAN | URB2_HUMAN | VPS16_HUMAN |
| U5S1_HUMAN | UBP1_HUMAN | USMG5_HUMAN | VPS29_HUMAN |
| UACA_HUMAN | UBP20_HUMAN | USO1_HUMAN | VPS35_HUMAN |
| UAP1_HUMAN | UBP22_HUMAN | USP9X_HUMAN | VPS36_HUMAN |
| UB2D1_HUMAN | UBP24_HUMAN | UTP15_HUMAN | VPS39_HUMAN |
| VPS45_HUMAN | XPO7_HUMAN | ZMAT2_HUMAN | ##PYGL_HUMAN |
| VPS4A_HUMAN | XPOT_HUMAN | ZMYM1_HUMAN | ##RL6_HUMAN |
| VPS4B_HUMAN | XPP1_HUMAN | ZMYM2_HUMAN | ##SMC1A_HUMAN |
| VRK1_HUMAN | XRCC1_HUMAN | ZMYM3_HUMAN | ##TCPQ_HUMAN |
| VRK3_HUMAN | XRCC4_HUMAN | ZN207_HUMAN | ##TITIN_HUMAN |
| VTA1_HUMAN | XRCC5_HUMAN | ZN264_HUMAN | ##TXND3_HUMAN |
| WAC_HUMAN | XRCC6_HUMAN | ZN281_HUMAN | |
| WAP53_HUMAN | XRN2_HUMAN | ZN326_HUMAN | |
| WASH1_HUMAN | XRP2_HUMAN | ZN330_HUMAN | |
| WBP11_HUMAN | YAF2_HUMAN | ZN346_HUMAN | |
| WBP2_HUMAN | YAP1_HUMAN | ZN451_HUMAN | |
| WBS22_HUMAN | YBOX1_HUMAN | ZN460_HUMAN | |
| WDHD1_HUMAN | YETS4_HUMAN | ZN503_HUMAN | |
| WDR11_HUMAN | YI017_HUMAN | ZN598_HUMAN | |
| WDR12_HUMAN | YIPF3_HUMAN | ZN622_HUMAN | |
| WDR1_HUMAN | YKT6_HUMAN | ZN638_HUMAN | |
| WDR26_HUMAN | YMEL1_HUMAN | ZN711_HUMAN | |
| WDR36_HUMAN | YTHD1_HUMAN | ZN768_HUMAN | |
| WDR41_HUMAN | YTHD2_HUMAN | ZNF24_HUMAN | |
| WDR43_HUMAN | Z280C_HUMAN | ZNT1_HUMAN | |
| WDR44_HUMAN | Z3H7A_HUMAN | ZO1_HUMAN | |
| WDR48_HUMAN | ZBT10_HUMAN | ZO2_HUMAN | |

(continued)

| | | |
|---|---|---|
| WDR59_HUMAN | ZC11A_HUMAN | ZPR1_HUMAN |
| WDR61_HUMAN | ZC3HE_HUMAN | ZRAB2_HUMAN |
| WDR67_HUMAN | ZC3HF_HUMAN | ZSWM6_HUMAN |
| WDR6_HUMAN | ZCCHV_HUMAN | ZUFSP_HUMAN |
| WDR74_HUMAN | ZCH10_HUMAN | ZW10_HUMAN |
| WDR75_HUMAN | ZCH12_HUMAN | ZWILC_HUMAN |
| WDR82_HUMAN | ZCHC2_HUMAN | ZWINT_HUMAN |
| WDR85_HUMAN | ZCHC3_HUMAN | ZYX_HUMAN |
| WDTC1_HUMAN | ZCHC8_HUMAN | ZZEF1_HUMAN |
| WIZ_HUMAN | ZDH13_HUMAN | ##AHNK2_HUMAN |
| WLS_HUMAN | ZEB1_HUMAN | ##AHNK_HUMAN |
| WPB5_HUMAN | ZF106_HUMAN | ##BAP31_HUMAN |
| WRB_HUMAN | ZF161_HUMAN | ##CENPF_HUMAN |
| WRIP1_HUMAN | ZFAN5_HUMAN | ##CLH1_HUMAN |
| WRP73_HUMAN | ZFAN6_HUMAN | ##CNTRL_HUMAN |
| WWP1_HUMAN | ZFN2B_HUMAN | ##ENOA_HUMAN |
| XIAP_HUMAN | ZFR_HUMAN | ##FAS_HUMAN |
| XPC_HUMAN | ZFX_HUMAN | ##HUWE1_HUMAN |
| XPO1_HUMAN | ZFY16_HUMAN | ##MCM7_HUMAN |
| XPO2_HUMAN | ZFY19_HUMAN | ##NBN_HUMAN |
| XPO5_HUMAN | ZKSC1_HUMAN | ##PRKDC_HUMAN |

## Claims

1. A peptide comprising the amino acid sequence:
$X_1X_2CX_3X_4X_5X_6X_7X_8X_9X_{10}X_{11}CX_{12}$(SEQ ID NO: 48)
wherein:
$X_1$ is selected from L, M, A, S, and V;
$X_2$ is selected from Y, D, E, I, L, N, and S;
$X_3$ is selected from F, I, and Y;
$X_4$ is selected from F, I, and Y;
$X_5$ is selected from D and E;
$X_6$ is selected from L, M, V, and P;
$X_7$ is selected from S, N, D, A, and T;
$X_8$ is selected from Y, D, F, N, and W;
$X_9$ is selected from G, D, and E;
$X_{10}$ is selected from Y and F;
$X_{11}$ is selected from L, V, M, Q, and W; and
$X_{12}$ is selected from F, L, C, V, and Y;
wherein the peptide inhibits USP30 with an IC50 of less than 10 $\mu$M.

2. The peptide according to claim 1, wherein the IC50 of the peptide for at least one, at least two, or at least three peptidases selected from USP7, USP5, UCHL3, and USP2 is greater than 20 $\mu$M, greater than 30 $\mu$M, greater than 40 $\mu$M, or greater than 50 $\mu$M.

3. The peptide according to any one of claims 1 to 2, wherein the peptide comprises an amino acid sequence that is at least 80%, at least 85%, at least 90%, at least 95%, or 100% identical to an amino acid sequence selected from SEQ ID NOs: 1 to 22.

4. An inhibitor of USP30 according to any one of claims 1 to 3 for use in the treatment of Parkinson's disease in a subject.

**5.** An inhibitor of USP30 according to any one of claims 1 to 3 in combination with at least one additional therapeutic agent.

**6.** An inhibitor of USP30 according to claim 5, wherein the at least one additional therapeutic agent is selected from levodopa, a dopamine agonist, a monoamino oxygenase (MAO) B inhibitor, a catechol O-methyltransferase (COMT) inhibitor, an anticholinergic, amantadine, riluzole, a cholinesterase inhibitor, memantine, tetrabenazine, an antipsychotic, clonazepam, diazepam, an antidepressant, and an anti-convulsant.

## Patentansprüche

**1.** Peptid, umfassend die Aminosäuresequenz:
$X_1X_2CX_3X_4X_5X_6X_7X_8X_9X_{1o}X_{11}CX_{12}$ (SEQ ID NO: 48)
wobei:
$X_1$ ausgewählt ist aus L, M, A, S und V;
$X_2$ ausgewählt ist aus Y, D, E, I, L, N und S;
$X_3$ ausgewählt ist aus F, I und Y;
$X_4$ ausgewählt ist aus F, I und Y;
$X_5$ ausgewählt ist aus D und E;
$X_6$ ausgewählt ist aus L, M, V und P;
$X_7$ ausgewählt ist aus S, N, D, A und T;
$X_8$ ausgewählt ist aus Y, D, F, N und W;
$X_9$ ausgewählt ist aus G, D und E;
$X_{10}$ ausgewählt ist aus Y und F;
$X_{11}$ ausgewählt ist aus L, V, M, Q und W; und
$X_{12}$ ausgewählt ist aus F, L, C, V und Y;
wobei das Peptid USP30 mit einer IC50 von weniger als 10 $\mu$M inhibiert.

**2.** Peptid nach Anspruch 1, wobei die IC50 des Peptids für mindestens eine, mindestens zwei oder mindestens drei Peptidasen, ausgewählt aus USP7, USP5, UCHL3 und USP2, größer als 20 $\mu$M, größer als 30 $\mu$M, größer als 40 $\mu$M oder größer als 50 $\mu$M, ist.

**3.** Peptid nach einem der Ansprüche 1 bis 2, wobei das Peptid eine Aminosäuresequenz umfasst, die zu mindestens 80 %, mindestens 85 %, mindestens 90 %, mindestens 95 % oder 100 % identisch zu einer Aminosäuresequenz, ausgewählt aus SEQ ID NO: 1 bis 22, ist.

**4.** USP30-Inhibitor nach einem der Ansprüche 1 bis 3 zur Verwendung bei der Behandlung der Parkinson-Krankheit bei einem Individuum.

**5.** USP30-Inhibitor nach einem der Ansprüche 1 bis 3 in Kombination mit mindestens einem zusätzlichen therapeutischen Mittel.

**6.** USP30-Inhibitor nach Anspruch 5, wobei das mindestens eine zusätzliche therapeutische Mittel ausgewählt ist aus Levodopa, einem Dopamin-Agonisten, einem Monoamino-Oxygenase-B-Inhibitor (MAO-B-Inhibitor), einem Catechol-O-Methyltransferase-Inhibitor (COMT-Inhibitor), einem Anticholinergikum, Amantadin, Riluzol, einem Cholinesterase-Inhibitor, Memantin, Tetrabenazin, einem Antipsychotikum, Clonazepam, Diazepam, einem Antidepressivum und einem Antikonvulsivum.

## Revendications

**1.** Peptide comprenant la séquence d’acides aminés :
$X_1X_2CX_3X_4X_5X_6X_7X_8X_9X_{10}X_{11}CX_{12}$ (SEQ ID NO: 48)
dans laquelle :
$X_1$ est choisi parmi L, M, A, S et V ;
$X_2$ est choisi parmi Y, D, E, I, L, N et S ;
$X_3$ est choisi parmi F, I et Y ;
$X_4$ est choisi parmi F, I et Y ;

$X_5$ est choisi parmi D et E ;
$X_6$ est choisi parmi L, M, V et P ;
$X_7$ est choisi parmi S, N, D, A et T ;
$X_8$ est choisi parmi Y, D, F, N et W ;
$X_9$ est choisi parmi G, D et E ;
$X_{10}$ est choisi parmi Y et F ;
$X_{11}$ est choisi parmi L, V, M, Q et W ; et
$X_{12}$ est choisi parmi F, L, C, V et Y ;
dans lequel le peptide inhibe l'USP30 avec une CI50 de moins de 10 μM.

**2.** Peptide selon la revendication 1, dans lequel la CI50 du peptide vis-à-vis d'au moins une, d'au moins deux, ou d'au moins trois peptidases choisies parmi USP7, USP5, UCHL3 et USP2 est supérieure à 20 μM, supérieure à 30 μM, supérieure à 40 μM, ou supérieure à 50 μM.

**3.** Peptide selon l'une quelconque des revendications 1 à 2, dans lequel le peptide comprend une séquence d'acides aminés qui est identique à au moins 80 %, à au moins 85 %, à au moins 90 %, à au moins 95 %, ou à 100 % à une séquence d'acides aminés choisie parmi les SEQ ID NO: 1 à 22.

**4.** Inhibiteur de l'USP30 selon l'une quelconque des revendications 1 à 3 pour utilisation dans le traitement de la maladie de Parkinson chez un sujet.

**5.** Inhibiteur de l'USP30 selon l'une quelconque des revendications 1 à 3 en association avec au moins un agent thérapeutique supplémentaire.

**6.** Inhibiteur de l'USP30 selon la revendication 5, dans lequel l'au moins un agent thérapeutique supplémentaire est choisi parmi la lévodopa, un agoniste de dopamine, un inhibiteur de monoamino-oxygénase (MAO) B, un inhibiteur de catéchol O-méthyltransférase (COMT), un anticholinergique, l'amantadine, le riluzole, un inhibiteur de cholinestérase, la mémantine, la tétrabénazine, un antipsychotique, le clonazépam, le diazépam, un antidépresseur, et un antiépileptique.

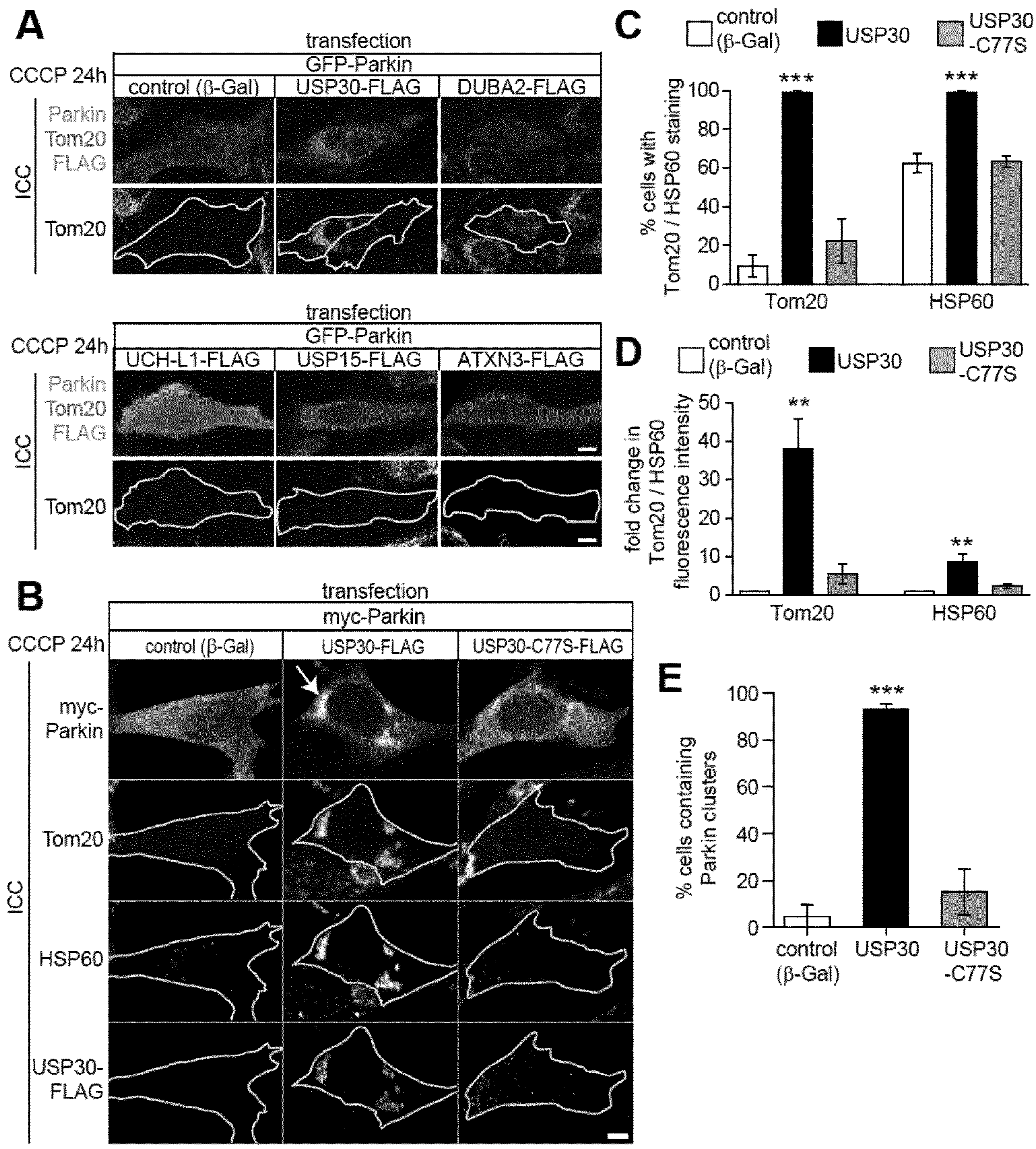
A
transfection
GFP-Parkin
CCCP 24h
control (β-Gal)
USP30-FLAG
DUBA2-FLAG
ICC
Parkin
Tom20
FLAG
Tom20
transfection
GFP-Parkin
CCCP 24h
UCH-L1-FLAG
USP15-FLAG
ATXN3-FLAG
ICC
Parkin
Tom20
FLAG
Tom20
B
transfection
myc-Parkin
CCCP 24h
control (β-Gal)
USP30-FLAG
USP30-C77S-FLAG
ICC
myc-Parkin
Tom20
HSP60
USP30-FLAG
C
control (β-Gal)
USP30
USP30 -C77S
% cells with Tom20 / HSP60 staining
0
20
40
60
80
100
***
***
Tom20
HSP60
D
control (β-Gal)
USP30
USP30 -C77S
fold change in Tom20 / HSP60 fluorescence intensity
0
10
20
30
40
50
**
**
Tom20
HSP60
E
% cells containing Parkin clusters
0
20
40
60
80
100
***
control (β-Gal)
USP30
USP30 -C77S

*FIG. 1*

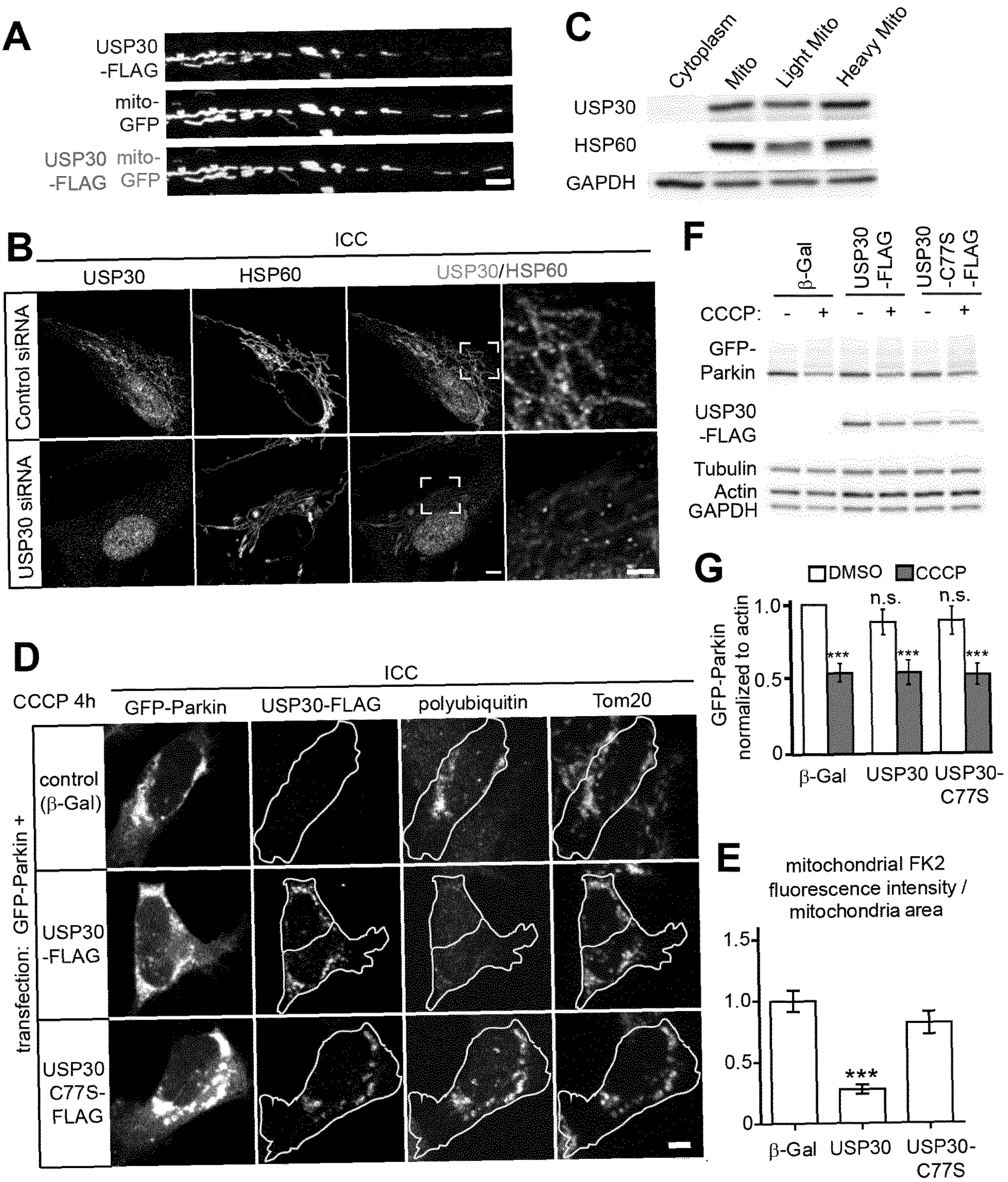
A
USP30 -FLAG
mito- GFP
USP30 -FLAG mito- GFP
C
Cytoplasm
Mito
Light Mito
Heavy Mito
USP30
HSP60
GAPDH
B
ICC
USP30
HSP60
USP30/HSP60
Control siRNA
USP30 siRNA
F
β-Gal
USP30 -FLAG
USP30 -C77S -FLAG
CCCP:
GFP- Parkin
USP30 -FLAG
Tubulin
Actin
GAPDH
G
DMSO
CCCP
GFP-Parkin normalized to actin
n.s.
***
β-Gal
USP30
USP30- C77S
D
ICC
CCCP 4h
GFP-Parkin
USP30-FLAG
polyubiquitin
Tom20
transfection: GFP-Parkin +
control (β-Gal)
USP30 -FLAG
USP30 C77S- FLAG
E
mitochondrial FK2 fluorescence intensity / mitochondria area
1.5
1.0
0.5
0
***
β-Gal
USP30
USP30- C77S

*FIG. 2*

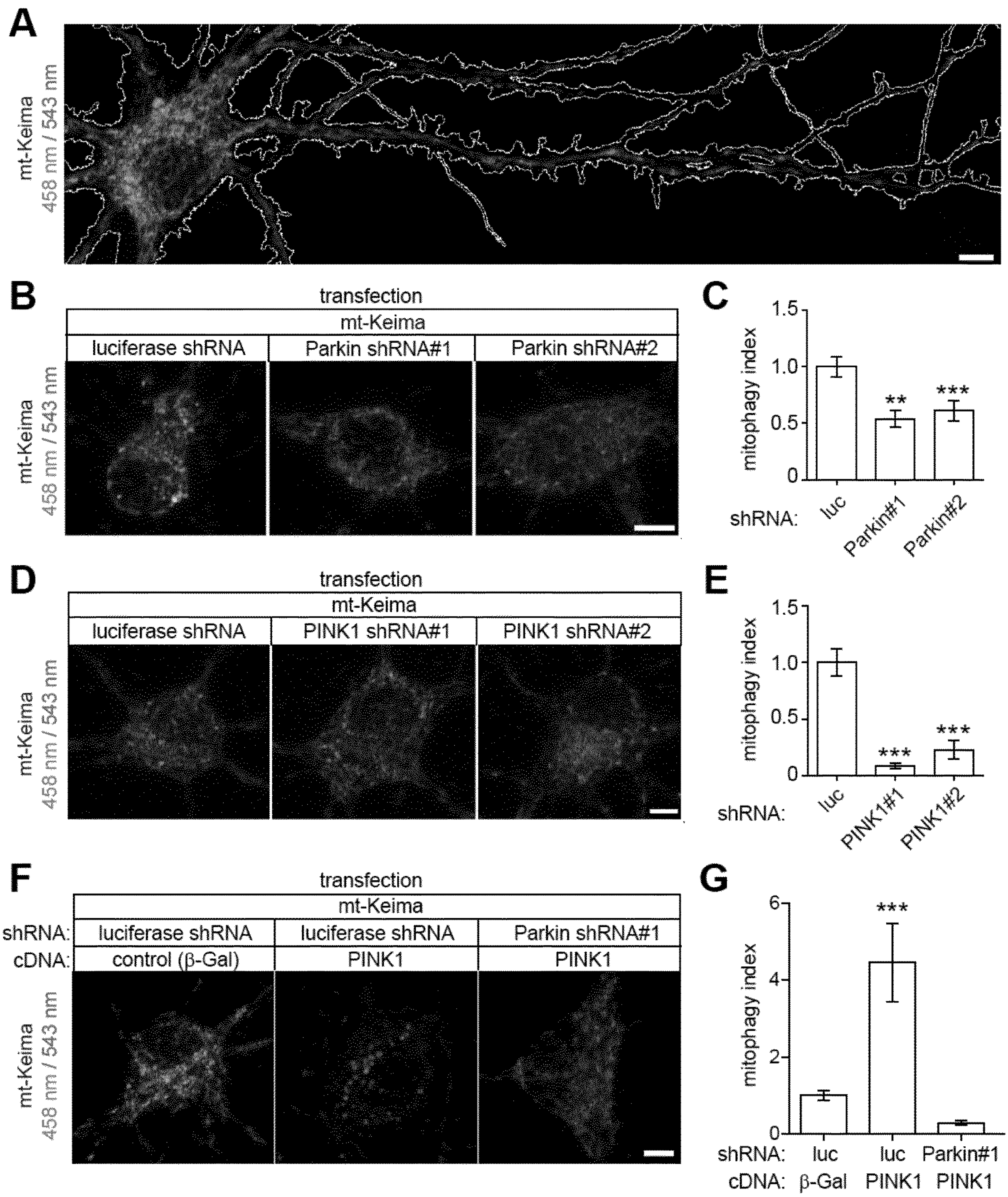
A
mt-Keima
458 nm / 543 nm
B
transfection
mt-Keima
luciferase shRNA
Parkin shRNA#1
Parkin shRNA#2
mt-Keima
458 nm / 543 nm
C
mitophagy index
1.5
1.0
0.5
0
**
***
shRNA:
luc
Parkin#1
Parkin#2
D
transfection
mt-Keima
luciferase shRNA
PINK1 shRNA#1
PINK1 shRNA#2
mt-Keima
458 nm / 543 nm
E
mitophagy index
1.5
1.0
0.5
0
***
***
shRNA:
luc
PINK1#1
PINK1#2
F
transfection
mt-Keima
shRNA:
luciferase shRNA
luciferase shRNA
Parkin shRNA#1
cDNA:
control (β-Gal)
PINK1
PINK1
mt-Keima
458 nm / 543 nm
G
mitophagy index
6
4
2
0
***
shRNA: luc luc Parkin#1
cDNA: β-Gal PINK1 PINK1

*FIG. 3*

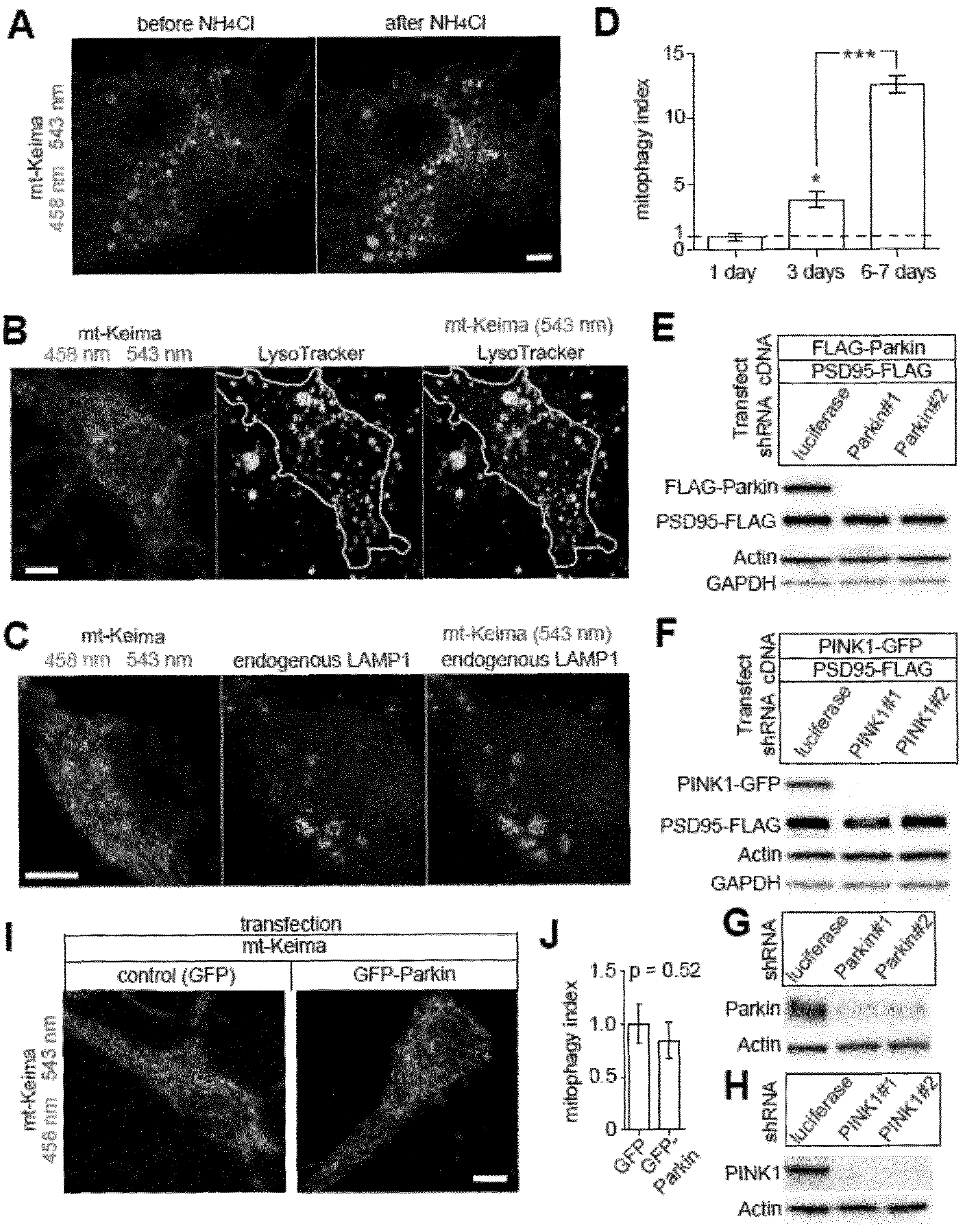
A
before NH4Cl
after NH4Cl
mt-Keima 458 nm 543 nm
D
mitophagy index
15
10
5
1
0
***
*
1 day
3 days
6-7 days
B
mt-Keima
458 nm 543 nm
LysoTracker
mt-Keima (543 nm)
LysoTracker
E
Transfect cDNA
shRNA
FLAG-Parkin
PSD95-FLAG
luciferase
Parkin#1
Parkin#2
FLAG-Parkin
PSD95-FLAG
Actin
GAPDH
C
mt-Keima
458 nm 543 nm
endogenous LAMP1
mt-Keima (543 nm)
endogenous LAMP1
F
Transfect cDNA
shRNA
PINK1-GFP
PSD95-FLAG
luciferase
PINK1#1
PINK1#2
PINK1-GFP
PSD95-FLAG
Actin
GAPDH
I
transfection
mt-Keima
control (GFP)
GFP-Parkin
mt-Keima 458 nm 543 nm
J
mitophagy index
1.5
1.0
0.5
0
p = 0.52
GFP
GFP-Parkin
G
shRNA
luciferase
Parkin#1
Parkin#2
Parkin
Actin
H
shRNA
luciferase
PINK1#1
PINK1#2
PINK1
Actin

*FIG. 4*

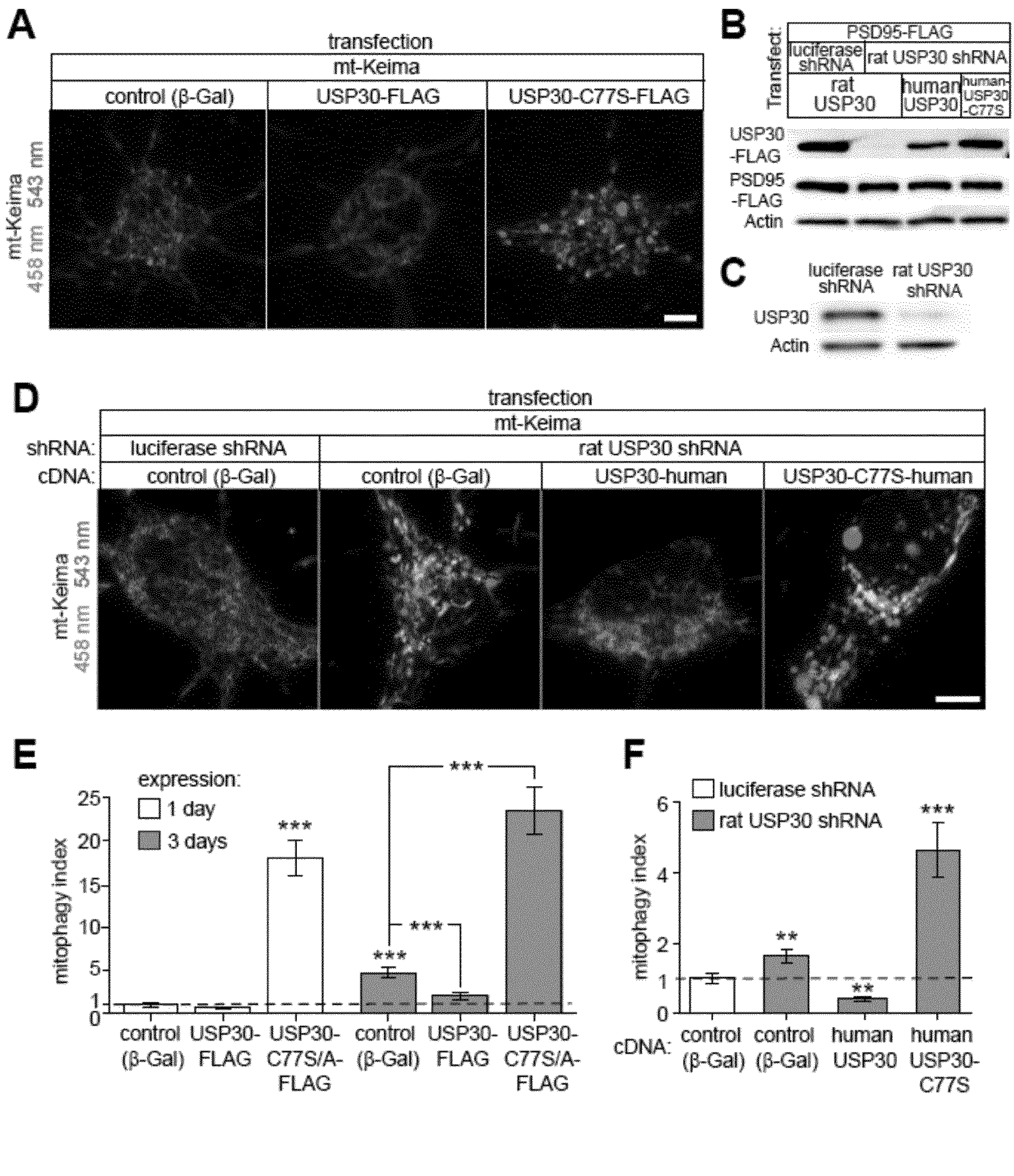
A
transfection
mt-Keima
control (β-Gal)
USP30-FLAG
USP30-C77S-FLAG
mt-Keima 458 nm 543 nm
B
Transfect:
PSD95-FLAG
luciferase shRNA
rat USP30 shRNA
rat USP30
human USP30
human-USP30-C77S
USP30-FLAG
PSD95-FLAG
Actin
C
luciferase shRNA
rat USP30 shRNA
USP30
Actin
D
transfection
mt-Keima
shRNA:
luciferase shRNA
rat USP30 shRNA
cDNA:
control (β-Gal)
control (β-Gal)
USP30-human
USP30-C77S-human
mt-Keima 458 nm 543 nm
E
expression:
1 day
3 days
mitophagy index
control (β-Gal)
USP30-FLAG
USP30-C77S/A-FLAG
control (β-Gal)
USP30-FLAG
USP30-C77S/A-FLAG
F
luciferase shRNA
rat USP30 shRNA
mitophagy index
cDNA:
control (β-Gal)
control (β-Gal)
human USP30
human USP30-C77S

*FIG. 5*

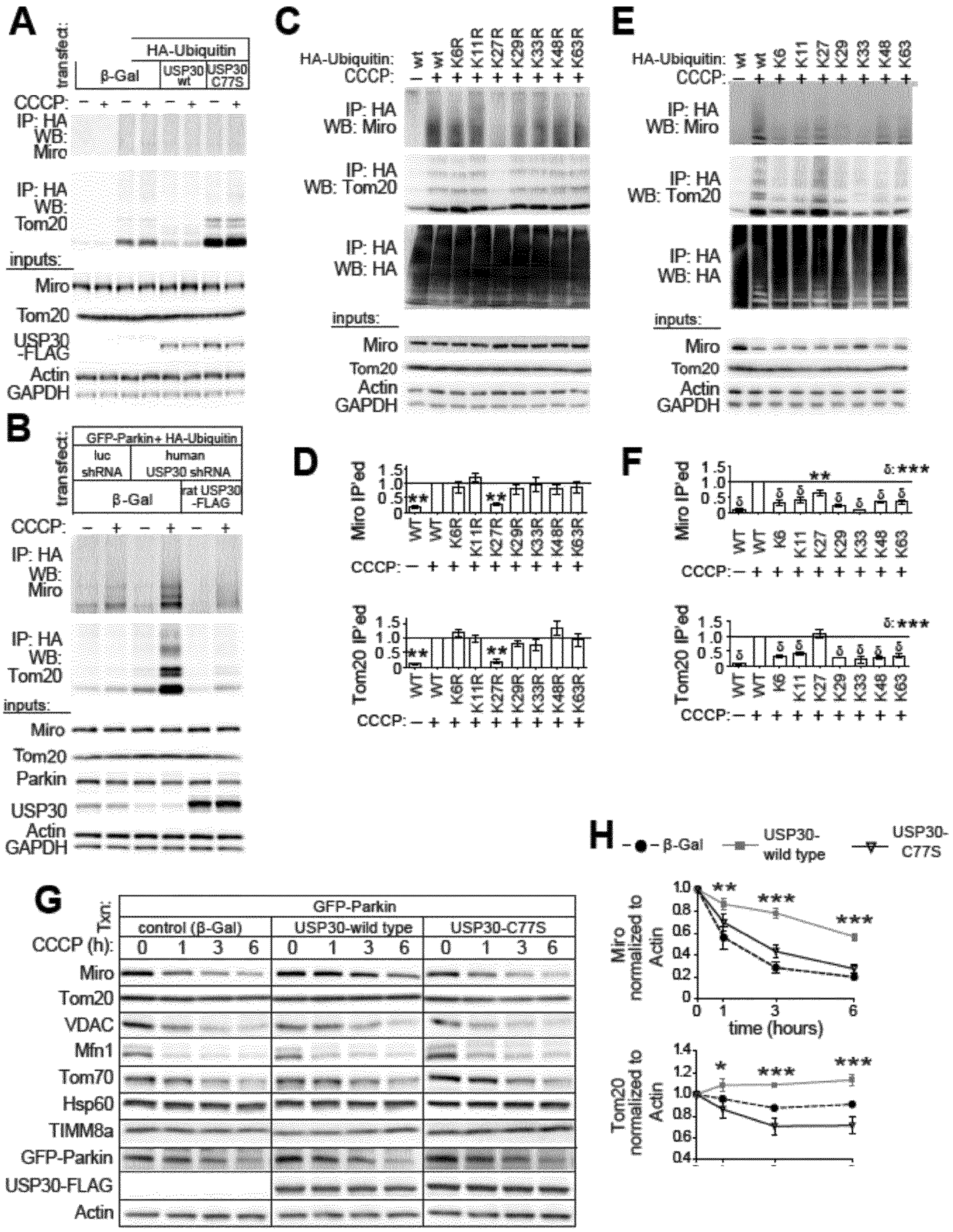
A
transfected:
HA-Ubiquitin
β-Gal
USP30 wt
USP30 C77S
CCCP:
IP: HA WB: Miro
IP: HA WB: Tom20
inputs:
Miro
Tom20
USP30 -FLAG
Actin
GAPDH
B
transfect:
GFP-Parkin+ HA-Ubiquitin
luc shRNA
human USP30 shRNA
β-Gal
rat USP30 -FLAG
CCCP:
IP: HA WB: Miro
IP: HA WB: Tom20
inputs:
Miro
Tom20
Parkin
USP30
Actin
GAPDH
C
HA-Ubiquitin: wt wt K6R K11R K27R K29R K33R K48R K63R
CCCP: − + + + + + + + +
IP: HA WB: Miro
IP: HA WB: Tom20
IP: HA WB: HA
inputs:
Miro
Tom20
Actin
GAPDH
D
Miro IP'ed
Tom20 IP'ed
WT WT K6R K11R K27R K29R K33R K48R K63R
CCCP: − + + + + + + + +
E
HA-Ubiquitin: wt wt K6 K11 K27 K29 K33 K48 K63
CCCP: − + + + + + + + +
IP: HA WB: Miro
IP: HA WB: Tom20
IP: HA WB: HA
inputs:
Miro
Tom20
Actin
GAPDH
F
Miro IP'ed
Tom20 IP'ed
δ:***
WT WT K6 K11 K27 K29 K33 K48 K63
CCCP: − + + + + + + + +
G
Txn:
GFP-Parkin
control (β-Gal)
USP30-wild type
USP30-C77S
CCCP (h): 0 1 3 6 0 1 3 6 0 1 3 6
Miro
Tom20
VDAC
Mfn1
Tom70
Hsp60
TIMM8a
GFP-Parkin
USP30-FLAG
Actin
H
β-Gal
USP30- wild type
USP30- C77S
Miro normalized to Actin
time (hours)
Tom20 normalized to Actin

FIG. 6

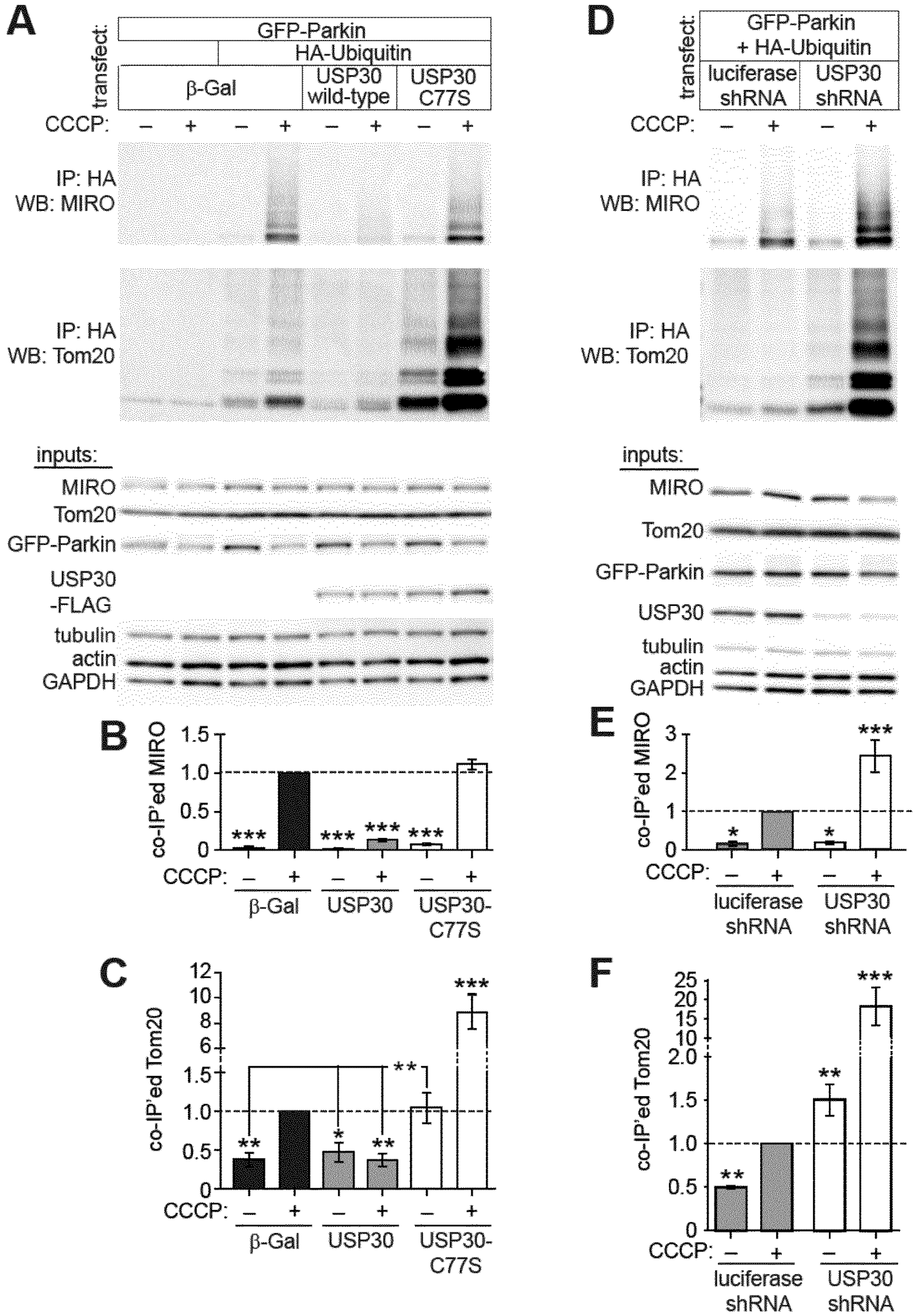
A
transfect:
GFP-Parkin
HA-Ubiquitin
β-Gal
USP30 wild-type
USP30 C77S
CCCP: – + – + – + – +
IP: HA
WB: MIRO
IP: HA
WB: Tom20
inputs:
MIRO
Tom20
GFP-Parkin
USP30 -FLAG
tubulin
actin
GAPDH
B
co-IP'ed MIRO
1.5
1.0
0.5
0
***
***
***
***
CCCP: – + – + – +
β-Gal
USP30
USP30-C77S
C
co-IP'ed Tom20
12
10
8
6
1.5
1.0
0.5
0
**
*
**
**
***
CCCP: – + – + – +
β-Gal
USP30
USP30-C77S
D
transfect:
GFP-Parkin + HA-Ubiquitin
luciferase shRNA
USP30 shRNA
CCCP: – + – +
IP: HA
WB: MIRO
IP: HA
WB: Tom20
inputs:
MIRO
Tom20
GFP-Parkin
USP30
tubulin
actin
GAPDH
E
co-IP'ed MIRO
3
2
1
0
*
*
***
CCCP: – + – +
luciferase shRNA
USP30 shRNA
F
co-IP'ed Tom20
25
20
15
10
2.0
1.5
1.0
0.5
0
**
**
***
CCCP: – + – +
luciferase shRNA
USP30 shRNA

*FIG. 7*

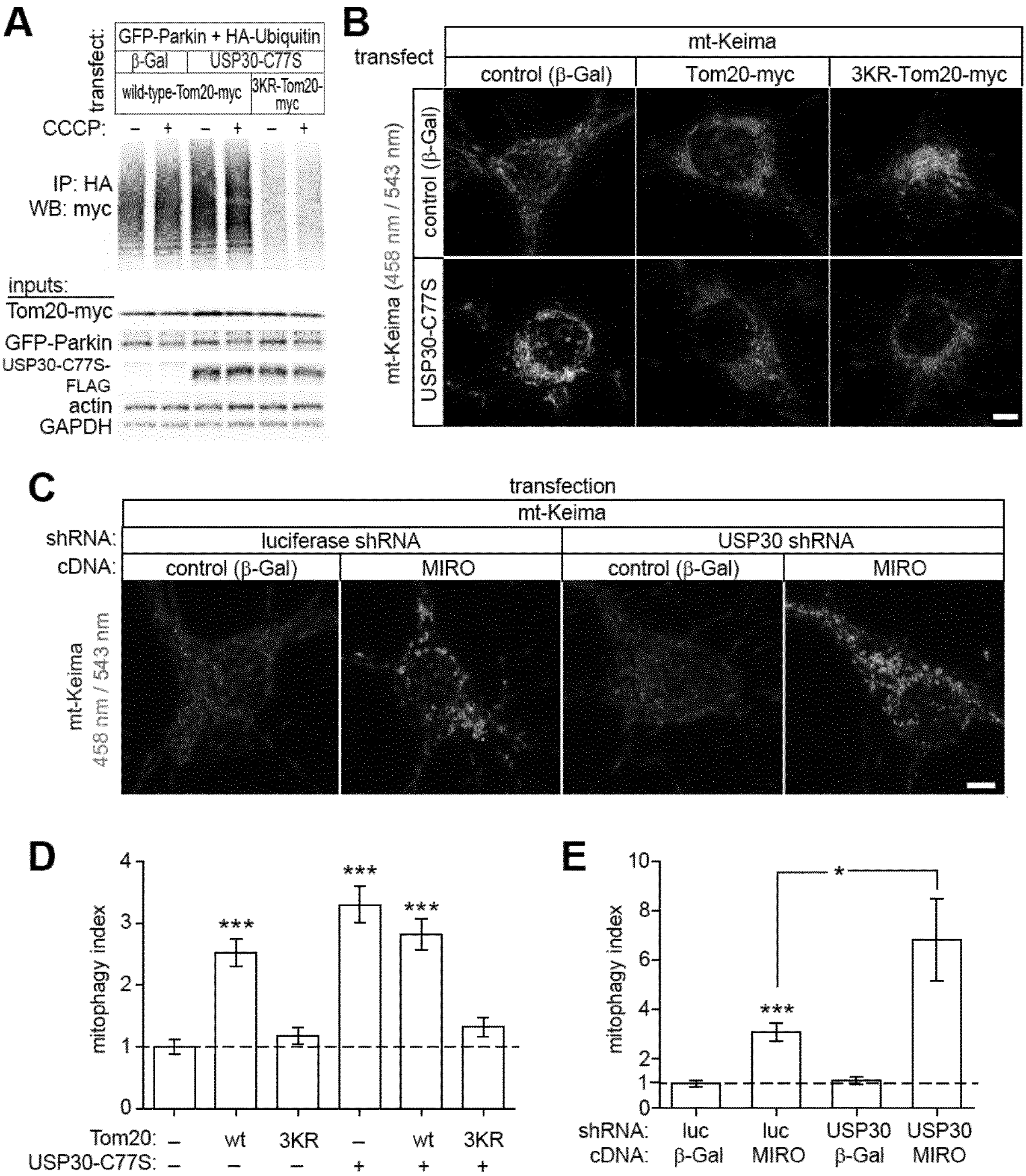
A
transfect:
GFP-Parkin + HA-Ubiquitin
β-Gal
USP30-C77S
wild-type-Tom20-myc
3KR-Tom20-myc
CCCP: − + − + − +
IP: HA
WB: myc
inputs:
Tom20-myc
GFP-Parkin
USP30-C77S-FLAG
actin
GAPDH
B
transfect
mt-Keima
control (β-Gal)
Tom20-myc
3KR-Tom20-myc
mt-Keima (458 nm / 543 nm)
control (β-Gal)
USP30-C77S
C
transfection
mt-Keima
shRNA:
luciferase shRNA
USP30 shRNA
cDNA:
control (β-Gal)
MIRO
control (β-Gal)
MIRO
mt-Keima
458 nm / 543 nm
D
mitophagy index
Tom20: – wt 3KR – wt 3KR
USP30-C77S: – – – + + +
E
mitophagy index
shRNA: luc luc USP30 USP30
cDNA: β-Gal MIRO β-Gal MIRO

*FIG. 8*

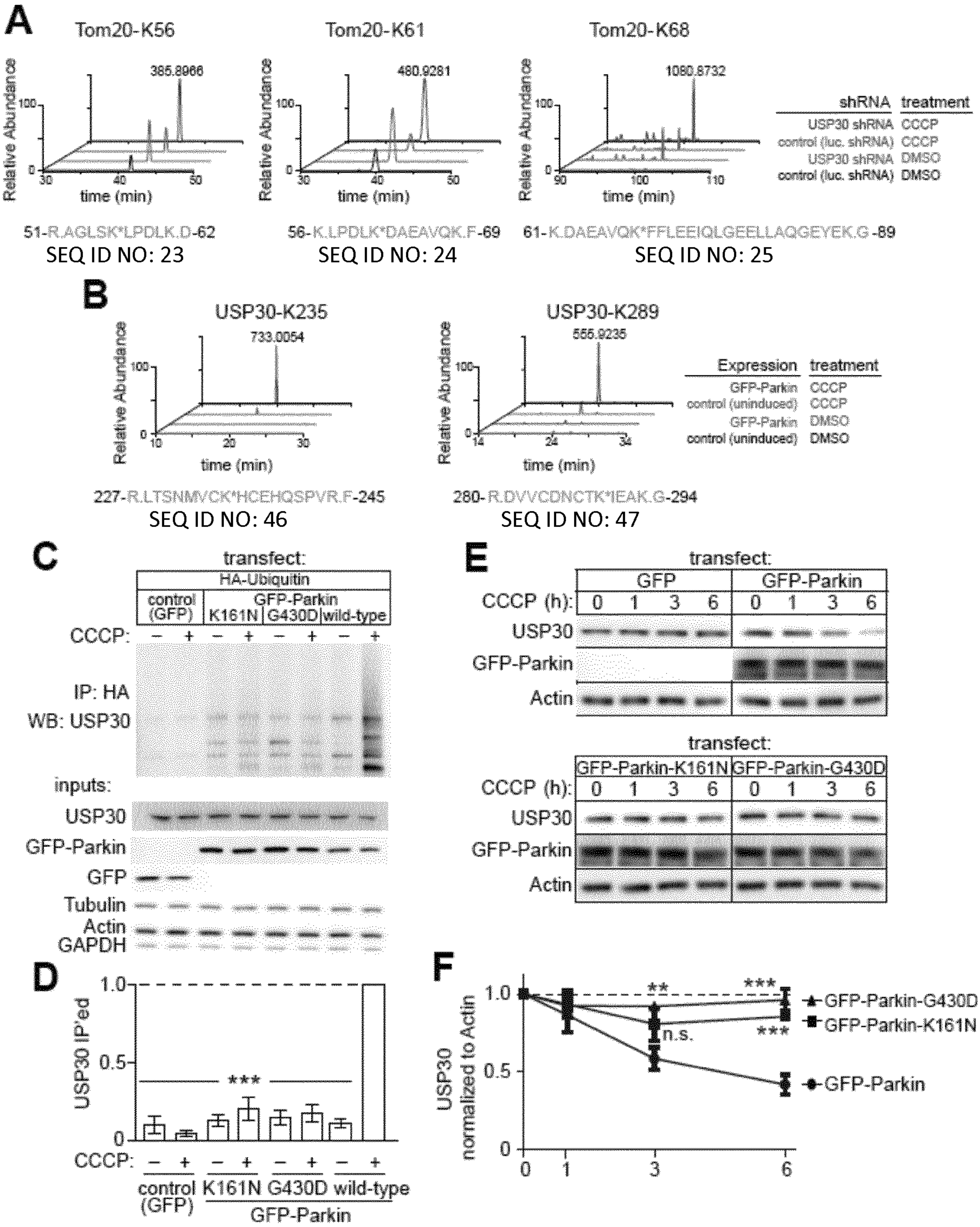
A
Tom20-K56
Tom20-K61
Tom20-K68
385.8966
480.9281
1080.8732
Relative Abundance
time (min)
shRNA treatment
USP30 shRNA CCCP
control (luc. shRNA) CCCP
USP30 shRNA DMSO
control (luc. shRNA) DMSO
51-R.AGLSK*LPDLK.D-62
SEQ ID NO: 23
56-K.LPDLK*DAEAVQK.F-69
SEQ ID NO: 24
61-K.DAEAVQK*FFLEEIQLGEELLAQGEYEK.G-89
SEQ ID NO: 25
B
USP30-K235
USP30-K289
733.0054
555.9235
Expression treatment
GFP-Parkin CCCP
control (uninduced) CCCP
GFP-Parkin DMSO
control (uninduced) DMSO
227-R.LTSNMVCK*HCEHQSPVR.F-245
SEQ ID NO: 46
280-R.DVVCDNCTK*IEAK.G-294
SEQ ID NO: 47
C
transfect:
HA-Ubiquitin
control (GFP)
GFP-Parkin
K161N G430D wild-type
CCCP:
IP: HA
WB: USP30
inputs:
USP30
GFP-Parkin
GFP
Tubulin
Actin
GAPDH
D
USP30 IP'ed
***
control (GFP)
K161N G430D wild-type
GFP-Parkin
E
transfect:
GFP
GFP-Parkin
CCCP (h): 0 1 3 6 0 1 3 6
USP30
GFP-Parkin
Actin
transfect:
GFP-Parkin-K161N
GFP-Parkin-G430D
F
USP30 normalized to Actin
GFP-Parkin-G430D
GFP-Parkin-K161N
GFP-Parkin
n.s.
**
***

*FIG. 9*

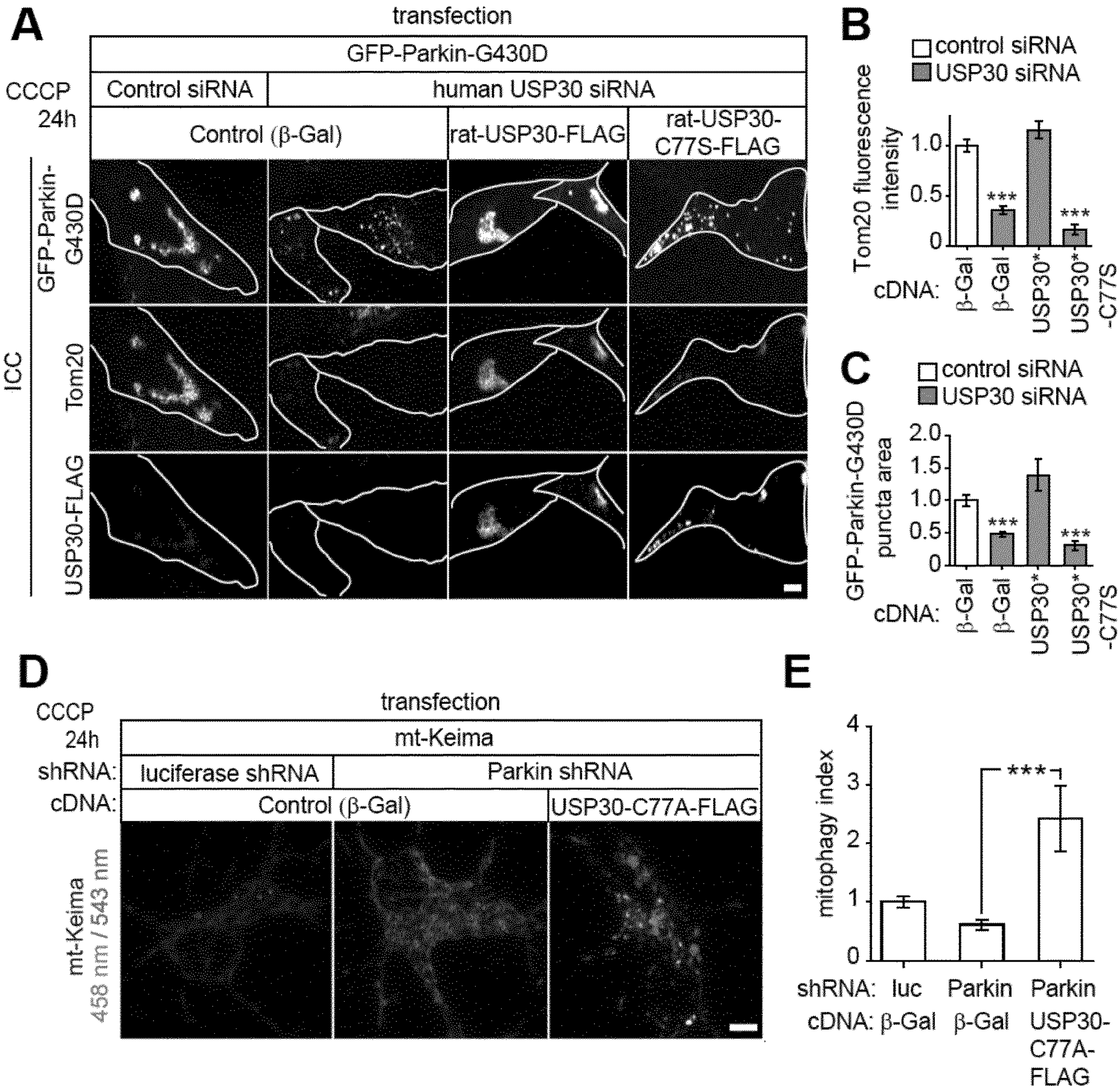
A
transfection
GFP-Parkin-G430D
CCCP 24h
Control siRNA
human USP30 siRNA
Control (β-Gal)
rat-USP30-FLAG
rat-USP30-C77S-FLAG
ICC
GFP-Parkin-G430D
Tom20
USP30-FLAG
B
control siRNA
USP30 siRNA
Tom20 fluorescence intensity
1.0
0.5
0
***
***
cDNA:
β-Gal
β-Gal
USP30*
USP30*-C77S
C
control siRNA
USP30 siRNA
GFP-Parkin-G430D puncta area
2.0
1.5
1.0
0.5
0
***
***
cDNA:
β-Gal
β-Gal
USP30*
USP30*-C77S
D
CCCP 24h
transfection
mt-Keima
shRNA:
luciferase shRNA
Parkin shRNA
cDNA:
Control (β-Gal)
USP30-C77A-FLAG
mt-Keima 458 nm / 543 nm
E
mitophagy index
4
3
2
1
0
***
shRNA: luc Parkin Parkin
cDNA: β-Gal β-Gal USP30-C77A-FLAG

*FIG. 10*

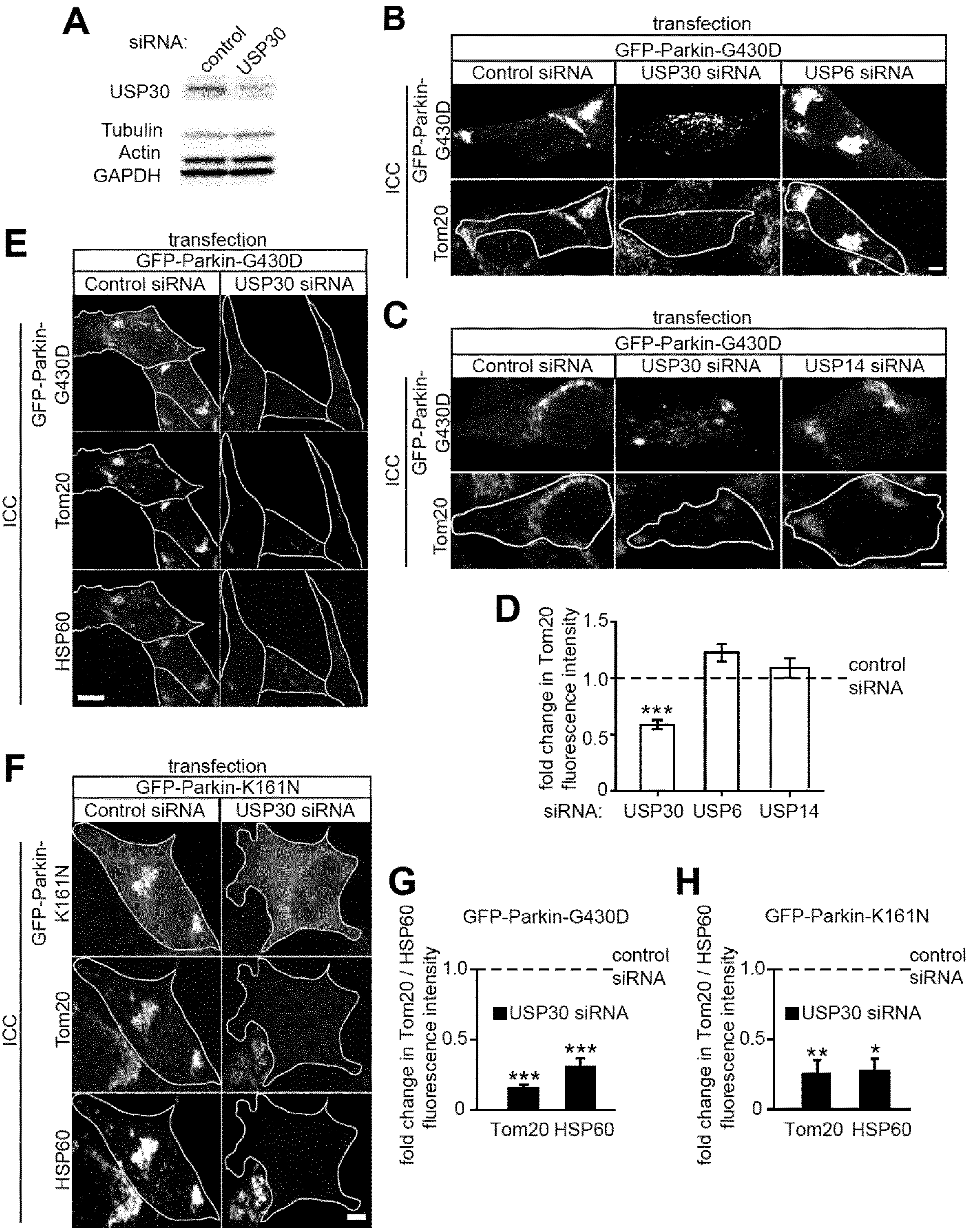
A
siRNA: control USP30
USP30
Tubulin
Actin
GAPDH
B
transfection
GFP-Parkin-G430D
Control siRNA
USP30 siRNA
USP6 siRNA
ICC
GFP-Parkin-G430D
Tom20
C
transfection
GFP-Parkin-G430D
Control siRNA
USP30 siRNA
USP14 siRNA
ICC
GFP-Parkin-G430D
Tom20
D
fold change in Tom20 fluorescence intensity
1.5
1.0
0.5
0
***
control siRNA
siRNA: USP30 USP6 USP14
E
transfection
GFP-Parkin-G430D
Control siRNA
USP30 siRNA
ICC
GFP-Parkin-G430D
Tom20
HSP60
F
transfection
GFP-Parkin-K161N
Control siRNA
USP30 siRNA
ICC
GFP-Parkin-K161N
Tom20
HSP60
G
GFP-Parkin-G430D
fold change in Tom20 / HSP60 fluorescence intensity
1.0
0.5
0
control siRNA
USP30 siRNA
***
***
Tom20 HSP60
H
GFP-Parkin-K161N
fold change in Tom20 / HSP60 fluorescence intensity
1.0
0.5
0
control siRNA
USP30 siRNA
**
*
Tom20 HSP60

*FIG. 11*

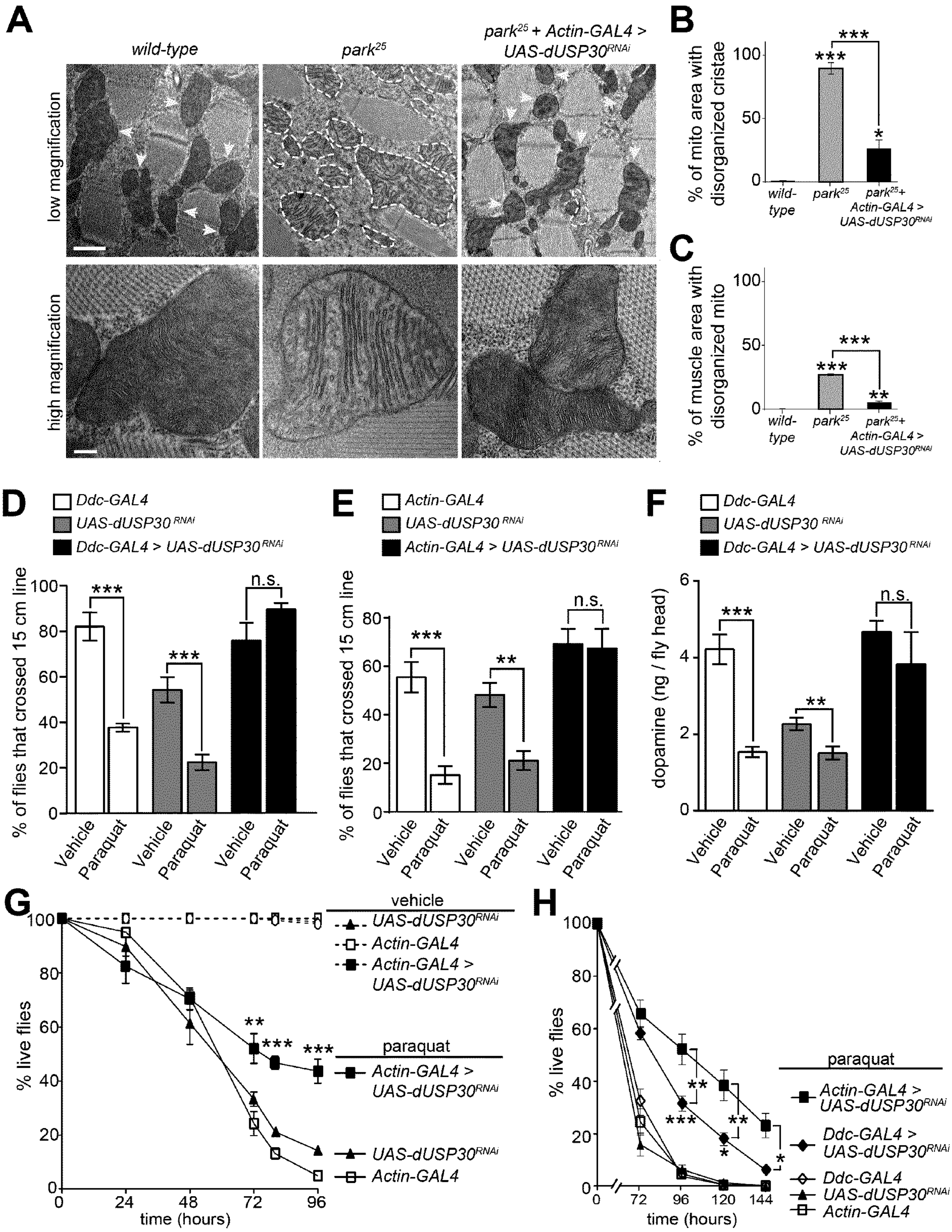

A
wild-type
park25
park25 + Actin-GAL4 > UAS-dUSP30RNAi
low magnification
high magnification
B
% of mito area with disorganized cristae
***
***
*
wild-type
park25
park25+ Actin-GAL4 > UAS-dUSP30RNAi
C
% of muscle area with disorganized mito
***
***
**
D
Ddc-GAL4
UAS-dUSP30RNAi
Ddc-GAL4 > UAS-dUSP30RNAi
% of flies that crossed 15 cm line
n.s.
Vehicle
Paraquat
E
Actin-GAL4
UAS-dUSP30RNAi
Actin-GAL4 > UAS-dUSP30RNAi
F
Ddc-GAL4
UAS-dUSP30RNAi
Ddc-GAL4 > UAS-dUSP30RNAi
dopamine (ng / fly head)
G
vehicle
UAS-dUSP30RNAi
Actin-GAL4
Actin-GAL4 > UAS-dUSP30RNAi
paraquat
% live flies
time (hours)
H
Ddc-GAL4 > UAS-dUSP30RNAi
Ddc-GAL4


*FIG. 12*

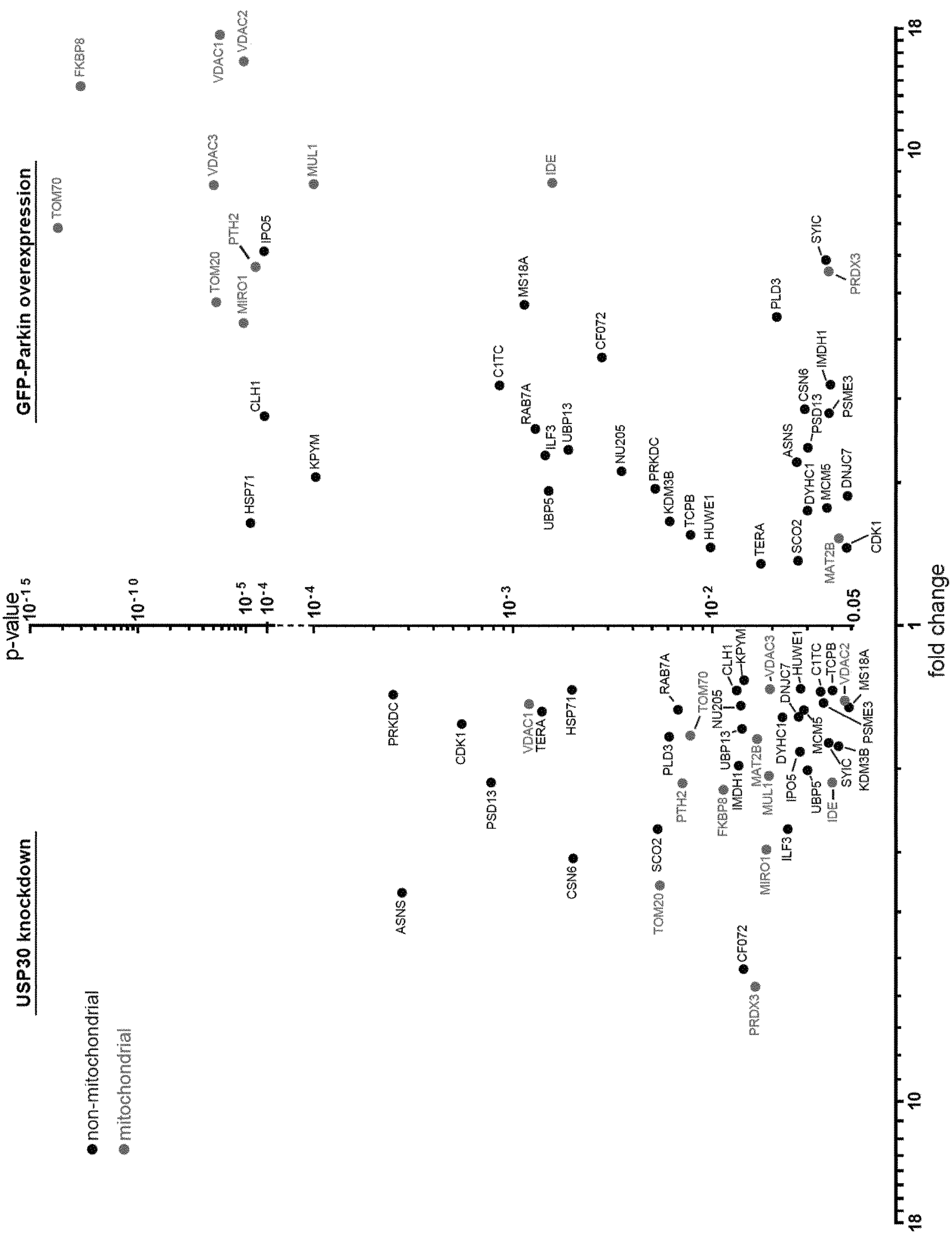

USP30 knockdown
GFP-Parkin overexpression
non-mitochondrial
mitochondrial
p-value
10-15
10-10
10-5
10-4
10-4
10-3
10-2
0.05
fold change
18
10
1
10
18
FKBP8
VDAC1
VDAC2
VDAC3
MUL1
TOM70
IDE
TOM20
MIRO1
PTH2
IPO5
MS18A
PLD3
SYIC
PRDX3
CF072
C1TC
CLH1
RAB7A
ILF3
UBP13
NU205
PRKDC
KDM3B
KPYM
HSP71
UBP5
TCPB
HUWE1
TERA
SCO2
MAT2B
CDK1
ASNS
CSN6
PSD13
IMDH1
PSME3
DYHC1
MCM5
DNJC7
ASNS
CDK1
PSD13
VDAC1
TERA
HSP71
CSN6
PLD3
RAB7A
TOM70
SCO2
TOM20
PTH2
FKBP8
IMDH1
UBP13
NU205
CLH1
KPYM
VDAC3
MAT2B
MUL1
MIRO1
ILF3
DNJC7
HUWE1
DYHC1
IPO5
UBP5
MCM5
C1TC
TCPB
VDAC2
SYIC
IDE
KDM3B
PSME3
MS18A
CF072
PRDX3


*FIG. 13*

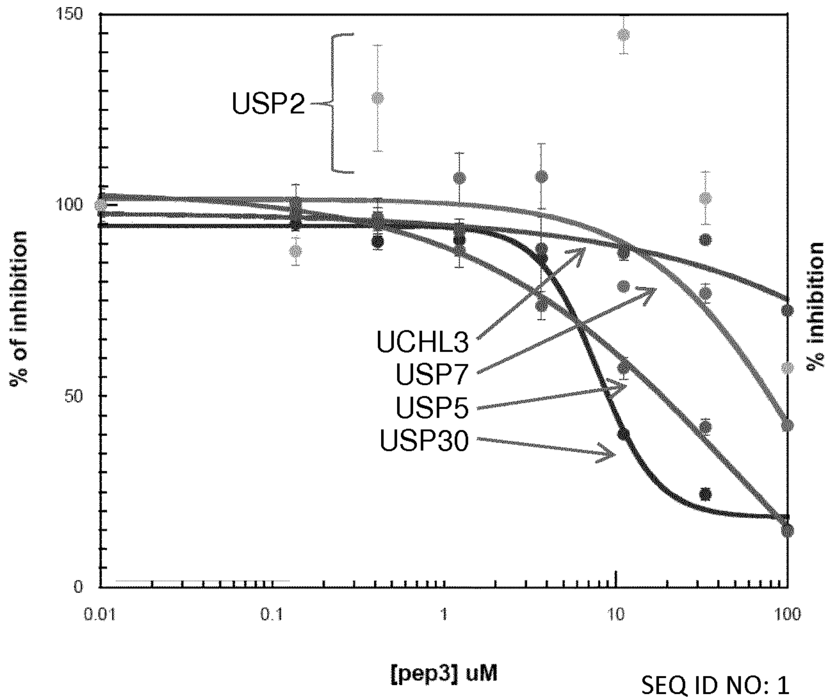


SEQ ID NO: 1

USP30_3 P L Y C F Y D L T Y G Y L C F Y

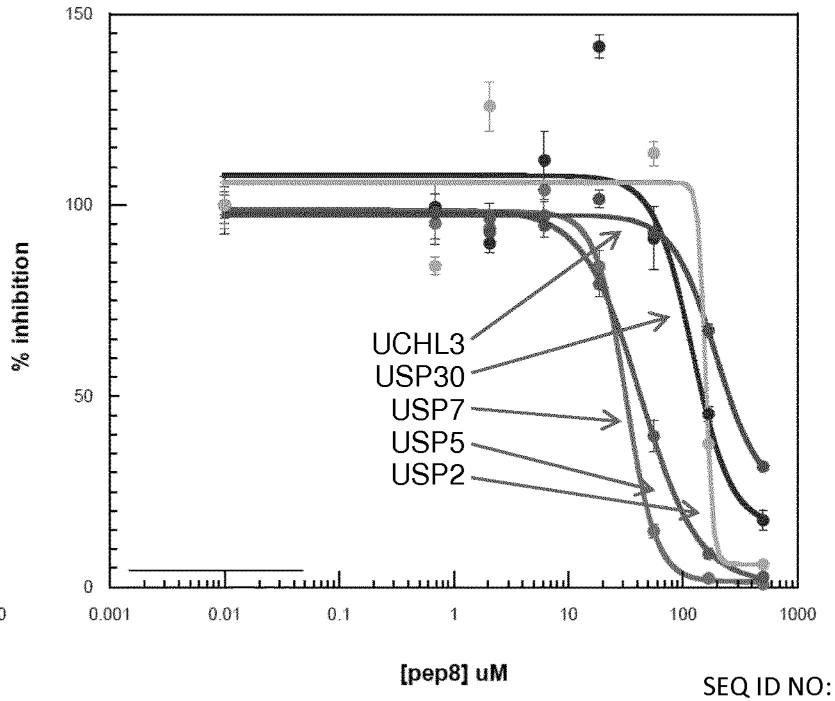


SEQ ID NO: 2

USP30_8 V S R C Y I F W N E M F C D V E

| | USP30 | USP7 | USP5 | UCHL3 | USP2 |
|---|---|---|---|---|---|
| pep3 IC50 | 8.0 | ~100 | 49.4 | >200 | >200 |
| Stdv | 1.0 | | 52.0 | | |
| Hill slope | 2.5 | | 0.6 | | |
| pep8 IC50 | 120.7 | 31.7 | 42.9 | 193.5 | ~200 |
| Stdv | 60 | 2.9 | 1.9 | 44.3 | |
| Hill slope | 2.6 | 3.3 | 1.7 | 2.3 | |

*FIG. 14*

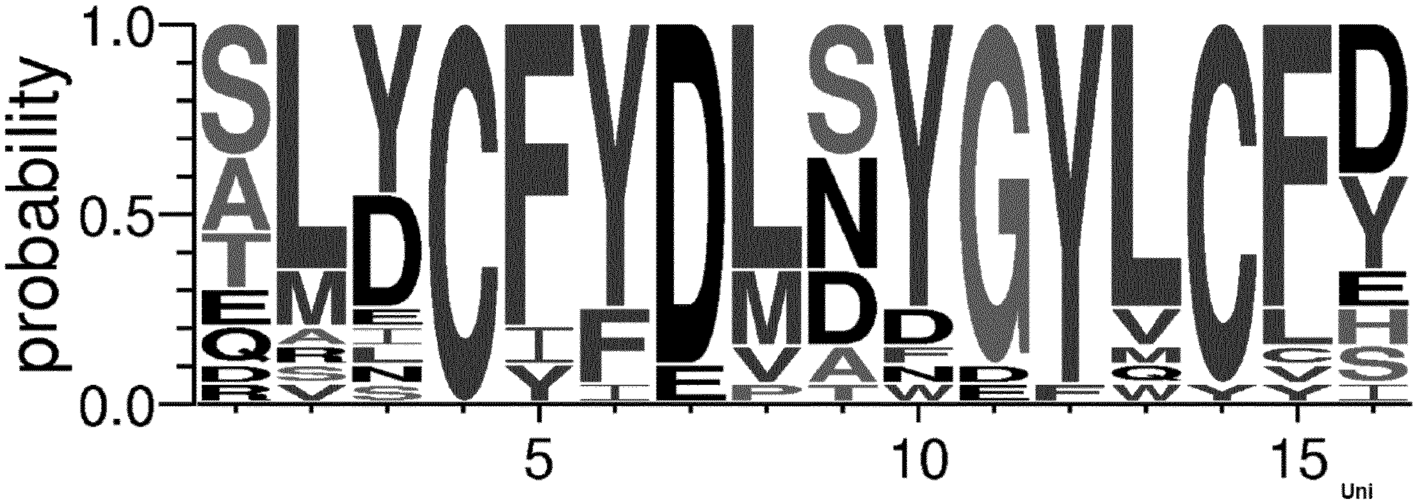


| SEQ ID NO: | Name | peptide seq | Number | S/N | signal | n | Total | Uni q |
|---|---|---|---|---|---|---|---|---|
| 1 | USP30-3 | P L Y C F Y D L T Y G Y L C F Y | | | | | | |
| 3 | 2 | S L D C F F D L S Y G Y L C F D | 2 | 28.3 | 1.99 | 45 | 66 | 20 |
| 4 | 3 | A M Y C F Y D M D Y G Y Q C L Y | 3 | 17.4 | 1.10 | 1 | | |
| 5 | 20 | T L N C Y Y D L D Y G Y L C F H | 20 | 24.9 | 1.75 | 1 | | |
| 6 | 23 | D L Y C F Y D L N D G Y L C F S | 23 | 33.3 | 3.10 | 1 | | |
| 7 | 24 | S L D C F F D L N Y G Y L C F D | 24 | 30.6 | 3.08 | 1 | | |
| 8 | 27 | S L Y C F Y D L S Y G Y V C L Y | 27 | 8.8 | 0.58 | 1 | | |
| 9 | 32 | Q S Y C F Y D V D W G Y L C Y H | 32 | 16.4 | 1.14 | 1 | | |
| 10 | 33 | S M L C F Y D V A Y G Y L C F D | 33 | 24.0 | 1.98 | 1 | | |
| 11 | 42 | S A I C F Y D M S Y G Y V C F E | 42 | 16.9 | 2.91 | 2 | | |
| 12 | 46 | T L Y C F F D M S Y G Y W C V D | 46 | 25.5 | 1.93 | 1 | | |
| 13 | 51 | T M Y C F Y D L S D D Y L C F Y | 51 | 30.2 | 2.33 | 2 | | |
| 14 | 52 | E L Y C F Y D L A N G Y L C F D | 52 | 13.0 | 1.34 | 1 | | |
| 15 | 53 | Q L D C I Y E L N Y G Y L C F D | 53 | 25.7 | 1.83 | 1 | | |
| 16 | 62 | S R D C F I D L N F G Y L Y C Y | 62 | 6.5 | 0.43 | 1 | | |
| 17 | 65 | A L D C F Y D L N Y G Y L C F D | 65 | 28.3 | 2.05 | 1 | | |
| 18 | 66 | A L D C I F E M S Y G F M C F D | 66 | 13.1 | 0.90 | 1 | | |
| 19 | 71 | E L S C F Y D L S Y G Y L C F Y | 71 | 19.8 | 1.84 | 1 | | |
| 20 | 77 | A L Y C Y Y D P N Y G Y L C F S | 77 | 20.3 | 1.51 | 1 | | |
| 21 | 85 | R V Y C F Y D L T Y E Y L C F I | 85 | 13.7 | 1.25 | 1 | | |
| 22 | 88 | S L E C F Y D L D Y G Y L C F E | 88 | 23.8 | 1.96 | 1 | | |

*FIG. 15*

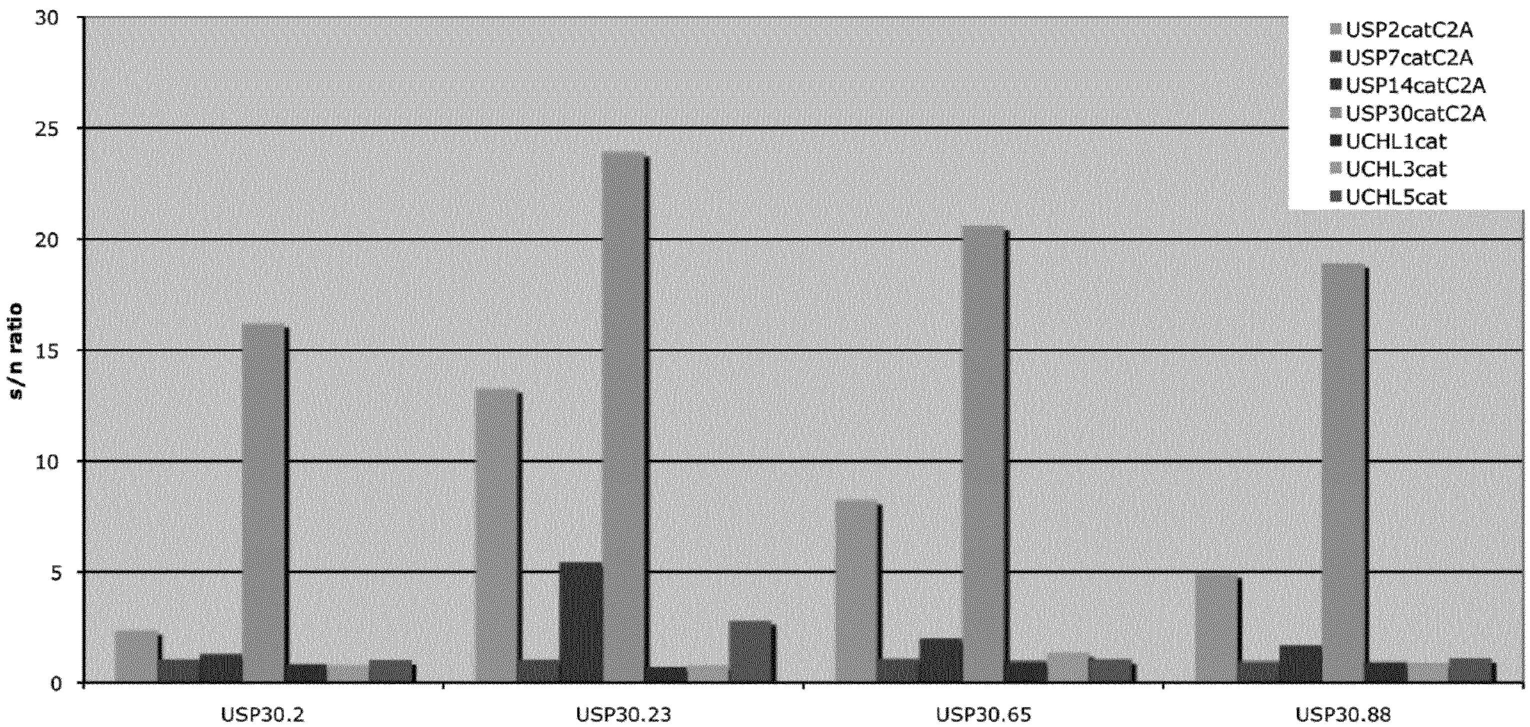


| SEQ: | | |
|---|---|---|
| 1 | **USP30-3** | P L Y C F Y D L T Y G Y L C F Y |
| 3 | **USP30-3.2** | S L D C F F D L S Y G Y L C F D |
| 6 | **USP30-3.23** | D L Y C F Y D L N D G Y L C F S |
| 17 | **USP30-3.65** | A L D C F Y D L N Y G Y L C F D |
| 22 | **USP30-3.88** | S L E C F Y D L D Y G Y L C F E |

***FIG. 16***

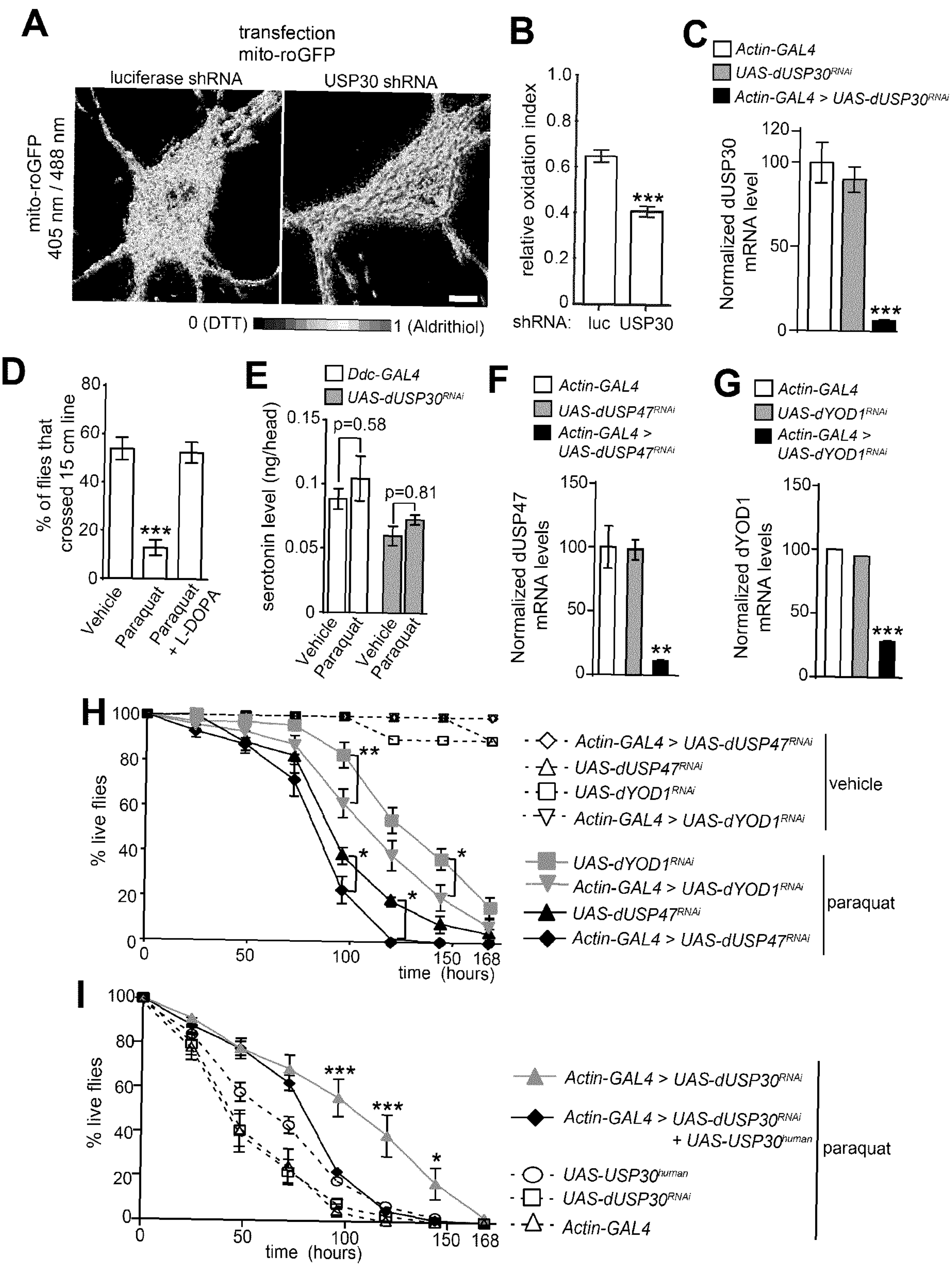
A
transfection
mito-roGFP
luciferase shRNA
USP30 shRNA
mito-roGFP
405 nm / 488 nm
0 (DTT)
1 (Aldrithiol)
B
relative oxidation index
***
shRNA: luc USP30
C
Actin-GAL4
UAS-dUSP30RNAi
Actin-GAL4 > UAS-dUSP30RNAi
Normalized dUSP30 mRNA level
***
D
% of flies that crossed 15 cm line
***
Vehicle
Paraquat
Paraquat + L-DOPA
E
Ddc-GAL4
UAS-dUSP30RNAi
serotonin level (ng/head)
p=0.58
p=0.81
Vehicle
Paraquat
Vehicle
Paraquat
F
Actin-GAL4
UAS-dUSP47RNAi
Actin-GAL4 > UAS-dUSP47RNAi
Normalized dUSP47 mRNA levels
**
G
Actin-GAL4
UAS-dYOD1RNAi
Actin-GAL4 > UAS-dYOD1RNAi
Normalized dYOD1 mRNA levels
***
H
% live flies
time (hours)
Actin-GAL4 > UAS-dUSP47RNAi
UAS-dUSP47RNAi
UAS-dYOD1RNAi
Actin-GAL4 > UAS-dYOD1RNAi
vehicle
UAS-dYOD1RNAi
Actin-GAL4 > UAS-dYOD1RNAi
UAS-dUSP47RNAi
Actin-GAL4 > UAS-dUSP47RNAi
paraquat
I
% live flies
time (hours)
Actin-GAL4 > UAS-dUSP30RNAi
Actin-GAL4 > UAS-dUSP30RNAi + UAS-USP30human
UAS-USP30human
UAS-dUSP30RNAi
Actin-GAL4
paraquat

*FIG. 17*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816567 A
- US 5530101 A
- US 5585089 A
- US 5693761 A
- US 5693762 A
- US 6180370 B
- US 20090136500 A
- US 5545807 A
- US 6713610 B
- US 6673986 B
- US 6162963 A
- US 6300129 B
- US 6255458 B
- US 5877397 A
- US 5874299 A
- US 5545806 A
- WO 9910494 A
- US 20080318838 A
- US 5010175 A
- WO 1996023899 A
- WO 1998015833 A
- US 5580723 A
- US 5834250 A
- US 4289872 A
- US 5229490 A
- WO 19930021259 A
- WO 20000024782 A
- US 6660843 B
- US 5475096 A
- US 7964356 B
- WO 2007017758 A
- WO 2004009023 A
- WO 2007009715 A
- US 20050260186 A
- US 20060104968 A
- US 61809927 B
- US 61701963 B


### Non-patent literature cited in the description


- **VAN ROON-MOM et al.** *Methods Mol. Biol.,* vol. 867, 79-96
- **PRAKASH.** *Chem. Biodivers.,* 2011, vol. 8, 1616-1641
- **YAMAMOTO et al.** *Future Med. Chem.,* 2011, vol. 3, 339-365
- **CHAN et al.** *Clin. Exper. Pharmacol. Physiol.,* 2006, vol. 33, 533-540
- **KURRECK et al.** *Nucl. Acids Res.,* 2002, vol. 30, 1911-1918
- **KURRECK.** *Eur. J. Biochem.,* 2003, vol. 270, 1628-1644
- **GEARY.** *Expert Opin. Drug Metab. Toxicol.,* 2009, vol. 5, 381-391
- Designing Antisense Oligonucleotides. *Integrated DNA Technologies,* 2011
- **FIRE et al.** *Nature,* 1998, vol. 391, 806-811
- **MCMANUS et al.** *Nat. Rev. Genet.,* 2002, vol. 3 (10), 737-747
- **PEACOCK et al.** *J. Org. Chem.,* 2011, vol. 76, 7295-7300
- **BRAMSEN et al.** *Methods Mol. Biol.,* 2011, vol. 721, 77-103
- **PASTERNAK et al.** *Org. Biomol. Chem.,* 2011, vol. 9, 3591-3597
- **GAGLIONE et al.** *Mini Rev. Med. Chem.,* 2010, vol. 10, 578-595
- **CHERNOLOVSKAYA et al.** *Curr. Opin. Mol. Ther.,* 2010, vol. 12, 158-167
- **SETH et al.** *Ther. Deliv.,* 2012, vol. 3, 245-261
- **KANASTY et al.** *Mol. Ther.,* 2012, vol. 20, 513-524
- *Methods Enzymol.,* 2012, vol. 502, 91-122
- **VADER et al.** *Curr. Top. Med. Chem.,* 2012, vol. 12, 108-119
- **NAEYE et al.** *Curr. Top. Med. Chem.,* 2012, vol. 12, 89-96
- **FOGED.** *Curr. Top. Med. Chem.,* 2012, vol. 12, 97-107
- **CHATURVEDI et al.** *Expert Opin. Drug Deliv.,* 2011, vol. 8, 1455-1468
- **GAO et al.** *Int. J. Nanomed.,* 2011, vol. 6, 1017-1025
- **SHEGOKAR et al.** *Pharmazie,* 2011, vol. 66, 313-318
- **KUMARI et al.** *Expert Opin. Drug Deliv.,* 2011, vol. 11, 1327-1339
- **MORRISON et al.** *Proc. Natl. Acad. Sci. USA,* 1984, vol. 81, 6851-55
- **JONES et al.** *Nature,* 1986, vol. 321, 522-525

- **RIECHMANN et al.** *Nature,* 1988, vol. 332, 323-27
- **VERHOEYEN et al.** *Science,* 1988, vol. 239, 1534-36
- **JAKOBOVITS et al.** *Proc. Natl. Acad. Sci. USA,* 1993, vol. 90, 2551-55
- **JAKOBOVITS et al.** *Nature,* 1993, vol. 362, 255-8
- **LONBERG et al.** *Nature,* 1994, vol. 368, 856-9
- **HOOGENBOOM et al.** *J. Mol. Biol.,* 1992, vol. 227, 381-8
- **MARKS et al.** *J. Mol. Biol.,* 1991, vol. 222, 581-97
- **GENTILUCCI et al.** *Curr. Pharm. Des.,* 2010, vol. 16, 3185-3203
- **BRATKOVIC.** *Cell. Mol. Life Sci.,* 2010, vol. 67, 749-767
- **PANDE et al.** *Biotech. Adv.,* 2010, vol. 28, 849-858
- **NI et al.** *Curr. Med. Chem.,* 2011, vol. 18, 4206
- **ELLINGTON et al.** *Nature,* 1990, vol. 346, 818
- **TUERK et al.** *Science,* 1990, vol. 249, 505
- **BEREZOVSKI et al.** *J. Am. Chem. Soc.,* 2008, vol. 130, 913
- **BRODY et al.** *Expert Rev. Mol. Diagn.,* 2010, vol. 10, 1013-22
- **THOMPSON et al.** *Chem. Rev.,* 1996, vol. 96, 555-600
- **GALLOP et al.** *J. Med. Chem.,* 1994, vol. 37, 1233-1250
- **COLLAND et al.** *Mol. Cancer Therap.,* 2009, vol. 8, 2286
- **LEE et al.** *Nature,* 2010, vol. 467, 179-184
- **TIAN et al.** *Assay Drug Develop. Technol.,* 2011, vol. 9, 165-173
- **LIU et al.** *Chemistry & Biology,* 2003, vol. 10, 837-846
- **LIU et al.** *Neurobiol. Disease,* 2010, vol. 41, 318-328
- **KOHARUDIN et al.** *PNAS,* 2010, vol. 107, 6835-6840
- **MULLALLY et al.** *J. Biol. Chem.,* 2001, vol. 276, 30366-73
- **QUESADA et al.** *Biochem. Biophys. Res. Commun,* 2004, vol. 314, 54-62
- Remington's Pharmaceutical Sciences. 1980
- **PATHAN et al.** *Recent Patents on Drug Delivery & Formulation,* 2009, vol. 3, 71-89
- **SEEBURG et al.** *Neuron,* 2008, vol. 58, 571-583
- **KATAYAMA et al.** *Chemistry & Biology,* 2011, vol. 18, 1042-1094
- **SEEBURG ; SHENG.** *J. Neurosci.,* 2008, vol. 28, 6583-6591
- **DOOLEY et al.** *J. Biol. Chem.,* 2004, vol. 279, 22284-22293
- **XU et al.** *Nat. Biotech.,* 2010, vol. 28, 868-873
- **KIM et al.** *Mol. Cell,* 2011, vol. 44, 325-340
- **RAPPSILBER et al.** *Anal. Chem.,* 2003, vol. 75, 663-670
- **BEAUSOLEIL et al.** *Nat. Biotech.,* 2006, vol. 24 (10), 1285-1292
- **BUSTOS et al.** *Mol. Cell. Proteomics,* 23 June 2012
- **SEYFRIED et al.** *Anal. Chem.,* 2008, vol. 80, 4161-4169
- **BAKALARSKI et al.** *J. Proteome Res.,* 2008, vol. 7, 4756-4765
- **PINHEIRO et al.** nlme: Linear and Nonlinear Mixed Effects Models. *R package version,* 2011, vol. 3, 1-101
- **GREENE et al.** *Proc. Natl. Acad. Sci. USA,* 2003, vol. 100, 4078-4083
- **NARENDRA et al.** *J. Cell Biol.,* 2008, vol. 183, 795-803
- **NAKAMURA ; HIROSE.** *Mol. Biol. Cell,* 2008, vol. 19, 1903-1914
- **LEE et al.** *J. Cell Biol.,* 2010, vol. 189, 671-680
- **GEISLER et al.** *Nat. Cell Biol.,* 2010, vol. 12, 119-131
- **YOULE ; NARENDRA.** *Nat. Rev. Mol. Cell Biol.,* 2011, vol. 12, 9-14
- **CLARK et al.** *Nature,* 2006, vol. 441, 1162-1166
- **PARK et al.** *Nature,* 2006, vol. 441, 1157-1161
- **BERLIN et al.** *J. Biol. Chem.,* 2010, vol. 285, 34909-34921
- **BOMBERGER et al.** *J. Biol. Chem.,* 2009, vol. 284, 18778-18789
- **OGAWA et al.** *J. Biol. Chem.,* 2011, vol. 286, 41455-41465
- **CHAN et al.** *Human Mol. Genet.,* 2011, vol. 20, 1726-1737
- **FRANSSON et al.** *Bioch. Biophys. Res. Comm.,* 2006, vol. 344, 500-510
- **SRIRAM et al.** *Human Mol. Genet.,* 2005, vol. 14, 2571-2586
- **LEE et al.** *Biochem. J.,* 2012, vol. 441, 523-540
- **GUZMAN et al.** *Nature,* 2010, vol. 468, 696-700
- **GUO.** *Cold Spring Harb. Perspect. Med.,* 2012, vol. 2 (11
- **BRAND et al.** *Development,* 1993, vol. 118, 401-415
- **CASTELLO et al.** *J. Biol. Chem.,* 2007, vol. 282, 14186-14193
- **COCHEME et al.** *J. Biol. Chem.,* 2008, vol. 283, 1786-1798
- **TANNER et al.** *Environmental Health Perspect.,* 2011, vol. 119, 866-872
- **SCHAPIRA et al.** *Lancet,* 1989, vol. 1, 1269

- **BENDER et al.** *Nature Genet.,* 2006, vol. 38, 515-517
- **KRAYTSBERG et al.** *Nature Genet.,* 2006, vol. 38, 518-520
- **ZHENG et al.** *Science Transl. Med.,* 2010, vol. 2, 52ra73
- **YOULE et al.** *J. Biol. Chem.,* 2011, vol. 12, 9-23
- **TANAKA et al.** *J. Cell. Biol.,* 2010, vol. 191, 1367-1380
- **TWIG et al.** *EMBO J.,* 2008, vol. 27, 433-446
- **NARENDRA et al.** *PLoS Biology,* 2010, vol. 8, e1000298
- **SHIN et al.** *Cell,* 2011, vol. 144, 689-702
- **MATSUDA et al.** *J. Cell Biol.,* 2010, vol. 189, 211-221
- **KONDAPALLI et al.** *Open Biology,* 2012, vol. 2, 120080
- **VIVES-BAUZA et al.** *Proc. Natl. Acad. Sci. USA,* 2010, vol. 107, 378-383
- **PAGLIARINI et al.** *Cell,* 2008, vol. 134, 112-123
- **HAMPE et al.** *Human Mol. Genet.,* 2006, vol. 15, 2059-2075
- **MATSUDA et al.** *J. Biol. Chem.,* 2006, vol. 281, 3204-3209
- **WANG et al.** *J. Neurochem.,* 2005, vol. 93, 422-431
- **WINKLHOFER et al.** *J. Biol. Chem.,* 2003, vol. 278, 47199-47208
- **CORTI et al.** *Physiol. Rev.,* 2011, vol. 91, 1161-1218
- **SCHAPIRA et al.** *Parkinson's Dis.,* 2011, vol. 2011, 159160
- **GOTTLIEB et al.** *Am. J. Physiol. Cell Physiol.,* 2010, vol. 299, C203-210
- **HUANG et al.** *PLoS One,* 2011, vol. 6, e20975
- **BAYONA-BAFALUY et al.** *Proc. Natl. Acad. Sci. USA,* 2005, vol. 102, 14392-14397
- **SUEN et al.** *Proc. Natl. Acad. Sci. USA,* 2010, vol. 107, 11835-11840
- **STANGER et al.** *Nat. Chem. Bio.,* 2012, vol. 7, 655-660

SEQUENCE LISTING

<110> Genentech, Inc.

<120> USP30 INHIBITORS AND METHODS OF USE

<130> P4986R1-WO

<150> US 61/809,927
<151> 2013-04-09

<150> US 61/701,963
<151> 2012-09-17

<160> 49

<170> PatentIn version 3.5

<210> 1
<211> 16
<212> PRT
<213> Artificial sequence

<220>
<223> Synthetic

<400> 1

```
Pro Leu Tyr Cys Phe Tyr Asp Leu Thr Tyr Gly Tyr Leu Cys Phe Tyr
1               5                   10                  15
```

<210> 2
<211> 16
<212> PRT
<213> Artificial sequence

<220>
<223> Synthetic

<400> 2

```
Val Ser Arg Cys Tyr Ile Phe Trp Asn Glu Met Phe Cys Asp Val Glu
1               5                   10                  15
```

<210> 3
<211> 16
<212> PRT
<213> Artificial sequence

<220>
<223> Synthetic

<400> 3

```
Ser Leu Asp Cys Phe Phe Asp Leu Ser Tyr Gly Tyr Leu Cys Phe Asp
1               5                   10                  15
```

<210> 4
<211> 16
<212> PRT
<213> Artificial sequence

<220>
<223> Synthetic

<400> 4

```
Ala Met Tyr Cys Phe Tyr Asp Met Asp Tyr Gly Tyr Gln Cys Leu Tyr
1               5                   10                  15
```

<210> 5
<211> 16
<212> PRT
<213> Artificial sequence

<220>
<223> Synthetic

<400> 5

```
Thr Leu Asn Cys Tyr Tyr Asp Leu Asp Tyr Gly Tyr Leu Cys Phe His
1               5                   10                  15
```

<210> 6
<211> 16
<212> PRT
<213> Artificial sequence

<220>

<223> Synthetic

<400> 6

```
Asp Leu Tyr Cys Phe Tyr Asp Leu Asn Asp Gly Tyr Leu Cys Phe Ser
1               5                   10                  15
```

<210> 7
<211> 16
<212> PRT
<213> Artificial sequence

<220>
<223> Synthetic

<400> 7

```
Ser Leu Asp Cys Phe Phe Asp Leu Asn Tyr Gly Tyr Leu Cys Phe Asp
1               5                   10                  15
```

<210> 8
<211> 16
<212> PRT
<213> Artificial sequence

<220>
<223> Synthetic

<400> 8

```
Ser Leu Tyr Cys Phe Tyr Asp Leu Ser Tyr Gly Tyr Val Cys Leu Tyr
1               5                   10                  15
```

<210> 9
<211> 16
<212> PRT
<213> Artificial sequence

<220>
<223> Synthetic

<400> 9

```
Gln Ser Tyr Cys Phe Tyr Asp Val Asp Trp Gly Tyr Leu Cys Tyr His
1               5                   10                  15
```

<210> 10
<211> 16
<212> PRT
<213> Artificial sequence

<220>
<223> Synthetic

<400> 10

```
Ser Met Leu Cys Phe Tyr Asp Val Ala Tyr Gly Tyr Leu Cys Phe Asp
1               5                   10                  15
```

<210> 11
<211> 16
<212> PRT
<213> Artificial sequence

<220>
<223> Synthetic

<400> 11

```
Ser Ala Ile Cys Phe Tyr Asp Met Ser Tyr Gly Tyr Val Cys Phe Glu
1               5                   10                  15
```

<210> 12
<211> 16
<212> PRT
<213> Artificial sequence

<220>
<223> Synthetic

<400> 12

```
Thr Leu Tyr Cys Phe Phe Asp Met Ser Tyr Gly Tyr Trp Cys Val Asp
1               5                   10                  15
```

<210> 13
<211> 16
<212> PRT
<213> Artificial sequence

<220>
<223> Synthetic

<400> 13

```
Thr Met Tyr Cys Phe Tyr Asp Leu Ser Asp Asp Tyr Leu Cys Phe Tyr
1               5                   10                  15
```

<210> 14
<211> 16
<212> PRT
<213> Artificial sequence

<220>
<223> Synthetic

<400> 14

```
Glu Leu Tyr Cys Phe Tyr Asp Leu Ala Asn Gly Tyr Leu Cys Phe Asp
1               5                   10                  15
```

<210> 15
<211> 16
<212> PRT
<213> Artificial sequence

<220>

<223> Synthetic

<400> 15

```
Gln Leu Asp Cys Ile Tyr Glu Leu Asn Tyr Gly Tyr Leu Cys Phe Asp
1               5                   10                  15
```

<210> 16
<211> 16
<212> PRT
<213> Artificial sequence

<220>
<223> Synthetic

<400> 16

```
Ser Arg Asp Cys Phe Ile Asp Leu Asn Phe Gly Tyr Leu Tyr Cys Tyr
1               5                   10                  15
```

<210> 17
<211> 16
<212> PRT
<213> Artificial sequence

<220>
<223> Synthetic

<400> 17

```
Ala Leu Asp Cys Phe Tyr Asp Leu Asn Tyr Gly Tyr Leu Cys Phe Asp
1               5                   10                  15
```

<210> 18
<211> 16
<212> PRT
<213> Artificial sequence

<220>
<223> Synthetic

<400> 18

```
Ala Leu Asp Cys Ile Phe Glu Met Ser Tyr Gly Phe Met Cys Phe Asp
1               5                   10                  15
```

<210> 19
<211> 16
<212> PRT
<213> Artificial sequence

<220>
<223> Synthetic

<400> 19

```
Glu Leu Ser Cys Phe Tyr Asp Leu Ser Tyr Gly Tyr Leu Cys Phe Tyr
1               5                   10                  15
```

<210> 20
<211> 16
<212> PRT
<213> Artificial sequence

<220>
<223> Synthetic

<400> 20

```
Ala Leu Tyr Cys Tyr Tyr Asp Pro Asn Tyr Gly Tyr Leu Cys Phe Ser
1               5                   10                  15
```

<210> 21
<211> 16
<212> PRT
<213> Artificial sequence

<220>
<223> Synthetic

<400> 21

```
Arg Val Tyr Cys Phe Tyr Asp Leu Thr Tyr Glu Tyr Leu Cys Phe Ile
1               5                   10                  15
```

<210> 22
<211> 16
<212> PRT
<213> Artificial sequence

<220>
<223> Synthetic

<400> 22

```
Ser Leu Glu Cys Phe Tyr Asp Leu Asp Tyr Gly Tyr Leu Cys Phe Glu
1               5                   10                  15
```

<210> 23
<211> 12
<212> PRT
<213> Artificial sequence

<220>
<223> Synthetic

<400> 23

```
Arg Ala Gly Leu Ser Lys Leu Pro Asp Leu Lys Asp
1               5                   10
```

<210> 24
<211> 14
<212> PRT
<213> Artificial sequence

<220>

<223> Synthetic

<400> 24

Lys Leu Pro Asp Leu Lys Asp Ala Glu Ala Val Gln Lys Phe
1 5 10

<210> 25
<211> 29
<212> PRT
<213> Artificial sequence

<220>
<223> Synthetic

<400> 25

Lys Asp Ala Glu Ala Val Gln Lys Phe Phe Leu Glu Glu Ile Gln Leu
1 5 10 15

Gly Glu Glu Leu Leu Ala Gln Gly Glu Tyr Glu Lys Gly
20 25

<210> 26
<211> 517
<212> PRT
<213> Homo sapiens

<400> 26

```
Met Leu Ser Ser Arg Ala Glu Ala Ala Met Thr Ala Ala Asp Arg Ala
1               5                   10                  15

Ile Gln Arg Phe Leu Arg Thr Gly Ala Ala Val Arg Tyr Lys Val Met
            20                  25                  30

Lys Asn Trp Gly Val Ile Gly Gly Ile Ala Ala Ala Leu Ala Ala Gly
        35                  40                  45

Ile Tyr Val Ile Trp Gly Pro Ile Thr Glu Arg Lys Lys Arg Arg Lys
    50                  55                  60

Gly Leu Val Pro Gly Leu Val Asn Leu Gly Asn Thr Cys Phe Met Asn
65                  70                  75                  80

Ser Leu Leu Gln Gly Leu Ser Ala Cys Pro Ala Phe Ile Arg Trp Leu
                85                  90                  95

Glu Glu Phe Thr Ser Gln Tyr Ser Arg Asp Gln Lys Glu Pro Pro Ser
            100                 105                 110

His Gln Tyr Leu Ser Leu Thr Leu Leu His Leu Leu Lys Ala Leu Ser
        115                 120                 125

Cys Gln Glu Val Thr Asp Asp Glu Val Leu Asp Ala Ser Cys Leu Leu
    130                 135                 140

Asp Val Leu Arg Met Tyr Arg Trp Gln Ile Ser Ser Phe Glu Glu Gln
145                 150                 155                 160

Asp Ala His Glu Leu Phe His Val Ile Thr Ser Ser Leu Glu Asp Glu
                165                 170                 175

Arg Asp Arg Gln Pro Arg Val Thr His Leu Phe Asp Val His Ser Leu
            180                 185                 190

Glu Gln Gln Ser Glu Ile Thr Pro Lys Gln Ile Thr Cys Arg Thr Arg
        195                 200                 205

Gly Ser Pro His Pro Thr Ser Asn His Trp Lys Ser Gln His Pro Phe
    210                 215                 220

His Gly Arg Leu Thr Ser Asn Met Val Cys Lys His Cys Glu His Gln
225                 230                 235                 240

Ser Pro Val Arg Phe Asp Thr Phe Asp Ser Leu Ser Leu Ser Ile Pro
                245                 250                 255
```

```
Ala Ala Thr Trp Gly His Pro Leu Thr Leu Asp His Cys Leu His His
            260                 265                 270

Phe Ile Ser Ser Glu Ser Val Arg Asp Val Val Cys Asp Asn Cys Thr
        275                 280                 285

Lys Ile Glu Ala Lys Gly Thr Leu Asn Gly Glu Lys Val Glu His Gln
    290                 295                 300

Arg Thr Thr Phe Val Lys Gln Leu Lys Leu Gly Lys Leu Pro Gln Cys
305                 310                 315                 320

Leu Cys Ile His Leu Gln Arg Leu Ser Trp Ser Ser His Gly Thr Pro
                325                 330                 335

Leu Lys Arg His Glu His Val Gln Phe Asn Glu Phe Leu Met Met Asp
            340                 345                 350

Ile Tyr Lys Tyr His Leu Leu Gly His Lys Pro Ser Gln His Asn Pro
        355                 360                 365

Lys Leu Asn Lys Asn Pro Gly Pro Thr Leu Glu Leu Gln Asp Gly Pro
    370                 375                 380

Gly Ala Pro Thr Pro Val Leu Asn Gln Pro Gly Ala Pro Lys Thr Gln
385                 390                 395                 400

Ile Phe Met Asn Gly Ala Cys Ser Pro Ser Leu Leu Pro Thr Leu Ser
                405                 410                 415

Ala Pro Met Pro Phe Pro Leu Pro Val Val Pro Asp Tyr Ser Ser Ser
            420                 425                 430

Thr Tyr Leu Phe Arg Leu Met Ala Val Val Val His His Gly Asp Met
        435                 440                 445

His Ser Gly His Phe Val Thr Tyr Arg Arg Ser Pro Pro Ser Ala Arg
    450                 455                 460

Asn Pro Leu Ser Thr Ser Asn Gln Trp Leu Trp Val Ser Asp Asp Thr
465                 470                 475                 480

Val Arg Lys Ala Ser Leu Gln Glu Val Leu Ser Ser Ser Ala Tyr Leu
                485                 490                 495

Leu Phe Tyr Glu Arg Val Leu Ser Arg Met Gln His Gln Ser Gln Glu
```

500 505 510

Cys Lys Ser Glu Glu
515

<210> 27
<211> 145
<212> PRT
<213> Homo sapiens

<400> 27

Met Val Gly Arg Asn Ser Ala Ile Ala Ala Gly Val Cys Gly Ala Leu
1 5 10 15

Phe Ile Gly Tyr Cys Ile Tyr Phe Asp Arg Lys Arg Arg Ser Asp Pro
20 25 30

Asn Phe Lys Asn Arg Leu Arg Glu Arg Arg Lys Lys Gln Lys Leu Ala
35 40 45

Lys Glu Arg Ala Gly Leu Ser Lys Leu Pro Asp Leu Lys Asp Ala Glu
50 55 60

Ala Val Gln Lys Phe Phe Leu Glu Glu Ile Gln Leu Gly Glu Glu Leu
65 70 75 80

Leu Ala Gln Gly Glu Tyr Glu Lys Gly Val Asp His Leu Thr Asn Ala
85 90 95

Ile Ala Val Cys Gly Gln Pro Gln Gln Leu Leu Gln Val Leu Gln Gln
100 105 110

Thr Leu Pro Pro Pro Val Phe Gln Met Leu Leu Thr Lys Leu Pro Thr
115 120 125

Ile Ser Gln Arg Ile Val Ser Ala Gln Ser Leu Ala Glu Asp Asp Val
130 135 140

Glu
145

<210> 28
<211> 618
<212> PRT
<213> Homo sapiens

<400> 28

```
Met Lys Lys Asp Val Arg Ile Leu Leu Val Gly Glu Pro Arg Val Gly
1               5                   10                  15
```

```
Lys Thr Ser Leu Ile Met Ser Leu Val Ser Glu Glu Phe Pro Glu Glu
            20                  25                  30

Val Pro Pro Arg Ala Glu Glu Ile Thr Ile Pro Ala Asp Val Thr Pro
        35                  40                  45

Glu Arg Val Pro Thr His Ile Val Asp Tyr Ser Glu Ala Glu Gln Ser
    50                  55                  60

Asp Glu Gln Leu His Gln Glu Ile Ser Gln Ala Asn Val Ile Cys Ile
65                  70                  75                  80

Val Tyr Ala Val Asn Asn Lys His Ser Ile Asp Lys Val Thr Ser Arg
                85                  90                  95

Trp Ile Pro Leu Ile Asn Glu Arg Thr Asp Lys Asp Ser Arg Leu Pro
            100                 105                 110

Leu Ile Leu Val Gly Asn Lys Ser Asp Leu Val Glu Tyr Ser Ser Met
        115                 120                 125

Glu Thr Ile Leu Pro Ile Met Asn Gln Tyr Thr Glu Ile Glu Thr Cys
    130                 135                 140

Val Glu Cys Ser Ala Lys Asn Leu Lys Asn Ile Ser Glu Leu Phe Tyr
145                 150                 155                 160

Tyr Ala Gln Lys Ala Val Leu His Pro Thr Gly Pro Leu Tyr Cys Pro
                165                 170                 175

Glu Glu Lys Glu Met Lys Pro Ala Cys Ile Lys Ala Leu Thr Arg Ile
            180                 185                 190

Phe Lys Ile Ser Asp Gln Asp Asn Asp Gly Thr Leu Asn Asp Ala Glu
        195                 200                 205

Leu Asn Phe Phe Gln Arg Ile Cys Phe Asn Thr Pro Leu Ala Pro Gln
    210                 215                 220

Ala Leu Glu Asp Val Lys Asn Val Val Arg Lys His Ile Ser Asp Gly
225                 230                 235                 240

Val Ala Asp Ser Gly Leu Thr Leu Lys Gly Phe Leu Phe Leu His Thr
                245                 250                 255

Leu Phe Ile Gln Arg Gly Arg His Glu Thr Thr Trp Thr Val Leu Arg
```

```
            260                 265                 270

Arg Phe Gly Tyr Asp Asp Asp Leu Asp Leu Thr Pro Glu Tyr Leu Phe
        275                 280                 285

Pro Leu Leu Lys Ile Pro Pro Asp Cys Thr Thr Glu Leu Asn His His
    290                 295                 300

Ala Tyr Leu Phe Leu Gln Ser Thr Phe Asp Lys His Asp Leu Asp Arg
305                 310                 315                 320

Asp Cys Ala Leu Ser Pro Asp Glu Leu Lys Asp Leu Phe Lys Val Phe
                325                 330                 335

Pro Tyr Ile Pro Trp Gly Pro Asp Val Asn Asn Thr Val Cys Thr Asn
            340                 345                 350

Glu Arg Gly Trp Ile Thr Tyr Gln Gly Phe Leu Ser Gln Trp Thr Leu
        355                 360                 365

Thr Thr Tyr Leu Asp Val Gln Arg Cys Leu Glu Tyr Leu Gly Tyr Leu
    370                 375                 380

Gly Tyr Ser Ile Leu Thr Glu Gln Glu Ser Gln Ala Ser Ala Val Thr
385                 390                 395                 400

Val Thr Arg Asp Lys Lys Ile Asp Leu Gln Lys Lys Gln Thr Gln Arg
                405                 410                 415

Asn Val Phe Arg Cys Asn Val Ile Gly Val Lys Asn Cys Gly Lys Ser
            420                 425                 430

Gly Val Leu Gln Ala Leu Leu Gly Arg Asn Leu Met Arg Gln Lys Lys
        435                 440                 445

Ile Arg Glu Asp His Lys Ser Tyr Tyr Ala Ile Asn Thr Val Tyr Val
    450                 455                 460

Tyr Gly Gln Glu Lys Tyr Leu Leu Leu His Asp Ile Ser Glu Ser Glu
465                 470                 475                 480

Phe Leu Thr Glu Ala Glu Ile Ile Cys Asp Val Val Cys Leu Val Tyr
                485                 490                 495

Asp Val Ser Asn Pro Lys Ser Phe Glu Tyr Cys Ala Arg Ile Phe Lys
            500                 505                 510
```

Gln His Phe Met Asp Ser Arg Ile Pro Cys Leu Ile Val Ala Ala Lys
515 520 525

Ser Asp Leu His Glu Val Lys Gln Glu Tyr Ser Ile Ser Pro Thr Asp
530 535 540

Phe Cys Arg Lys His Lys Met Pro Pro Pro Gln Ala Phe Thr Cys Asn
545 550 555 560

Thr Ala Asp Ala Pro Ser Lys Asp Ile Phe Val Lys Leu Thr Thr Met
565 570 575

Ala Met Tyr Pro His Val Thr Gln Ala Asp Leu Lys Ser Ser Thr Phe
580 585 590

Trp Leu Arg Ala Ser Phe Gly Ala Thr Val Phe Ala Val Leu Gly Phe
595 600 605

Ala Met Tyr Lys Ala Leu Leu Lys Gln Arg
610 615

<210> 29
<211> 465
<212> PRT
<213> Homo sapiens

<400> 29

Met Ile Val Phe Val Arg Phe Asn Ser Ser His Gly Phe Pro Val Glu
1 5 10 15

Val Asp Ser Asp Thr Ser Ile Phe Gln Leu Lys Glu Val Val Ala Lys
20 25 30

Arg Gln Gly Val Pro Ala Asp Gln Leu Arg Val Ile Phe Ala Gly Lys
35 40 45

Glu Leu Arg Asn Asp Trp Thr Val Gln Asn Cys Asp Leu Asp Gln Gln
50 55 60

Ser Ile Val His Ile Val Gln Arg Pro Trp Arg Lys Gly Gln Glu Met
65 70 75 80

Asn Ala Thr Gly Gly Asp Asp Pro Arg Asn Ala Ala Gly Gly Cys Glu
85 90 95

Arg Glu Pro Gln Ser Leu Thr Arg Val Asp Leu Ser Ser Ser Val Leu
100 105 110

```
Pro Gly Asp Ser Val Gly Leu Ala Val Ile Leu His Thr Asp Ser Arg
        115                 120                 125

Lys Asp Ser Pro Pro Ala Gly Ser Pro Ala Gly Arg Ser Ile Tyr Asn
    130                 135                 140

Ser Phe Tyr Val Tyr Cys Lys Gly Pro Cys Gln Arg Val Gln Pro Gly
145                 150                 155                 160

Lys Leu Arg Val Gln Cys Ser Thr Cys Arg Gln Ala Thr Leu Thr Leu
                165                 170                 175

Thr Gln Gly Pro Ser Cys Trp Asp Asp Val Leu Ile Pro Asn Arg Met
            180                 185                 190

Ser Gly Glu Cys Gln Ser Pro His Cys Pro Gly Thr Ser Ala Glu Phe
        195                 200                 205

Phe Phe Lys Cys Gly Ala His Pro Thr Ser Asp Lys Glu Thr Ser Val
    210                 215                 220

Ala Leu His Leu Ile Ala Thr Asn Ser Arg Asn Ile Thr Cys Ile Thr
225                 230                 235                 240

Cys Thr Asp Val Arg Ser Pro Val Leu Val Phe Gln Cys Asn Ser Arg
                245                 250                 255

His Val Ile Cys Leu Asp Cys Phe His Leu Tyr Cys Val Thr Arg Leu
            260                 265                 270

Asn Asp Arg Gln Phe Val His Asp Pro Gln Leu Gly Tyr Ser Leu Pro
        275                 280                 285

Cys Val Ala Gly Cys Pro Asn Ser Leu Ile Lys Glu Leu His His Phe
    290                 295                 300

Arg Ile Leu Gly Glu Glu Gln Tyr Asn Arg Tyr Gln Gln Tyr Gly Ala
305                 310                 315                 320

Glu Glu Cys Val Leu Gln Met Gly Gly Val Leu Cys Pro Arg Pro Gly
                325                 330                 335

Cys Gly Ala Gly Leu Leu Pro Glu Pro Asp Gln Arg Lys Val Thr Cys
            340                 345                 350

Glu Gly Gly Asn Gly Leu Gly Cys Gly Phe Ala Phe Cys Arg Glu Cys
        355                 360                 365
```

Lys Glu Ala Tyr His Glu Gly Glu Cys Ser Ala Val Phe Glu Ala Ser
370 375 380

Gly Thr Thr Thr Gln Ala Tyr Arg Val Asp Glu Arg Ala Ala Glu Gln
385 390 395 400

Ala Arg Trp Glu Ala Ala Ser Lys Glu Thr Ile Lys Lys Thr Thr Lys
405 410 415

Pro Cys Pro Arg Cys His Val Pro Val Glu Lys Asn Gly Gly Cys Met
420 425 430

His Met Lys Cys Pro Gln Pro Gln Cys Arg Leu Glu Trp Cys Trp Asn
435 440 445

Cys Gly Cys Glu Trp Asn Arg Val Cys Met Gly Asp His Trp Phe Asp
450 455 460

Val
465

<210> 30
<211> 581
<212> PRT
<213> Homo sapiens

<400> 30

Met Ala Val Arg Gln Ala Leu Gly Arg Gly Leu Gln Leu Gly Arg Ala
1 5 10 15

Leu Leu Leu Arg Phe Thr Gly Lys Pro Gly Arg Ala Tyr Gly Leu Gly
20 25 30

Arg Pro Gly Pro Ala Ala Gly Cys Val Arg Gly Glu Arg Pro Gly Trp
35 40 45

Ala Ala Gly Pro Gly Ala Glu Pro Arg Arg Val Gly Leu Gly Leu Pro
50 55 60

Asn Arg Leu Arg Phe Phe Arg Gln Ser Val Ala Gly Leu Ala Ala Arg
65 70 75 80

Leu Gln Arg Gln Phe Val Val Arg Ala Trp Gly Cys Ala Gly Pro Cys
85 90 95

Gly Arg Ala Val Phe Leu Ala Phe Gly Leu Gly Leu Gly Leu Ile Glu
100 105 110

```
Glu Lys Gln Ala Glu Ser Arg Arg Ala Val Ser Ala Cys Gln Glu Ile
        115                 120                 125

Gln Ala Ile Phe Thr Gln Lys Ser Lys Pro Gly Pro Asp Pro Leu Asp
    130                 135                 140

Thr Arg Arg Leu Gln Gly Phe Arg Leu Glu Glu Tyr Leu Ile Gly Gln
145                 150                 155                 160

Ser Ile Gly Lys Gly Cys Ser Ala Ala Val Tyr Glu Ala Thr Met Pro
                165                 170                 175

Thr Leu Pro Gln Asn Leu Glu Val Thr Lys Ser Thr Gly Leu Leu Pro
            180                 185                 190

Gly Arg Gly Pro Gly Thr Ser Ala Pro Gly Glu Gly Gln Glu Arg Ala
        195                 200                 205

Pro Gly Ala Pro Ala Phe Pro Leu Ala Ile Lys Met Met Trp Asn Ile
    210                 215                 220

Ser Ala Gly Ser Ser Ser Glu Ala Ile Leu Asn Thr Met Ser Gln Glu
225                 230                 235                 240

Leu Val Pro Ala Ser Arg Val Ala Leu Ala Gly Glu Tyr Gly Ala Val
                245                 250                 255

Thr Tyr Arg Lys Ser Lys Arg Gly Pro Lys Gln Leu Ala Pro His Pro
            260                 265                 270

Asn Ile Ile Arg Val Leu Arg Ala Phe Thr Ser Ser Val Pro Leu Leu
        275                 280                 285

Pro Gly Ala Leu Val Asp Tyr Pro Asp Val Leu Pro Ser Arg Leu His
    290                 295                 300

Pro Glu Gly Leu Gly His Gly Arg Thr Leu Phe Leu Val Met Lys Asn
305                 310                 315                 320

Tyr Pro Cys Thr Leu Arg Gln Tyr Leu Cys Val Asn Thr Pro Ser Pro
                325                 330                 335

Arg Leu Ala Ala Met Met Leu Leu Gln Leu Leu Glu Gly Val Asp His
            340                 345                 350

Leu Val Gln Gln Gly Ile Ala His Arg Asp Leu Lys Ser Asp Asn Ile
        355                 360                 365
```

```
Leu Val Glu Leu Asp Pro Asp Gly Cys Pro Trp Leu Val Ile Ala Asp
    370                 375                 380

Phe Gly Cys Cys Leu Ala Asp Glu Ser Ile Gly Leu Gln Leu Pro Phe
385                 390                 395                 400

Ser Ser Trp Tyr Val Asp Arg Gly Gly Asn Gly Cys Leu Met Ala Pro
                405                 410                 415

Glu Val Ser Thr Ala Arg Pro Gly Pro Arg Ala Val Ile Asp Tyr Ser
            420                 425                 430

Lys Ala Asp Ala Trp Ala Val Gly Ala Ile Ala Tyr Glu Ile Phe Gly
        435                 440                 445

Leu Val Asn Pro Phe Tyr Gly Gln Gly Lys Ala His Leu Glu Ser Arg
    450                 455                 460

Ser Tyr Gln Glu Ala Gln Leu Pro Ala Leu Pro Glu Ser Val Pro Pro
465                 470                 475                 480

Asp Val Arg Gln Leu Val Arg Ala Leu Leu Gln Arg Glu Ala Ser Lys
                485                 490                 495

Arg Pro Ser Ala Arg Val Ala Ala Asn Val Leu His Leu Ser Leu Trp
            500                 505                 510

Gly Glu His Ile Leu Ala Leu Lys Asn Leu Lys Leu Asp Lys Met Val
        515                 520                 525

Gly Trp Leu Leu Gln Gln Ser Ala Ala Thr Leu Leu Ala Asn Arg Leu
    530                 535                 540

Thr Glu Lys Cys Cys Val Glu Thr Lys Met Lys Met Leu Phe Leu Ala
545                 550                 555                 560

Asn Leu Glu Cys Glu Thr Leu Cys Gln Ala Ala Leu Leu Leu Cys Ser
                565                 570                 575

Trp Arg Ala Ala Leu
            580
```

<210> 31
<211> 3766
<212> DNA
<213> Homo sapiens

<400> 31

```
tgcggccgca ggttccgctg tctcgggaac cgtcgtatcc ctcggtccgg cggcggcggc      60
ggcggtagcg gaggagacgg tttcaggcct ccggtgcggc tgcaatgctg agctcccggg     120
ccgaggcggc gatgaccgcg gccgacaggg ccatccagcg cttcctgcgg accggggcgg     180
ccgtcagata taaagtcatg aagaactggg gagttatagg tggaattgct gctgctcttg     240
cagcaggaat atatgttatt tggggtccca ttacagaaag aaagaagcgt agaaaagggc     300
ttgtgcctgg ccttgttaat ttagggaaca cctgcttcat gaactccctg ctacaaggcc     360
tgtctgcctg tcctgctttc atcaggtggc tggaagagtt cacctcccag tactccaggg     420
atcagaagga gcccccctca caccagtatt tatccttaac actcttgcac cttctgaaag     480
ccttgtcctg ccaagaagtt actgatgatg aggtcttaga tgcaagctgc ttgttggatg     540
tcttaagaat gtacagatgg cagatctcat catttgaaga acaggatgct cacgaattat     600
tccatgtcat tacctcgtca ttggaagatg agcgagaccg ccagcctcgg gtcacacatt     660
tgtttgatgt gcattccctg gagcagcagt cagaaataac tcccaaacaa attacctgcc     720
gcacaagagg gtcacctcac cctacatcca atcactggaa gtctcaacat ccttttcatg     780
gaagactcac tagtaatatg gtctgcaaac actgtgaaca ccagagtcct gttcgatttg     840
ataccttga tagcctttca ctaagtattc cagccgccac atggggtcac ccattgaccc     900
tggaccactg ccttcaccac ttcatctcat cagaatcagt gcgggatgtt gtgtgtgaca     960
actgtacaaa gattgaagcc aagggaacgt tgaacgggga aaaggtggaa caccagagga    1020
ccacttttgt taaacagtta aaactaggga agctccctca gtgtctctgc atccacctac    1080
agcggctgag ctggtccagc cacggcacgc ctctgaagcg gcatgagcac gtgcagttca    1140
atgagttcct gatgatggac atttacaagt accacctcct tggacataaa cctagtcaac    1200
acaaccctaa actgaacaag aacccagggc ctacactgga gctgcaggat gggccgggag    1260
cccccacacc agttctgaat cagccagggg cccccaaaac acagattttt atgaatggcg    1320
cctgctcccc atctttattg ccaacgctgt cagcgccgat gcccttccct ctcccagttg    1380
ttcccgacta cagctcctcc acatacctct tccggctgat ggcagttgtc gtccaccatg    1440
gagacatgca ctctggacac tttgtcactt accgacggtc cccaccttct gccaggaacc    1500
ctctctcaac tagcaatcag tggctgtggg tctccgatga cactgtccgc aaggccagcc    1560
tgcaggaggt cctgtcctcc agcgcctacc tgctgttcta cgagcgcgtc ctttccagga    1620
tgcagcacca gagccaggag tgcaagtctg aagaatgact gtgccctcct gcaaggctag    1680
agctgatggc actgtctgca ctgtccagga aaaaagtaaa actgtactgt tgcgtgtgca    1740
agcggcccca ctagagcctt ccagccttct ggtgtgttct aagagcaggc tccacctggg    1800
agccagcccc agttcacacc aaaccaggct ccctgaacag tcctgttcat gtgtgtaggt    1860
```

ggttctgttg tgttaagaaa gcattcatta tgtccggagt gtctttttac tcatctgata 1920
caggtaatta aaagaactca gattcttgaa gccaccgttt tcatattgta atgttaggtg 1980
ttctcagagg ggaggtacct ttgtctaatc aacgtttcca cttagatctt ttatttttaa 2040
taagcaggcc cataaaaatt gttgacaaga attaatgaaa ttattaaagg caacaattta 2100
gaagaaaaag tgcctttcac tttcgattgc ttttgtagca cgtccattgt gaaatattcc 2160
ttccaggcta ctcaaaggat agcaagagaa caggtaaatg atgcctaaag aacaccttcc 2220
tttttctatg ccttttctaa tctttcaatt ctttctatgg agtaaaggct catctgccaa 2280
atctgccccc tggggaaact ctttcactac tttgtcagtt ataagtgaag agcttacttg 2340
ttgcttttat cttttgtata ttggactgag atgtaattac actgtattat aaaactctgt 2400
gaatagccag aactgagctg gatctttgca acacctgatt cctctgctct gtggaaaact 2460
ttttcttaca caaggatcca ctgtggacgg ttactttcat ctgtttattt attgcccatg 2520
cagagctctt aaggtttaca ggtgggagct tggggctgta taaaaaaata atccctgccc 2580
tgagttgaca cctggcttag gaaggaaggg ctgactatgg ggctgcagtc tctctgaacc 2640
tcagtttcct catttgtgaa gtgaagggtt agatttgatg accaccaaag ttcagcccttt 2700
ttcacgaaaa ggagaaagca gcttttgact ttttaaaaaa catataacta cagctggcat 2760
ctagtattgt catgttgctc taggtccata ttctgaattt attcatttcc aatagcctaa 2820
tacaaaaagt atatattgag cactttcttc ccttttcagg taagtctctg aatgcagccc 2880
agggccaaag gaattttgat gacacagtag tacctatgtt ttaagctata tttttaattt 2940
agaaaaatgg ataccaaatt caaaccgact catcagaggt aagatttgga atcagacctt 3000
tccaaaaggt catctgaggt aaggctaaga ccgcacttcc tctgctgggg gtgagctggc 3060
agacacacca aacagtgcct tggcagcagc tcacagtgca ggaagcccag gtgatcactc 3120
ttctgctggg cccaggctgc accctgagga ctcagtaact cactctcaac agaatattct 3180
gtgcaggctc tccaggctct gggcgtcagg gtgcaagggg cagcttgaac tgtacggtcc 3240
gtcctgcact cacccgatgc agaccttgac tttgatgttg aaatgaacac acttgtttta 3300
cccaagtctg gtggaacaaa tgcccaatca tgtgacctta aagtgtactg caaagctgta 3360
gctttaagta attgctgttc tgccactgct tactctgaaa tctaccatca aagaaagata 3420
gagaaaaggg gctgagcctt ggaatatatg gttataagca gatctttctt tggtcagaga 3480
ccagggtttg agccaaggct gtaaatgtga acaatagctg tgcaaagcct tttaacctga 3540
cttcttcatt ttgtaaatta ttatgcatta agtagcagcc caataatctg atttctagtt 3600
ttattttcaa agtaagtagc ttcttttggg aaaaacctaa gttaaactag tagttttgcc 3660
ataataactg ctgatttatg tatttgctaa aggtactttt gtatctgctg tgtattatag 3720
caataaaata atcattttgt tagaaaaaaa tcaaaaaaaa aaaaaa 3766

<210> 32
<211> 352
<212> PRT
<213> Homo sapiens

<400> 32

```
Met Glu Ser Gly Gly Arg Pro Ser Leu Cys Gln Phe Ile Leu Leu Gly
1               5                   10                  15

Thr Thr Ser Val Val Thr Ala Ala Leu Tyr Ser Val Tyr Arg Gln Lys
            20                  25                  30

Ala Arg Val Ser Gln Glu Leu Lys Gly Ala Lys Lys Val His Leu Gly
        35                  40                  45

Glu Asp Leu Lys Ser Ile Leu Ser Glu Ala Pro Gly Lys Cys Val Pro
    50                  55                  60

Tyr Ala Val Ile Glu Gly Ala Val Arg Ser Val Lys Glu Thr Leu Asn
65                  70                  75                  80

Ser Gln Phe Val Glu Asn Cys Lys Gly Val Ile Gln Arg Leu Thr Leu
                85                  90                  95

Gln Glu His Lys Met Val Trp Asn Arg Thr Thr His Leu Trp Asn Asp
            100                 105                 110

Cys Ser Lys Ile Ile His Gln Arg Thr Asn Thr Val Pro Phe Asp Leu
        115                 120                 125

Val Pro His Glu Asp Gly Val Asp Val Ala Val Arg Val Leu Lys Pro
    130                 135                 140

Leu Asp Ser Val Asp Leu Gly Leu Glu Thr Val Tyr Glu Lys Phe His
145                 150                 155                 160

Pro Ser Ile Gln Ser Phe Thr Asp Val Ile Gly His Tyr Ile Ser Gly
                165                 170                 175

Glu Arg Pro Lys Gly Ile Gln Glu Thr Glu Glu Met Leu Lys Val Gly
            180                 185                 190

Ala Thr Leu Thr Gly Val Gly Glu Leu Val Leu Asp Asn Asn Ser Val
        195                 200                 205

Arg Leu Gln Pro Pro Lys Gln Gly Met Gln Tyr Tyr Leu Ser Ser Gln
    210                 215                 220
```

Asp Phe Asp Ser Leu Leu Gln Arg Gln Glu Ser Ser Val Arg Leu Trp
225 230 235 240

Lys Val Leu Ala Leu Val Phe Gly Phe Ala Thr Cys Ala Thr Leu Phe
245 250 255

Phe Ile Leu Arg Lys Gln Tyr Leu Gln Arg Gln Glu Arg Leu Arg Leu
260 265 270

Lys Gln Met Gln Glu Glu Phe Gln Glu His Glu Ala Gln Leu Leu Ser
275 280 285

Arg Ala Lys Pro Glu Asp Arg Glu Ser Leu Lys Ser Ala Cys Val Val
290 295 300

Cys Leu Ser Ser Phe Lys Ser Cys Val Phe Leu Glu Cys Gly His Val
305 310 315 320

Cys Ser Cys Thr Glu Cys Tyr Arg Ala Leu Pro Glu Pro Lys Lys Cys
325 330 335

Pro Ile Cys Arg Gln Ala Ile Thr Arg Val Ile Pro Leu Tyr Asn Ser
340 345 350

<210> 33
<211> 561
<212> PRT
<213> Homo sapiens

<400> 33

Met Cys Gly Ile Trp Ala Leu Phe Gly Ser Asp Asp Cys Leu Ser Val
1 5 10 15

Gln Cys Leu Ser Ala Met Lys Ile Ala His Arg Gly Pro Asp Ala Phe
20 25 30

Arg Phe Glu Asn Val Asn Gly Tyr Thr Asn Cys Cys Phe Gly Phe His
35 40 45

Arg Leu Ala Val Val Asp Pro Leu Phe Gly Met Gln Pro Ile Arg Val
50 55 60

Lys Lys Tyr Pro Tyr Leu Trp Leu Cys Tyr Asn Gly Glu Ile Tyr Asn
65 70 75 80

His Lys Lys Met Gln Gln His Phe Glu Phe Glu Tyr Gln Thr Lys Val
85 90 95

```
Asp Gly Glu Ile Ile Leu His Leu Tyr Asp Lys Gly Gly Ile Glu Gln
            100                 105                 110

Thr Ile Cys Met Leu Asp Gly Val Phe Ala Phe Val Leu Leu Asp Thr
        115                 120                 125

Ala Asn Lys Lys Val Phe Leu Gly Arg Asp Thr Tyr Gly Val Arg Pro
    130                 135                 140

Leu Phe Lys Ala Met Thr Glu Asp Gly Phe Leu Ala Val Cys Ser Glu
145                 150                 155                 160

Ala Lys Gly Leu Val Thr Leu Lys His Ser Ala Thr Pro Phe Leu Lys
                165                 170                 175

Val Glu Pro Phe Leu Pro Gly His Tyr Glu Val Leu Asp Leu Lys Pro
            180                 185                 190

Asn Gly Lys Val Ala Ser Val Glu Met Val Lys Tyr His His Cys Arg
        195                 200                 205

Asp Val Pro Leu His Ala Leu Tyr Asp Asn Val Glu Lys Leu Phe Pro
    210                 215                 220

Gly Phe Glu Ile Glu Thr Val Lys Asn Asn Leu Arg Ile Leu Phe Asn
225                 230                 235                 240

Asn Ala Val Lys Lys Arg Leu Met Thr Asp Arg Arg Ile Gly Cys Leu
                245                 250                 255

Leu Ser Gly Gly Leu Asp Ser Ser Leu Val Ala Ala Thr Leu Leu Lys
            260                 265                 270

Gln Leu Lys Glu Ala Gln Val Gln Tyr Pro Leu Gln Thr Phe Ala Ile
        275                 280                 285

Gly Met Glu Asp Ser Pro Asp Leu Leu Ala Ala Arg Lys Val Ala Asp
    290                 295                 300

His Ile Gly Ser Glu His Tyr Glu Val Leu Phe Asn Ser Glu Glu Gly
305                 310                 315                 320

Ile Gln Ala Leu Asp Glu Val Ile Phe Ser Leu Glu Thr Tyr Asp Ile
                325                 330                 335

Thr Thr Val Arg Ala Ser Val Gly Met Tyr Leu Ile Ser Lys Tyr Ile
```

```
            340                 345                 350

Arg Lys Asn Thr Asp Ser Val Val Ile Phe Ser Gly Glu Gly Ser Asp
        355                 360                 365

Glu Leu Thr Gln Gly Tyr Ile Tyr Phe His Lys Ala Pro Ser Pro Glu
    370                 375                 380

Lys Ala Glu Glu Glu Ser Glu Arg Leu Leu Arg Glu Leu Tyr Leu Phe
385                 390                 395                 400

Asp Val Leu Arg Ala Asp Arg Thr Thr Ala Ala His Gly Leu Glu Leu
                405                 410                 415

Arg Val Pro Phe Leu Asp His Arg Phe Ser Ser Tyr Tyr Leu Ser Leu
            420                 425                 430

Pro Pro Glu Met Arg Ile Pro Lys Asn Gly Ile Glu Lys His Leu Leu
        435                 440                 445

Arg Glu Thr Phe Glu Asp Ser Asn Leu Ile Pro Lys Glu Ile Leu Trp
    450                 455                 460

Arg Pro Lys Glu Ala Phe Ser Asp Gly Ile Thr Ser Val Lys Asn Ser
465                 470                 475                 480

Trp Phe Lys Ile Leu Gln Glu Tyr Val Glu His Gln Val Asp Asp Ala
                485                 490                 495

Met Met Ala Asn Ala Ala Gln Lys Phe Pro Phe Asn Thr Pro Lys Thr
            500                 505                 510

Lys Glu Gly Tyr Tyr Tyr Arg Gln Val Phe Glu Arg His Tyr Pro Gly
        515                 520                 525

Arg Ala Asp Trp Leu Ser His Tyr Trp Met Pro Lys Trp Ile Asn Ala
    530                 535                 540

Thr Asp Pro Ser Ala Arg Thr Leu Thr His Tyr Lys Ser Ala Val Lys
545                 550                 555                 560

Ala
```

<210> 34
<211> 412
<212> PRT
<213> Homo sapiens

<400> 34

```
Met Ala Ser Cys Ala Glu Pro Ser Glu Pro Ser Ala Pro Leu Pro Ala
1               5                   10                  15

Gly Val Pro Pro Leu Glu Asp Phe Glu Val Leu Asp Gly Val Glu Asp
            20                  25                  30

Ala Glu Gly Glu Glu Glu Glu Glu Glu Glu Glu Glu Glu Glu Asp Asp
        35                  40                  45

Leu Ser Glu Leu Pro Pro Leu Glu Asp Met Gly Gln Pro Pro Ala Glu
    50                  55                  60

Glu Ala Glu Gln Pro Gly Ala Leu Ala Arg Glu Phe Leu Ala Ala Met
65                  70                  75                  80

Glu Pro Glu Pro Ala Pro Ala Pro Ala Pro Glu Glu Trp Leu Asp Ile
                85                  90                  95

Leu Gly Asn Gly Leu Leu Arg Lys Lys Thr Leu Val Pro Gly Pro Pro
            100                 105                 110

Gly Ser Ser Arg Pro Val Lys Gly Gln Val Val Thr Val His Leu Gln
        115                 120                 125

Thr Ser Leu Glu Asn Gly Thr Arg Val Gln Glu Glu Pro Glu Leu Val
    130                 135                 140

Phe Thr Leu Gly Asp Cys Asp Val Ile Gln Ala Leu Asp Leu Ser Val
145                 150                 155                 160

Pro Leu Met Asp Val Gly Glu Thr Ala Met Val Thr Ala Asp Ser Lys
                165                 170                 175

Tyr Cys Tyr Gly Pro Gln Gly Arg Ser Pro Tyr Ile Pro Pro His Ala
            180                 185                 190

Ala Leu Cys Leu Glu Val Thr Leu Lys Thr Ala Val Asp Gly Pro Asp
        195                 200                 205

Leu Glu Met Leu Thr Gly Gln Glu Arg Val Ala Leu Ala Asn Arg Lys
    210                 215                 220

Arg Glu Cys Gly Asn Ala His Tyr Gln Arg Ala Asp Phe Val Leu Ala
225                 230                 235                 240
```

Ala Asn Ser Tyr Asp Leu Ala Ile Lys Ala Ile Thr Ser Ser Ala Lys
245 250 255

Val Asp Met Thr Phe Glu Glu Glu Ala Gln Leu Leu Gln Leu Lys Val
260 265 270

Lys Cys Leu Asn Asn Leu Ala Ala Ser Gln Leu Lys Leu Asp His Tyr
275 280 285

Arg Ala Ala Leu Arg Ser Cys Ser Leu Val Leu Glu His Gln Pro Asp
290 295 300

Asn Ile Lys Ala Leu Phe Arg Lys Gly Lys Val Leu Ala Gln Gln Gly
305 310 315 320

Glu Tyr Ser Glu Ala Ile Pro Ile Leu Arg Ala Ala Leu Lys Leu Glu
325 330 335

Pro Ser Asn Lys Thr Ile His Ala Glu Leu Ser Lys Leu Val Lys Lys
340 345 350

His Ala Ala Gln Arg Ser Thr Glu Thr Ala Leu Tyr Arg Lys Met Leu
355 360 365

Gly Asn Pro Ser Arg Leu Pro Ala Lys Cys Pro Gly Lys Gly Ala Trp
370 375 380

Ser Ile Pro Trp Lys Trp Leu Phe Gly Ala Thr Ala Val Ala Leu Gly
385 390 395 400

Gly Val Ala Leu Ser Val Val Ile Ala Ala Arg Asn
405 410

<210> 35
<211> 608
<212> PRT
<213> Homo sapiens

<400> 35

Met Ala Ala Ser Lys Pro Val Glu Ala Ala Val Val Ala Ala Ala Val
1 5 10 15

Pro Ser Ser Gly Ser Gly Val Gly Gly Gly Gly Thr Ala Gly Pro Gly
20 25 30

Thr Gly Gly Leu Pro Arg Trp Gln Leu Ala Leu Ala Val Gly Ala Pro
35 40 45

Leu Leu Leu Gly Ala Gly Ala Ile Tyr Leu Trp Ser Arg Gln Gln Arg
    50                  55                  60

Arg Arg Glu Ala Arg Gly Arg Gly Asp Ala Ser Gly Leu Lys Arg Asn
65                  70                  75                  80

Ser Glu Arg Lys Thr Pro Glu Gly Arg Ala Ser Pro Ala Pro Gly Ser
                85                  90                  95

Gly His Pro Glu Gly Pro Gly Ala His Leu Asp Met Asn Ser Leu Asp
            100                 105                 110

Arg Ala Gln Ala Ala Lys Asn Lys Gly Asn Lys Tyr Phe Lys Ala Gly
        115                 120                 125

Lys Tyr Glu Gln Ala Ile Gln Cys Tyr Thr Glu Ala Ile Ser Leu Cys
    130                 135                 140

Pro Thr Glu Lys Asn Val Asp Leu Ser Thr Phe Tyr Gln Asn Arg Ala
145                 150                 155                 160

Ala Ala Phe Glu Gln Leu Gln Lys Trp Lys Glu Val Ala Gln Asp Cys
                165                 170                 175

Thr Lys Ala Val Glu Leu Asn Pro Lys Tyr Val Lys Ala Leu Phe Arg
            180                 185                 190

Arg Ala Lys Ala His Glu Lys Leu Asp Asn Lys Lys Glu Cys Leu Glu
        195                 200                 205

Asp Val Thr Ala Val Cys Ile Leu Glu Gly Phe Gln Asn Gln Gln Ser
    210                 215                 220

Met Leu Leu Ala Asp Lys Val Leu Lys Leu Leu Gly Lys Glu Lys Ala
225                 230                 235                 240

Lys Glu Lys Tyr Lys Asn Arg Glu Pro Leu Met Pro Ser Pro Gln Phe
                245                 250                 255

Ile Lys Ser Tyr Phe Ser Ser Phe Thr Asp Asp Ile Ile Ser Gln Pro
            260                 265                 270

Met Leu Lys Gly Glu Lys Ser Asp Glu Asp Lys Asp Lys Glu Gly Glu
        275                 280                 285

Ala Leu Glu Val Lys Glu Asn Ser Gly Tyr Leu Lys Ala Lys Gln Tyr
    290                 295                 300

Met Glu Glu Glu Asn Tyr Asp Lys Ile Ile Ser Glu Cys Ser Lys Glu
305 310 315 320

Ile Asp Ala Glu Gly Lys Tyr Met Ala Glu Ala Leu Leu Leu Arg Ala
325 330 335

Thr Phe Tyr Leu Leu Ile Gly Asn Ala Asn Ala Ala Lys Pro Asp Leu
340 345 350

Asp Lys Val Ile Ser Leu Lys Glu Ala Asn Val Lys Leu Arg Ala Asn
355 360 365

Ala Leu Ile Lys Arg Gly Ser Met Tyr Met Gln Gln Gln Gln Pro Leu
370 375 380

Leu Ser Thr Gln Asp Phe Asn Met Ala Ala Asp Ile Asp Pro Gln Asn
385 390 395 400

Ala Asp Val Tyr His His Arg Gly Gln Leu Lys Ile Leu Leu Asp Gln
405 410 415

Val Glu Glu Ala Val Ala Asp Phe Asp Glu Cys Ile Arg Leu Arg Pro
420 425 430

Glu Ser Ala Leu Ala Gln Ala Gln Lys Cys Phe Ala Leu Tyr Arg Gln
435 440 445

Ala Tyr Thr Gly Asn Asn Ser Ser Gln Ile Gln Ala Ala Met Lys Gly
450 455 460

Phe Glu Glu Val Ile Lys Lys Phe Pro Arg Cys Ala Glu Gly Tyr Ala
465 470 475 480

Leu Tyr Ala Gln Ala Leu Thr Asp Gln Gln Gln Phe Gly Lys Ala Asp
485 490 495

Glu Met Tyr Asp Lys Cys Ile Asp Leu Glu Pro Asp Asn Ala Thr Thr
500 505 510

Tyr Val His Lys Gly Leu Leu Gln Leu Gln Trp Lys Gln Asp Leu Asp
515 520 525

Arg Gly Leu Glu Leu Ile Ser Lys Ala Ile Glu Ile Asp Asn Lys Cys
530 535 540

Asp Phe Ala Tyr Glu Thr Met Gly Thr Ile Glu Val Gln Arg Gly Asn
545 550 555 560

Met Glu Lys Ala Ile Asp Met Phe Asn Lys Ala Ile Asn Leu Ala Lys
565 570 575

Ser Glu Met Glu Met Ala His Leu Tyr Ser Leu Cys Asp Ala Ala His
580 585 590

Ala Gln Thr Glu Val Ala Lys Lys Tyr Gly Leu Lys Pro Pro Thr Leu
595 600 605

<210> 36
<211> 334
<212> PRT
<213> Homo sapiens

<400> 36

Met Val Gly Arg Glu Lys Glu Leu Ser Ile His Phe Val Pro Gly Ser
1 5 10 15

Cys Arg Leu Val Glu Glu Glu Val Asn Ile Pro Asn Arg Arg Val Leu
20 25 30

Val Thr Gly Ala Thr Gly Leu Leu Gly Arg Ala Val His Lys Glu Phe
35 40 45

Gln Gln Asn Asn Trp His Ala Val Gly Cys Gly Phe Arg Arg Ala Arg
50 55 60

Pro Lys Phe Glu Gln Val Asn Leu Leu Asp Ser Asn Ala Val His His
65 70 75 80

Ile Ile His Asp Phe Gln Pro His Val Ile Val His Cys Ala Ala Glu
85 90 95

Arg Arg Pro Asp Val Val Glu Asn Gln Pro Asp Ala Ala Ser Gln Leu
100 105 110

Asn Val Asp Ala Ser Gly Asn Leu Ala Lys Glu Ala Ala Ala Val Gly
115 120 125

Ala Phe Leu Ile Tyr Ile Ser Ser Asp Tyr Val Phe Asp Gly Thr Asn
130 135 140

Pro Pro Tyr Arg Glu Glu Asp Ile Pro Ala Pro Leu Asn Leu Tyr Gly
145 150 155 160

Lys Thr Lys Leu Asp Gly Glu Lys Ala Val Leu Glu Asn Asn Leu Gly
165 170 175

Ala Ala Val Leu Arg Ile Pro Ile Leu Tyr Gly Glu Val Glu Lys Leu
180 185 190

Glu Glu Ser Ala Val Thr Val Met Phe Asp Lys Val Gln Phe Ser Asn
195 200 205

Lys Ser Ala Asn Met Asp His Trp Gln Gln Arg Phe Pro Thr His Val
210 215 220

Lys Asp Val Ala Thr Val Cys Arg Gln Leu Ala Glu Lys Arg Met Leu
225 230 235 240

Asp Pro Ser Ile Lys Gly Thr Phe His Trp Ser Gly Asn Glu Gln Met
245 250 255

Thr Lys Tyr Glu Met Ala Cys Ala Ile Ala Asp Ala Phe Asn Leu Pro
260 265 270

Ser Ser His Leu Arg Pro Ile Thr Asp Ser Pro Val Leu Gly Ala Gln
275 280 285

Arg Pro Arg Asn Ala Gln Leu Asp Cys Ser Lys Leu Glu Thr Leu Gly
290 295 300

Ile Gly Gln Arg Thr Pro Phe Arg Ile Gly Ile Lys Glu Ser Leu Trp
305 310 315 320

Pro Phe Leu Ile Asp Lys Arg Trp Arg Gln Thr Val Phe His
325 330

<210> 37
<211> 256
<212> PRT
<213> Homo sapiens

<400> 37

Met Ala Ala Ala Val Gly Arg Leu Leu Arg Ala Ser Val Ala Arg His
1 5 10 15

Val Ser Ala Ile Pro Trp Gly Ile Ser Ala Thr Ala Ala Leu Arg Pro
20 25 30

Ala Ala Cys Gly Arg Thr Ser Leu Thr Asn Leu Leu Cys Ser Gly Ser
35 40 45

Ser Gln Ala Lys Leu Phe Ser Thr Ser Ser Ser Cys His Ala Pro Ala
50 55 60

Val Thr Gln His Ala Pro Tyr Phe Lys Gly Thr Ala Val Val Asn Gly
65 70 75 80

Glu Phe Lys Asp Leu Ser Leu Asp Asp Phe Lys Gly Lys Tyr Leu Val
85 90 95

Leu Phe Phe Tyr Pro Leu Asp Phe Thr Phe Val Cys Pro Thr Glu Ile
100 105 110

Val Ala Phe Ser Asp Lys Ala Asn Glu Phe His Asp Val Asn Cys Glu
115 120 125

Val Val Ala Val Ser Val Asp Ser His Phe Ser His Leu Ala Trp Ile
130 135 140

Asn Thr Pro Arg Lys Asn Gly Gly Leu Gly His Met Asn Ile Ala Leu
145 150 155 160

Leu Ser Asp Leu Thr Lys Gln Ile Ser Arg Asp Tyr Gly Val Leu Leu
165 170 175

Glu Gly Ser Gly Leu Ala Leu Arg Gly Leu Phe Ile Ile Asp Pro Asn
180 185 190

Gly Val Ile Lys His Leu Ser Val Asn Asp Leu Pro Val Gly Arg Ser
195 200 205

Val Glu Glu Thr Leu Arg Leu Val Lys Ala Phe Gln Tyr Val Glu Thr
210 215 220

His Gly Glu Val Cys Pro Ala Asn Trp Thr Pro Asp Ser Pro Thr Ile
225 230 235 240

Lys Pro Ser Pro Ala Ala Ser Lys Glu Tyr Phe Gln Lys Val Asn Gln
245 250 255

<210> 38
<211> 1019
<212> PRT
<213> Homo sapiens

<400> 38

Met Arg Tyr Arg Leu Ala Trp Leu Leu His Pro Ala Leu Pro Ser Thr
1 5 10 15

Phe Arg Ser Val Leu Gly Ala Arg Leu Pro Pro Pro Glu Arg Leu Cys
20 25 30

```
Gly Phe Gln Lys Lys Thr Tyr Ser Lys Met Asn Asn Pro Ala Ile Lys
        35                  40                  45

Arg Ile Gly Asn His Ile Thr Lys Ser Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Ala Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Glu
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Gln Glu
225                 230                 235                 240

Leu Leu Lys Phe His Ser Ala Tyr Tyr Ser Ser Asn Leu Met Ala Val
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
```

275 280 285

Pro Glu His Pro Phe Gln Glu Glu His Leu Lys Gln Leu Tyr Lys Ile
290 295 300

Val Pro Ile Lys Asp Ile Arg Asn Leu Tyr Val Thr Phe Pro Ile Pro
305 310 315 320

Asp Leu Gln Lys Tyr Tyr Lys Ser Asn Pro Gly His Tyr Leu Gly His
325 330 335

Leu Ile Gly His Glu Gly Pro Gly Ser Leu Leu Ser Glu Leu Lys Ser
340 345 350

Lys Gly Trp Val Asn Thr Leu Val Gly Gly Gln Lys Glu Gly Ala Arg
355 360 365

Gly Phe Met Phe Phe Ile Ile Asn Val Asp Leu Thr Glu Glu Gly Leu
370 375 380

Leu His Val Glu Asp Ile Ile Leu His Met Phe Gln Tyr Ile Gln Lys
385 390 395 400

Leu Arg Ala Glu Gly Pro Gln Glu Trp Val Phe Gln Glu Cys Lys Asp
405 410 415

Leu Asn Ala Val Ala Phe Arg Phe Lys Asp Lys Glu Arg Pro Arg Gly
420 425 430

Tyr Thr Ser Lys Ile Ala Gly Ile Leu His Tyr Tyr Pro Leu Glu Glu
435 440 445

Val Leu Thr Ala Glu Tyr Leu Leu Glu Glu Phe Arg Pro Asp Leu Ile
450 455 460

Glu Met Val Leu Asp Lys Leu Arg Pro Glu Asn Val Arg Val Ala Ile
465 470 475 480

Val Ser Lys Ser Phe Glu Gly Lys Thr Asp Arg Thr Glu Glu Trp Tyr
485 490 495

Gly Thr Gln Tyr Lys Gln Glu Ala Ile Pro Asp Glu Val Ile Lys Lys
500 505 510

Trp Gln Asn Ala Asp Leu Asn Gly Lys Phe Lys Leu Pro Thr Lys Asn
515 520 525

Glu Phe Ile Pro Thr Asn Phe Glu Ile Leu Pro Leu Glu Lys Glu Ala
530 535 540

Thr Pro Tyr Pro Ala Leu Ile Lys Asp Thr Ala Met Ser Lys Leu Trp
545 550 555 560

Phe Lys Gln Asp Asp Lys Phe Phe Leu Pro Lys Ala Cys Leu Asn Phe
565 570 575

Glu Phe Phe Ser Pro Phe Ala Tyr Val Asp Pro Leu His Cys Asn Met
580 585 590

Ala Tyr Leu Tyr Leu Glu Leu Leu Lys Asp Ser Leu Asn Glu Tyr Ala
595 600 605

Tyr Ala Ala Glu Leu Ala Gly Leu Ser Tyr Asp Leu Gln Asn Thr Ile
610 615 620

Tyr Gly Met Tyr Leu Ser Val Lys Gly Tyr Asn Asp Lys Gln Pro Ile
625 630 635 640

Leu Leu Lys Lys Ile Ile Glu Lys Met Ala Thr Phe Glu Ile Asp Glu
645 650 655

Lys Arg Phe Glu Ile Ile Lys Glu Ala Tyr Met Arg Ser Leu Asn Asn
660 665 670

Phe Arg Ala Glu Gln Pro His Gln His Ala Met Tyr Tyr Leu Arg Leu
675 680 685

Leu Met Thr Glu Val Ala Trp Thr Lys Asp Glu Leu Lys Glu Ala Leu
690 695 700

Asp Asp Val Thr Leu Pro Arg Leu Lys Ala Phe Ile Pro Gln Leu Leu
705 710 715 720

Ser Arg Leu His Ile Glu Ala Leu Leu His Gly Asn Ile Thr Lys Gln
725 730 735

Ala Ala Leu Gly Ile Met Gln Met Val Glu Asp Thr Leu Ile Glu His
740 745 750

Ala His Thr Lys Pro Leu Leu Pro Ser Gln Leu Val Arg Tyr Arg Glu
755 760 765

Val Gln Leu Pro Asp Arg Gly Trp Phe Val Tyr Gln Gln Arg Asn Glu
770 775 780

```
Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ser Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Phe Asp Arg Asp Asn Thr Glu Val Ala Tyr Leu
        915                 920                 925

Lys Thr Leu Thr Lys Glu Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Cys Gln Asn
                965                 970                 975

Asp Ile Asn Leu Ser Gln Ala Pro Ala Leu Pro Gln Pro Glu Val Ile
            980                 985                 990

Gln Asn Met Thr Glu Phe Lys Arg  Gly Leu Pro Leu Phe  Pro Leu Val
        995                 1000                1005

Lys Pro  His Ile Asn Phe Met  Ala Ala Lys Leu
    1010                1015
```

<210> 39
<211> 283

<212> PRT
<213> Homo sapiens

<400> 39

```
Met Ala Val Pro Pro Thr Tyr Ala Asp Leu Gly Lys Ser Ala Arg Asp
1               5                   10                  15

Val Phe Thr Lys Gly Tyr Gly Phe Gly Leu Ile Lys Leu Asp Leu Lys
            20                  25                  30

Thr Lys Ser Glu Asn Gly Leu Glu Phe Thr Ser Ser Gly Ser Ala Asn
        35                  40                  45

Thr Glu Thr Thr Lys Val Thr Gly Ser Leu Glu Thr Lys Tyr Arg Trp
    50                  55                  60

Thr Glu Tyr Gly Leu Thr Phe Thr Glu Lys Trp Asn Thr Asp Asn Thr
65                  70                  75                  80

Leu Gly Thr Glu Ile Thr Val Glu Asp Gln Leu Ala Arg Gly Leu Lys
                85                  90                  95

Leu Thr Phe Asp Ser Ser Phe Ser Pro Asn Thr Gly Lys Lys Asn Ala
            100                 105                 110

Lys Ile Lys Thr Gly Tyr Lys Arg Glu His Ile Asn Leu Gly Cys Asp
        115                 120                 125

Met Asp Phe Asp Ile Ala Gly Pro Ser Ile Arg Gly Ala Leu Val Leu
    130                 135                 140

Gly Tyr Glu Gly Trp Leu Ala Gly Tyr Gln Met Asn Phe Glu Thr Ala
145                 150                 155                 160

Lys Ser Arg Val Thr Gln Ser Asn Phe Ala Val Gly Tyr Lys Thr Asp
                165                 170                 175

Glu Phe Gln Leu His Thr Asn Val Asn Asp Gly Thr Glu Phe Gly Gly
            180                 185                 190

Ser Ile Tyr Gln Lys Val Asn Lys Lys Leu Glu Thr Ala Val Asn Leu
        195                 200                 205

Ala Trp Thr Ala Gly Asn Ser Asn Thr Arg Phe Gly Ile Ala Ala Lys
    210                 215                 220

Tyr Gln Ile Asp Pro Asp Ala Cys Phe Ser Ala Lys Val Asn Asn Ser
225                 230                 235                 240
```

Ser Leu Ile Gly Leu Gly Tyr Thr Gln Thr Leu Lys Pro Gly Ile Lys
245 250 255

Leu Thr Leu Ser Ala Leu Leu Asp Gly Lys Asn Val Asn Ala Gly Gly
260 265 270

His Lys Leu Gly Leu Gly Leu Glu Phe Gln Ala
275 280

<210> 40
<211> 1097
<212> PRT
<213> Homo sapiens

<400> 40

Met Ala Ala Ala Ala Ala Glu Gln Gln Gln Phe Tyr Leu Leu Leu Gly
1 5 10 15

Asn Leu Leu Ser Pro Asp Asn Val Val Arg Lys Gln Ala Glu Glu Thr
20 25 30

Tyr Glu Asn Ile Pro Gly Gln Ser Lys Ile Thr Phe Leu Leu Gln Ala
35 40 45

Ile Arg Asn Thr Thr Ala Ala Glu Glu Ala Arg Gln Met Ala Ala Val
50 55 60

Leu Leu Arg Arg Leu Leu Ser Ser Ala Phe Asp Glu Val Tyr Pro Ala
65 70 75 80

Leu Pro Ser Asp Val Gln Thr Ala Ile Lys Ser Glu Leu Leu Met Ile
85 90 95

Ile Gln Met Glu Thr Gln Ser Ser Met Arg Lys Lys Val Cys Asp Ile
100 105 110

Ala Ala Glu Leu Ala Arg Asn Leu Ile Asp Glu Asp Gly Asn Asn Gln
115 120 125

Trp Pro Glu Gly Leu Lys Phe Leu Phe Asp Ser Val Ser Ser Gln Asn
130 135 140

Val Gly Leu Arg Glu Ala Ala Leu His Ile Phe Trp Asn Phe Pro Gly
145 150 155 160

Ile Phe Gly Asn Gln Gln Gln His Tyr Leu Asp Val Ile Lys Arg Met
165 170 175

```
Leu Val Gln Cys Met Gln Asp Gln Glu His Pro Ser Ile Arg Thr Leu
            180                 185                 190

Ser Ala Arg Ala Thr Ala Ala Phe Ile Leu Ala Asn Glu His Asn Val
        195                 200                 205

Ala Leu Phe Lys His Phe Ala Asp Leu Leu Pro Gly Phe Leu Gln Ala
    210                 215                 220

Val Asn Asp Ser Cys Tyr Gln Asn Asp Asp Ser Val Leu Lys Ser Leu
225                 230                 235                 240

Val Glu Ile Ala Asp Thr Val Pro Lys Tyr Leu Arg Pro His Leu Glu
                245                 250                 255

Ala Thr Leu Gln Leu Ser Leu Lys Leu Cys Gly Asp Thr Ser Leu Asn
            260                 265                 270

Asn Met Gln Arg Gln Leu Ala Leu Glu Val Ile Val Thr Leu Ser Glu
        275                 280                 285

Thr Ala Ala Ala Met Leu Arg Lys His Thr Asn Ile Val Ala Gln Thr
    290                 295                 300

Ile Pro Gln Met Leu Ala Met Met Val Asp Leu Glu Glu Asp Glu Asp
305                 310                 315                 320

Trp Ala Asn Ala Asp Glu Leu Glu Asp Asp Asp Phe Asp Ser Asn Ala
                325                 330                 335

Val Ala Gly Glu Ser Ala Leu Asp Arg Met Ala Cys Gly Leu Gly Gly
            340                 345                 350

Lys Leu Val Leu Pro Met Ile Lys Glu His Ile Met Gln Met Leu Gln
        355                 360                 365

Asn Pro Asp Trp Lys Tyr Arg His Ala Gly Leu Met Ala Leu Ser Ala
    370                 375                 380

Ile Gly Glu Gly Cys His Gln Gln Met Glu Gly Ile Leu Asn Glu Ile
385                 390                 395                 400

Val Asn Phe Val Leu Leu Phe Leu Gln Asp Pro His Pro Arg Val Arg
                405                 410                 415

Tyr Ala Ala Cys Asn Ala Val Gly Gln Met Ala Thr Asp Phe Ala Pro
```

420 425 430

Gly Phe Gln Lys Lys Phe His Glu Lys Val Ile Ala Ala Leu Leu Gln
435 440 445

Thr Met Glu Asp Gln Gly Asn Gln Arg Val Gln Ala His Ala Ala Ala
450 455 460

Ala Leu Ile Asn Phe Thr Glu Asp Cys Pro Lys Ser Leu Leu Ile Pro
465 470 475 480

Tyr Leu Asp Asn Leu Val Lys His Leu His Ser Ile Met Val Leu Lys
485 490 495

Leu Gln Glu Leu Ile Gln Lys Gly Thr Lys Leu Val Leu Glu Gln Val
500 505 510

Val Thr Ser Ile Ala Ser Val Ala Asp Thr Ala Glu Glu Lys Phe Val
515 520 525

Pro Tyr Tyr Asp Leu Phe Met Pro Ser Leu Lys His Ile Val Glu Asn
530 535 540

Ala Val Gln Lys Glu Leu Arg Leu Leu Arg Gly Lys Thr Ile Glu Cys
545 550 555 560

Ile Ser Leu Ile Gly Leu Ala Val Gly Lys Glu Lys Phe Met Gln Asp
565 570 575

Ala Ser Asp Val Met Gln Leu Leu Leu Lys Thr Gln Thr Asp Phe Asn
580 585 590

Asp Met Glu Asp Asp Asp Pro Gln Ile Ser Tyr Met Ile Ser Ala Trp
595 600 605

Ala Arg Met Cys Lys Ile Leu Gly Lys Glu Phe Gln Gln Tyr Leu Pro
610 615 620

Val Val Met Gly Pro Leu Met Lys Thr Ala Ser Ile Lys Pro Glu Val
625 630 635 640

Ala Leu Leu Asp Thr Gln Asp Met Glu Asn Met Ser Asp Asp Asp Gly
645 650 655

Trp Glu Phe Val Asn Leu Gly Asp Gln Gln Ser Phe Gly Ile Lys Thr
660 665 670

```
Ala Gly Leu Glu Glu Lys Ser Thr Ala Cys Gln Met Leu Val Cys Tyr
        675                 680                 685

Ala Lys Glu Leu Lys Glu Gly Phe Val Glu Tyr Thr Glu Gln Val Val
    690                 695                 700

Lys Leu Met Val Pro Leu Leu Lys Phe Tyr Phe His Asp Gly Val Arg
705                 710                 715                 720

Val Ala Ala Ala Glu Ser Met Pro Leu Leu Leu Glu Cys Ala Arg Val
                725                 730                 735

Arg Gly Pro Glu Tyr Leu Thr Gln Met Trp His Phe Met Cys Asp Ala
            740                 745                 750

Leu Ile Lys Ala Ile Gly Thr Glu Pro Asp Ser Asp Val Leu Ser Glu
        755                 760                 765

Ile Met His Ser Phe Ala Lys Cys Ile Glu Val Met Gly Asp Gly Cys
    770                 775                 780

Leu Asn Asn Glu His Phe Glu Glu Leu Gly Gly Ile Leu Lys Ala Lys
785                 790                 795                 800

Leu Glu Glu His Phe Lys Asn Gln Glu Leu Arg Gln Val Lys Arg Gln
                805                 810                 815

Asp Glu Asp Tyr Asp Glu Gln Val Glu Glu Ser Leu Gln Asp Glu Asp
            820                 825                 830

Asp Asn Asp Val Tyr Ile Leu Thr Lys Val Ser Asp Ile Leu His Ser
        835                 840                 845

Ile Phe Ser Ser Tyr Lys Glu Lys Val Leu Pro Trp Phe Glu Gln Leu
    850                 855                 860

Leu Pro Leu Ile Val Asn Leu Ile Cys Pro His Arg Pro Trp Pro Asp
865                 870                 875                 880

Arg Gln Trp Gly Leu Cys Ile Phe Asp Asp Val Ile Glu His Cys Ser
                885                 890                 895

Pro Ala Ser Phe Lys Tyr Ala Glu Tyr Phe Leu Arg Pro Met Leu Gln
            900                 905                 910

Tyr Val Cys Asp Asn Ser Pro Glu Val Arg Gln Ala Ala Ala Tyr Gly
        915                 920                 925
```

Leu Gly Val Met Ala Gln Tyr Gly Gly Asp Asn Tyr Arg Pro Phe Cys
930 935 940

Thr Glu Ala Leu Pro Leu Leu Val Arg Val Ile Gln Ser Ala Asp Ser
945 950 955 960

Lys Thr Lys Glu Asn Val Asn Ala Thr Glu Asn Cys Ile Ser Ala Val
965 970 975

Gly Lys Ile Met Lys Phe Lys Pro Asp Cys Val Asn Val Glu Glu Val
980 985 990

Leu Pro His Trp Leu Ser Trp Leu Pro Leu His Glu Asp Lys Glu Glu
995 1000 1005

Ala Val Gln Thr Phe Asn Tyr Leu Cys Asp Leu Ile Glu Ser Asn
1010 1015 1020

His Pro Ile Val Leu Gly Pro Asn Asn Thr Asn Leu Pro Lys Ile
1025 1030 1035

Phe Ser Ile Ile Ala Glu Gly Glu Met His Glu Ala Ile Lys His
1040 1045 1050

Glu Asp Pro Cys Ala Lys Arg Leu Ala Asn Val Val Arg Gln Val
1055 1060 1065

Gln Thr Ser Gly Gly Leu Trp Thr Glu Cys Ile Ala Gln Leu Ser
1070 1075 1080

Pro Glu Gln Gln Ala Ala Ile Gln Glu Leu Leu Asn Ser Ala
1085 1090 1095

<210> 41
<211> 179
<212> PRT
<213> Homo sapiens

<400> 41

Met Pro Ser Lys Ser Leu Val Met Glu Tyr Leu Ala His Pro Ser Thr
1 5 10 15

Leu Gly Leu Ala Val Gly Val Ala Cys Gly Met Cys Leu Gly Trp Ser
20 25 30

Leu Arg Val Cys Phe Gly Met Leu Pro Lys Ser Lys Thr Ser Lys Thr
35 40 45

His Thr Asp Thr Glu Ser Glu Ala Ser Ile Leu Gly Asp Ser Gly Glu
50 55 60

Tyr Lys Met Ile Leu Val Val Arg Asn Asp Leu Lys Met Gly Lys Gly
65 70 75 80

Lys Val Ala Ala Gln Cys Ser His Ala Ala Val Ser Ala Tyr Lys Gln
85 90 95

Ile Gln Arg Arg Asn Pro Glu Met Leu Lys Gln Trp Glu Tyr Cys Gly
100 105 110

Gln Pro Lys Val Val Val Lys Ala Pro Asp Glu Glu Thr Leu Ile Ala
115 120 125

Leu Leu Ala His Ala Lys Met Leu Gly Leu Thr Val Ser Leu Ile Gln
130 135 140

Asp Ala Gly Arg Thr Gln Ile Ala Pro Gly Ser Gln Thr Val Leu Gly
145 150 155 160

Ile Gly Pro Gly Pro Ala Asp Leu Ile Asp Lys Val Thr Gly His Leu
165 170 175

Lys Leu Tyr

<210> 42
<211> 376
<212> PRT
<213> Homo sapiens

<400> 42

Met Lys Asp Val Pro Gly Phe Leu Gln Gln Ser Gln Asn Ser Gly Pro
1 5 10 15

Gly Gln Pro Ala Val Trp His Arg Leu Glu Glu Leu Tyr Thr Lys Lys
20 25 30

Leu Trp His Gln Leu Thr Leu Gln Val Leu Asp Phe Val Gln Asp Pro
35 40 45

Cys Phe Ala Gln Gly Asp Gly Leu Ile Lys Leu Tyr Glu Asn Phe Ile
50 55 60

Ser Glu Phe Glu His Arg Val Asn Pro Leu Ser Leu Val Glu Ile Ile
65 70 75 80

```
Leu His Val Val Arg Gln Met Thr Asp Pro Asn Val Ala Leu Thr Phe
                85                  90                  95

Leu Glu Lys Thr Arg Glu Lys Val Lys Ser Ser Asp Glu Ala Val Ile
            100                 105                 110

Leu Cys Lys Thr Ala Ile Gly Ala Leu Lys Leu Asn Ile Gly Asp Leu
        115                 120                 125

Gln Val Thr Lys Glu Thr Ile Glu Asp Val Glu Glu Met Leu Asn Asn
    130                 135                 140

Leu Pro Gly Val Thr Ser Val His Ser Arg Phe Tyr Asp Leu Ser Ser
145                 150                 155                 160

Lys Tyr Tyr Gln Thr Ile Gly Asn His Ala Ser Tyr Tyr Lys Asp Ala
                165                 170                 175

Leu Arg Phe Leu Gly Cys Val Asp Ile Lys Asp Leu Pro Val Ser Glu
            180                 185                 190

Gln Gln Glu Arg Ala Phe Thr Leu Gly Leu Ala Gly Leu Leu Gly Glu
        195                 200                 205

Gly Val Phe Asn Phe Gly Glu Leu Leu Met His Pro Val Leu Glu Ser
    210                 215                 220

Leu Arg Asn Thr Asp Arg Gln Trp Leu Ile Asp Thr Leu Tyr Ala Phe
225                 230                 235                 240

Asn Ser Gly Asn Val Glu Arg Phe Gln Thr Leu Lys Thr Ala Trp Gly
                245                 250                 255

Gln Gln Pro Asp Leu Ala Ala Asn Glu Ala Gln Leu Leu Arg Lys Ile
            260                 265                 270

Gln Leu Leu Cys Leu Met Glu Met Thr Phe Thr Arg Pro Ala Asn His
        275                 280                 285

Arg Gln Leu Thr Phe Glu Glu Ile Ala Lys Ser Ala Lys Ile Thr Val
    290                 295                 300

Asn Glu Val Glu Leu Leu Val Met Lys Ala Leu Ser Val Gly Leu Val
305                 310                 315                 320

Lys Gly Ser Ile Asp Glu Val Asp Lys Arg Val His Met Thr Trp Val
                325                 330                 335
```

```
Gln Pro Arg Val Leu Asp Leu Gln Gln Ile Lys Gly Met Lys Asp Arg
            340                 345                 350

Leu Glu Phe Trp Cys Thr Asp Val Lys Ser Met Glu Met Leu Val Glu
        355                 360                 365

His Gln Ala His Asp Ile Leu Thr
    370                 375
```

<210> 43
<211> 863
<212> PRT
<213> Homo sapiens

<400> 43

```
Met Gln Arg Arg Gly Ala Leu Phe Gly Met Pro Gly Gly Ser Gly Gly
1               5                   10                  15

Arg Lys Met Ala Ala Gly Asp Ile Gly Glu Leu Leu Val Pro His Met
            20                  25                  30

Pro Thr Ile Arg Val Pro Arg Ser Gly Asp Arg Val Tyr Lys Asn Glu
        35                  40                  45

Cys Ala Phe Ser Tyr Asp Ser Pro Asn Ser Glu Gly Gly Leu Tyr Val
    50                  55                  60

Cys Met Asn Thr Phe Leu Ala Phe Gly Arg Glu His Val Glu Arg His
65                  70                  75                  80

Phe Arg Lys Thr Gly Gln Ser Val Tyr Met His Leu Lys Arg His Val
                85                  90                  95

Arg Glu Lys Val Arg Gly Ala Ser Gly Gly Ala Leu Pro Lys Arg Arg
            100                 105                 110

Asn Ser Lys Ile Phe Leu Asp Leu Asp Thr Asp Asp Asp Leu Asn Ser
        115                 120                 125

Asp Asp Tyr Glu Tyr Glu Asp Glu Ala Lys Leu Val Ile Phe Pro Asp
    130                 135                 140

His Tyr Glu Ile Ala Leu Pro Asn Ile Glu Glu Leu Pro Ala Leu Val
145                 150                 155                 160

Thr Ile Ala Cys Asp Ala Val Leu Ser Ser Lys Ser Pro Tyr Arg Lys
                165                 170                 175
```

```
Gln Asp Pro Asp Thr Trp Glu Asn Glu Leu Pro Val Ser Lys Tyr Ala
            180                 185                 190

Asn Asn Leu Thr Gln Leu Asp Asn Gly Val Arg Ile Pro Pro Ser Gly
        195                 200                 205

Trp Lys Cys Ala Arg Cys Asp Leu Arg Glu Asn Leu Trp Leu Asn Leu
    210                 215                 220

Thr Asp Gly Ser Val Leu Cys Gly Lys Trp Phe Phe Asp Ser Ser Gly
225                 230                 235                 240

Gly Asn Gly His Ala Leu Glu His Tyr Arg Asp Met Gly Tyr Pro Leu
                245                 250                 255

Ala Val Lys Leu Gly Thr Ile Thr Pro Asp Gly Ala Asp Val Tyr Ser
            260                 265                 270

Phe Gln Glu Glu Glu Pro Val Leu Asp Pro His Leu Ala Lys His Leu
        275                 280                 285

Ala His Phe Gly Ile Asp Met Leu His Met His Gly Thr Glu Asn Gly
    290                 295                 300

Leu Gln Asp Asn Asp Ile Lys Leu Arg Val Ser Glu Trp Glu Val Ile
305                 310                 315                 320

Gln Glu Ser Gly Thr Lys Leu Lys Pro Met Tyr Gly Pro Gly Tyr Thr
                325                 330                 335

Gly Leu Lys Asn Leu Gly Asn Ser Cys Tyr Leu Ser Ser Val Met Gln
            340                 345                 350

Ala Ile Phe Ser Ile Pro Glu Phe Gln Arg Ala Tyr Val Gly Asn Leu
        355                 360                 365

Pro Arg Ile Phe Asp Tyr Ser Pro Leu Asp Pro Thr Gln Asp Phe Asn
    370                 375                 380

Thr Gln Met Thr Lys Leu Gly His Gly Leu Leu Ser Gly Gln Tyr Ser
385                 390                 395                 400

Lys Pro Pro Val Lys Ser Glu Leu Ile Glu Gln Val Met Lys Glu Glu
                405                 410                 415

His Lys Pro Gln Gln Asn Gly Ile Ser Pro Arg Met Phe Lys Ala Phe
```

420 425 430

Val Ser Lys Ser His Pro Glu Phe Ser Ser Asn Arg Gln Gln Asp Ala
435 440 445

Gln Glu Phe Phe Leu His Leu Val Asn Leu Val Glu Arg Asn Arg Ile
450 455 460

Gly Ser Glu Asn Pro Ser Asp Val Phe Arg Phe Leu Val Glu Glu Arg
465 470 475 480

Ile Gln Cys Cys Gln Thr Arg Lys Val Arg Tyr Thr Glu Arg Val Asp
485 490 495

Tyr Leu Met Gln Leu Pro Val Ala Met Glu Ala Ala Thr Asn Lys Asp
500 505 510

Glu Leu Ile Ala Tyr Glu Leu Thr Arg Arg Glu Ala Glu Ala Asn Arg
515 520 525

Arg Pro Leu Pro Glu Leu Val Arg Ala Lys Ile Pro Phe Ser Ala Cys
530 535 540

Leu Gln Ala Phe Ser Glu Pro Glu Asn Val Asp Asp Phe Trp Ser Ser
545 550 555 560

Ala Leu Gln Ala Lys Ser Ala Gly Val Lys Thr Ser Arg Phe Ala Ser
565 570 575

Phe Pro Glu Tyr Leu Val Val Gln Ile Lys Lys Phe Thr Phe Gly Leu
580 585 590

Asp Trp Val Pro Lys Lys Phe Asp Val Ser Ile Asp Met Pro Asp Leu
595 600 605

Leu Asp Ile Asn His Leu Arg Ala Arg Gly Leu Gln Pro Gly Glu Glu
610 615 620

Glu Leu Pro Asp Ile Ser Pro Pro Ile Val Ile Pro Asp Asp Ser Lys
625 630 635 640

Asp Arg Leu Met Asn Gln Leu Ile Asp Pro Ser Asp Ile Asp Glu Ser
645 650 655

Ser Val Met Gln Leu Ala Glu Met Gly Phe Pro Leu Glu Ala Cys Arg
660 665 670

```
Lys Ala Val Tyr Phe Thr Gly Asn Met Gly Ala Glu Val Ala Phe Asn
        675                 680                 685

Trp Ile Ile Val His Met Glu Glu Pro Asp Phe Ala Glu Pro Leu Thr
    690                 695                 700

Met Pro Gly Tyr Gly Gly Ala Ala Ser Ala Gly Ala Ser Val Phe Gly
705                 710                 715                 720

Ala Ser Gly Leu Asp Asn Gln Pro Pro Glu Glu Ile Val Ala Ile Ile
                725                 730                 735

Thr Ser Met Gly Phe Gln Arg Asn Gln Ala Ile Gln Ala Leu Arg Ala
            740                 745                 750

Thr Asn Asn Asn Leu Glu Arg Ala Leu Asp Trp Ile Phe Ser His Pro
        755                 760                 765

Glu Phe Glu Glu Asp Ser Asp Phe Val Ile Glu Met Glu Asn Asn Ala
    770                 775                 780

Asn Ala Asn Ile Ile Ser Glu Ala Lys Pro Glu Gly Pro Arg Val Lys
785                 790                 795                 800

Asp Gly Ser Gly Thr Tyr Glu Leu Phe Ala Phe Ile Ser His Met Gly
                805                 810                 815

Thr Ser Thr Met Ser Gly His Tyr Ile Cys His Ile Lys Lys Glu Gly
            820                 825                 830

Arg Trp Val Ile Tyr Asn Asp His Lys Val Cys Ala Ser Glu Arg Pro
        835                 840                 845

Pro Lys Asp Leu Gly Tyr Met Tyr Phe Tyr Arg Arg Ile Pro Ser
    850                 855                 860
```

<210> 44
<211> 294
<212> PRT
<213> Homo sapiens

<400> 44

```
Met Ala Thr His Gly Gln Thr Cys Ala Arg Pro Met Cys Ile Pro Pro
1               5                   10                  15

Ser Tyr Ala Asp Leu Gly Lys Ala Ala Arg Asp Ile Phe Asn Lys Gly
            20                  25                  30
```

```
Phe Gly Phe Gly Leu Val Lys Leu Asp Val Lys Thr Lys Ser Cys Ser
        35                  40                  45

Gly Val Glu Phe Ser Thr Ser Gly Ser Ser Asn Thr Asp Thr Gly Lys
    50                  55                  60

Val Thr Gly Thr Leu Glu Thr Lys Tyr Lys Trp Cys Glu Tyr Gly Leu
65                  70                  75                  80

Thr Phe Thr Glu Lys Trp Asn Thr Asp Asn Thr Leu Gly Thr Glu Ile
                85                  90                  95

Ala Ile Glu Asp Gln Ile Cys Gln Gly Leu Lys Leu Thr Phe Asp Thr
            100                 105                 110

Thr Phe Ser Pro Asn Thr Gly Lys Lys Ser Gly Lys Ile Lys Ser Ser
        115                 120                 125

Tyr Lys Arg Glu Cys Ile Asn Leu Gly Cys Asp Val Asp Phe Asp Phe
    130                 135                 140

Ala Gly Pro Ala Ile His Gly Ser Ala Val Phe Gly Tyr Glu Gly Trp
145                 150                 155                 160

Leu Ala Gly Tyr Gln Met Thr Phe Asp Ser Ala Lys Ser Lys Leu Thr
                165                 170                 175

Arg Asn Asn Phe Ala Val Gly Tyr Arg Thr Gly Asp Phe Gln Leu His
            180                 185                 190

Thr Asn Val Asn Asp Gly Thr Glu Phe Gly Gly Ser Ile Tyr Gln Lys
        195                 200                 205

Val Cys Glu Asp Leu Asp Thr Ser Val Asn Leu Ala Trp Thr Ser Gly
    210                 215                 220

Thr Asn Cys Thr Arg Phe Gly Ile Ala Ala Lys Tyr Gln Leu Asp Pro
225                 230                 235                 240

Thr Ala Ser Ile Ser Ala Lys Val Asn Asn Ser Ser Leu Ile Gly Val
                245                 250                 255

Gly Tyr Thr Gln Thr Leu Arg Pro Gly Val Lys Leu Thr Leu Ser Ala
            260                 265                 270

Leu Val Asp Gly Lys Ser Ile Asn Ala Gly Gly His Lys Val Gly Leu
        275                 280                 285
```

Ala Leu Glu Leu Glu Ala
290

<210> 45
<211> 283
<212> PRT
<213> Homo sapiens

<400> 45

Met Cys Asn Thr Pro Thr Tyr Cys Asp Leu Gly Lys Ala Ala Lys Asp
1 5 10 15

Val Phe Asn Lys Gly Tyr Gly Phe Gly Met Val Lys Ile Asp Leu Lys
20 25 30

Thr Lys Ser Cys Ser Gly Val Glu Phe Ser Thr Ser Gly His Ala Tyr
35 40 45

Thr Asp Thr Gly Lys Ala Ser Gly Asn Leu Glu Thr Lys Tyr Lys Val
50 55 60

Cys Asn Tyr Gly Leu Thr Phe Thr Gln Lys Trp Asn Thr Asp Asn Thr
65 70 75 80

Leu Gly Thr Glu Ile Ser Trp Glu Asn Lys Leu Ala Glu Gly Leu Lys
85 90 95

Leu Thr Leu Asp Thr Ile Phe Val Pro Asn Thr Gly Lys Lys Ser Gly
100 105 110

Lys Leu Lys Ala Ser Tyr Lys Arg Asp Cys Phe Ser Val Gly Ser Asn
115 120 125

Val Asp Ile Asp Phe Ser Gly Pro Thr Ile Tyr Gly Trp Ala Val Leu
130 135 140

Ala Phe Glu Gly Trp Leu Ala Gly Tyr Gln Met Ser Phe Asp Thr Ala
145 150 155 160

Lys Ser Lys Leu Ser Gln Asn Asn Phe Ala Leu Gly Tyr Lys Ala Ala
165 170 175

Asp Phe Gln Leu His Thr His Val Asn Asp Gly Thr Glu Phe Gly Gly
180 185 190

Ser Ile Tyr Gln Lys Val Asn Glu Lys Ile Glu Thr Ser Ile Asn Leu
195 200 205

```
Ala Trp Thr Ala Gly Ser Asn Asn Thr Arg Phe Gly Ile Ala Ala Lys
    210                 215                 220

Tyr Met Leu Asp Cys Arg Thr Ser Leu Ser Ala Lys Val Asn Asn Ala
225                 230                 235                 240

Ser Leu Ile Gly Leu Gly Tyr Thr Gln Thr Leu Arg Pro Gly Val Lys
                245                 250                 255

Leu Thr Leu Ser Ala Leu Ile Asp Gly Lys Asn Phe Ser Ala Gly Gly
            260                 265                 270

His Lys Val Gly Leu Gly Phe Glu Leu Glu Ala
        275                 280
```

<210> 46
<211> 19
<212> PRT
<213> Artificial sequence

<220>
<223> Synthetic

<400> 46

```
Arg Leu Thr Ser Asn Met Val Cys Lys His Cys Glu His Gln Ser Pro
1               5                   10                  15

Val Arg Phe
```

<210> 47
<211> 15
<212> PRT
<213> Artificial sequence

<220>
<223> Synthetic

<400> 47

```
Arg Asp Val Val Cys Asp Asn Cys Thr Lys Ile Glu Ala Lys Gly
1               5                   10                  15
```

<210> 48
<211> 14
<212> PRT
<213> Artificial sequence

<220>
<223> Synthetic

<220>
<221> misc_feature
<222> (1)..(1)

<220>
<221> misc_feature
<222> (2)..(2)
<223> Xaa is selected from Tyr, Asp, Glu, Ile, Leu, Asn, and Ser

<220>
<221> misc_feature
<222> (4)..(4)
<223> Xaa is selected from Phe, Ile, and Tyr

<220>
<221> misc_feature
<222> (5)..(5)
<223> Xaa is selected from Phe, Ile, and Tyr

<220>
<221> misc_feature
<222> (6)..(6)
<223> Xaa is selected from Asp and Glu

<220>
<221> misc_feature
<222> (7)..(7)
<223> Xaa is selected from Leu, Met, Val, and Pro

<220>
<221> misc_feature
<222> (8)..(8)
<223> Xaa is selected from Ser, Asn, Asp, Ala, and Thr

<220>
<221> misc_feature
<222> (9)..(9)
<223> Xaa is selected from Tyr, Asp, Phe, Asn, and Trp

<220>
<221> misc_feature
<222> (10)..(10)
<223> Xaa is selected from Gly, Asp, and Glu

<220>
<221> misc_feature
<222> (11)..(11)
<223> Xaa is selected from Tyr and Phe

<220>
<221> misc_feature
<222> (12)..(12)
<223> Xaa is selected from Leu, Val, Met, Gln, and Trp

<220>
<221> misc_feature
<222> (14)..(14)
<223> Xaa is selected from Phe, Leu, Cys, Val, and Tyr

<400> 48

```
Xaa Xaa Cys Xaa Xaa Xaa Xaa Xaa Xaa Xaa Xaa Xaa Cys Xaa
1               5                   10
```

<210> 49
<211> 16
<212> PRT
<213> Artificial sequence

<220>
<223> Synthetic

<220>
<221> MISC_FEATURE
<222> (1)..(1)
<223> Xaa is selected from any amino acid

<220>
<221> MISC_FEATURE
<222> (2)..(2)
<223> Xaa is selected from Leu, Met, Ala, Ser, and Val

<220>
<221> MISC_FEATURE
<222> (3)..(3)
<223> Xaa is selected from Try, Asp, Glu, Ile, Leu, Asn, and Ser

<220>
<221> MISC_FEATURE
<222> (5)..(5)
<223> Xaa is selected from Phe, Ile, and Tyr

<220>
<221> MISC_FEATURE
<222> (6)..(6)
<223> Xaa is selected from Phe, Ile, and Tyr

<220>
<221> MISC_FEATURE
<222> (7)..(7)
<223> Xaa is selected from Asp and Glu

<220>
<221> MISC_FEATURE
<222> (8)..(8)
<223> Xaa is selected from Leu, Met, Val, and Pro

<220>
<221> MISC_FEATURE
<222> (9)..(9)
<223> Xaa is selected from Ser, Asn, Asp, Ala, and Thr

<220>
<221> MISC_FEATURE
<222> (10)..(10)
<223> Xaa is selected from Tyr, Asp, Phe, Asn, and Trp

<220>
<221> MISC_FEATURE

<222> (11)..(11)
<223> Xaa is selected from Gly, Asp, and Glu

<220>
<221> MISC_FEATURE
<222> (12)..(12)
<223> Xaa is selected from Tyr and Phe

<220>
<221> MISC_FEATURE
<222> (13)..(13)
<223> Xaa is selected from Leu, Val, Met, Gln, and Trp

<220>
<221> MISC_FEATURE
<222> (15)..(15)
<223> Xaa is selected from Phe, Leu, Cys, Val, and Tyr

<220>
<221> MISC_FEATURE
<222> (16)..(16)
<223> Xaa is selected from any amino acid

<400> 49

```
Xaa Xaa Xaa Cys Xaa Xaa Xaa Xaa Xaa Xaa Xaa Xaa Xaa Cys Xaa Xaa
1               5                   10                  15
```